# EUROPEAN PATENT SPECIFICATION

(11) **EP 2 544 716 B1**
(45) Date of publication and mention of the grant of the patent: **01.01.2020**
(21) Application number: 11709301.3
(22) Date of filing: 11.03.2011
(51) Int. Cl.: A61K 39/135, C12N 15/82, A61K 39/15, A61K 39/00, C07K 14/005, A61K 39/12

(54) **FOOT AND MOUTH DISEASE VIRUS RECOMBINANT VACCINES AND USES THEREOF**
REKOMBINANTE IMPFSTOFFE GEGEN DEN MAUL-UND-KLAUENSEUCHEVIRUS UND VERWENDUNGEN DAVON
VACCINS DE RECOMBINAISON CONTRE LE VIRUS DE LA FIÈVRE APHTEUSE ET UTILISATIONS DE CEUX-CI

(30) Priority: 21.07.2010 US 366363 P; 12.03.2010 US 313164 P
(43) Date of publication of application: 16.01.2013
(73) Proprietor: Boehringer Ingelheim Animal Health USA Inc., Duluth, GA 30096 (US)
(72) Inventor: AUDONNET, Jean-Christophe, F-69006 Lyon (FR); GUO, Xuan, Suwanee, GA 30024 (US); FEILMEIER, Bradley, J., Watkinsville, GA 30677 (US); TROUPE, Karolyn, Marie, Athens, GA 30605 (US); BUBLOT, Michel, F-69630 Chaponost (FR); COX, Kevin, Raleigh, NC 27609 (US)
(74) Representative: D Young & Co LLP
(86) International application number: PCT/US2011/028115
(87) International publication number: WO 2011/112945

(56) References cited:
- WO-A1-02/00251
- US-A1- 2005 287 672
- PAN ET AL: "Foliar extracts from transgenic tomato plants expressing the structural polyprotein, P1-2A, and protease, 3C, from foot-and-mouth disease virus elicit a protective response in guinea pigs", VETERINARY IMMUNOLOGY AND IMMUNOPATHOLOGY, ELSEVIER BV, AMSTERDAM, NL, vol. 121, no. 1-2, 12 December 2007 (2007-12-12), pages 83-90, XP022386813, ISSN: 0165-2427, DOI: DOI:10.1016/J.VETIMM.2007.08.010
- Guo, Xuan et al: "Lemna (duckweed) expressed hemagglutinin from avian influenza H5N1 protects chickens against H5N1 high pathogenicity avian influenza virus challenge", Abstracts of the 7th International Symposium on Avian Influenza, April 5-8, 2009, Athens, Georgia, 5 April 2009 (2009-04-05), XP002636123, Retrieved from the Internet: URL:http://www.ars.usda.gov/research/publi cations/Publications.htm?seq_no_115=234836 [retrieved on 2011-05-10]

## Description

### FIELD OF THE INVENTION

The present invention relates to compositions for combating Foot and Mouth Disease Virus (FMDV) infection in animals. The present invention provides pharmaceutical compositions comprising an FMDV antigen, methods of vaccination against FMDV, and kits for use with such methods and compositions.

### BACKGROUND OF THE INVENTION

Foot-and-mouth disease (FMD) is one of the most virulent and contagious diseases affecting farm animals. This disease is endemic in numerous countries in the world, especially in Africa, Asia and South America. In addition, epidemic outbreaks can occur periodically. The presence of this disease in a country may have very severe economic consequences resulting from loss of productivity, loss of weight and milk production in infected herds, and from trade embargoes imposed on these countries. The measures taken against this disease consist of strict application of import restrictions, hygiene controls and quarantine, slaughtering sick animals and vaccination programs using inactivated vaccines, either as a preventive measure at the national or regional level, or periodically when an epidemic outbreak occurs.

FMD is characterized by its short incubation period, its highly contagious nature, the formation of ulcers in the mouth and on the feet and sometimes, the death of young animals. FMD affects a number of animal species, in particular cattle, pigs, sheep and goats. The agent responsible for this disease is a ribonucleic acid (RNA) virus belonging to the Aphthovirus genus of the *Picornaviridae* family (Cooper et al., Intervirology, 1978, 10, 165-180). At present, at least seven types of foot-and-mouth disease virus (FMDV) are known: the European types (A, O and C), the African types (SAT1, SAT2 and SAT3) and an Asiatic type (Asia 1). Numerous sub-types have also been distinguished (Kleid et al. Science (1981), 214, 1125-1129).

FMDV is a naked icosahedral virus of about 25 nm in diameter, containing a single-stranded RNA molecule consisting of about 8500 nucleotides, with a positive polarity. This RNA molecule comprises a single open reading frame (ORF), encoding a single polyprotein containing, *inter alia,* the capsid precursor also known as protein P1 or P88. The protein P1 is myristylated at its amino-terminal end. During the maturation process, the protein PI is cleaved by the protease 3C into three proteins known as VP0, VP1 and VP3 (or 1AB, 1D and 1C respectively; Belsham G. J., Progress in Biophysics and Molecular Biology, 1993, 60, 241-261). In the virion, the protein VP0 is then cleaved into two proteins, VP4 and VP2 (or 1A and 1B respectively). The mechanism for the conversion of the proteins VP0 into VP4 and VP2, and for the formation of mature virions is not known. The proteins VP1, VP2 and VP3 have a molecular weight of about 26,000 Da, while the protein VP4 is smaller at about 8,000 Da.

The simple combination of the capsid proteins forms the protomer or 5S molecule, which is the elementary constituent of the FMDV capsid. This protomer is then complexed into a pentamer to form the 12S molecule. The virion results from the encapsidation of a genomic RNA molecule by assembly of twelve 12S pentamers, thus constituting the 146S particles. The viral capsid may also be formed without the presence of an RNA molecule inside it (hereinafter "empty capsid"). The empty capsid is also designated as particle 70S. The formation of empty capsids may occur naturally during viral replication or may be produced artificially by chemical treatment.

Many hypotheses, research routes, and proposals have been developed in an attempt to design effective vaccines against FMD. Currently, the only vaccines on the market comprise inactivated virus. Concerns about safety of the FMDV vaccine exist, as outbreaks of FMD in Europe have been associated with shortcomings in vaccine manufacture (King, A.M.Q. et al, (1981) Nature 293: 479-480). The inactivated vaccines do not confer long-term immunity, thus requiring booster injections given every year, or more often in the event of epidemic outbreaks. In addition, there are risks linked to incomplete inactivation and/or to the escape of virus during the production of inactivated vaccines (King, A.M.Q., *ibid*). A goal in the art has been to construct conformationally correct immunogens lacking the infective FMDV genome to make effective and safe vaccines.

Vaccinia virus has been used successfully to immunize against smallpox, culminating in the worldwide eradication of smallpox in 1980. Thus, a new role for poxviruses became important, that of a genetically engineered vector for the expression of foreign genes (Panicali and Paoletti, 1982; Paoletti *et al.,* 1984). Genes encoding heterologous antigens have been expressed in vaccinia, often resulting in protective immunity against challenge by the corresponding pathogen (reviewed in Tartaglia *et al.,* 1990). A highly attenuated strain of vaccines, designated MVA, has also been used as a vector for poxvirus-based vaccines. Use of MVA is described in U.S. Patent No. 5,185,146.

Additional vaccine vector systems involve the use of avipox viruses, which are naturally host-restricted poxviruses. Both fowlpoxvirus (FPV; Taylor *et al.* 1988a, b) and canarypoxvirus (CPV; Taylor *et al.,* 1991 & 1992) have been engineered to express foreign gene products. Fowlpox virus (FPV) is the prototypic virus of the Avipox genus of the Poxvirus family. The virus causes an economically important disease of poultry that has been well controlled since the 1920's by the use of live attenuated vaccines. Replication of the avipox viruses is limited to avian species (Matthews, 1982) and there are no reports in the literature of avipox virus causing a productive infection in any non-avian species including man. This host restriction provides an inherent safety barrier against transmission of the virus to other species and makes the use of avipox virus based vaccine vectors in veterinary and human applications an attractive proposition.

Other attenuated poxvirus vectors have been prepared by genetic modifications of wild type strains of virus. The NYVAC vector, derived by deletion of specific virulence and host-range genes from the Copenhagen strain of vaccinia (Tartaglia *et al.,* 1992) has proven useful as a recombinant vector in eliciting a protective immune response against an expressed foreign antigen. Another engineered poxvirus vector is ALVAC, derived from canarypox virus (see U.S. Patent No. 5,756,103). ALVAC does not productively replicate in non-avian hosts, a characteristic thought to improve its safety profile (Taylor *et al.,* 1991 & 1992). ALVAC was deposited under the terms of the Budapest Treaty with the American Type Culture Collection under accession number VR-2547. Yet another engineered poxvirus vector is TROVAC, derived from fowlpox virus (see U.S. Patent No. 5,766,599).

Recombinant poxviruses can be constructed in two steps known in the art and analogous to the methods for creating synthetic recombinants of poxviruses such as the vaccinia virus and avipox virus described in U.S. Patent Nos. 4,769,330; 4,722,848; 4,603,112; 5,110,587; 5,174,993; 5,494,807; and 5,505,941, the disclosures of which are incorporated herein by reference. It can thus be appreciated that provision of an FMDV recombinant poxvirus, and of compositions and products therefrom, particularly ALVAC or TROVAC-based FMDV recombinants and compositions and products therefrom, especially such recombinants containing the PI genes and/or 3C protease gene of FMDV, and compositions and products therefrom, would be a highly desirable advance over the current state of technology.

Recently, plants have been investigated as a source for the production of therapeutic agents such as vaccines, antibodies, and biopharmaceuticals. However, the production of vaccines, antibodies, proteins, and biopharmaceuticals from plants is far from a remedial process, and there are numerous obstacles that are commonly associated with such vaccine production. Limitations to successfully producing plant vaccines include low yield of the bioproduct or expressed antigen (Chargelegue et al., Trends in Plant Science 2001, 6, 495-496), protein instability, inconsistencies in product quality (Schillberg et al., Vaccine 2005, 23, 1764-1769), and insufficient capacity to produce viral-like products of expected size and immunogenicity (Arntzen et al., Vaccine 2005, 23, 1753-1756). In order to address these problems, codon optimization, careful approaches to harvesting and purifying plant products, use of plant parts such as chloroplasts to increase uptake of the material, and improved subcellular targeting are all being considered as potential strategies (Koprowski, Vaccine 2005, 23, 1757-1763).

Considering the susceptibility of animals (including humans, albeit rarely), to FMDV, a method of preventing FMDV infection and protecting animals is essential. Accordingly, there is a need for an effective vaccine against FMDV.

Foliar extracts from transgenic tomato plants expressing the structural polyprotein, P1-2A, and protease, 3C, from foot-and-mouth disease virus have been shown to elicit a protective response in guinea pigs (Pan et al., Veterinary Immunology and Immunopathology 2007, 121, 83-90).

### SUMMARY OF THE INVENTION

The invention is as described in the accompanying claims.

In a first embodiment, the present invention provides an immunological or vaccine composition comprising a duckweed-expressed foot-and-mouth disease virus (FMDV) antigen and a pharmaceutical or veterinarily acceptable carrier, excipient or vehicle, wherein said antigen comprises a virus-like particle (VLP), further wherein said antigen is encoded by a polynucleotide having at least 80% identity to the sequence as set forth in SEQ ID NO: 2.

In a second embodiment, the present invention provides composition according to the invention for use in a method of vaccinating a host susceptible to ovine, bovine, caprine, or porcine FMDV.

In a third embodiment, the present invention provides a duckweed plant transformed with a plasmid comprising a DNA fragment having at least 80% identity to the sequence as set forth in SEQ ID NO:2, wherein the plasmid is for plant transformation.

In a fourth embodiment, the present invention provides a stably transformed duckweed plant transformed with: (a) a plasmid comprising a DNA fragment having at least 80% identity to the sequence as set forth in SEQ ID NO:2, wherein the plasmid is for plant transformation; or (b) a gene expressing a FMDV antigen, wherein the antigen has at least 80% identity to the sequence as set forth in SEQ ID NO: 3.

Compositions comprising an antigenic FMDV polypeptide and fragments and variants thereof are described herein. The FMDV antigens and fragments and variants thereof possess immunogenic and protective properties. The FMDV antigens may be produced in a plant or algae.

The antigenic polypeptides and fragments and variants thereof can be formulated into vaccines and/or pharmaceutical compositions. Such vaccines can be used to vaccinate an animal and provide protection against at least one FMDV strain.

Methods described herein include methods for making the antigenic polypeptides in duckweed plant. Methods also include methods of use including administering to an animal an effective amount of an antigenic polypeptide or fragment or variant thereof to produce a protective immunogenic response. After production in duckweed the antigenic polypeptide can be partially or substantially purified for use as a vaccine.

Kits comprising at least one antigenic polypeptide or fragment or variant thereof and instructions for use are also described herein.

### BRIEF DESCRIPTION OF THE DRAWINGS

The following detailed description, given by way of example, but not intended to limit the invention solely to the specific embodiments described, may best be understood in conjunction with the accompanying drawings, in which:

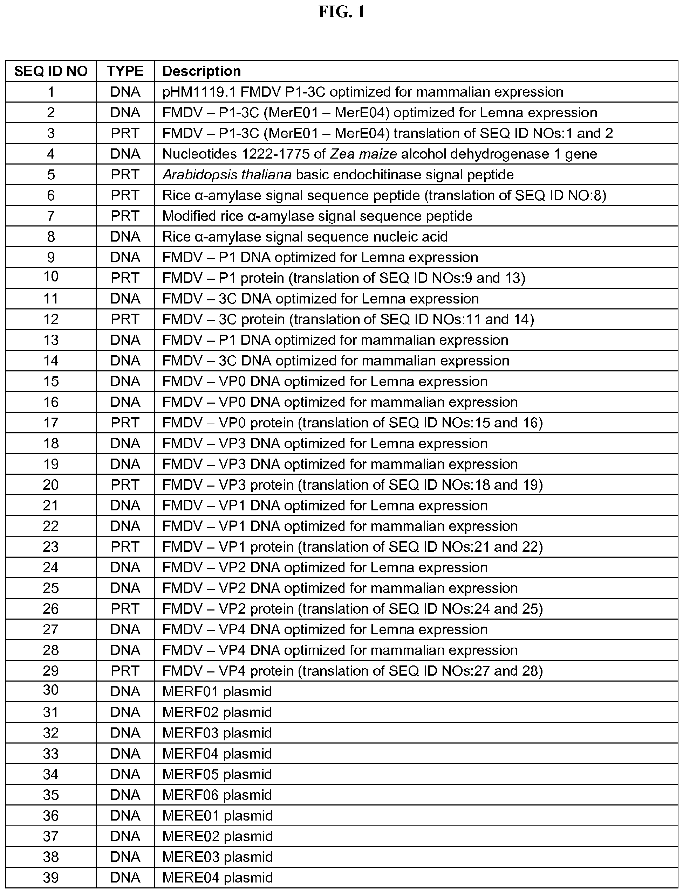

FIG. 1 depicts a table summarizing the DNA and Protein sequences, presented as a listing in the appendix of the instant application;

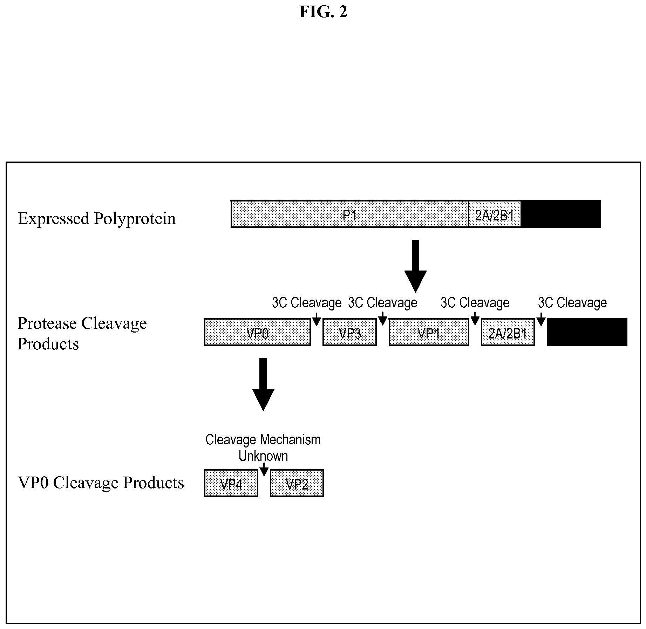

FIG. 2 represents the native and optimized FMDV sequence that was expressed in duckweed. The expressed polypeptide is cleaved into its individual proteins by the 3C protease that folds after translation, self-cleaves to release itself from the polypeptide, and finally perform internal cleavages within the PI polypeptide.
FIG. 3 depicts the identity and placement of the duckweed-optimized FMDV antigens for the 4 "MerE" duckweed expression constructs;

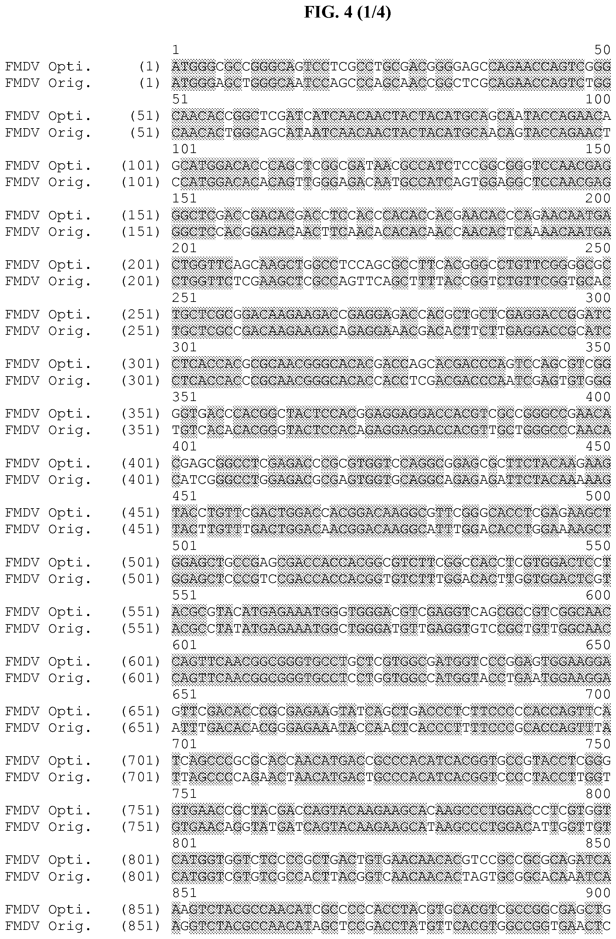

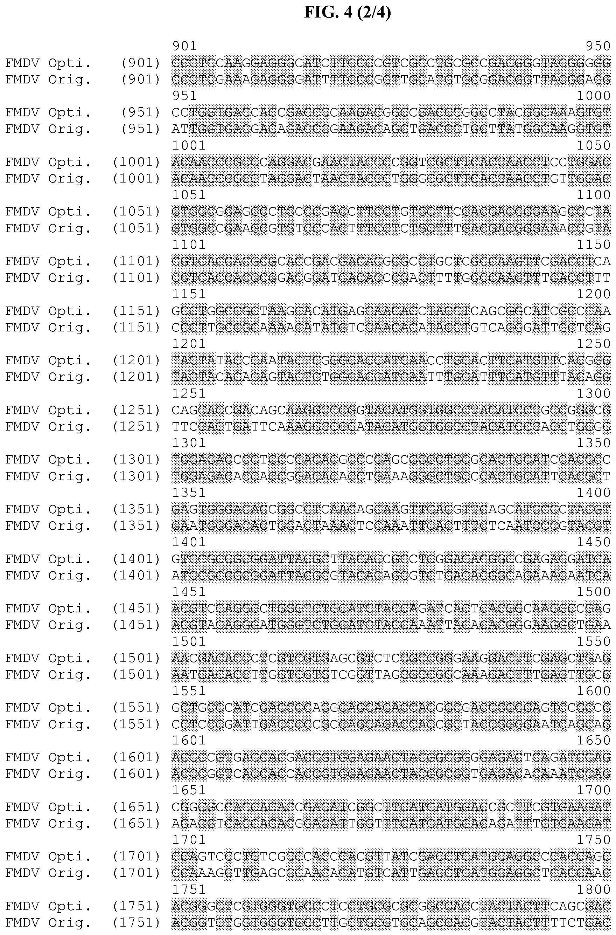

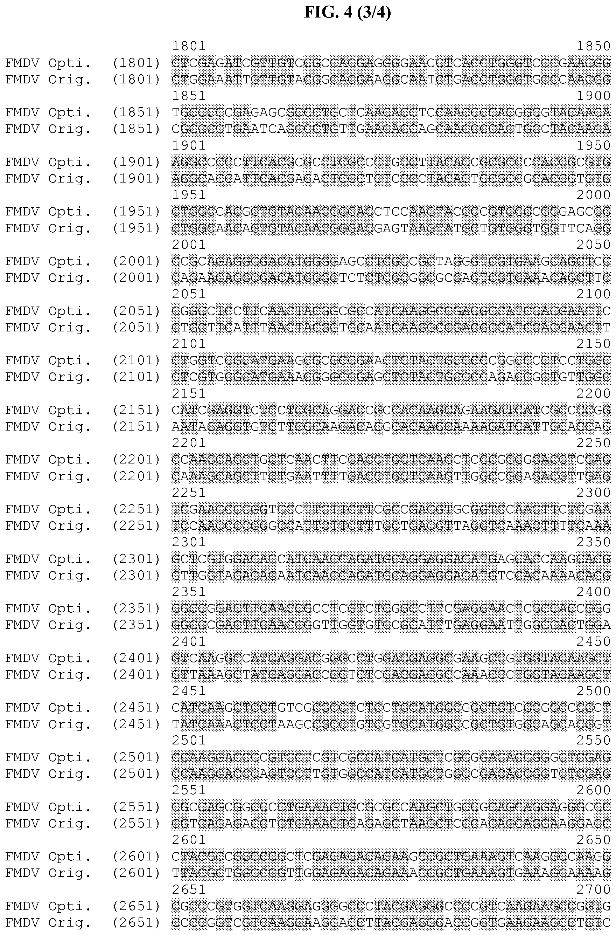

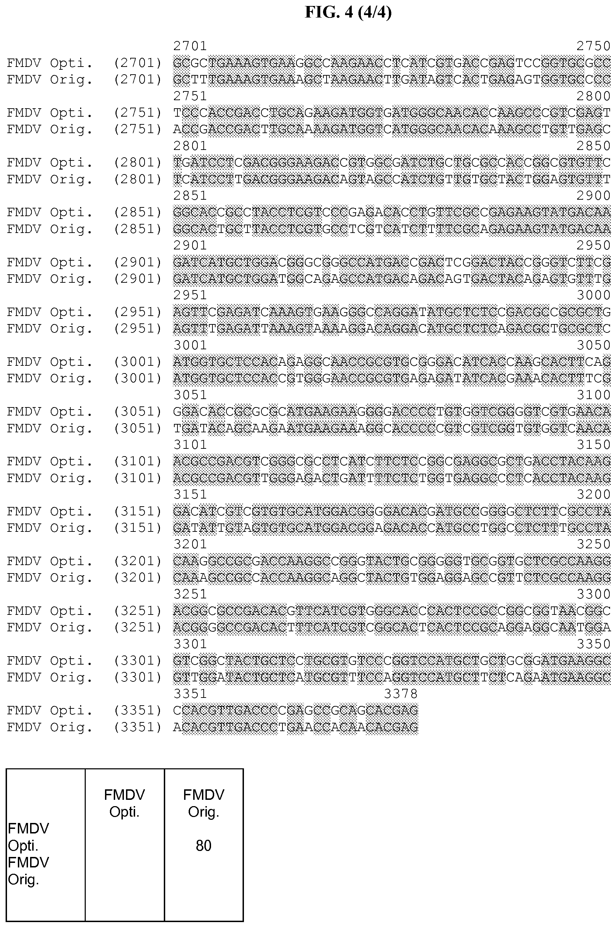

FIG. 4 is a sequence alignment of FMDV optimized for expression in duckweed (SEQ ID NO:2) and FMDV optimized for expression in mammalian cells (SEQ ID NO:1). The sequence identity is indicated;

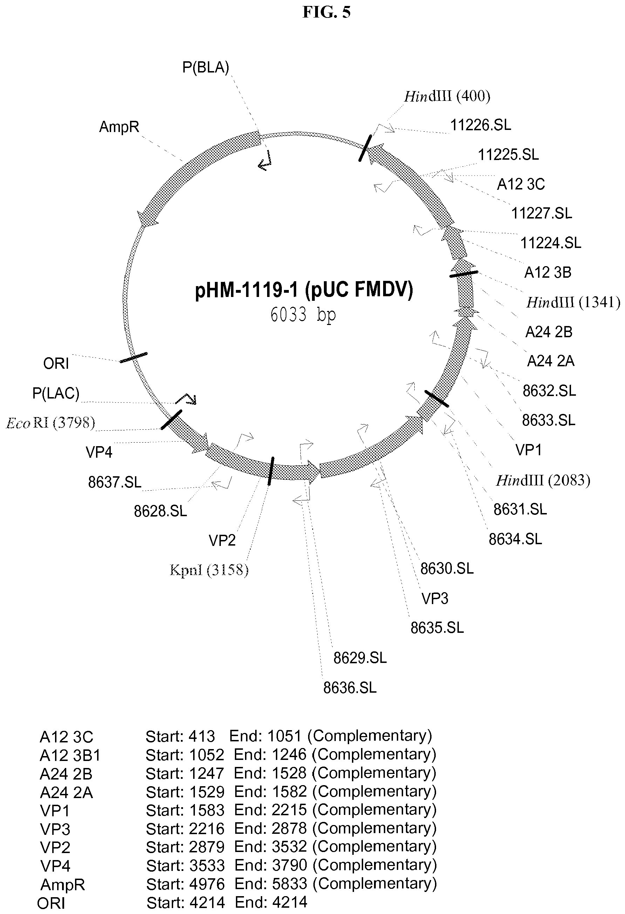

FIG. 5 depicts the pHM-1119-1 plasmid, which contains nucleic acid sequence encoding the mammalian optimized FMDV P1-3C (SEQ ID NO:1);
FIG. 6 depicts the pMerE01 plasmid, Strong Promoter + P1+2A/2B1 (A24) + 3C (A12);
FIG. 7 depicts the pMerE02, Strong Promoter + P1;
FIG. 8 depicts the pMerE03 plasmid, Strong Promoter + P1+2A/2B1 (A24) + Weak Promoter 3C (A12);
FIG.9 depicts the pMerE04 plasmid, Strong Promoter + P1+2A/2B1 (A24) + Weak Promoter 3C (A12) with optimized 5'UTR;
FIG. 10 depicts a representative RNA dot blot used to screen recombinant duckweed cell lines for expression of genes encoding FMDV antigens;
FIG. 11 depicts a representative quantitative PCR results for duckweed cell lines expressing FMDV constructs;
FIG. 12 depicts the Western blot for duckweed cell lines harboring and expressing MerE01 and MerE02;

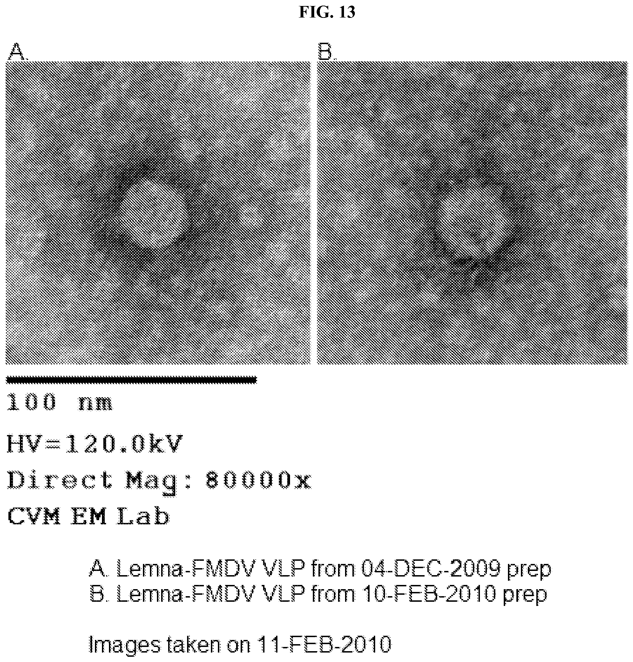

FIG. 13 depicts electron micrographs of inventive FMDV virus like particles (VLP);

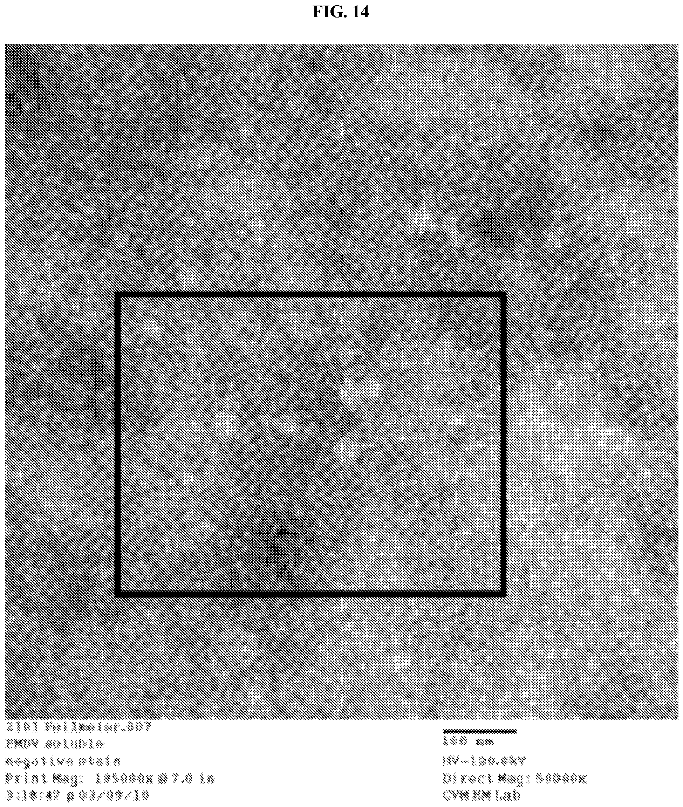

FIG. 14 depicts electron micrograph of clusters of FMDV VLP;

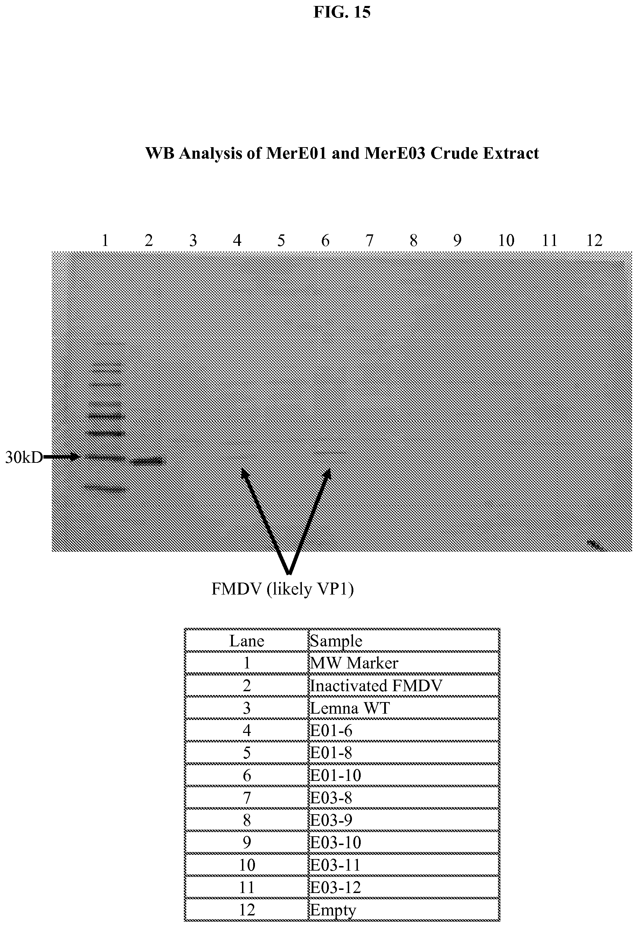

FIG. 15 depicts WB Analysis of MerE01 and MerE03 Crude Extract (5ug TSP/lane) using Guinea Pig Serum. For MerE01, VP1 (VP3) band(s) observed, suggesting expression of both P1 and 3C and further processing of P1. For MerE03, neither PI nor VP1 (VP3) were observed, suggesting expression of 3C and degradation of P1;
FIG. 16 presents an FMD viral particle schematic diagram;
FIG. 17 presents OD of decreasing concentrations of various extracts from duckweed expressing FMDV antigens as measured by ELISA;

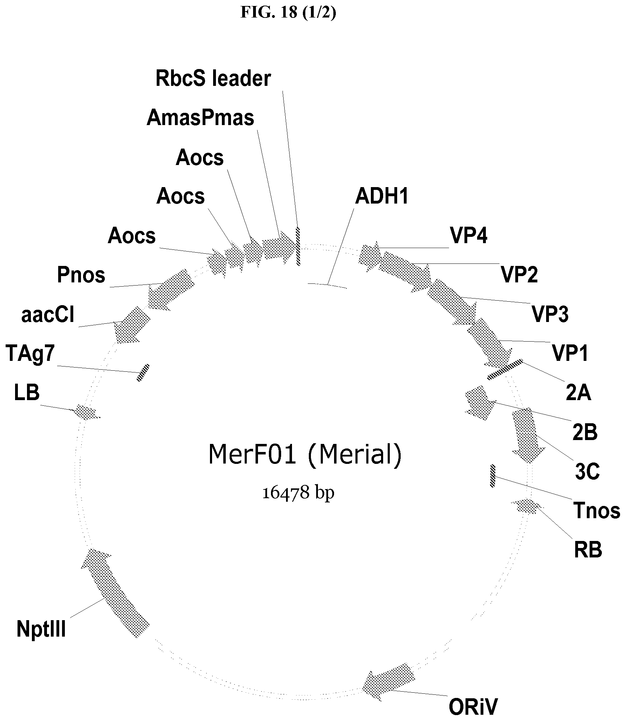

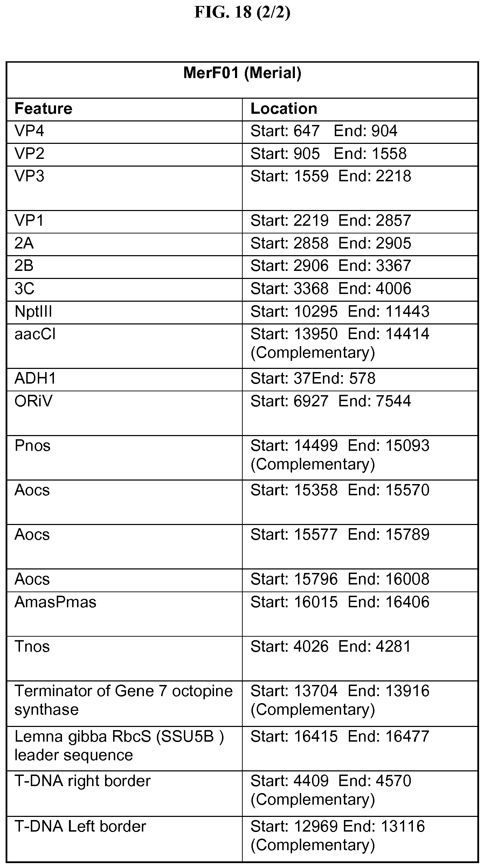

FIG. 18 presents MerF01 plasmid map with feature summary table;

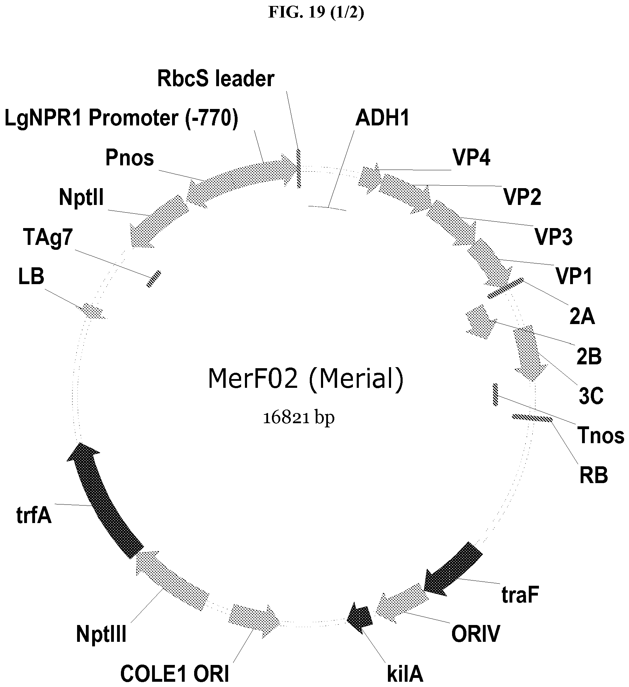

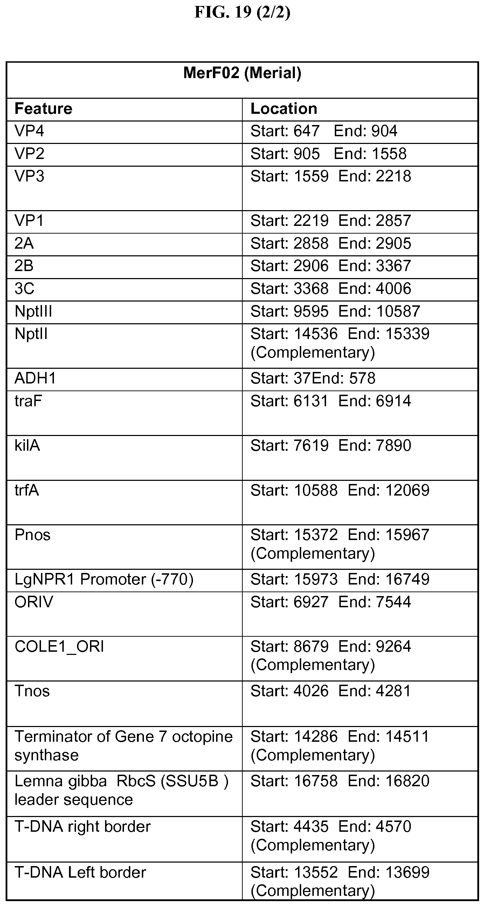

FIG. 19 presents MerF02 plasmid map with feature summary table;

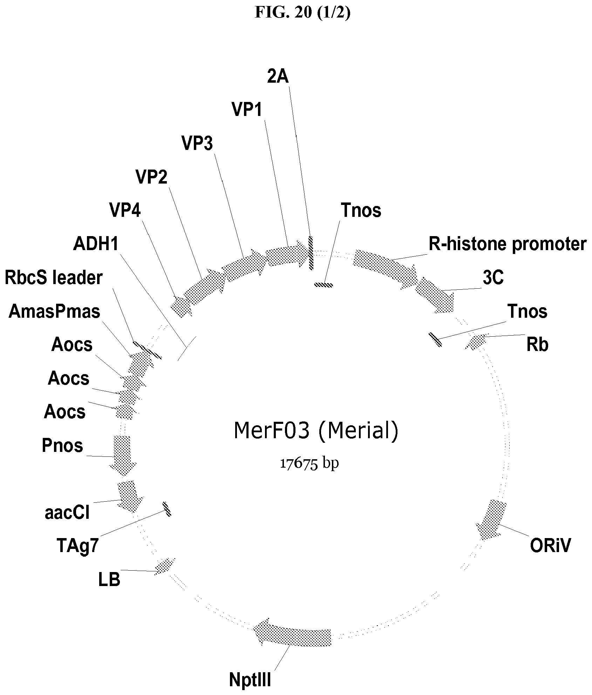

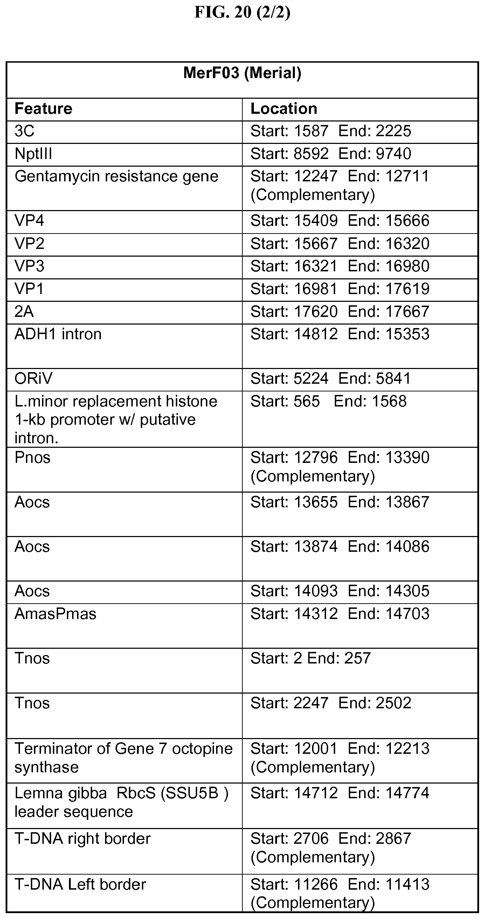

FIG. 20 presents MerF03 plasmid map with feature summary table;

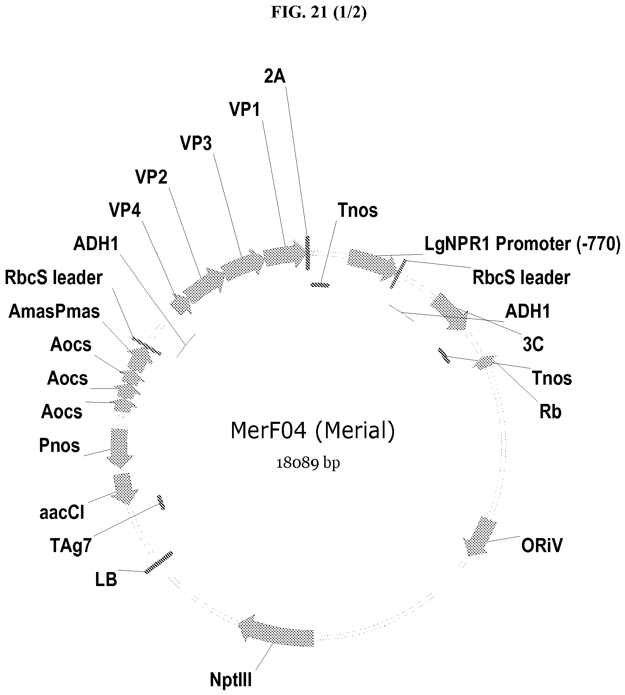

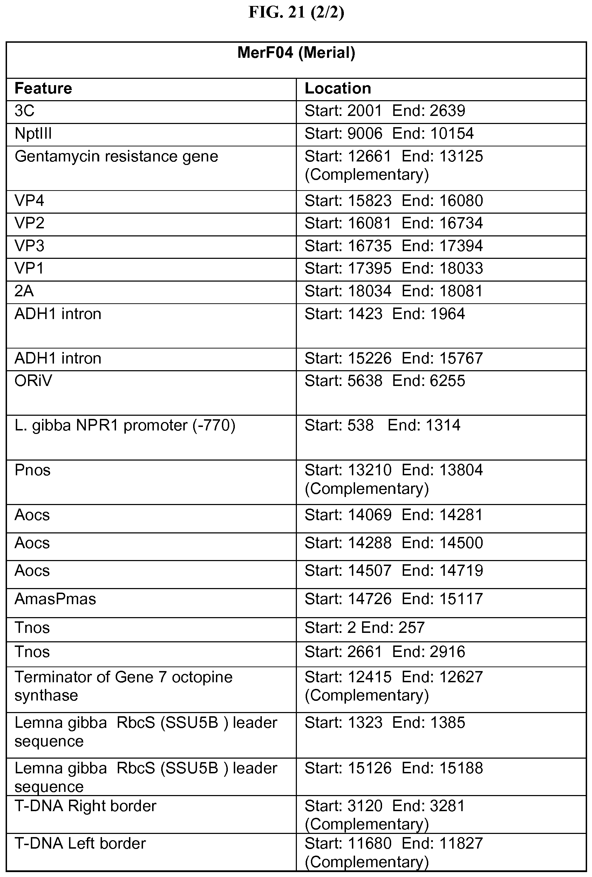

FIG. 21 presents MerF04 plasmid map with feature summary table;

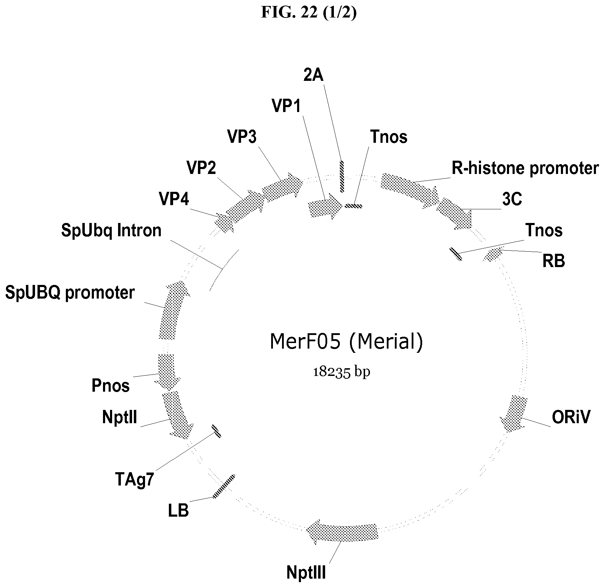

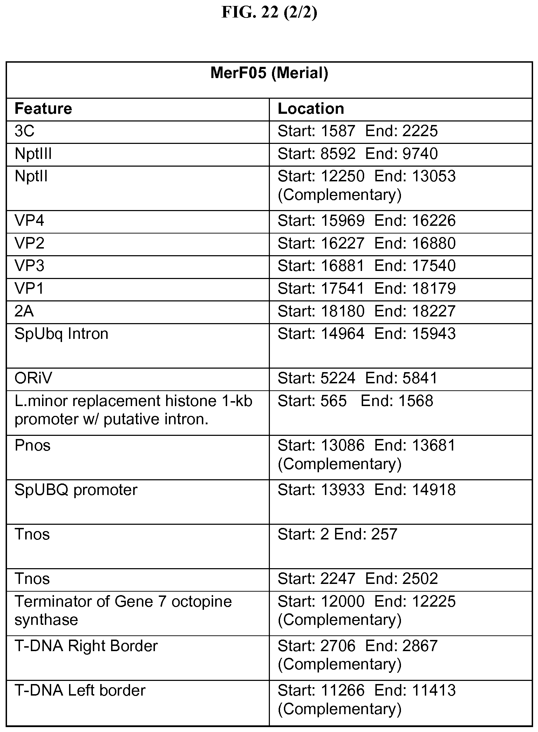

FIG. 22 presents MerF05 plasmid map with feature summary table;

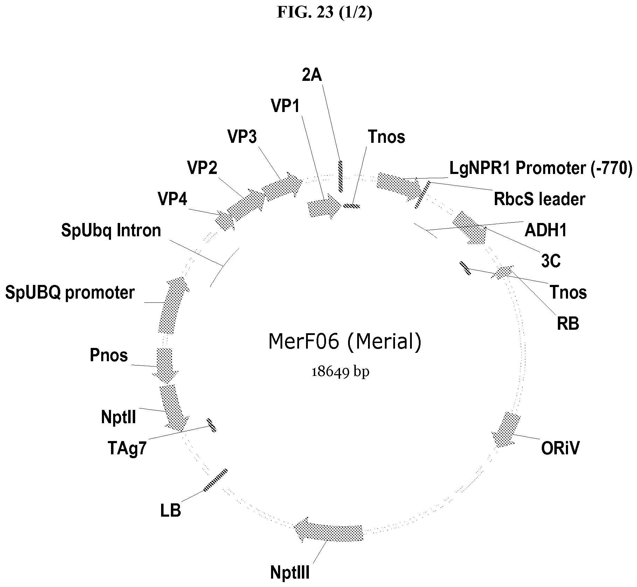

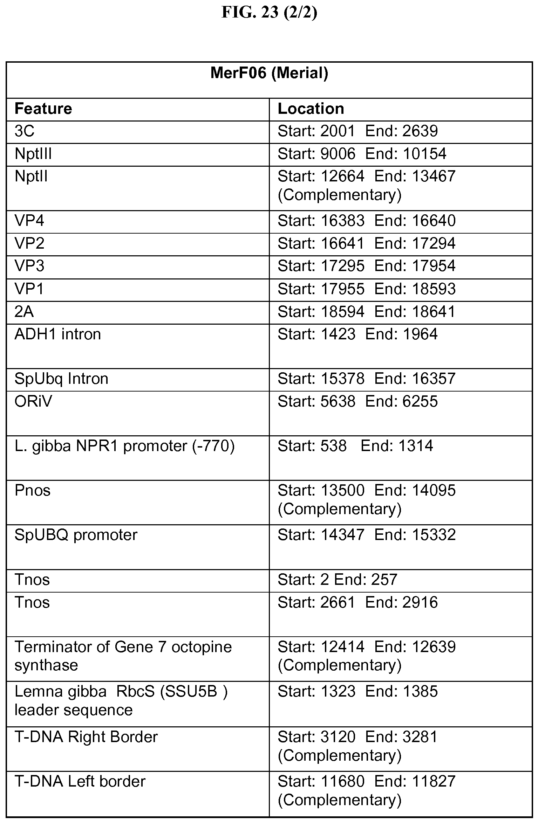

FIG. 23 presents MerF06 plasmid map with feature summary table.

### DETAILED DESCRIPTION

Compositions comprising an FMDV polypeptide, antigen and fragments and variants thereof that elicit an immunogenic response in an animal are described herein. The antigenic polypeptides or fragments or variants thereof are produced in a plant or algae. The antigenic polypeptides or fragments or variants may be formulated into vaccines or pharmaceutical compositions and used to elicit or stimulate a protective response in an animal. In one embodiment the polypeptide antigen is an FMDV PI or 3C polypeptide or active fragment or variant thereof.

It is recognized that the antigenic polypeptides described herein may be full length polypeptides or active fragments or variants thereof. By "active fragments" or "active variants" is intended that the fragments or variants retain the antigenic nature of the polypeptide. Thus described herein is any FMDV polypeptide, antigen, epitope or immunogen that elicits an immunogenic response in an animal. The FMDV polypeptide, antigen, epitope or immunogen may be any FMDV polypeptide, antigen, epitope or immunogen, such as, but not limited to, a protein, peptide or fragment or variant thereof, that elicits, induces or stimulates a response in an animal, such as an ovine, bovine, caprine or porcine.

Particular FMDV antigenic polypeptides include PI and 3C. FMDV is a non-enveloped icosahedral virus of about 25 nm in diameter, containing a single-stranded RNA molecule consisting of about 8500 nucleotides, with a positive polarity. This RNA molecule comprises a single open reading frame (ORF), encoding a single polyprotein containing, *inter alia,* the capsid precursor also known as protein PI or P88. The protein PI is myristylated at its amino-terminal end. During the maturation process, the protein PI is cleaved by the protease 3C into three proteins known as VP0, VP1 and VP3 (or 1AB, 1D and 1C respectively; Belsham G. J., Progress in Biophysics and Molecular Biology, 1993, 60, 241-261). In the virion, the protein VP0 is then cleaved into two proteins, VP4 and VP2 (or 1A and 1B respectively). The mechanism for the conversion of the proteins VP0 into VP4 and VP2, and for the formation of mature virions is not known. The proteins VP1, VP2 and VP3 have a molecular weight of about 26,000 Da, while the protein VP4 is smaller at about 8,000 Da. FMDV sequences are also described in US Patent numbers US 7,527,960 and US 7,531,182.

The, simple combination of the capsid proteins forms the protomer or 5S molecule, which is the elementary constituent of the FMDV capsid. This protomer is then complexed into a pentamer to form the 12S molecule. The virion results from the encapsidation of a genomic RNA molecule by assembly of twelve 12S pentamers, thus constituting the 146S particles. The viral capsid may also be formed without the presence of an RNA molecule inside it (hereinafter "empty capsid"). The empty capsid is also designated as particle 70S. The formation of empty capsids may occur naturally during viral replication or may be produced artificially by chemical treatment.

The present invention relates to bovine, ovine, caprine, or porcine vaccines or compositions which may comprise an effective amount of a recombinant FMDV antigen and a pharmaceutically or veterinarily acceptable carrier, excipient, or vehicle.

In some embodiments, the vaccines further comprise adjuvants, such as the oil-in-water (O/W) emulsions described in US Patent 7371395.

In still other embodiments, the adjuvants include EMULSIGEN, Aluminum Hydroxide, Saponin, and CpG, or combinations thereof.

In some embodiments, the response in the animal is a protective immune response.

By "animal" it is intended mammals, birds, and the like. Animal or host includes mammals and human. The animal may be selected from the group consisting of equine (e.g., horse), canine (e.g., dogs, wolves, foxes, coyotes, jackals), feline (e.g., lions, tigers, domestic cats, wild cats, other big cats, and other felines including cheetahs and lynx), ovine (e.g., sheep), bovine (e.g., cattle), porcine (e.g., pig), caprine (e.g., goat), avian (e.g., chicken, duck, goose, turkey, quail, pheasant, parrot, finches, hawk, crow, ostrich, emu and cassowary), primate (e.g., prosimian, tarsier, monkey, gibbon, ape), and fish.

The term "plants" as used herein includes both dicotyledonous (dicot) plants and monocotyledonous (monocot) plant. Dicot plants include, but are not limited to, legumes such as pea, alfalfa and soybean, carrot, celery, tomato, potato, tobacco, pepper, oilseed rape, beet, cabbage, cauliflower, broccoli, lettuce, peanut, and the like. Monocot plants include, but are not limited to, cereals such as wheat, barley, sorghum and millet, rye, triticale, maize, rice or oats, sugarcane, members of the microalgae family, grasses, and the like. The term "plant" also include non-flowering plants including, but not limited to, ferns, horsetails, club mosses, mosses, liverworts, hornworts, algae, for example, red, brown, and green algae, gametophytes, and the like.

The term "algae" and "alga" as used herein includes any strain of algae capable of producing a polypeptide or fragment or variant thereof. The algae may be microalgae. The microalgae may be *Thraustochytriaceae,* for example, *Schizochytrium, Thraustochytrium, Labyrinthuloides, and Japonochytrium.*

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of' and "consists essentially of' have the meaning ascribed to them in U.S. Patent law, e.g., they allow for elements not explicitly recited, but exclude elements that are found in the prior art or that affect a basic or novel characteristic of the invention.
The antigenic polypeptides of the invention are capable of protecting against FMDV. That is, they are capable of stimulating an immune response in an animal. By "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a polypeptide, an epitope, a hapten, or any combination thereof. Alternately, the immunogen or antigen may comprise a toxin or antitoxin.

The term "immunogenic protein, polypeptide, or peptide" as used herein includes polypeptides that are immunologically active in the sense that once administered to the host, it is able to evoke an immune response of the humoral and/or cellular type directed against the protein. Preferably the protein fragment is such that it has substantially the same immunological activity as the total protein. Thus, a protein fragment described herein comprises or consists essentially of or consists of at least one epitope or antigenic determinant. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. See, e.g., Epitope Mapping Protocols in Methods in Molecular Biology, Vol. 66 (Glenn E. Morris, Ed., 1996). For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Pat. No. 4,708,871; Geysen et al., 1984; Geysen et al., 1986. Similarly, conformational epitopes are readily identified by determining spatial conformation of amino acids such as by, e.g., x-ray crystallography and 2-dimensional nuclear magnetic resonance. See, e.g., Epitope Mapping Protocols, supra. Methods especially applicable to the proteins of *T. parva* are fully described in PCT/US2004/022605.

As discussed, described herein are active fragments and variants of the antigenic polypeptide. Thus, the term "immunogenic protein, polypeptide, or peptide" further contemplates deletions, additions and substitutions to the sequence, so long as the polypeptide functions to produce an immunological response as defined herein. The term "conservative variation" denotes the replacement of an amino acid residue by another biologically similar residue, or the replacement of a nucleotide in a nucleic acid sequence such that the encoded amino acid residue does not change or is another biologically similar residue. In this regard, particularly preferred substitutions will generally be conservative in nature, i.e., those substitutions that take place within a family of amino acids. For example, amino acids are generally divided into four families: (1) acidic--aspartate and glutamate; (2) basic--lysine, arginine, histidine; (3) non-polar--alanine, valine, leucine, isoleucine, proline, phenylalanine, methionine, tryptophan; and (4) uncharged polar--glycine, asparagine, glutamine, cysteine, serine, threonine, tyrosine. Phenylalanine, tryptophan, and tyrosine are sometimes classified as aromatic amino acids. Examples of conservative variations include the substitution of one hydrophobic residue such as isoleucine, valine, leucine or methionine for another hydrophobic residue, or the substitution of one polar residue for another polar residue, such as the substitution of arginine for lysine, glutamic acid for aspartic acid, or glutamine for asparagine, and the like; or a similar conservative replacement of an amino acid with a structurally related amino acid that will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide. All of the polypeptides produced by these modifications are included herein. The term "conservative variation" also includes the use of a substituted amino acid in place of an unsubstituted parent amino acid provided that antibodies raised to the substituted polypeptide also immunoreact with the unsubstituted polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

Synthetic antigens are also included within the definition, for example, polyepitopes, flanking epitopes, and other recombinant or synthetically derived antigens. See, e.g., Bergmann et al., 1993; Bergmann et al., 1996; Suhrbier, 1997; Gardner et al., 1998. Immunogenic fragments described herein will usually include at least about 3 amino acids, at least about 5 amino acids, at least about 10-15 amino acids, or about 15-25 amino acids or more amino acids, of the molecule. There is no critical upper limit to the length of the fragment, which could comprise nearly the full-length of the protein sequence, or even a fusion protein comprising at least one epitope of the protein.

Accordingly, a minimum structure of a polynucleotide expressing an epitope is that it comprises or consists essentially of or consists of nucleotides encoding an epitope or antigenic determinant of an FMDV polypeptide. A polynucleotide encoding a fragment of an FMDV polypeptide may comprise or consist essentially of or consist of a minimum of 15 nucleotides, about 30-45 nucleotides, about 45-75, or at least 57, 87 or 150 consecutive or contiguous nucleotides of the sequence encoding the polypeptide. Epitope determination procedures, such as, generating overlapping peptide libraries (Hemmer et al., 1998), Pepscan (Geysen et al., 1984; Geysen et al., 1985; Van der Zee R. et al., 1989; Geysen, 1990; Multipin. RTM. Peptide Synthesis Kits de Chiron) and algorithms (De Groot et al., 1999; PCT/US2004/022605) can be used.

The term "nucleic acid" and "polynucleotide" refers to RNA or DNA that is linear or branched, single or double stranded, or a hybrid thereof. The term also encompasses RNA/DNA hybrids. The following are non-limiting examples of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes and primers. A polynucleotide may comprise modified nucleotides, such as methylated nucleotides and nucleotide analogs, uracyl, other sugars and linking groups such as fluororibose and thiolate, and nucleotide branches. The sequence of nucleotides may be further modified after polymerization, such as by conjugation, with a labeling component. Other types of modifications included in this definition are caps, substitution of one or more of the naturally occurring nucleotides with an analog, and introduction of means for attaching the polynucleotide to proteins, metal ions, labeling components, other polynucleotides or solid support. The polynucleotides can be obtained by chemical synthesis or derived from a microorganism.

The term "gene" is used broadly to refer to any segment of polynucleotide associated with a biological function. Thus, genes include introns and exons as in genomic sequence, or just the coding sequences as in cDNAs and/or the regulatory sequences required for their expression. For example, gene also refers to a nucleic acid fragment that expresses mRNA or functional RNA, or encodes a specific protein, and which includes regulatory sequences.

Described herein is a complementary strand to a polynucleotide encoding an FMDV antigen, epitope or immunogen. The complementary strand can be polymeric and of any length, and can contain deoxyribonucleotides, ribonucleotides, and analogs in any combination.

The terms "protein", "peptide", "polypeptide" and "polypeptide fragment" are used interchangeably herein to refer to polymers of amino acid residues of any length. The polymer can be linear or branched, it may comprise modified amino acids or amino acid analogs, and it may be interrupted by chemical moieties other than amino acids. The terms also encompass an amino acid polymer that has been modified naturally or by intervention; for example disulfide bond formation, glycosylation, lipidation, acetylation, phosphorylation, or any other manipulation or modification, such as conjugation with a labeling or bioactive component.

An "isolated" biological component (such as a nucleic acid or protein or organelle) refers to a component that has been substantially separated or purified away from other biological components in the cell of the organism in which the component naturally occurs, for instance, other chromosomal and extra-chromosomal DNA and RNA, proteins, and organelles. Nucleic acids and proteins that have been "isolated" include nucleic acids and proteins purified by standard purification methods. The term also embraces nucleic acids and proteins prepared by recombinant technology as well as chemical synthesis.

The term "purified" as used herein does not require absolute purity; rather, it is intended as a relative term. Thus, for example, a purified polypeptide preparation is one in which the polypeptide is more enriched than the polypeptide is in its natural environment. That is the polypeptide is separated from cellular components. By "substantially purified" it is intended that such that the polypeptide represents several embodiments at least 60%, at least 70%, at least 80%, at least 90%, at least 95%, or at least 98%, or more of the cellular components or materials have been removed. Likewise, the polypeptide may be partially purified. By "partially purified" is intended that less than 60% of the cellular components or material is removed. The same applies to polynucleotides. The polypeptides disclosed herein can be purified by any of the means known in the art.

As noted above, the antigenic polypeptides or fragments or variants thereof are FMDV antigenic polypeptides that are produced in duckweed. Fragments and variants of the disclosed polynucleotides and polypeptides encoded thereby are also e described herein. By "fragment" is intended a portion of the polynucleotide or a portion of the antigenic amino acid sequence encoded thereby. Fragments of a polynucleotide may encode protein fragments that retain the biological activity of the native protein and hence have immunogenic activity as noted elsewhere herein. Fragments of the polypeptide sequence retain the ability to induce a protective immune response in an animal.

"Variants" is intended to mean substantially similar sequences. For polynucleotides, a variant comprises a deletion and/or addition of one or more nucleotides at one or more sites within the native polynucleotide and/or a substitution of one or more nucleotides at one or more sites in the native polynucleotide. As used herein, a "native" polynucleotide or polypeptide comprises a naturally occurring nucleotide sequence or amino acid sequence, respectively. Variants of a particular polynucleotide of the invention (i.e., the reference polynucleotide) can also be evaluated by comparison of the percent sequence identity between the polypeptide encoded by a variant polynucleotide and the polypeptide encoded by the reference polynucleotide. "Variant" protein is intended to mean a protein derived from the native protein by deletion or addition of one or more amino acids at one or more sites in the native protein and/or substitution of one or more amino acids at one or more sites in the native protein. Variant proteins encompassed by the present invention are biologically active, that is they the ability to elicit an immune response.

In one aspect, the present invention provides FMDV polypeptides from ovine, bovine, caprine, or porcine. described herein is a polypeptide having a sequence as set forth in SEQ ID NOs:3, 10, 12, 17, 20, 23, 26, or 29, and variant or fragment thereof.

Moreover, homologs of FMDV polypeptides from ovine, bovine, caprine, or porcine are described herein. As used herein, the term "homologs" includes orthologs, analogs and paralogs. The term "analogs" refers to two polynucleotides or polypeptides that have the same or similar function, but that have evolved separately in unrelated organisms. The term "orthologs" refers to two polynucleotides or polypeptides from different species, but that have evolved from a common ancestral gene by speciation. Normally, orthologs encode polypeptides having the same or similar functions. The term "paralogs" refers to two polynucleotides or polypeptides that are related by duplication within a genome. Paralogs usually have different functions, but these functions may be related. Analogs, orthologs, and paralogs of a wild-type FMDV polypeptide can differ from the wild-type FMDV polypeptide by post-translational modifications, by amino acid sequence differences, or by both. In particular, homologs of the invention will generally exhibit at least 80-85%, 85-90%, 90-95%, or 95%, 96%, 97%, 98% , 99% sequence identity, with all or part of the wild-type FMDV or polynucleotide sequences, and will exhibit a similar function. Variants include allelic variants. The term "allelic variant" refers to a polynucleotide or a polypeptide containing polymorphisms that lead to changes in the amino acid sequences of a protein and that exist within a natural population (e.g., a virus species or variety). Such natural allelic variations can typically result in 1- 5% variance in a polynucleotide or a polypeptide. Allelic variants can be identified by sequencing the nucleic acid sequence of interest in a number of different species, which can be readily carried out by using hybridization probes to identify the same gene genetic locus in those species. Any and all such nucleic acid variations and resulting amino acid polymorphisms or variations that are the result of natural allelic variation and that do not alter the functional activity of gene of interest, are described herein.

As used herein, the term "derivative" or "variant" refers to a polypeptide, or a nucleic acid encoding a polypeptide, that has one or more conservative amino acid variations or other minor modifications such that (1) the corresponding polypeptide has substantially equivalent function when compared to the wild type polypeptide or (2) an antibody raised against the polypeptide is immunoreactive with the wild-type polypeptide. These variants or derivatives include polypeptides having minor modifications of the FMDV polypeptide primary amino acid sequences that may result in peptides which have substantially equivalent activity as compared to the unmodified counterpart polypeptide. Such modifications may be deliberate, as by site-directed mutagenesis, or may be spontaneous. The term "variant" further contemplates deletions, additions and substitutions to the sequence, so long as the polypeptide functions to produce an immunological response as defined herein.

The term "conservative variation" denotes the replacement of an amino acid residue by another biologically similar residue, or the replacement of a nucleotide in a nucleic acid sequence such that the encoded amino acid residue does not change or is another biologically similar residue. In this regard, particularly preferred substitutions will generally be conservative in nature, as described above.

The polynucleotides of the disclosure include sequences that are degenerate as a result of the genetic code, e.g., optimized codon usage for a specific host. As used herein, "optimized" refers to a polynucleotide that is genetically engineered to increase its expression in a given species. To provide optimized polynucleotides coding for FMDV polypeptides, the DNA sequence of the FMDV protein gene can be modified to 1) comprise codons preferred by highly expressed genes in a particular species; 2) comprise an A+T or G+C content in nucleotide base composition to that substantially found in said species; 3) form an initiation sequence of said species; or 4) eliminate sequences that cause destabilization, inappropriate polyadenylation, degradation and termination of RNA, or that form secondary structure hairpins or RNA splice sites. Increased expression of FMDV protein in said species can be achieved by utilizing the distribution frequency of codon usage in eukaryotes and prokaryotes, or in a particular species. The term "frequency of preferred codon usage" refers to the preference exhibited by a specific host cell in usage of nucleotide codons to specify a given amino acid. There are 20 natural amino acids, most of which are specified by more than one codon. Therefore, all degenerate nucleotide sequences are included in the disclosure as long as the amino acid sequence of the FMDV polypeptide encoded by the nucleotide sequence is functionally unchanged.

The sequence identity between two amino acid sequences may be established by the NCBI (National Center for Biotechnology Information) pairwise blast and the blosum62 matrix, using the standard parameters (see, e.g., the BLAST or BLASTX algorithm available on the "National Center for Biotechnology Information" (NCBI, Bethesda, Md., USA) server, as well as in Altschul *et al.*; and thus, this document speaks of using the algorithm or the BLAST or BLASTX and BLOSUM62 matrix by the term "blasts").

The "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur and Lipman), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics™ Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the invention and can be derived from DNA sequences, by thymidine (T) in the DNA sequence being considered equal to uracil (U) in RNA sequences.

The sequence identity or sequence similarity of two amino acid sequences, or the sequence identity between two nucleotide sequences can be determined using Vector NTI software package (Invitrogen, 1600 Faraday Ave., Carlsbad, CA).

The following documents provide algorithms for comparing the relative identity or homology of sequences, and additionally or alternatively with respect to the foregoing, the teachings in these references can be used for determining percent homology or identity: Needleman SB and Wunsch CD; Smith TF and Waterman MS; Smith TF, Waterman MS and Sadler JR; Feng DF and Dolittle RF; Higgins DG and Sharp PM; Thompson JD, Higgins DG and Gibson TJ; and, Devereux J, Haeberlie P and Smithies O. And, without undue experimentation, the skilled artisan can consult with many other programs or references for determining percent homology.

Hybridization reactions can be performed under conditions of different "stringency." Conditions that increase stringency of a hybridization reaction are well known. See for example, "Molecular Cloning: A Laboratory Manual", second edition (Sambrook et al., 1989).

The invention further encompasses the FMDV polynucleotides contained in a vector molecule or an expression vector and operably linked to a promoter element and optionally to an enhancer.

A "vector" refers to a recombinant DNA or RNA plasmid or virus that comprises a heterologous polynucleotide to be delivered to a target cell, either *in vitro* or *in vivo.* The heterologous polynucleotide may comprise a sequence of interest for purposes of prevention or therapy, and may optionally be in the form of an expression cassette. As used herein, a vector needs not be capable of replication in the ultimate target cell or subject. The term includes cloning vectors and viral vectors.

The term "recombinant" means a polynucleotide semisynthetic, or synthetic origin which either does not occur in nature or is linked to another polynucleotide in an arrangement not found in nature.

"Heterologous" means derived from a genetically distinct entity from the rest of the entity to which it is being compared. For example, a polynucleotide may be placed by genetic engineering techniques into a plasmid or vector derived from a different source, and is a heterologous polynucleotide. A promoter removed from its native coding sequence and operatively linked to a coding sequence other than the native sequence is a heterologous promoter.

The present invention relates to ovine, bovine, caprine and porcine vaccines or pharmaceutical or immunological compositions which may comprise an effective amount of a recombinant FMDV antigens and a pharmaceutically or veterinarily acceptable carrier, excipient, or vehicle.

The subject matter described herein is directed in part, to compositions and methods related to the FMDV antigen prepared in a plant or alga expression system that was highly immunogenic and protected animals against challenge from homologous and heterologous FMDV strains.

### Compositions

The present invention relates to an FMDV vaccine or composition which may comprise an effective amount of a recombinant FMDV antigen and a pharmaceutically or veterinarily acceptable carrier, excipient, or vehicle. Described herein, the recombinant FMDV antigen is expressed in a plant or alga.

In an embodiment, the subject matter disclosed herein is directed to a composition comprising an FMDV antigen produced by a duckweed expression system and plant material from duckweed, including the genus *Lemna,* and a pharmaceutical or veterinarily acceptable carrier, excipient or vehicle. In another embodiment, the subject matter disclosed herein is directed to an optionally aglycosylated protein produced by a duckweed expression system comprising an FMDV antigen.

In one embodiment, the recombinant FMDV antigen is expressed in algae. In yet another embodiment, the algae are selected from *Schizochytrium.* In one embodiment, the recombinant FMDV antigen may be expressed in a *Schizochytrium* protein expression system, as described, for example, in US 7,001,772, US 2008/0022422, US provisional App. No. 61/160,618, and US provisional applications concurrently filed on December 28, 2009 by Martek BioScience Corp. (MD, USA).

In an embodiment, the subject matter disclosed herein is directed to a protein produced by a plant or alga expression system comprising an FMDV antigen and material from the plant or alga.

In an embodiment, the subject matter disclosed herein is directed to a vaccine or composition comprising an FMDV antigen produced by a duckweed expression system.

In an embodiment, the subject matter disclosed herein is directed to a vaccine or composition comprising an FMDV antigen produced by a duckweed expression system and plant material from duckweed.

In an embodiment, the subject matter disclosed herein is directed to a stably transformed plant or plant culture that expresses an FMDV antigen wherein the plant or plant culture is selected from duckweed.

Described herein is any FMDV polypeptide, antigen, epitope or immunogen that elicits an immunogenic response in an animal, such as an ovine, bovine, caprine or porcine. The FMDV polypeptide, antigen, epitope or immunogen may be any FMDV polypeptide, antigen, epitope or immunogen, such as, but not limited to, a protein, peptide or fragment thereof, that elicits, induces or stimulates a response in an animal, such as an ovine, bovine, caprine or porcine.

In an embodiment wherein the FMDV immunological composition or vaccine is a recombinant immunological composition or vaccine, the composition or vaccine comprising a recombinant vector and a pharmaceutical or veterinary acceptable excipient, carrier or vehicle; the recombinant vector is plant expression vector which may comprise a polynucleotide encoding a polypeptide, antigen, epitope or immunogen. The FMDV polypeptide, antigen, epitope or immunogen, may be VP1, VP2, VP3, VP4, VP5, NS1, VP7, NS2, VP6, NS3, NS3a, PI, VP0, 3C, or any fragment thereof.

In another embodiment, the FMDV antigen is PI, VP0, VP3, VP1, VP2, VP4, 2A, 2B, or 3C.

In an embodiment wherein the FMDV immunological composition or vaccine is a recombinant immunological composition or vaccine, the composition or vaccine comprising a recombinant vector and a pharmaceutical or veterinary acceptable excipient, carrier or vehicle; the recombinant vector is plant expression vector which may comprise a polynucleotide encoding an FMDV polypeptide, antigen, epitope or immunogen. The FMDV polypeptide, antigen, epitope or immunogen, may be an FMDV polypeptide VP1, VP2, VP3, VP4, 2A, 2B or 3C. In one embodiment, the nucleic acid molecule encoding one or more foot-and-mouth disease virus (FMDV) antigen(s) is a cDNA encoding FMDV PI region and a cDNA encoding FMDV 3C protease of FMDV.

In one embodiment, the FMDV antigen may be a P1-3C polypeptide. In another embodiment, the FMDV antigen may be PI alone, or P1-2A/2B1. In yet another embodiment, the FMDV antigen may be VP0-VP3. In another embodiment, the FMDV antigen may be VP4-VP2. In still another embodiment, the FMDV antigen may be 3C, or may be 3C with a 5'UTR optimized for expression in duckweed. In one embodiment, both P1-2A/2B1 and 3C polypeptides may be expressed in duckweed using a single construct and the expression may be regulated by one or more than one promoter sequences.

In another embodiment, the FMDV antigen may be *FMDV O1 Manisa, O1 BFS or Campos, A24 Cruzeiro, Asia 1 Shamir, A Iran'96, A22 Iraq, SAT2 Saudi Arabia.*

The present invention relates to an FMDV vaccine which may comprise an effective amount of a recombinant FMDV antigen and a pharmaceutically or veterinarily acceptable carrier, excipient, or vehicle. Described herein, the FMDV antigen may be FMDV PI, VP0, VP3, VP1, VP2, VP4, or 3C.

Further described herein, the recombinant FMDV antigen is expressed in a plant or alga. In yet another embodiment, the plant is a duckweed plant, including a *Lemna* plant. In yet another embodiment, the plant is *Lemna minor.* In one embodiment, the recombinant FMDV antigen may be expressed in a proprietary *Lemna minor* protein expression system, advantageously Biolex's LEX system^{SM}. Described herein, the algae are selected from *Schizochytrium.* Described herein, the recombinant FMDV antigen may be expressed in a *Schizochytrium* protein expression system, as described, for example, in US 7,001,772, US 2008/0022422, US 2010/0233760 A1 by Martek BioScience Corp. (MD, USA).

In another embodiment, pharmaceutically or veterinarily acceptable carrier, excipient, or vehicle may be a water-in-oil emulsion. In yet another embodiment, the water-in-oil emulsion may be an oil-in-water emulsion.

The invention further encompasses the FMDV polynucleotides contained in a vector molecule or an expression vector and operably linked to a promoter element and optionally to an enhancer.

Described herein are FMDV polypeptides, particularly ovine, bovine, caprine or porcine polypeptides having a sequence as set forth in SEQ ID NO:3 and variants or fragments thereof.

Described herein is a polypeptide having at least 70%, at least 75%, at least 80%, at least 85%, at least 90%, at least 95%, 96%, 97%, 98% or 99% sequence identity to an antigenic polypeptide of the invention, particularly to the polypeptides having a sequence as set forth in SEQ ID NOs:3, 10, 12, 17, 20, 23, 26, or 29.

Described herein are fragments and variants of the FMDV polypeptides identified above (SEQ ID NOs:3, 10, 12, 17, 20, 23, 26, or 29) which may readily be prepared by one of skill in the art using well-known molecular biology techniques.

Variants are homologous polypeptides having an amino acid sequence at least 75%, 80%, 85%, 90%, 95%, 96%, 97%, 98%, or 99% identity to the amino acid sequence as set forth in SEQ ID NOs:3, 10, 12, 17, 20, 23, 26, or 29.

An immunogenic fragment of an FMDV polypeptide includes at least 8, 10, 15, or 20 consecutive amino acids, at least 21 amino acids, at least 23 amino acids, at least 25 amino acids, or at least 30 amino acids of an FMDV polypeptide having a sequence as set forth in SEQ ID NOs:3, 10, 12, 17, 20, 23, 26, or 29, or variants thereof. In another embodiment, a fragment of an FMDV polypeptide includes a specific antigenic epitope found on a full-length FMDV polypeptide.

Further described herein is a polynucleotide encoding an FMDV polypeptide, such as a polynucleotide encoding a polypeptide having a sequence as set forth in SEQ ID NOs:3, 10, 12, 17, 20, 23, 26, or 29. Further described herein is a polynucleotide encoding a polypeptide having at least 70%, at least 75%, at least 80%, at least 85%, at least 90%, at least 95%, 96%, 97%, 98% or 99% sequence identity to a polypeptide having a sequence as set forth in SEQ ID NOs:3, 10, 12, 17, 20, 23, 26, or 29, or a conservative variant, an allelic variant, a homolog or an immunogenic fragment comprising at least eight or at least ten consecutive amino acids of one of these polypeptides, or a combination of these polypeptides.

Further described herein is a polynucleotide having a nucleotide sequence as set forth in SEQ ID NOs:1, 2, 4, 8, 9, 11, 13, 14, 15, 16, 18, 19, 21, 22, 24, 25, 27, 28, 30-35, or a variant thereof. Further described herein is a polynucleotide having at least 70%, at least 75%, at least 80%, at least 85%, at least 90%, at least 95%, at least 95%, 96%, 97%, 98% or 99% sequence identity to one of a polynucleotide having a sequence as set forth in SEQ ID NOs:1, 2, 4, 8, 9, 11, 13, 14, 15, 16, 18, 19, 21, 22, 24, 25, 27, 28, 30-35, or a variant thereof.

The polynucleotides of the invention may comprise additional sequences, such as additional encoding sequences within the same transcription unit, controlling elements such as promoters, ribosome binding sites, 5'UTR, 3'UTR, transcription terminators, polyadenylation sites, additional transcription units under control of the same or a different promoter, sequences that permit cloning, expression, homologous recombination, and transformation of a host cell, and any such construct as may be desirable to provide embodiments of this invention.

Elements for the expression of an FMDV polypeptide, antigen, epitope or immunogen are advantageously present in an inventive vector. In minimum manner, this comprises, consists essentially of, or consists of an initiation codon (ATG), a stop codon and a promoter, and optionally also a polyadenylation sequence for certain vectors such as plasmid and certain viral vectors, e.g., viral vectors other than poxviruses. When the polynucleotide encodes a polyprotein fragment, e.g. an FMDV peptide, advantageously, in the vector, an ATG is placed at 5' of the reading frame and a stop codon is placed at 3'. Other elements for controlling expression may be present, such as enhancer sequences, stabilizing sequences, such as intron and signal sequences permitting the secretion of the protein.

Further described herein are preparations comprising vectors, such as expression vectors, e.g., therapeutic compositions. The preparations can comprise one or more vectors, e.g., expression vectors, such as *in vivo* expression vectors, comprising and expressing one or more FMDV polypeptides, antigens, epitopes or immunogens. In one embodiment, the vector contains and expresses a polynucleotide that comprises, consists essentially of, or consists of a polynucleotide coding for (and advantageously expressing) an FMDV antigen, epitope or immunogen, in a pharmaceutically or veterinarily acceptable carrier, excipient or vehicle. Thus, described herein, the other vector or vectors in the preparation comprises, consists essentially of or consists of a polynucleotide that encodes, and under appropriate circumstances the vector expresses one or more other proteins of an FMDV polypeptide, antigen, epitope or immunogen, or a fragment thereof.

According to another embodiment, the vector or vectors in the preparation comprise, or consist essentially of, or consist of polynucleotide(s) encoding one or more proteins or fragment(s) thereof of an FMDV polypeptide, antigen, epitope or immunogen, the vector or vectors expressing the polynucleotide(s). In another embodiment, the preparation comprises one, two, or more vectors comprising polynucleotides encoding and expressing, advantageously *in vivo,* an FMDV polypeptide, antigen, fusion protein or an epitope thereof. Further described herein are mixtures of vectors that comprise polynucleotides encoding and expressing different FMDV polypeptides, antigens, epitopes or immunogens, e.g., an FMDV polypeptide, antigen, epitope or immunogen from different animal species such as, but not limited to, ovine, bovine, caprine or porcine
According to a yet further embodiment of the invention, the expression vector is a plasmid vector or a DNA plasmid vector, in particular an *in vivo* expression vector. In a specific, non-limiting example, the pVR1020 or 1012 plasmid (VICAL Inc.; Luke et al., 1997; Hartikka et al., 1996, see, e.g., U.S. Patent Nos. 5,846,946 and 6,451,769) can be utilized as a vector for the insertion of a polynucleotide sequence. The pVR1020 plasmid is derived from pVR1012 and contains the human tPA signal sequence. In one embodiment the human tPA signal comprises from amino acid M(1) to amino acid S(23) in Genbank under the accession number HUMTPA14. In another specific, non-limiting example, the plasmid utilized as a vector for the insertion of a polynucleotide sequence can contain the signal peptide sequence of equine IGF1 from amino acid M(24) to amino acid A(48) in Genbank under the accession number U28070. Additional information on DNA plasmids which may be consulted or employed in the practice are found, for example, in U.S. Patent Nos. 6,852,705; 6,818,628; 6,586,412; 6,576,243; 6,558,674; 6,464,984; 6,451,770; 6,376,473 and 6,221,362.

The term plasmid covers any DNA transcription unit comprising a polynucleotide according to the invention and the elements necessary for its *in vivo* expression in a cell or cells of the desired host or target; and, in this regard, it is noted that a supercoiled or non-supercoiled, circular plasmid, as well as a linear form, are intended to be within the scope of the invention.

Each plasmid comprises or contains or consists essentially of, in addition to the polynucleotide encoding an FMDV antigen, epitope or immunogen, optionally fused with a heterologous peptide sequence, variant, analog or fragment, operably linked to a promoter or under the control of a promoter or dependent upon a promoter. In general, it is advantageous to employ a strong promoter functional in eukaryotic cells. The strong promoter may be, but not limited to, the immediate early cytomegalovirus promoter (CMV-IE) of human or murine origin, or optionally having another origin such as the rat or guinea pig, the Super promoter (Ni, M. et al., Plant J. 7, 661-676, 1995.). The CMV-IE promoter can comprise the actual promoter part, which may or may not be associated with the enhancer part. Reference can be made to EP-A-260 148, EP-A-323 597, U.S. Patents Nos. 5,168,062, 5,385,839, and 4,968,615, as well as to PCT Application No WO87/03905. The CMV-IE promoter is advantageously a human CMV-IE (Boshart et al., 1985) or murine CMV-IE.

In more general terms, the promoter has either a viral, a plant, or a cellular origin. A strong viral promoter other than CMV-IE that may be usefully employed in the practice of the invention is the early/late promoter of the SV40 virus or the LTR promoter of the Rous sarcoma virus. A strong cellular promoter that may be usefully employed in the practice of the invention is the promoter of a gene of the cytoskeleton, such as e.g. the desmin promoter (Kwissa et al., 2000), or the actin promoter (Miyazaki et al., 1989).

Any of constitutive, regulatable, or stimulus-dependent promoters may be used. For example, constitutive promoters may include the mannopine synthase promoter from *Agrobacterium tumefaciens.* Alternatively, it may be advantageous to use heat shock gene promoters, drought-inducible gene promoters, pathogen-inducible gene promoters, wound-inducible gene promoters, and light/dark-inducible gene promoters. It may be useful to use promoters that are controlled by plant growth regulators, such as abscissic acid, auxins, cytokinins, and gibberellic acid. Promoters may also be chosen that give tissue-specific expression (e.g., root, leaf, and floral-specific promoters).

The plasmids may comprise other expression control elements. It is particularly advantageous to incorporate stabilizing sequence(s), e.g., intron sequence(s), for example, maize alcohol dehydrogenase intron (Callis et al. Genes & Dev.1(10):1183-1200, Dec. 1987), the first intron of the hCMV-IE (PCT Application No. WO1989/01036), the intron II of the rabbit β-globin gene (van Ooyen et al., 1979). In another embodiment, the plasmids may comprise 3' UTR. The 3' UTR may be, but not limited to, *agrobacterium* nopaline synthase (Nos) 3' UTR (Nopaline synthase: transcript mapping and DNA sequence. Depicker, A. et al. J. Mol. Appl. Genet., 1982; Bevan, NAR, 1984, 12(22): 8711-8721).

As to the polyadenylation signal (polyA) for the plasmids and viral vectors other than poxviruses, use can more be made of the poly(A) signal of the bovine growth hormone (bGH) gene (see U.S. 5,122,458), or the poly(A) signal of the rabbit β-globin gene or the poly(A) signal of the SV40 virus.

A "host cell" denotes a prokaryotic or eukaryotic cell that has been genetically altered, or is capable of being genetically altered by administration of an exogenous polynucleotide, such as a recombinant plasmid or vector. When referring to genetically altered cells, the term refers both to the originally altered cell and to the progeny thereof.

In one embodiment, the recombinant FMDV antigen is expressed in a transgenic plant or alga. In another embodiment, the transgenic plant is a *Lemna* plant. In yet another embodiment, the transgenic plant is *Lemna minor* (duckweed). In yet another embodiment, the recombinant FMDV antigen may be expressed in the *Lemna minor* (duckweed) protein expression system, the Biolex's LEX system^{SM}. Details of the *Lemna minor* (duckweed) protein expression system may be found, for example, in U.S. Patent Nos. 6,815,184, 7,022,309, 7,160,717, 7,176,024, 6,040,498, and 7,161,064. In yet another embodiment, the transgenic alga is *Schizochytrium.* Details of the algal protein expression system may be found, for example, in US 7,001,772, US 2008/0022422, US provisional application 61/160,618. The FMDV antigen in the embodiments may be any polypeptide disclosed herein, or a polypeptide encoded by any polynucleotide disclosed herein.

### Methods for Expressing FMDV polypeptides in Duckweed or Microalga

Thus, in some embodiments described herein, antigenic FMDV polypeptides, or fragments or variants thereof, are expressed in duckweed or microalga. These methods comprise the use of expression cassettes that are introduced into a duckweed plant or microalga using any suitable transformation method known in the art. Polynucleotides within these expression cassettes can be modified for enhanced expression of the antigenic FMDV polypeptide, or fragment or variant thereof, in duckweed or microalga, as follows.

### Cassettes for Duckweed or Microalga Expression of Antigenic FMDV Polypeptides

Transgenic duckweed or microalga expressing an antigenic FMDV polypeptide, or fragment or variant thereof, is obtained by transformation of duckweed or microalga with an expression cassette comprising a polynucleotide encoding the antigenic FMDV polypeptide, or fragment or variant thereof. In this manner, a polynucleotide encoding the antigenic FMDV polypeptide of interest, or fragment or variant thereof, is constructed within an expression cassette and introduced into a duckweed plant or microalga culture by any suitable transformation method known in the art.

In some embodiments, the duckweed plant or microalga that is transformed with an expression cassette comprising polynucleotide encoding the antigenic FMDV polypeptide of interest, or fragment or variant thereof, has also been transformed with an expression cassette that provides for expression of another heterologous polypeptide of interest, for example, another antigenic FMDV polypeptide, fragment, or variant thereof. The expression cassette providing for expression of another heterologous polypeptide of interest can be provided on the same polynucleotide (for example, on the same transformation vector) for introduction into a duckweed plant or microalga, or on a different polynucleotide (for example, on different transformation vectors) for introduction into the duckweed plant or microalga at the same time or at different times, by the same or by different methods of introduction, for example, by the same or different transformation methods.

The expression cassettes for use in transformation of duckweed or microalga comprise expression control elements that at least comprise a transcriptional initiation region (e.g., a promoter) operably linked to the polynucleotide of interest, i.e., a polynucleotide encoding an antigenic FMDV polypeptide, fragment, or variant thereof. "Operably linked" as used herein in reference to nucleotide sequences refers to multiple nucleotide sequences that are placed in a functional relationship with each other. Generally, operably linked DNA sequences are contiguous and, where necessary to join two protein coding regions, in reading frame. Such an expression cassette is provided with a plurality of restriction sites for insertion of the polynucleotide or polynucleotides of interest (e.g., one polynucleotide of interest, two polynucleotides of interest, etc.) to be under the transcriptional regulation of the promoter and other expression control elements. In particular embodiments of the invention, the polynucleotide to be transferred contains two or more expression cassettes, each of which contains at least one polynucleotide of interest.

By "expression control element" is intended a regulatory region of DNA, usually comprising a TATA box, capable of directing RNA polymerase II, or in some embodiments, RNA polymerase III, to initiate RNA synthesis at the appropriate transcription initiation site for a particular coding sequence. An expression control element may additionally comprise other recognition sequences generally positioned upstream or 5' to the TATA box, which influence (e.g., enhance) the transcription initiation rate. Furthermore, an expression control element may additionally comprise sequences generally positioned downstream or 3' to the TATA box, which influence (e.g., enhance) the transcription initiation rate.

The transcriptional initiation region (e.g., a promoter) may be native or homologous or foreign or heterologous to the duckweed or microalga host, or could be the natural sequence or a synthetic sequence. By foreign, it is intended that the transcriptional initiation region is not found in the wild-type duckweed or microalga host into which the transcriptional initiation region is introduced. By "functional promoter" is intended the promoter, when operably linked to a sequence encoding an antigenic FMDV polypeptide of interest, or fragment or variant thereof, is capable of driving expression (i.e., transcription and translation) of the encoded polypeptide, fragment, or variant. The promoters can be selected based on the desired outcome. Thus the expression cassettes of the invention can comprise constitutive, inducible, tissue-preferred, or other promoters for expression in duckweed.

Any suitable promoter known in the art can be employed in the expression cassettes according to the present invention, including bacterial, yeast, fungal, insect, mammalian, and plant promoters. For example, plant promoters, including duckweed or microalga promoters, may be used. Exemplary promoters include, but are not limited to, the Cauliflower Mosaic Virus 35S promoter, the opine synthetase promoters (e.g., nos, mas, ocs, etc.), the ubiquitin promoter, the actin promoter, the ribulose bisphosphate (RubP) carboxylase small subunit promoter, and the alcohol dehydrogenase promoter. The duckweed RubP carboxylase small subunit promoter is known in the art (Silverthorne et al. (1990) Plant Mol. Biol. 15:49). Other promoters from viruses that infect plants or microalgae are also suitable, including, but not limited to, promoters isolated from Dasheen mosaic virus, Chlorella virus (e.g., the Chlorella virus adenine methyltransferase promoter; Mitra et al. (1994) Plant Mol. Biol. 26:85), tomato spotted wilt virus, tobacco rattle virus, tobacco necrosis virus, tobacco ring spot virus, tomato ring spot virus, cucumber mosaic virus, peanut stump virus, alfalfa mosaic virus, sugarcane baciliform badnavirus and the like.

Expression control elements, including promoters, can be chosen to give a desired level of regulation. For example, in some instances, it may be advantageous to use a promoter that confers constitutive expression (e.g., the mannopine synthase promoter from *Agrobacterium tumefaciens*). Alternatively, in other situations, it may be advantageous to use promoters that are activated in response to specific environmental stimuli (e.g., heat shock gene promoters, drought-inducible gene promoters, pathogen-inducible gene promoters, wound-inducible gene promoters, and light/dark-inducible gene promoters) or plant growth regulators (e.g., promoters from genes induced by abscissic acid, auxins, cytokinins, and gibberellic acid). As a further alternative, promoters can be chosen that give tissue-specific expression (e.g., root, leaf, and floral-specific promoters).

The overall strength of a given promoter can be influenced by the combination and spatial organization of cis-acting nucleotide sequences such as upstream activating sequences. For example, activating nucleotide sequences derived from the *Agrobacterium tumefaciens* octopine synthase gene can enhance transcription from the *Agrobacterium tumefaciens* mannopine synthase promoter (see U.S. Patent 5,955,646 to Gelvin et al.). In the present invention, the expression cassette can contain activating nucleotide sequences inserted upstream of the promoter sequence to enhance the expression of the antigenic FMDV polypeptide of interest, or fragment or variant thereof. In one embodiment, the expression cassette includes three upstream activating sequences derived from the *Agrobacterium tumefaciens* octopine synthase gene operably linked to a promoter derived from an Agrobacterium tumefaciens mannopine synthase gene (see U.S Patent 5,955,646).

The expression cassette thus includes in the 5'-3' direction of transcription, an expression control element comprising a transcriptional and translational initiation region, a polynucleotide of encoding an antigenic FMDV polypeptide of interest (or fragment or variant thereof), and a transcriptional and translational termination region functional in plants. Any suitable termination sequence known in the art may be used in accordance with the present invention. The termination region may be native with the transcriptional initiation region, may be native with the coding sequence of interest, or may be derived from another source. Convenient termination regions are available from the Ti-plasmid *of A. tumefaciens,* such as the octopine synthetase and nopaline synthetase termination regions. See also Guerineau et al. (1991) Mol. Gen. Genet. 262:141; Proudfoot (1991) Cell 64:671; Sanfacon et al. (1991) Genes Dev. 5:141; Mogen et al. (1990) Plant Cell 2:1261; Munroe et al. (1990) Gene 91:151; Ballas et al. (1989) Nucleic Acids Res. 17:7891; and Joshi et al. (1987) Nucleic Acids Res. 15:9627. Additional exemplary termination sequences are the pea RubP carboxylase small subunit termination sequence and the Cauliflower Mosaic Virus 35S termination sequence.

Generally, the expression cassette will comprise a selectable marker gene for the selection of transformed duckweed cells or tissues. Selectable marker genes include genes encoding antibiotic resistance, such as those encoding neomycin phosphotransferase II (NEO) and hygromycin phosphotransferase (HPT), as well as genes conferring resistance to herbicidal compounds. Herbicide resistance genes generally code for a modified target protein insensitive to the herbicide or for an enzyme that degrades or detoxifies the herbicide in the plant before it can act. See DeBlock et al. (1987) EMBO J. 6:2513; DeBlock et al.(1989) Plant Physiol. 91:691; Fromm et al. (1990) BioTechnology 8:833; Gordon-Kamm et al. (1990) Plant Cell 2:603. For example, resistance to glyphosate or sulfonylurea herbicides has been obtained using genes coding for the mutant target enzymes, 5-enolpyruvylshikimate-3-phosphate synthase (EPSPS) and acetolactate synthase (ALS). Resistance to glufosinate ammonium, boromoxynil, and 2,4-dichlorophenoxyacetate (2,4-D) have been obtained by using bacterial genes encoding phosphinothricin acetyltransferase, a nitrilase, or a 2,4-dichlorophenoxyacetate monooxygenase, which detoxify the respective herbicides.

For purposes of the present invention, selectable marker genes include, but are not limited to, genes encoding neomycin phosphotransferase II (Fraley et al. (1986) CRC Critical Reviews in Plant Science 4:1); cyanamide hydratase (Maier-Greiner et al. (1991) Proc. Natl. Acad. Sci. USA 88:4250); aspartate kinase; dihydrodipicolinate synthase (Perl et al. (1993) BioTechnology 11:715); bar gene (Toki et al. (1992) Plant Physiol. 100:1503; Meagher et al. (1996) Crop Sci. 36:1367); tryptophan decarboxylase (Goddijn et al. (1993) Plant Mol. Biol. 22:907); neomycin phosphotransferase (NEO; Southern et al. (1982) J. Mol. Appl. Gen. 1:327); hygromycin phosphotransferase (HPT or HYG; Shimizu et al. (1986) Mol. Cell. Biol. 6:1074); dihydrofolate reductase (DHFR; Kwok et al. (1986) Proc. Natl. Acad. Sci. USA 83:4552); phosphinothricin acetyltransferase (DeBlock et al. (1987) EMBO J. 6:2513); 2,2-dichloropropionic acid dehalogenase (Buchanan-Wollatron et al. (1989) J. Cell. Biochem. 13D:330); acetohydroxyacid synthase (U.S. Pat. No. 4,761,373 to Anderson et al.; Haughn et al. (1988) Mol. Gen. Genet. 221:266); 5-enolpyruvyl-shikimate-phosphate synthase (aroA; Comai et al. (1985) Nature 317:741); haloarylnitrilase (WO 87/04181 to Stalker et al.); acetyl-coenzyme A carboxylase (Parker et al. (1990) Plant Physiol. 92:1220); dihydropteroate synthase (sulI; Guerineau et al. (1990) Plant Mol. Biol. 15:127); and 32 kDa photosystem II polypeptide (psbA; Hirschberg et al. (1983) Science 222:1346 (1983).

Also included are genes encoding resistance to: gentamycin (e.g., aacCl, Wohlleben et al. (1989) Mol. Gen. Genet. 217:202-208); chloramphenicol (Herrera-Estrella et al. (1983) EMBO J. 2:987); methotrexate (Herrera-Estrella et al. (1983) Nature 303:209; Meijer et al. (1991) Plant Mol. Biol. 16:807); hygromycin (Waldron et al. (1985) Plant Mol. Biol. 5:103; Zhijian et al. (1995) Plant Science 108:219; Meijer et al. (1991) Plant Mol. Bio. 16:807); streptomycin (Jones et al. (1987) Mol. Gen. Genet. 210:86); spectinomycin (Bretagne-Sagnard et al. (1996) Transgenic Res. 5:131); bleomycin (Hille et al. (1986) Plant Mol. Biol. 7:171); sulfonamide (Guerineau et al. (1990) Plant Mol. Bio. 15:127); bromoxynil (Stalker et al. (1988) Science 242:419); 2,4-D (Streber et al. (1989) BioTechnology 7:811); phosphinothricin (DeBlock et al. (1987) EMBO J. 6:2513); spectinomycin (Bretagne-Sagnard and Chupeau, Transgenic Research 5:131).

The bar gene confers herbicide resistance to glufosinate-type herbicides, such as phosphinothricin (PPT) or bialaphos, and the like. As noted above, other selectable markers that could be used in the vector constructs include, but are not limited to, the pat gene, also for bialaphos and phosphinothricin resistance, the ALS gene for imidazolinone resistance, the HPH or HYG gene for hygromycin resistance, the EPSP synthase gene for glyphosate resistance, the Hm1 gene for resistance to the Hc-toxin, and other selective agents used routinely and known to one of ordinary skill in the art. See Yarranton (1992) Curr. Opin. Biotech. 3:506; Chistopherson et al. (1992) Proc. Natl. Acad. Sci. USA 89:6314; Yao et al. (1992) Cell 71:63; Reznikoff (1992) Mol. Microbiol. 6:2419; Barkley et al. (1980) The Operon 177-220; Hu et al. (1987) Cell 48:555; Brown et al. (1987) Cell 49:603; Figge et al. (1988) Cell 52:713; Deuschle et al. (1989) Proc. Natl. Acad. Sci. USA 86:5400; Fuerst et al. (1989) Proc. Natl. Acad. Sci. USA 86:2549; Deuschle et al. (1990) Science 248:480; Labow et al. (1990) Mol. Cell. Biol. 10:3343; Zambretti et al. (1992) Proc. Natl. Acad. Sci. USA 89:3952; Baim et al. (1991) Proc. Natl. Acad. Sci. USA 88:5072; Wyborski et al. (1991) Nuc. Acids Res. 19:4647; Hillenand-Wissman (1989) Topics in Mol. And Struc. Biol. 10:143; Degenkolb et al. (1991) Antimicrob. Agents Chemother. 35:1591; Kleinschnidt et al. (1988) Biochemistry 27:1094; Gatz et al. (1992) Plant J. 2:397; Gossen et al. (1992) Proc. Natl. Acad. Sci. USA 89:5547; Oliva et al. (1992) Antimicrob. Agents Chemother. 36:913; Hlavka et al. (1985) Handbook of Experimental Pharmacology 78; and Gill et al. (1988) Nature 334:721.

The above list of selectable marker genes is not meant to be limiting. Any selectable marker gene can be used in the present invention.

### Modification of Nucleotide Sequences for Enhanced Expression in a Plant or microalga Host

Where the antigenic FMDV polypeptide or fragment or variant thereof is expressed within duckweed or microalga, the expressed polynucleotide sequence encoding the FMDV polypeptide or fragment or variant thereof can be modified to enhance its expression in duckweed or microalga, respectively. One such modification is the synthesis of the polynucleotide using plant-preferred codons, particularly duckweed-preferred codons, or using microalga-preferred codons, such as *Schizochytrium*-preferred codons. Methods are available in the art for synthesizing nucleotide sequences with plant-preferred codons. See, e.g., U.S. Patent Nos. 5,380,831 and 5,436,391; EP 0 359 472; EP 0 385 962; WO 91/16432; Perlak et al. (1991) Proc. Natl. Acad. Sci. USA 15:3324; Iannacome et al. (1997) Plant Mol. Biol. 34:485; and Murray et al. (1989) Nucleic Acids. Res. 17:477. Synthesis can be accomplished using any method known to one of skill in the art. The preferred codons may be determined from the codons of highest frequency in the proteins expressed in duckweed or microalga. For example, the frequency of codon usage for *Lemna minor* is found in Table 1, the frequency of codon usage for *Schizochytrium* is found in Table 2.

**Table 1. Lemna minor [gbpln]: 4 CDS's (1597 codons)**

| fields: [triplet] [frequency: **per thousand]** ([numbeer]) | | | | | | | |
|---|---|---|---|---|---|---|---|
| UUU | 17.5(28) | UCU | 13.8(22) | UAU | 8.8(14) | UGU | 5.0(8) |
| UUC | 36.3(58) | UCC | 17.5(28) | UAC | 15.7(25) | UGC | 14.4(23) |
| UUA | 5.6(9) | UCA | 14.4(23) | UAA | 0.0(0) | UGA | 1.9(3) |
| UUG | 13.8(22) | UCG | 13.8(22) | UAG | 0.6(1) | UGG | 16.3(26) |
| | | | | | | | |
| CUU | 15.7(25) | CCU | 11.9(19) | CAU | 6.9(11) | CGU | 4.4(7) |
| CUC | 25.7(41) | CCC | 15.7(25) | CAC | 16.9(27) | CGC | 18.2(29) |
| CUA | 5.0(8) | CCA | 11.3(18) | CAA | 10.0(16) | CGA | 6.3(10) |
| CUG | 21.3(34) | CCG | 14.4(23) | CAG | 22.5(36) | CGG | 10.6(17) |
| | | | | | | | |
| AUU | 18.8(30) | ACU | 9.4(15) | AAU | 13.8(22) | AGU | 10.0(16) |
| AUC | 19.4(31) | ACC | 17.5(28) | AAC | 21.9(35) | AGC | 15.0(24) |
| AUA | 1.9(3) | ACA | 5.0 (8) | AAA | 15.7(25) | AGA | 20.7(33) |
| AUG | 20.7(33) | ACG | 10.0(16) | AAG | 35.7(57) | AGG | 17.5(28) |
| | | | | | | | |
| GUU | 15.0(24) | GCU | 25.0(40) | GAU | 20.0(32) | GGU | 8.1(13) |
| GUC | 25.0(40) | GCC | 22.5(36) | GAC | 26.3(42) | GGC | 21.9(35) |
| GUA | 6.3(10) | GCA | 14.4(23) | GAA | 26.3(42) | GGA | 16.9(27) |
| GUG | 30.7(49) | GCG | 18.2(29) | GAG | 40.1(64) | GGG | 18.2(29) |

**Table 2. Schizochytrium sp. ATCC_20888 [gbpln]: 3 CDS's (6473 codons)**

| fields: [triplet] [frequency: **per thousand]** ([number]) | | | | | | | |
|---|---|---|---|---|---|---|---|
| UUU | 12.2(79) | UCU | 7.0(45) | UAU | 1.1(7) | UGU | 0.8(5) |
| UUC | 19.9(129) | UCC | 23.8(154) | UAC | 21.5(139) | UGC | 15.3(99) |
| UUA | 0.0(0) | UCA | 0.5(3) | UAA | 0.5(3) | UGA | 0.0(0) |
| UUG | 0.6(4) | UCG | 18.8(122) | UAG | 0.0(0) | UGG | 8.3(54) |
| | | | | | | | |
| CUU | 12.7(82) | CCU | 11.7(76) | CAU | 2.3(15) | CGU | 7.1(46) |
| CUC | 61.2(396) | CCC | 23.8(154) | CAC | 12.8(83) | CGC | 42.9(278) |
| CUA | 0.0(0) | CCA | 1.5(10) | CAA | 2.3(15) | CGA | 0.3(2) |
| CUG | 7.4(48) | CCG | 16.2(105) | CAG | 27.7(179) | CGG | 0.8(5) |
| | | | | | | | |
| AUU | 13.9 (90) | ACU | 9.1(59) | AAU | 1.9(12) | AGU | 1.5(10) |
| AUC | 33.5(217) | ACC | 29.2(189) | AAC | 32.4(210) | AGC | 15.6(101) |
| AUA | 0.0(0) | ACA | 1.5(10) | AAA | 2.2(14) | AGA | 0.2(1) |
| AUG | 27.8(180) | ACG | 9.6(62) | AAG | 54.5(353) | AGG | 0.0(0) |
| | | | | | | | |
| GUU | 8.3(54) | GCU | 24.4(158) | GAU | 13.4(87) | GGU | 13.0(84) |
| GUC | 53.0(343) | GCC | 86.0(557) | GAC | 45.0(291) | GGC | 54.5(353) |
| GUA | 0.2(1) | GCA | 4.0(26) | GAA | 7.3(47) | GGA | 3.9(25) |
| GUG | 14.4(93) | GCG | 15.9(103) | GAG | 62.3(403) | GGG | 0.5(3) |

For purposes of the present invention, "duckweed-preferred codons" refers to codons that have a frequency of codon usage in duckweed of greater than 17%. "*Lemna*-preferred codons" as used herein refers to codons that have a frequency of codon usage in the genus *Lemna* of greater than 17%. *"Lemna minor*-preferred codons" as used herein refers to codons that have a frequency of codon usage in *Lemna minor* of greater than 17% where the frequency of codon usage in *Lemna minor* is obtained from the Codon Usage Database (GenBank Release 160.0, June 15, 2007). "Microalgae-preferred codons" refers to codons that have a frequency of codon usage in microalgae of greater than 17%. "microalgae-preferred codons" as used herein refers to codons that have a frequency of codon usage in the family *Thraustochytriaceae* of greater than 17%. *"Schizochytrium-*preferred codons" as used herein refers to codons that have a frequency of codon usage in *schizochytrium* of greater than 17% where the frequency of codon usage in *schizochytrium* is obtained from the Codon Usage Database.

It is further recognized that all or any part of the polynucleotide encoding the antigenic FMDV polypeptide of interest, or fragment or variant thereof, may be optimized or synthetic. In other words, fully optimized or partially optimized sequences may also be used. For example, 40%, 45%, 50%, 55%, 60%, 65%, 70%, 75%, 80%, 85%, 87%, 90%, 91%, 92%, 93%, 94%, 95%, 96%, 97%, 98%, 99%, or 100% of the codons may be duckweed-preferred or microalgae-preferred codons. In one embodiment, between 90 and 96% of the codons are duckweed-preferred or microalgae-preferred codons. The coding sequence of a polynucleotide sequence encoding an antigenic FMDV polypeptide of interest, or fragment or variant thereof, may comprise codons used with a frequency of at least 17% in *Lemna gibba* or at least 17% in *Lemna minor.* In another such embodiment, the expression cassette comprises SEQ ID NO:9, which contains *Lemna minor*-preferred codons encoding the PI polypeptide set forth in SEQ ID NO:10. In a related embodiment, the FMDV polypeptide is a P1-3C polypeptide, for example, the P1-3C polypeptide as set forth in SEQ ID NO:3, and the expression cassette comprises an optimized coding sequence for this P1-3C polypeptide, where the coding sequence comprises duckweed-preferred codons, for example, *Lemna minor*-preferred or *Lemna gibba*-preferred codons. In one such embodiment, the expression cassette comprises SEQ ID NO:2, which contains *Lemna minor*-preferred codons encoding the FMDV polypeptide as set forth in SEQ ID NO:3.

Other modifications can also be made to the polynucleotide encoding the antigenic FMDV polypeptide of interest, or fragment or variant thereof, to enhance its expression in duckweed or microalga. These modifications include, but are not limited to, elimination of sequences encoding spurious polyadenylation signals, exon-intron splice site signals, transposon-like repeats, and other such well characterized sequences that may be deleterious to gene expression. The G-C content of the sequence may be adjusted to levels average for duckweed, as calculated by reference to known genes expressed in this plant. When possible, the polynucleotide encoding the heterologous polypeptide of interest may be modified to avoid predicted hairpin secondary mRNA structures.

There are known differences between the optimal translation initiation context nucleotide sequences for translation initiation codons in animals, plants and algae. "Translation initiation context nucleotide sequence" as used herein refers to the identity of the three nucleotides directly 5' of the translation initiation codon. "Translation initiation codon" refers to the codon that initiates the translation of the mRNA transcribed from the nucleotide sequence of interest. The composition of these translation initiation context nucleotide sequences can influence the efficiency of translation initiation. See, for example, Lukaszewicz et al. (2000) Plant Science 154:89-98; and Joshi et al. (1997); Plant Mol. Biol. 35:993-1001. In the present invention, the translation initiation context nucleotide sequence for the translation initiation codon of the polynucleotide encoding the antigenic FMDV polypeptide of interest, or fragment or variant thereof, may be modified to enhance expression in duckweed. In one embodiment, the nucleotide sequence is modified such that the three nucleotides directly upstream of the translation initiation codon are "ACC." In a second embodiment, these nucleotides are "ACA."

Expression of an antigenic FMDV polypeptide in duckweed or alga can also be enhanced by the use of 5' leader sequences. Such leader sequences can act to enhance translation. Translation leaders are known in the art and include, but are not limited to, picornavirus leaders, e.g., EMCV leader (Encephalomyocarditis 5' noncoding region; Elroy-Stein et al. (1989) Proc. Natl. Acad. Sci USA 86:6126); potyvirus leaders, e.g., TEV leader (Tobacco Etch Virus; Allison et al. (1986) Virology 154:9); human immunoglobulin heavy-chain binding protein (BiP; Macajak and Sarnow (1991) Nature 353:90); untranslated leader from the coat protein mRNA of alfalfa mosaic virus (AMV RNA 4; Jobling and Gehrke (1987) Nature 325:622); tobacco mosaic virus leader (TMV; Gallie (1989) Molecular Biology of RNA, 23:56); potato etch virus leader (Tomashevskaya et al. (1993) J. Gen. Virol. 74:2717-2724); Fed-1 5' untranslated region (Dickey (1992) EMBO J. 11:2311-2317); RbcS 5' untranslated region (Silverthorne et al. (1990) J. Plant. Mol. Biol. 15:49-58); and maize chlorotic mottle virus leader (MCMV; Lommel et al. (1991) Virology 81:382). See also, Della-Cioppa et al. (1987) Plant Physiology 84:965. Leader sequence comprising plant intron sequence, including intron sequence from the maize alcohol dehydrogenase 1 (ADH1) gene, the castor bean catalase gene, or the *Arabidopsis* tryptophan pathway gene PAT1 has also been shown to increase translational efficiency in plants (Callis et al. (1987) Genes Dev. 1:1183-1200; Mascarenhas et al. (1990) Plant Mol. Biol. 15:913-920).

Described herein, a nucleotide sequence corresponding to nucleotides 1222-1775 of the maize alcohol dehydrogenase 1 gene (SEQ ID NO:4; ADH1; GenBank Accession Number X04049) is inserted upstream of the polynucleotide encoding the antigenic FMDV polypeptide of interest, or fragment or variant thereof, to enhance the efficiency of its translation. In an embodiment, the expression cassette contains the leader from the *Lemna gibba* ribulose-bis-phosphate carboxylase small subunit 5B gene (RbcS leader; see Buzby et al. (1990) Plant Cell 2:805-814).

It is recognized that any of the expression-enhancing nucleotide sequence modifications described above can be used in the present invention, including any single modification or any possible combination of modifications. The phrase "modified for enhanced expression" in duckweed, as used herein, refers to a polynucleotide sequence that contains any one or any combination of these modifications.

### Transformed Duckweed Plants and Duckweed Nodule Cultures or Transformed Microalgae

The present invention provides transformed duckweed plants expressing an antigenic FMDV polypeptide of interest, or fragment or variant thereof. The term "duckweed" refers to members of the family *Lemnaceae.* This family currently is divided into five genera and 38 species of duckweed as follows: genus *Lemna* (*L. aequinoctialis, L. disperma, L. ecuadoriensis, L. gibba, L. japonica, L. minor, L. miniscula, L. obscura, L. perpusilla, L. tenera, L. trisulca, L. turionifera, L. valdiviana*); genus *Spirodela* (*S. intermedia, S. polyrrhiza, S. punctata*); genus *Wolffia* (*Wa. angusta, Wa. arrhiza, Wa. australina, Wa. borealis, Wa. brasiliensis, Wa. columbiana, Wa. elongata, Wa. globosa, Wa. microscopica, Wa. neglecta*); genus *Wolfiella* (*Wl. caudata, Wl. denticulata, Wl. gladiata, Wl. hyalina, Wl. lingulata, Wl. repunda, Wl. rotunda,* and *Wl. neotropica*) and genus *Landoltia* (*L. punctata*). Any other genera or species of *Lemnaceae,* if they exist, are also aspects of the present invention. *Lemna* species can be classified using the taxonomic scheme described by Landolt (1986) Biosystematic Investigation on the Family of Duckweeds: The family of Lemnaceae-A Monograph Study (Geobatanischen Institut ETH, Stiftung Rubel, Zurich).

As used herein, "plant" includes whole plants, plant organs (e.g., fronds (leaves), stems, roots, *etc.*), seeds, plant cells, and progeny of same. Parts of transgenic plants are to be understood within the scope of the invention to comprise, e.g., plant cells, plant protoplasts, plant cell tissue cultures from which plants can be regenerated, tissues, plant calli, embryos as well as flowers, ovules, stems, fruits, leaves, roots, root tips, nodules, and the like originating in transgenic plants or their progeny previously transformed with a polynucleotide of interest and therefore consisting at least in part of transgenic cells. As used herein, the term "plant cell" includes cells of seeds, embryos, ovules, meristematic regions, callus tissue, leaves, fronds, roots, nodules, shoots, anthers, and pollen.

As used herein, "duckweed nodule" means duckweed tissue comprising duckweed cells where at least about 50%, 55%, 60%, 65%, 70%, 75%, 80%, 85%, 90%, 95% or 100% of the cells are differentiated cells. As used herein, "differentiated cell," means a cell with at least one phenotypic characteristic (*e.g.*, a distinctive cell morphology or the expression of a marker nucleic acid or protein) that distinguishes it from undifferentiated cells or from cells found in other tissue types. The differentiated cells of the duckweed nodule culture described herein form a tiled smooth surface of interconnected cells fused at their adjacent cell walls, with nodules that have begun to organize into frond primordium scattered throughout the tissue. The surface of the tissue of the nodule culture has epidermal cells connected to each other via plasmadesmata.

The growth habit of the duckweeds is ideal for culturing methods. The plant rapidly proliferates through vegetative budding of new fronds, in a macroscopic manner analogous to asexual propagation in yeast. This proliferation occurs by vegetative budding from meristematic cells. The meristematic region is small and is found on the ventral surface of the frond. Meristematic cells lie in two pockets, one on each side of the frond midvein. The small midvein region is also the site from which the root originates and the stem arises that connects each frond to its mother frond. The meristematic pocket is protected by a tissue flap. Fronds bud alternately from these pockets. Doubling times vary by species and are as short as 20-24 hours (Landolt (1957) Ber. Schweiz. Bot. Ges. 67:271; Chang et al. (1977) Bull. Inst. Chem. Acad. Sin. 24:19; Datko and Mudd (1970) Plant Physiol. 65:16; Venkataraman et al. (1970) Z. Pflanzenphysiol. 62: 316). Intensive culture of duckweed results in the highest rates of biomass accumulation per unit time (Landolt and Kandeler (1987) The Family of Lemnaceae-A Monographic Study Vol. 2: Phytochemistry, Physiology, Application, Bibliography (Veroffentlichungen des Geobotanischen Institutes ETH, Stiftung Rubel, Zurich)), with dry weight accumulation ranging from 6-15% of fresh weight (Tillberg et al. (1979) Physiol. Plant. 46:5; Landolt (1957) Ber. Schweiz. Bot. Ges. 67:271; Stomp, unpublished data). Protein content of a number of duckweed species grown under varying conditions has been reported to range from 15-45% dry weight (Chang et al. (1977) Bull. Inst. Chem. Acad. Sin. 24:19; Chang and Chui (1978) Z. Pflanzenphysiol. 89:91; Porath et al. (1979) Aquatic Botany 7:272; Appenroth et al. (1982) Biochem. Physiol. Pflanz. 177:251). Using these values, the level of protein production per liter of medium in duckweed is on the same order of magnitude as yeast gene expression systems.

Described herein are transformed microalgae plants expressing an FMDV polypeptide of interest, or fragment or variant thereof. The term "microalgae" or "microalga" refers to members of the family *Thraustochytriaceae.* This family currently is divided into four genera: *Schizochytrium, Thraustochytrium, Labyrinthuloides, and Japonochytrium.*

The transformed duckweed plants or microalgae described herein can be obtained by introducing an expression construct comprising a polynucleotide encoding an antigenic FMDV polypeptide, or fragment or variant thereof, into the duckweed plant or microalga of interest.

The term "introducing" in the context of a polynucleotide, for example, an expression construct comprising a polynucleotide encoding an antigenic FMDV polypeptide, or fragment or variant thereof, is intended to mean presenting to the duckweed plant or microalga the polynucleotide in such a manner that the polynucleotide gains access to the interior of a cell of the duckweed plant or microalga. Where more than one polynucleotide is to be introduced, these polynucleotides can be assembled as part of a single nucleotide construct, or as separate nucleotide constructs, and can be located on the same or different transformation vectors. Accordingly, these polynucleotides can be introduced into the duckweed or microalga host cell of interest in a single transformation event, in separate transformation events, or, for example, as part of a breeding protocol. The compositions and methods described herein do not depend on a particular method for introducing one or more polynucleotides into a duckweed plant or microalga, only that the polynucleotide(s) gains access to the interior of at least one cell of the duckweed plant or microalga. Methods for introducing polynucleotides into plants or algae are known in the art including, but not limited to, transient transformation methods, stable transformation methods, and virus-mediated methods.

"Transient transformation" in the context of a polynucleotide such as a polynucleotide encoding an antigenic FMDV polypeptide, or fragment or variant thereof, is intended to mean that a polynucleotide is introduced into the duckweed plant or microalga and does not integrate into the genome of the duckweed plant or microalga.

By "stably introducing" or "stably introduced" in the context of a polynucleotide (such as a polynucleotide encoding an antigenic FMDV polypeptide, or fragment or variant thereof) introduced into a duckweed plant or microalga is intended the introduced polynucleotide is stably incorporated into the duckweed or microalga genome, and thus the duckweed plant or microalga is stably transformed with the polynucleotide.

"Stable transformation" or "stably transformed" is intended to mean that a polynucleotide, for example, a polynucleotide encoding an antigenic FMDV polypeptide, or fragment or variant thereof, introduced into a duckweed plant or microalga integrates into the genome of the plant or alga and is capable of being inherited by the progeny thereof, more particularly, by the progeny of multiple successive generations. In some embodiments, successive generations include progeny produced vegetatively (i.e., asexual reproduction), for example, with clonal propagation. In other embodiments, successive generations include progeny produced via sexual reproduction.

An expression construct comprising a polynucleotide encoding an antigenic FMDV polypeptide, or fragment or variant thereof, can be introduced into a duckweed plant or microalga of interest using any transformation protocol known to those of skill in art. Suitable methods of introducing nucleotide sequences into duckweed plants or plant cells or nodules or microalgae include microinjection (Crossway et al. (1986) Biotechniques 4:320-334), electroporation (Riggs et al. (1986) Proc. Natl. Acad. Sci. USA 83:5602-5606), *Agrobacterium*-mediated transformation (U.S. Patent Nos. 5,563,055 and 5,981,840), direct gene transfer (Paszkowski et al. (1984) EMBO J. 3:2717-2722), ballistic particle acceleration (see, *e.g.,* U.S. Patent Nos. 4,945,050; 5,879,918; 5,886,244; and 5,932,782; and Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); McCabe et al. (1988) Biotechnology 6:923-926). The cells that have been transformed may be grown into plants in accordance with conventional ways.

As noted above, stably transformed duckweed or microalgae can be obtained by any gene transfer method known in the art, such as one of the gene transfer methods disclosed in U.S. Patent No. 6,040,498 or U.S. Patent Application Publication Nos. 2003/0115640, 2003/0033630 or 2002/0088027. Duckweed plant or nodule cultures or microalga can be efficiently transformed with an expression cassette containing a nucleic acid sequence as described herein by any one of a number of methods including *Agrobacterium*-mediated gene transfer, ballistic bombardment or electroporation. The *Agrobacterium* used can be *Agrobacterium tumefaciens* or *Agrobacterium rhizogenes.* Stable duckweed or microalga transformants can be isolated by transforming the duckweed or microalga cells with both the nucleic acid sequence of interest and a gene that confers resistance to a selection agent, followed by culturing the transformed cells in a medium containing the selection agent. *See,* for example, U.S. Patent No. 6,040,498.

The stably transformed duckweed plants or microalgae utilized in these methods should exhibit normal morphology and be fertile by sexual reproduction and/or able to reproduce vegetatively (i.e., asexual reproduction), for example, with clonal propagation. Preferably, transformed duckweed plants or microalgae described herein contain a single copy of the transferred nucleic acid comprising a polynucleotide encoding an antigenic FMDV polypeptide, or fragment or variant thereof, and the transferred nucleic acid has no notable rearrangements therein. It is recognized that the transformed duckweed plants or microalgae described herein may contain the transferred nucleic acid present in low copy numbers (i.e., no more than twelve copies, no more than eight copies, no more than five copies, alternatively, no more than three copies, as a further alternative, fewer than three copies of the nucleic acid per transformed cell).

Transformed plants or microalgae expressing an antigenic FMDV polypeptide, or fragment or variant thereof, can be cultured under suitable conditions for expressing the antigenic FMDV polypeptide, or fragment or variant thereof. The antigenic FMDV polypeptide, or fragment or variant thereof, can then be harvested from the duckweed plant or microalgae, the culture medium, or the duckweed plant or microalgae and the culture medium, and, where desired, purified using any conventional isolation and purification method known in the art, as described elsewhere herein. The antigenic FMDV polypeptide, or fragment or variant thereof, can then be formulated as a vaccine for therapeutic applications, as described elsewhere herein.

### Methods of Preparing an FMDV Polypeptide

As described fully herein, in an embodiment, a method of producing an FMDV polypeptide comprises: (a) culturing within a duckweed or microalgae culture medium a duckweed or microalga culture, wherein the duckweed or microalga culture is stably transformed to express the polypeptide, and wherein the polypeptide is expressed from a nucleotide sequence comprising a coding sequence for said polypeptide; and (b) collecting the antigenic polypeptide from said culture medium. The term collecting includes, but is not limited to, harvesting from the culture medium or purifying.

After production of the recombinant polypeptide in duckweed or microalgae, any method available in the art may be used for protein purification. The various steps include freeing the protein from the nonprotein or plant or microalga material, followed by the purification of the protein of interest from other proteins. Initial steps in the purification process include centrifugation, filtration or a combination thereof. Proteins secreted within the extracellular space of tissues can be obtained using vacuum or centrifugal extraction. Minimal processing could also involve preparation of crude products. Other methods include maceration and extraction in order to permit the direct use of the extract.

Such methods to purify the protein of interest can exploit differences in protein size, physicochemical properties, and binding affinity. Such methods include chromatography, including procainamide affinity, size exclusion, high pressure liquid, reversed-phase, and anion-exchange chromatography, affinity tags, filtration, etc. In particular, immobilized Ni-ion affinity chromatography can be used to purify the expressed protein. See, Favacho et al. (2006) Protein expression and purification 46:196-203. See also, Zhou et al. (2007) The Protein J 26:29-37; Wang et al. (2006) Vaccine 15:2176-2185; and WO/2009/076778. Protectants may be used in the purification process such as osmotica, antioxidants, phenolic oxidation inhibitors, protease inhibitors, and the like.

### Methods of Use

In an embodiment, the subject matter disclosed herein is directed to a method of vaccinating an ovine, bovine, caprine, or porcine comprising administering to the ovine, bovine, caprine, or porcine an effective amount of a vaccine which may comprise an effective amount of a recombinant FMDV antigen and a pharmaceutically or veterinarily acceptable carrier, excipient, or vehicle.

In one embodiment of the present invention, the method comprises a single administration of a vaccine composition formulated with an emulsion according to the invention. For example, in one embodiment, the immunological or vaccine composition comprises duckweed-expressed FMDV antigens, including polypeptides and VLPs (virus-like particles). Electron microscopy indicates the duckweed transformed with MerE expression vectors likely produce FMDV VLP, and so immunological or vaccine compositions according to the instant invention encompass those comprising FMDV VLP.

In an embodiment, the subject matter disclosed herein is directed to a method of vaccinating an ovine, bovine, caprine, or porcine comprising administering to the ovine, bovine, caprine, or porcine an ovine, bovine, caprine, or porcine FMDV antigen produced in a plant or alga, and plant material from the genus *Lemna* or microalga material from *schizochytrium.*

In an embodiment, the subject matter disclosed herein is directed to a method of eliciting an immune response comprising administering to the ovine, bovine, caprine, or porcine a vaccine comprising an ovine, bovine, caprine, or porcine FMDV antigen expressed in a plant or alga, wherein an immune response is elicited.

In an embodiment, the subject matter disclosed herein is directed to a method of eliciting an immune response comprising administering to the ovine, bovine, caprine, or porcine a vaccine comprising an ovine, bovine, caprine, or porcine FMDV antigen produced in a plant or alga, and plant material from the genus *Lemna* or microalga material from *schizochytrium.,* wherein an immune response is elicited.

In an embodiment, the subject matter disclosed herein is directed to a method of preparing a stably transformed duckweed plant or microalga culture comprising, (a) introducing into the plant or microalga a genetic construct comprising an FMDV antigen gene; and (b) cultivating the plant or microalga. Methods for transformation of duckweed or microalga are available in the art.

In an embodiment, the subject matter disclosed herein is directed to a method of preparing a vaccine or composition comprising isolating an FMDV antigen produced by a duckweed or microalgal expression system and optionally combining with a pharmaceutically or veterinarily acceptable carrier, excipient or vehicle.

In an embodiment, the subject matter disclosed herein is directed to a method of preparing a vaccine or composition comprising combining an FMDV antigen produced by a *Lemna* expression system and plant material from the genus *Lemna* and optionally a pharmaceutically or veterinarily acceptable carrier, excipient, or vehicle.

In another embodiment, the subject matter disclosed herein is directed to a method of preparing a vaccine or composition comprising combining an FMDV antigen produced by a *Schizochytrium* expression system and *Schizochytrium* material and optionally a pharmaceutically or veterinarily acceptable carrier, excipient, or vehicle.

The administering may be subcutaneously or intramuscularly. The administering may be needle free (for example Pigjet or Bioject).

In one embodiment of the invention, a prime-boost regimen can be employed, which is comprised of at least one primary administration and at least one booster administration using at least one common polypeptide, antigen, epitope or immunogen. Typically the immunological composition or vaccine used in primary administration is different in nature from those used as a booster. However, it is noted that the same composition can be used as the primary administration and the boost. This administration protocol is called "prime-boost".

A prime-boost according to the present invention can include a recombinant viral vector is used to express an FMDV coding sequence or fragments thereof encoding an antigenic polypeptide or fragment or variant thereof. Specifically, the viral vector can express an FMDV gene or fragment thereof that encodes an antigenic polypeptide. Viral vector contemplated herein includes, but not limited to, poxvirus [e.g., vaccinia virus or attenuated vaccinia virus, avipox virus or attenuated avipox virus (e.g., canarypox, fowlpox, dovepox, pigeonpox, quailpox, ALVAC, TROVAC; see e.g., US 5,505,941, US 5,494,8070), raccoonpox virus, swinepox virus, etc.], adenovirus (e.g., human adenovirus, canine adenovirus), herpesvirus (e.g. canine herpesvirus, herpesvirus of turkey, Marek's disease virus, infectious laryngotracheitis virus, feline herpesvirus, laryngotracheitis virus (ILTV), bovine herpesvirus, swine herpesvirus), baculovirus, retrovirus, etc. In another embodiment, the avipox expression vector may be a canarypox vector, such as, ALVAC. In yet another embodiment, the avipox expression vector may be a fowlpox vector, such as, TROVAC. The FMDV antigen of the invention to be expressed is inserted under the control of a specific poxvirus promoter, e.g., the entomopoxvirus *Amsacta moorei* 42K promoter (Barcena, Lorenzo et al. 2000), the vaccinia promoter 7.5 kDa (Cochran et al., 1985), the vaccinia promoter I3L (Riviere et al., 1992), the vaccinia promoter HA (Shida, 1986), the cowpox promoter ATI (Funahashi et al., 1988), the vaccinia promoter H6 (Taylor et al., 1988b; Guo et al., 1989; Perkus et al., 1989), *inter alia.*

In another embodiment, the avipox expression vector may be a canarypox vector, such as, ALVAC. The FMDV antigen, epitope or immunogen may be FMDV P1-3C. The FMDV viral vector may be a canarypox virus such as vCP2186, vCP2181, or vCP2176, or a fowlpox virus such as vFP2215 (see US 7,527,960).

In another aspect of the prime-boost protocol of the invention, a composition comprising the FMDV antigen of the invention is administered followed by the administration of vaccine or composition comprising a recombinant viral vector that contains and expresses an FMDV antigen *in vivo,* or an inactivated viral vaccine or composition comprising an FMDV antigen, or a DNA plasmid vaccine or composition that contains or expresses an FMDV antigen. Likewise, a prime-boost protocol may comprise the administration of vaccine or composition comprising a recombinant viral vector that contains and expresses an FMDV antigen *in vivo,* or an inactivated viral vaccine or composition comprising an FMDV antigen, or a DNA plasmid vaccine or composition that contains or expresses an FMDV antigen, followed by the administration of a composition comprising the FMDV antigen of the invention. It is further noted that both the primary and the secondary administrations may comprise the composition comprising the FMDV antigen of the invention.

A prime-boost protocol comprises at least one prime-administration and at least one boost administration using at least one common polypeptide and/or variants or fragments thereof. The vaccine used in prime-administration may be different in nature from those used as a later booster vaccine. The prime-administration may comprise one or more administrations. Similarly, the boost administration may comprise one or more administrations.

The dose volume of compositions for target species that are mammals, e.g., the dose volume of ovine, bovine, caprine or porcine compositions, based on viral vectors, e.g., non-poxvirus-viral-vector-based compositions, is generally between about 0.1 to about 5.0 ml, between about 0.1 to about 3.0 ml, and between about 0.5 ml to about 2.5 ml.

The efficacy of the vaccines may be tested about 2 to 4 weeks after the last immunization by challenging animals, such as ovine, bovine, caprine or porcines, with a virulent strain of FMDV, advantageously the FMDV *O1 Manisa, O1 BFS or Campos, A24 Cruzeiro, Asia 1 Shamir, A Iran'96, A22 Iraq, SAT2 Saudi Arabia* strains.

Still other strains may include FMDV strains A10-61, A5, A12, A24/Cruzeiro, C3/Indaial, O1, C1-Santa Pau, C1-C5, A22/550/Azerbaijan/65, SAT1-SAT3, A, A/TNC/71/94, A/IND/2/68, A/IND/3/77, A/IND/5/68, A/IND/7/82, A/IND/16/82, A/IND/17/77, A/IND/17/82, A/IND/19/76, A/IND/20/82, A/IND/22/82, A/IND/25/81, A/IND/26/82, A/IND/54/79, A/IND/57/79, A/IND/73/79, A/IND/85/79, A/IND/86/79, A/APA/25/84, A/APN/41/84, A/APS/44/05, A/APS/50/05, A/APS/55/05, A/APS/66/05, A/APS/68/05, A/BIM/46/95, A/GUM/33/84, A/ORS/66/84, A/ORS/75/88, A/TNAn/60/947/Asia/1, A/IRN/05, Asia/IRN/05, O/HK/2001, O/UKG/3952/2001, O/UKG/4141/2001, Asia 1/HNK/CHA/05 (GenBank accession number EF149010, herein incorporated by reference), Asia I/XJ (Li, ZhiYong et al. Chin Sci Bull, 2007), HK/70 (Chin Sci Bull, 2006, 51(17): 2072-2078), O/UKG/7039/2001, O/UKG/9161/2001, O/UKG/7299/2001, O/UKG/4014/2001, O/UKG/4998/2001, O/UKG/9443/2001, O/UKG/5470/2001, O/UKG/5681/2001, O/ES/2001, HKN/2002, O5India, O/BKF/2/92, K/37/84/A, KEN/1/76/A, GAM/51/98/A, A10/Holland, O/KEN/1/91, O/IND49/97, O/IND65/98, O/IND64/98, O/IND48/98, O/IND47/98, O/IND82/97, O/IND81/99, O/IND81/98, O/IND79/97, O/IND78/97, O/IND75/97, O/IND74/97, O/IND70/97, O/IND66/98, O/IND63/97, O/IND61/97, O/IND57/98, O/IND56/98, O/IND55/98, O/IND54/98, O/IND469/98, O/IND465/97, O/IND464/97, O/IND424/97, O/IND423/97, O/IND420/97, O/IND414/97, O/IND411/97, O/IND410/97, O/IND409/97, O/IND407/97, O/IND399/97, O/IND39/97, O/IND391/97, O/IND38/97, O/IND384/97, O/IND380/97, O/IND37/97, O/IND352/97, O/IND33/97, O/IND31/97, O/IND296/97, O/IND23/99, O/IND463/97, O/IND461/97, O/IND427/98, O/IND28/97, O/IND287/99, O/IND285/99, O/IND282/99, O/IND281/97, O/IND27/97, O/IND278/97, O/IND256/99, O/IND249/99, O/IND210/99, O/IND208/99, O/IND207/99, O/IND205/99, O/IND185/99, O/IND175/99, O/IND170/97, O/IND164/99, O/IND160/99, O/IND153/99, O/IND148/99, O/IND146/99, O/SKR/2000, A22/India/17/77.

Further details of these FMDV strains may be found on the European Bioinformatics Information (EMBL-EBI) web page. The inventors contemplate that all FMDV strains, both herein listed, and those yet to be identified, could be expressed according to the teachings of the present disclosure to produce, for example, effective vaccine compositions. Both homologous and heterologous strains are used for challenge to test the efficacy of the vaccines. The animal may be challenged intradermally, subcutaneously, spray, intra-nasally, intra-ocularly, intra-tracheally, and/or orally.

The prime-boost administrations may be advantageously carried out 2 to 6 weeks apart, for example, about 3 weeks apart. According to one embodiment, a semi-annual booster or an annual booster, advantageously using the viral vector-based vaccine, is also envisaged. The animals are advantageously at least 6 to 8 weeks old at the time of the first administration.

The compositions comprising the recombinant antigenic polypeptides of the invention used in the prime-boost protocols are contained in a pharmaceutically or veterinary acceptable vehicle, diluent or excipient. The protocols of the invention protect the animal from ovine, bovine, caprine or porcine FMDV and/or prevent disease progression in an infected animal.

The various administrations are preferably carried out 1 to 6 weeks apart, and more particularly about 3 weeks apart. According to a preferred mode, an annual booster, preferably using the viral vector-based immunological composition of vaccine, is also envisaged. The animals are preferably at least one-day-old at the time of the first administration.

It should be understood by one of skill in the art that the disclosure herein is provided by way of example and the present invention is not limited thereto. From the disclosure herein and the knowledge in the art, the skilled artisan can determine the number of administrations, the administration route, and the doses to be used for each injection protocol, without any undue experimentation.

The present invention contemplates at least one administration to an animal of an efficient amount of the therapeutic composition made according to the invention. The animal may be male, female, pregnant female and newborn. This administration may be via various routes including, but not limited to, intramuscular (IM), intradermal (ID) or subcutaneous (SC) injection or via intranasal or oral administration. The therapeutic composition according to the invention can also be administered by a needleless apparatus (as, for example with a Pigjet, Dermojet, Biojector, Avijet (Merial, GA, USA), Vetjet or Vitajet apparatus (Bioject, Oregon, USA)). Another approach to administering plasmid compositions is to use electroporation (see, e.g. Tollefsen et al., 2002; Tollefsen et al., 2003; Babiuk et al., 2002; PCT Application No. WO99/01158). In another embodiment, the therapeutic composition is delivered to the animal by gene gun or gold particle bombardment.

In one embodiment, the invention provides for the administration of a therapeutically effective amount of a formulation for the delivery and expression of an FMDV antigen or epitope in a target cell. Determination of the therapeutically effective amount is routine experimentation for one of ordinary skill in the art. In one embodiment, the formulation comprises an expression vector comprising a polynucleotide that expresses an FMDV antigen or epitope and a pharmaceutically or veterinarily acceptable carrier, vehicle or excipient. In another embodiment, the pharmaceutically or veterinarily acceptable carrier, vehicle or excipient facilitates transfection or other means of transfer of polynucleotides to a host animal and/or improves preservation of the vector or protein in a host.

In one embodiment, the subject matter disclosed herein provides a detection method for differentiation between infected and vaccinated animals (DIVA).

It is disclosed herein that the use of the vaccine or composition of the present invention allows the detection of FMDV infection in an animal. It is disclosed herein that the use of the vaccine or composition of the present invention allows the detection of the infection in animals by differentiating between infected and vaccinated animals (DIVA). A method is disclosed herein for diagnosing the infection of FMDV in an animal using an FMDV non-structural protein (e.g. a FMDV 3ABC or 3D-specific ELISA).

### Article of Manufacture

In an embodiment, the subject matter disclosed herein is directed to a kit for performing a method of eliciting or inducing an immune response which may comprise any one of the recombinant FMDV immunological compositions or vaccines, or inactivated FMDV immunological compositions or vaccines, recombinant FMDV viral compositions or vaccines, and instructions for performing the method.

Described herein is a kit for performing a method of inducing an immunological or protective response against FMDV in an animal comprising a composition or vaccine comprising an FMDV antigen of the invention and a recombinant FMDV viral immunological composition or vaccine, and instructions for performing the method of delivery in an effective amount for eliciting an immune response in the animal.

Described herein is a kit for performing a method of inducing an immunological or protective response against FMDV in an animal comprising a composition or vaccine comprising an FMDV antigen of the invention and an inactivated FMDV immunological composition or vaccine, and instructions for performing the method of delivery in an effective amount for eliciting an immune response in the animal.

Described herein is a kit for prime-boost vaccination according to the present invention as described above. The kit may comprise at least two vials: a first vial containing a vaccine or composition for the prime-vaccination according to the present invention, and a second vial containing a vaccine or composition for the boost-vaccination according to the present invention. The kit may advantageously contain additional first or second vials for additional primo-vaccinations or additional boost-vaccinations.

The following embodiments are described herein. In an embodiment, a composition comprising an FMDV antigen or fragment or variant thereof and a pharmaceutical or veterinarily acceptable carrier, excipient, or vehicle is disclosed. In another embodiment, the composition described above wherein the FMDV antigen or fragment or variant thereof comprises an immunogenic fragment comprising at least 15 amino acids of an ovine, bovine, caprine, or porcine FMDV antigen is disclosed. In yet another embodiment, the above compositions wherein the FMDV antigen or fragment or variant thereof is produced in duckweed or microalgae are disclosed. In an embodiment, the above compositions wherein the FMDV antigen or fragment or variant thereof is partially purified are disclosed. In an embodiment, the above compositions wherein the FMDV antigen or fragment or variant thereof is substantially purified are disclosed.

In an embodiment, the above compositions wherein the FMDV antigen or fragment or variant thereof is an ovine, bovine, caprine, or porcine FMDV polypeptide are disclosed. In an embodiment, the above compositions wherein the FMDV polypeptide is a P1-3C polypeptide, PI polypeptide, VP0 polypeptide, VP1 polypeptide, VP3 polypeptide, VP2 polypeptide, VP4 polypeptide, 2A polypeptide, 2B1 polypeptide, or 3C polypeptide are disclosed. In an embodiment, the above compositions wherein the FMDV antigen or fragment or variant thereof has at least 80% sequence identity to the sequence as set forth in SEQ ID NOs:3, 10, 12, 17, 20, 23, 26, 29 are disclosed. In one embodiment, the above compositions wherein the FMDV antigen is encoded by a polynucleotide having at least 70% sequence identity to the sequence as set forth in SEQ ID NOs:1, 2, 9, 11, 13, 14, 15, 16, 18, 19, 21, 22, or 24, 25, 27, 28, 30-35 are disclosed. In an embodiment, the above compositions wherein the pharmaceutical or veterinarily acceptable carrier, excipient, or vehicle is a water-in-oil emulsion or an oil-in-water emulsion are disclosed. In another embodiment, a method of vaccinating an animal susceptible to ovine, bovine, caprine, or porcine FMDV comprising administering the compositions above to the animal is disclosed. In an embodiment, a method of vaccinating an animal susceptible to ovine, bovine, caprine, or porcine FMDV comprising a prime-boost regime is disclosed. In an embodiment, a substantially purified antigenic polypeptide expressed in duckweed or microalga, wherein the polypeptide comprises: an amino acid sequence having at least 80% sequence identity to a polypeptide having the sequence as set forth in SEQ ID NOs:3, 10, 12, 17, 20, 23, 26, 29 is disclosed. In any embodiment the animal is preferably an ovine, a bovine, a porcine, or a caprine. In one embodiment, a method of diagnosing FMDV infection in an animal is disclosed. In yet another embodiment, a kit for prime-boost vaccination comprising at least two vials, wherein a first vial containing the composition of the present invention, and a second vial containing a composition for the boost-vaccination comprising a composition comprising a recombinant viral vector, or a composition comprising an inactivated viral composition, or a DNA plasmid composition that contains or expresses the FMDV antigen is disclosed.

The pharmaceutically or veterinarily acceptable carriers or vehicles or excipients are well known to the one skilled in the art. For example, a pharmaceutically or veterinarily acceptable carrier or vehicle or excipient can be a 0.9% NaCl (e.g., saline) solution or a phosphate buffer. Other pharmaceutically or veterinarily acceptable carrier or vehicle or excipients that can be used for methods of this invention include, but are not limited to, poly-(L-glutamate) or polyvinylpyrrolidone. The pharmaceutically or veterinarily acceptable carrier or vehicle or excipients may be any compound or combination of compounds facilitating the administration of the vector (or protein expressed from an inventive vector *in vitro*); advantageously, the carrier, vehicle or excipient may facilitate transfection and/or improve preservation of the vector (or protein). Doses and dose volumes are herein discussed in the general description and can also be determined by the skilled artisan from this disclosure read in conjunction with the knowledge in the art, without any undue experimentation.

The cationic lipids containing a quaternary ammonium salt which are advantageously but not exclusively suitable for plasmids, are advantageously those having the following formula: 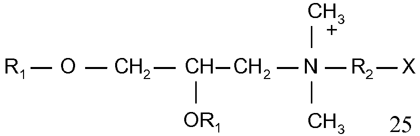 in which R1 is a saturated or unsaturated straight-chain aliphatic radical having 12 to 18 carbon atoms, R2 is another aliphatic radical containing 2 or 3 carbon atoms and X is an amine or hydroxyl group, e.g. the DMRIE. In another embodiment the cationic lipid can be associated with a neutral lipid, e.g. the DOPE.

Among these cationic lipids, preference is given to DMRIE (N-(2-hydroxyethyl)-N,N-dimethyl-2,3-bis(tetradecyloxy)-1-propane ammonium; WO96/34109), advantageously associated with a neutral lipid, advantageously DOPE (dioleoyl-phosphatidyl-ethanol amine; Behr, 1994), to form DMRIE-DOPE.

Advantageously, the plasmid mixture with the adjuvant is formed extemporaneously and advantageously contemporaneously with administration of the preparation or shortly before administration of the preparation; for instance, shortly before or prior to administration, the plasmid-adjuvant mixture is formed, advantageously so as to give enough time prior to administration for the mixture to form a complex, e.g. between about 10 and about 60 minutes prior to administration, such as approximately 30 minutes prior to administration.

When DOPE is present, the DMRIE:DOPE molar ratio is advantageously about 95: about 5 to about 5: about 95, more advantageously about 1: about 1, e.g., 1:1.

The DMRIE or DMRIE-DOPE adjuvant:plasmid weight ratio can be between about 50: about 1 and about 1: about 10, such as about 10: about 1 and about 1:about 5, and about 1: about 1 and about 1: about 2, e.g., 1:1 and 1:2.

In another embodiment, pharmaceutically or veterinarily acceptable carrier, excipient, or vehicle may be a water-in-oil emulsion. Examples of suitable water-in-oil emulsions include oil-based water-in-oil vaccinal emulsions which are stable and fluid at 4°C containing: from 6 to 50 v/v% of an antigen-containing aqueous phase, preferably from 12 to 25 v/v%, from 50 to 94 v/v% of an oil phase containing in total or in part a non-metabolizable oil (e.g., mineral oil such as paraffin oil) and/or metabolizable oil (e.g., vegetable oil, or fatty acid, polyol or alcohol esters), from 0.2 to 20 p/v% of surfactants, preferably from 3 to 8 p/v%, the latter being in total or in part, or in a mixture either polyglycerol esters, said polyglycerol esters being preferably polyglycerol (poly)ricinoleates, or polyoxyethylene ricin oils or else hydrogenated polyoxyethylene ricin oils. Examples of surfactants that may be used in a water-in-oil emulsion include ethoxylated sorbitan esters (e.g., polyoxyethylene (20) sorbitan monooleate (TWEEN 80®), available from AppliChem, Inc., Cheshire, CT) and sorbitan esters (e.g., sorbitan monooleate (SPAN 80®), available from Sigma Aldrich, St. Louis, MO). In addition, with respect to a water-in-oil emulsion, see also US Patent No. 6,919,084, e.g., Example 8 thereof, incorporated herein by reference. In some embodiments, the antigen-containing aqueous phase comprises a saline solution comprising one or more buffering agents. An example of a suitable buffering solution is phosphate buffered saline. In an advantageous embodiment, the water-in-oil emulsion may be a water/oil/water (W/O/W) triple emulsion (U.S. Patent No. 6,358,500). Examples of other suitable emulsions are described in U.S. Patent No. 7,371,395.

The immunological compositions and vaccines described herein may comprise or consist essentially of one or more adjuvants. Suitable adjuvants for use in the practice of the present invention are (1) polymers of acrylic or methacrylic acid, maleic anhydride and alkenyl derivative polymers, (2) immunostimulating sequences (ISS), such as oligodeoxyribonucleotide sequences having one or more non-methylated CpG units (Klinman et al., 1996; WO98/16247), (3) an oil in water emulsion, such as the SPT emulsion described on page 147 of "Vaccine Design, The Subunit and Adjuvant Approach" published by M. Powell, M. Newman, Plenum Press 1995, and the emulsion MF59 described on page 183 of the same work, (4) cation lipids containing a quaternary ammonium salt, e.g., DDA (5) cytokines, (6) aluminum hydroxide or aluminum phosphate, (7) saponin or (8) any combinations or mixtures thereof.

The oil in water emulsion (3), which is especially appropriate for viral vectors, can be based on: light liquid paraffin oil (European pharmacopoeia type), isoprenoid oil such as squalane, squalene, oil resulting from the oligomerization of alkenes, e.g. isobutene or decene, esters of acids or alcohols having a straight-chain alkyl group, such as vegetable oils, ethyl oleate, propylene glycol, di(caprylate/caprate), glycerol tri(caprylate/caprate) and propylene glycol dioleate, or esters of branched, fatty alcohols or acids, especially isostearic acid esters.

The oil is used in combination with emulsifiers to form an emulsion. The emulsifiers may be nonionic surfactants, such as: esters of on the one hand sorbitan, mannide (e.g. anhydromannitol oleate), glycerol, polyglycerol or propylene glycol and on the other hand oleic, isostearic, ricinoleic or hydroxystearic acids, said esters being optionally ethoxylated, or polyoxypropylene-polyoxyethylene copolymer blocks, such as Pluronic, e.g., L121.

Among the type (1) adjuvant polymers, preference is given to polymers of crosslinked acrylic or methacrylic acid, especially crosslinked by polyalkenyl ethers of sugars or polyalcohols. These compounds are known under the name carbomer (Pharmeuropa, vol. 8, no. 2, June 1996). One skilled in the art can also refer to U.S. Patent No. 2,909,462, which provides such acrylic polymers crosslinked by a polyhydroxyl compound having at least three hydroxyl groups, preferably no more than eight such groups, the hydrogen atoms of at least three hydroxyl groups being replaced by unsaturated, aliphatic radicals having at least two carbon atoms. The preferred radicals are those containing 2 to 4 carbon atoms, e.g. vinyls, allyls and other ethylenically unsaturated groups. The unsaturated radicals can also contain other substituents, such as methyl. Products sold under the name Carbopol (BF Goodrich, Ohio, USA) are especially suitable. They are crosslinked by allyl saccharose or by allyl pentaerythritol. Among them, reference is made to Carbopol 974P, 934P and 971P.

As to the maleic anhydride-alkenyl derivative copolymers, preference is given to EMA (Monsanto), which are straight-chain or crosslinked ethylene-maleic anhydride copolymers and they are, for example, crosslinked by divinyl ether. Reference is also made to J. Fields et al., 1960.

With regard to structure, the acrylic or methacrylic acid polymers and EMA are preferably formed by basic units having the following formula: 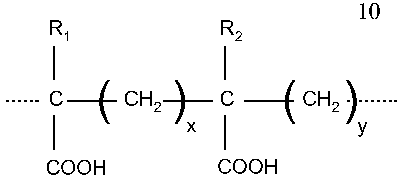 in which:
- R1 and R2, which can be the same or different, represent H or CH3
- x = 0 or 1, preferably x = 1
- y = 1 or 2, with x + y = 2.

For EMA, x = 0 and y = 2 and for carbomers x = y = 1.

These polymers are soluble in water or physiological salt solution (20 g/l NaCl) and the pH can be adjusted to 7.3 to 7.4, e.g., by soda (NaOH), to provide the adjuvant solution in which the expression vector(s) can be incorporated. The polymer concentration in the final immunological or vaccine composition can range between about 0.01 to about 1.5% w/v, about 0.05 to about 1% w/v, and about 0.1 to about 0.4% w/v.

The cytokine or cytokines (5) can be in protein form in the immunological or vaccine composition, or can be co-expressed in the host with the immunogen or immunogens or epitope(s) thereof. Preference is given to the co-expression of the cytokine or cytokines, either by the same vector as that expressing the immunogen or immunogens or epitope(s) thereof, or by a separate vector thereof.

The invention comprehends preparing such combination compositions; for instance by admixing the active components, advantageously together and with an adjuvant, carrier, cytokine, and/or diluent.

Cytokines that may be used in the present invention include, but are not limited to, granulocyte colony stimulating factor (G-CSF), granulocyte/macrophage colony stimulating factor (GM-CSF), interferon α (IFNα), interferon β (IFNβ), interferon γ, (IFNγ), interleukin-1α (IL-1α), interleukin-1β (IL-1β), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), tumor necrosis factor α (TNFα), tumor necrosis factor β (TNFβ), and transforming growth factor β (TGFβ). It is understood that cytokines can be co-administered and/or sequentially administered with the immunological or vaccine composition of the present invention. Thus, for instance, the vaccine of the instant invention can also contain an exogenous nucleic acid molecule that expresses *in vivo* a suitable cytokine, e.g., a cytokine matched to this host to be vaccinated or in which an immunological response is to be elicited (for instance, a bovine cytokine for preparations to be administered to bovines).

Advantageously, the immunological composition and/or vaccine according to the invention comprise or consist essentially of or consist of an effective quantity to elicit a therapeutic response of one or more Duckweed-expressed polypeptides as discussed herein; and, an effective quantity can be determined from this disclosure, including the documents incorporated herein, and the knowledge in the art, without undue experimentation.

In the case of immunological composition and/or vaccine based on a Duckweed-expressed polypeptides, a dose may include, about in 1 µg to about 2000 µg, advantageously about 50 µg to about 1000 µg and more advantageously from about 100 µg to about 500 µg of FMDV antigen, epitope or immunogen. The dose volumes can be between about 0.1 and about 10 ml, advantageously between about 0.2 and about 5 ml.

The invention will now be further described by way of the following non-limiting examples.

### EXAMPLES

Construction of DNA inserts, plasmids and recombinant viral or plant vectors was carried out using the standard molecular biology techniques described by J. Sambrook et al. (Molecular Cloning: A Laboratory Manual, 2nd Edition, Cold Spring Harbor Laboratory, Cold Spring Harbor, New York, 1989).

### Example 1 Generation and screening of FMDV-expressing duckweed lines

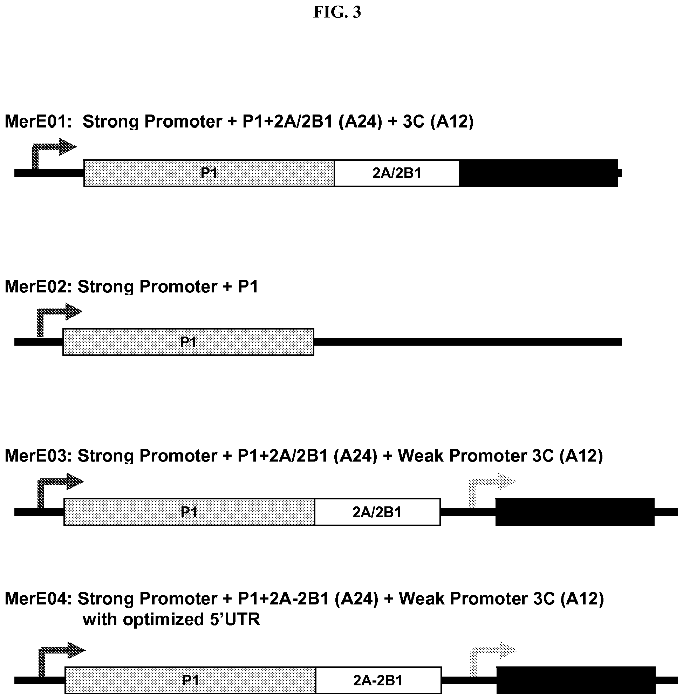

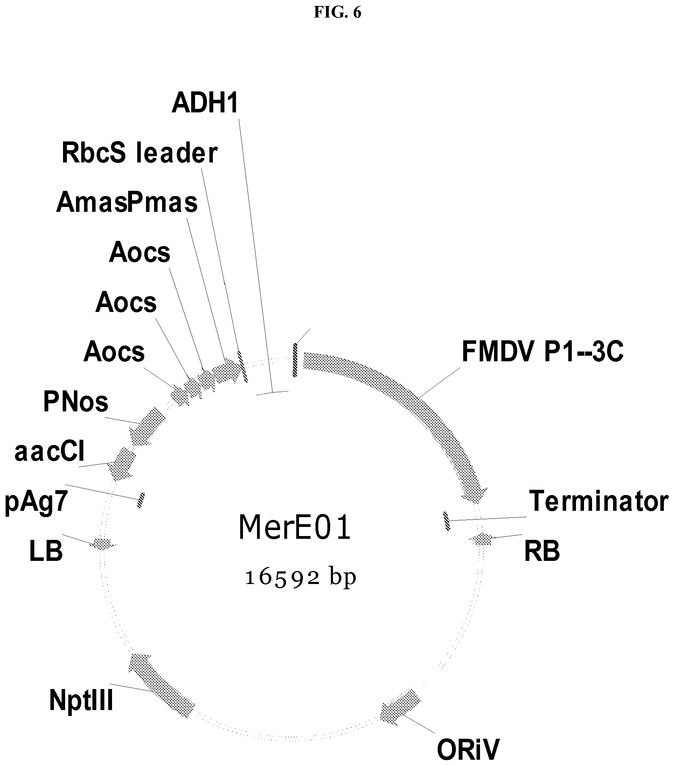

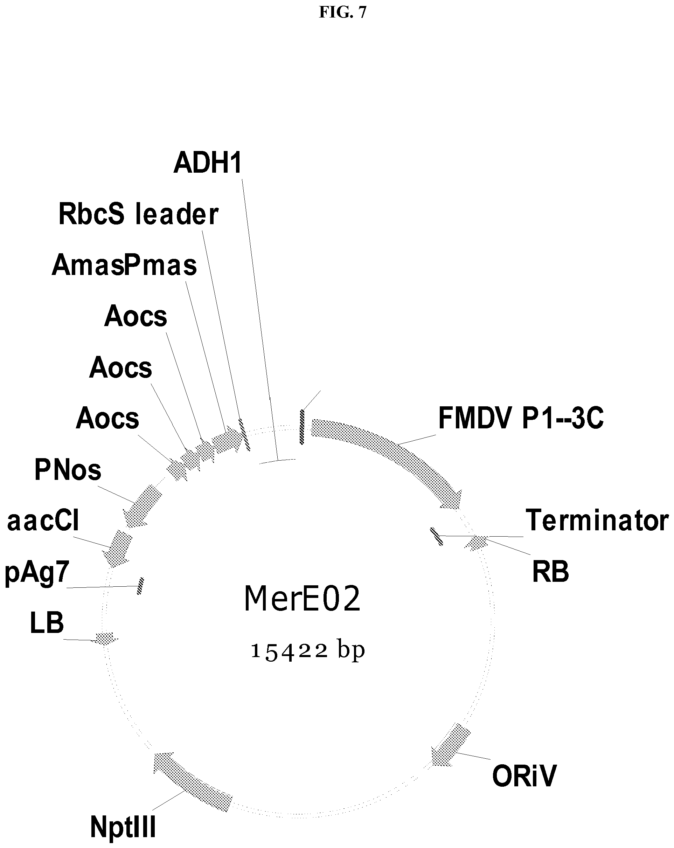

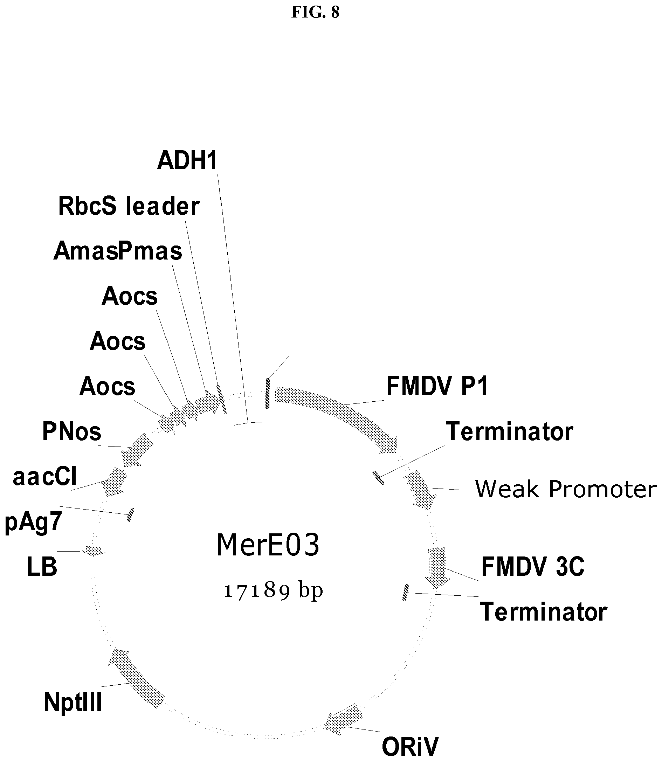

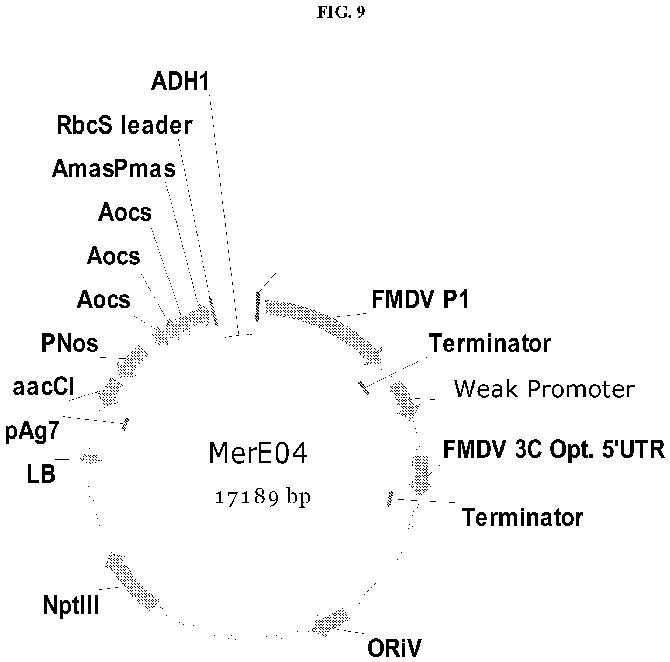

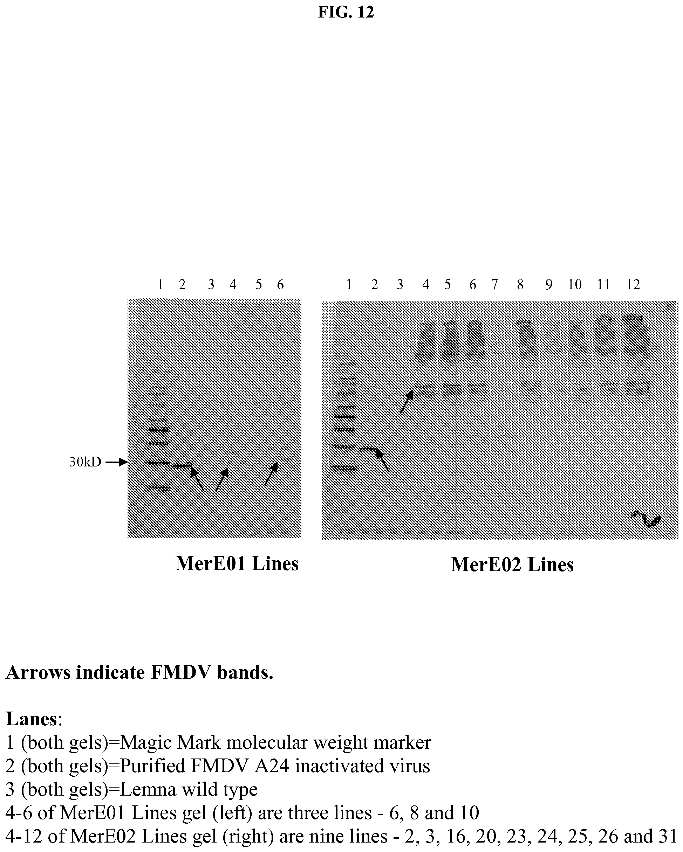

Duckweed optimized FMDV P1 and 3C sequences were produced and cloned into the parental plasmid to generate the MerE vectors depicted in FIGs. 6-9. Four independent constructs were designed for the FMDV project. Table 3 summarizes the number of transgenic lines that were generated and screened and FIG. 3 provides a schematic representation of gene structure for the FMDV inserts. Three lines express the PI capsid + 3C protease (MerE01, 3, 4), whereas the other express the PI capsid antigen alone (MerE02). ELISA and Agilent analyses were used to quantify the expression of the FMDV antigens. Western blots were performed to verify the correct size of expressed proteins (FIG. 12). The highest FMDV serotype A24-expressing duckweed lines, as determined by mRNA analysis and by western blot, were grown in scale vessels to provide biomass for use in characterization and animal studies.

**Table 3: Line generation and further screening of FMDV "MerE" lines**

| **Construct** | **Description** | **# of lines generated** | **# of lines screened** |
|---|---|---|---|
| MerE01 | P1-3C | 16 | 16 |
| MerE02 | P1 alone (Optimized 5' UTR) | 100 | 100 |
| MerE03 | P1-2A + 3C | 20 | 20 |
| MerE04 | P1-2A + 3C (Optimized 5 ' UTR) | 8 | 8 |

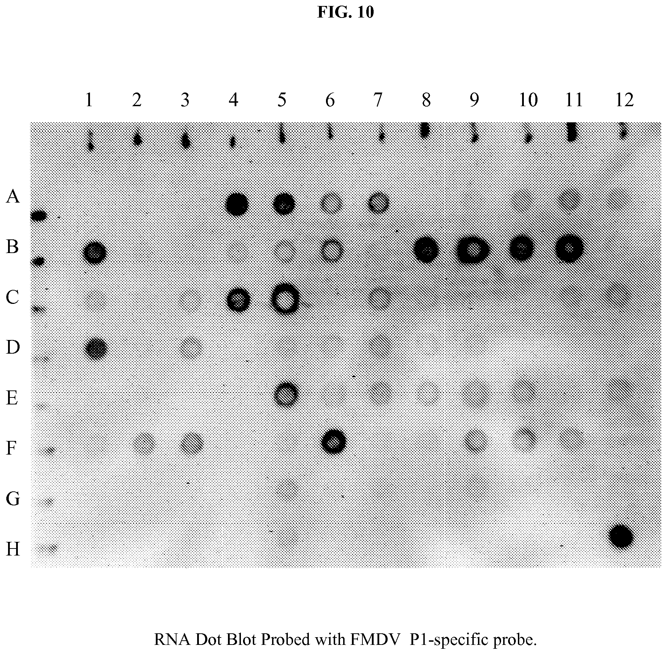

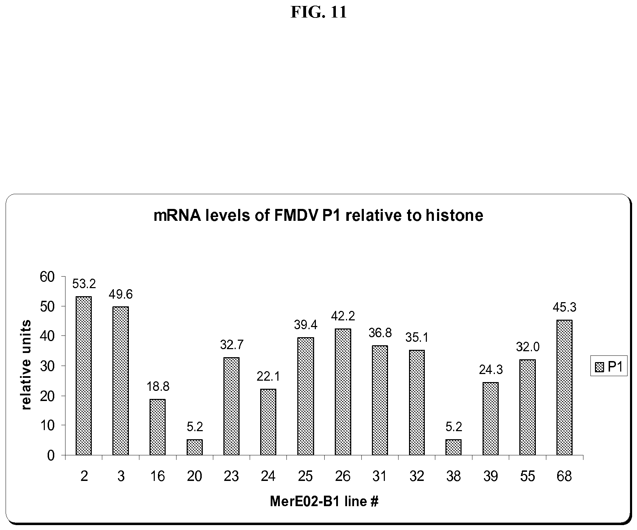

*Screening.* 144 transgenic FMDV lines were developed. After 144 FMDV expressing lines were developed, they were screened to determine the relative levels of FMDV expression in the tissue. FMDV mRNA levels were measured via RNA dot blot (FIG. 10) and real time rtPCR (FIG. 11). FMDV proteins levels were measured using western blot and ELISA. FMDV-expressing duckweed lines were screened via RNA dot blot using a labeled PI region to probe the blot. High expressing lines were confirmed by real time rtPCR, and there was reasonable agreement between the mRNA quantification methods. The results indicate that the FMDV P1 is highly expressed in duckweed.

*Methods.* The duckweed plants were grown for two weeks in small research vessels and the resulting tissue was collected and snap frozen in liquid N₂. Total RNA was extracted from 100 mg of frozen tissue samples using the Qiagen RNeasy 96-well RNA extraction kit (Qiagen, Valencia, CA, #74181). RNA was quantified, vacuum transferred to nylon membranes and probed with a gene fragment from the PI region of the FMDV gene sequence. Crude tissue extraction from lines containing FMDV antigens were prepared. All steps were taken place at 4°C. One hundred grams of frozen biomass was mixed with 200ml extraction buffer (50mM NaPO₄, 0.3M NaCl, 10mm EDTA, pH 7.4) and then homogenized in a Waring Blender with a 20 second burst for 4 times and 10-20 seconds cooling in between. The homogenate was centrifuged at 10,000 x g for 30 min at 4°C, clarified by passing through a cheese cloth to remove any large debris and finally cellulose acetate filter (0.22µm). The resulting homogenate was stored at 4°C or on ice for immediate testing. The remaining homogenate was frozen in aliquots at -80°C for further analysis. Total soluble protein (TSP) was determined using the Bradford assay with bovine serum albumin as a standard. Protein level analysis was performed for P1-expressing duckweed lines. Duplicate samples were read from two separate extractions (total of 4 measurements).

*RNA Results.* A broad range of RNA expression levels was observed between the various FMDV transgenic lines (FIG. 11). RNA expression from transgenic lines showing the strongest hybridization signals were then confirmed by real time rtPCR. Relative expression of these lines was shown to be comparable between the dot-blot and PCR methods. The top lines were then selected for further western blot characterization (FIG. 12).

*Protein Results.* The ELISA results are summarized in Table 4 and the densitometry and Agilent results are summarized in Table 5. FIG. 12 depicts the WB results, with arrows indicating the FMDV bands. Lane 1 (both gels) - marker, 2 (both gels) - sucrose-purified FMDV A24 inactivated virus, 3 (both gels) - duckweed wild type lysate. Lanes 4-6 of MerE01-expressing duckweed lysates (three lines - 6, 8 and 10). Lanes 4-12 MerE02-expressing duckweed lysates (nine lines - 2, 3, 16, 20, 23, 24, 25, 26 and 31). MerE01-expressing duckweed lines 6 and 10 appear to produce a properly cleaved protein (this line contains the 3C protease). MerE02-expressing duckweed lines (lacking the protease) appear to produce significant amounts of the uncleaved PI protein, as well as higher MW aggregate species.

**Table 4: ELISA Quantification Results for the MerE01 Lines.**

| **Duckweed line** | **Average Antigen Conc. (µg/ml)** | **Average TSP (mg/ml)** | **% TSP** |
|---|---|---|---|
| MerE01-6 | 11.35-16.84 | 5.0 | 0.23-0.34 |
| MerE01-10 | 0.79-3.4 | 5.0 | 0.02-0.07 |

**Table 5: Expression Level of Duckweed-FMDV MerE01 Lines.**

| **Duckweed line** | **Average Antigen Conc. (µg/ml)¹** | **Average%TSP²** |
|---|---|---|
| MerE02-2 | 54.6 ±4.3 | 2.35 |
| MerE02-3 | 36.8 ±6.6 | 1.70 |
| MerE02-26 | 91.6 ±9.0 | 3.95 |

| | | |
|---|---|---|
| ¹WT background band at the same MW (around 99-100 kDa) less than 5 µg/ml. ²Average total soluble protein between 2.1 and 2.3 mg/ml by Agilent analysis. | | |

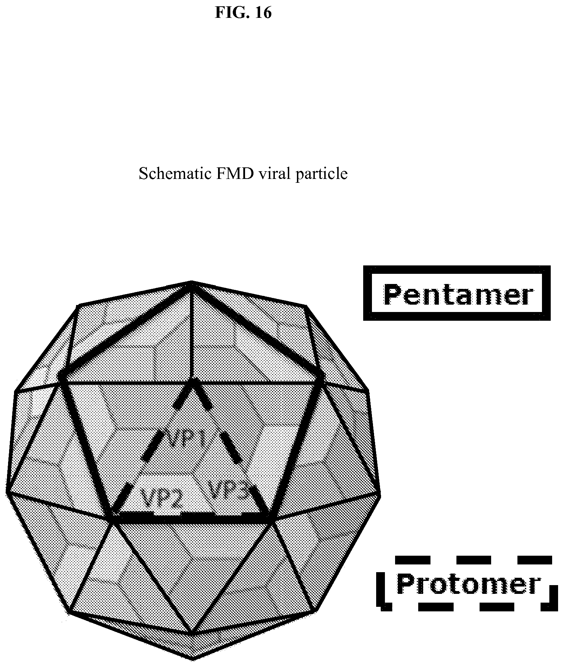

Inventors observed expression of the 3C protease was exerting toxic effects on the recombinant duckweed, causing reduced growth and antigen production, as compared to, for example, recombinant duckweed expressing only the P1 polypeptide. Therefore, new constructs were produced, some of which comprised a 3C expression cassette driven by an inducible promoter. Unhindered by the toxic effects of 3C expression, the duckweed grew and expressed robust levels of P1. Then, after the culture reached an optimum density, 3C expression was induced so it could cleave the established pools of PI into the various subunits. The subunits were then able to assemble into protomers, pentamers, and finally, the FMD viral particles (schematized in FIG. 16).

These new FMDV duckweed expression constructs included MerF01 (SEQ ID NO:30), which incorporated PI - 3C expressed driven by the Super Promoter in standard single gene expression vector (EC1.0, analogous to approach taken to produce MerE01). MerF02 (SEQ ID N0:31) used the *Lemna gibba* NPR promoter (ABA inducible promoter) to express PI - 3C and MerF03 (SEQ ID NO:32) expressed P1-2A and 3C with separate promoters in a single vector: PI - 2A expression driven by Super Promoter and 3C expression driven by *Lemna minor* R-histone promoter. MerF04 (SEQ ID NO:33) expressed P1-2A driven by Super promoter and expressed 3C driven by *Lemna gibba* NPR promoter. MerF05 (SEQ ID NO:34) expressed P1-2A (SpUbq promoter) and 3C (*Lemna minor* R-histone promoter), and MerF06 (SEQ ID NO:35) expressed P1-2A (SpUbq promoter) and 3C (*Lemna gibba* NPR promoter).

*Antigen preparation.* Crude extract was produced using a Waring Blender or bead beater (1:4 biomass to buffer ratio, PBS pH7.2), and the lysates were clarified by centrifugation (∼10K x g). To produce the concentrate, the clarified lysate was filtered through a sterile 0.22 µm filter. This material was then concentrated using 30KDa centricon filters (5-10x), and subjected to *in vitro* characterization or animal study.

### Example 2 - Expression of FMDV antigens in Schizochytrium

Codon-optimized FMDV P1 and 3C genes are cloned into the expression vector pAB0018 (ATCC deposit no. PTA9616). The specific nucleic acid sequence of FMDV gene is optimized for expression in *Schizochytrium* sp. Additionally, the expression vector contains a selection marker cassette conferring resistance to *Schizochytrium* transformants, a promoter from the *Schizochytrium* native gene to drive expression of the transgene, and a terminator.

*Schizochytrium* sp. (ATCC 20888) is used as a host for transformation with the expression vector containing the FDMV gene using electroporation method. Cryostocks of transgenic strains of *Schizochytrium* are grown in M50-20 (described in US 2008/0022422) to confluency. The propagated *Schizochytrium* cultures are transferred to 50mL conical tubes and centrifuged at 3000g for 15min or 100,000g for 1hour. The resulting pellet and the soluble fraction are used for expression analysis and in animal challenge study.

### Example 3 - Vaccination of pigs - safety assessment

Three (3) groups of five (5) pigs were vaccinated on days 0 and 21 (D0 and D21) according to the study design (Table 6). Details of the TS6 adjuvant (emulsions) may be found in US patent numbers US 7,608,279 B2 and US 7,371,395 B2 (both to Merial Limited).

*Assessment of Safety.* No adverse general/systemic reactions were observed after vaccination, though transient, slight to moderate increases of rectal temperature were observed in all groups. Locally, slight to moderate reactions were observed for the duckweed groups. The vaccines were globally acceptable for all groups.

**Table 6 - Vaccination of pigs - study design**

| **Group** | **Antigen** | **Dilution** | **Adjuvant** |
|---|---|---|---|
| G1 | MerE01 | Not diluted | TS6 |
| G2 | MerE01 | 1/10 diluted | TS6 |
| G3 | - | Control | - |

### Example 4 - Vaccination of Cattle

Seventeen (17) conventional cattle, free from FMDV, but not previously immunized against FMDV, were used for this study (study design summarized in Table 7). At D-1, the cattle were allocated into to three groups of 5 animals, and 1 group of 2 animals. Vaccination was performed on D0, via sub-cutaneous route on the left side of the neck. Challenge was administered on D21, and the final observations were made on D29. Tested vaccines expressing FMDV A24 P1-3C were formulated in TS6 adjuvant as described above. Separate vials of antigens and adjuvant were stored at 5 °C prior to administration. The contents of the vials were reconstituted extemporaneously by mixing antigen with adjuvant accordingly. The volume of a dose of the reconstituted vaccines was 2 mL. The challenge strain was FMD type A24 virus prepared to obtain 10 000 ID50 per 0.2 mL. The challenge strain was diluted in Hanks MEM 2% fetal bovine serum with antibiotics.

**Table 7 - cattle vaccine summary**

| Group | Antigen | Dose | # Cattle |
|---|---|---|---|
| A | Duckweed expressed FMDV | 2 ml | 5 |
| B | Experimental recombinant vaccine 1 expressed FMDV | 2 ml | 5 |
| C | Experimental recombinant vaccine 2 expressed FMDV | 2 ml | 5 |
| Control | NA | NA | 2 |

On D21, all animals were tranquilized by administration of Xylazine (0.03 - 0.10 mg per kg BW I.V. (0,15 - 0,5 ml per 100 kg BW I.V.) and challenged with 10 000 ID₅₀ of virus by intra-dermal route, into two locations of the tongue, 0.1 ml per location. The general well being of the animals was checked daily from D1 to D21. Any clinical observation and treatments administered (commercial name, active ingredient, preemption date, volume, route) were noted.

Necropsy results (Number of feet with at least 1 vesicle, min=0, max=4) for the animals were as follows: duckweed (4, 3, 2, 1, 1), experimental recombinant vaccine 1 (0, 0, 1, 1, 1), experimental recombinant vaccine 2 (3, 4, 4, 4, 4) and the Controls (4, 4). The challenge study was validated since all 2 control cattle showed FMD clinical signs, in addition, experimental vaccine 2 also exhibited clear FMD signs, and can be served as negative control. Even though the cattle in duckweed group were not well protected, clinical symptom was eased in two cattle. There were two cattle in experimental vaccine 1 group were considered to be protected, the result was consistent with other reports using the similar expression system and suggested that intact VLP was immunogenic and was able to provide protection against virulent FMDV.

It is likely the duckweed vaccine will be improved by 1) increasing percentage of intact VLP, and 2) improving the concentration/purification strategies, so that the antigen will be more accessible to the immune system in order to achieve greater protection.

### Example 5 - characterization of FMDV antigens by sandwich ELISA

*In vitro* characterization of duckweed expressed FMDV was conducted with a sandwich ELISA using FA24 005E9G (monoclonal antibody against FMDV A24) and biotinylated M3, a 12S-specific *llama* single chain domain antibody.

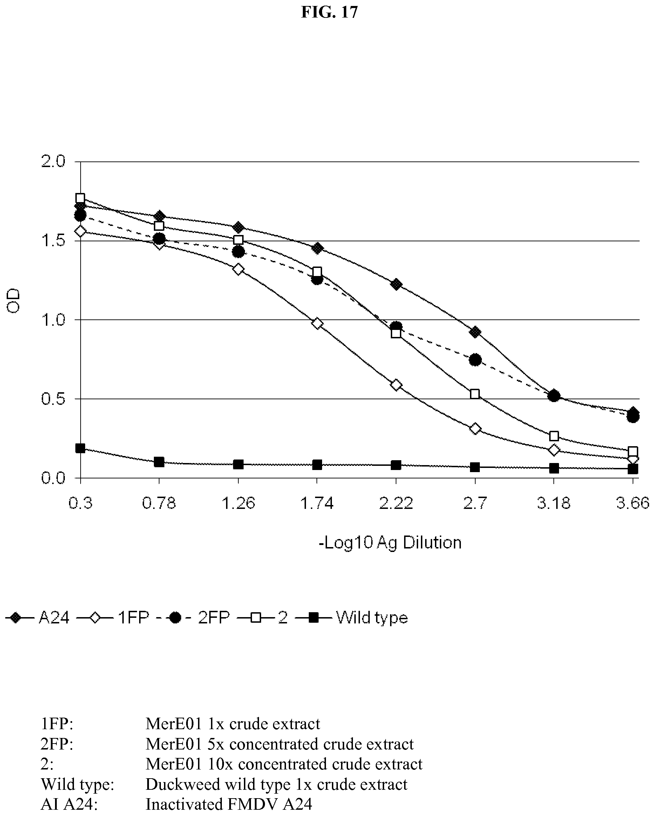

Results (FIG. 17) indicated that positive optical density at 450nm/630nm for 1x crude extract, 5x, and 10x concentrated crude extract, which was in good agreement with inactivated FMDV A24 as positive control, whereas crude extract prepared from duckweed wild type remained undetectable. ELISA titer was determined as the decimal logarithm per milliliter of the dilution for which 50% of the maximum OD was obtained. Table 8 demonstrated that ELISA titer for 5x was higher than 1x extract, however, equivalent to 10x extract, 5x concentrated duckweed crude extract was used in the cattle challenge study.

**Table 8. Summary of ELISA titer**

| **Sample** | **Titre log10OD50/ml** |
|---|---|
| **Inactivated FMDV A24** | **5.46** |
| **MerE01 1x crude extract** | **2.79** |
| **MerE01 5x concentrated crude extract** | **3.34** |
| **MerE01 10x concentrated crude extract** | **3.21** |
| **Duckweed wild type 1x crude extract** | ***Negative** |

### REFERENCES

Andreansky, S. S., B. He, et al. (1996). "The application of genetically engineered herpes simplex viruses to the treatment of experimental brain tumors." Proc Natl Acad Sci U SA 93(21): 11313-8.
Antoine, G., F. Scheiflinger, et al. (1998). "The complete genomic sequence of the modified vaccinia Ankara strain: comparison with other orthopoxviruses." Virology 244(2): 365-96.
Ballay, A., M. Levrero, et al. (1985). "In vitro and in vivo synthesis of the hepatitis B virus surface antigen and of the receptor for polymerized human serum albumin from recombinant human adenoviruses." Embo J 4(13B): 3861-5.
Barcena, J., M. M. Lorenzo, et al. (2000). "Sequence and analysis of a swinepox virus homologue of the vaccinia virus major envelope protein P37 (F13L)." J Gen Virol 81(Pt 4): 1073-85.
Boshart, M., F. Weber, et al. (1985). "A very strong enhancer is located upstream of an immediate early gene of human cytomegalovirus." Cell 41(2): 521-30.
Bradel-Tretheway, B. G., Z. Zhen, et al. (2003). "Effects of codon-optimization on protein expression by the human herpesvirus 6 and 7 U51 open reading frame." J Virol Methods 111(2): 145-56.
Carroll, M. W., W. W. Overwijk, et al. (1997). "Highly attenuated modified vaccinia virus Ankara (MVA) as an effective recombinant vector: a murine tumor model." Vaccine 15(4): 387-94.
Cochran, M. A., C. Puckett, et al. (1985). "In vitro mutagenesis of the promoter region for a vaccinia virus gene: evidence for tandem early and late regulatory signals." J Virol 54(1): 30-7.
De Groot, A. S. and F. G. Rothman (1999). "In silico predictions; in vivo veritas." Nat Biotechnol 17(6): 533-4.
Disbrow, G. L., I. Sunitha, et al. (2003). "Codon optimization of the HPV-16 E5 gene enhances protein expression." Virology 311(1): 105-14.
Feigner, J. H., R. Kumar, et al. (1994). "Enhanced gene delivery and mechanism studies with a novel series of cationic lipid formulations." J Biol Chem 269(4): 2550-61.
Frolov, I., T. A. Hoffman, et al. (1996). "Alphavirus-based expression vectors: strategies and applications." Proc Natl Acad Sci U S A 93(21): 11371-7.
Funahashi, S., T. Sato, et al. (1988). "Cloning and characterization of the gene encoding the major protein of the A-type inclusion body of cowpox virus." J Gen Virol 69 (Pt 1): 35-47.
Geysen, H. M. (1990). "Molecular technology: peptide epitope mapping and the pin technology." Southeast Asian J Trop Med Public Health 21(4): 523-33.
Geysen, H. M., S. J. Barteling, et al. (1985). "Small peptides induce antibodies with a sequence and structural requirement for binding antigen comparable to antibodies raised against the native protein." Proc Natl Acad Sci U S A 82(1): 178-82.
Geysen, H. M., R. H. Meloen, et al. (1984). "Use of peptide synthesis to probe viral antigens for epitopes to a resolution of a single amino acid." Proc Natl Acad Sci U S A 81(13): 3998-4002.
Graham, F. L. (1990). "Adenoviruses as expression vectors and recombinant vaccines." Trends Biotechnol 8(4): 85-7.
Guo, P. X., S. Goebel, et al. (1989). "Expression in recombinant vaccinia virus of the equine herpesvirus 1 gene encoding glycoprotein gpl3 and protection of immunized animals." J Virol 63(10): 4189-98.
Hartikka, J., M. Sawdey, et al. (1996). "An improved plasmid DNA expression vector for direct injection into skeletal muscle." Hum Gene Ther 7(10): 1205-17.
Hemmer, B., C. Pinilla, et al. (1998). "The use of soluble synthetic peptide combinatorial libraries to determine antigen recognition of T cells." J Pept Res 52(5): 338-45.
Ju, Q., D. Edelstein, et al. (1998). "Transduction of non-dividing adult human pancreatic beta cells by an integrating lentiviral vector." Diabetologia 41(6): 736-9.
Kim, C. H., Y. Oh, et al. (1997). "Codon optimization for high-level expression of human erythropoietin (EPO) in mammalian cells." Gene 199(1-2): 293-301.
Kitson, J. D., K. L. Burke, et al. (1991). "Chimeric polioviruses that include sequences derived from two independent antigenic sites of foot-and-mouth disease virus (FMDV) induce neutralizing antibodies against FMDV in guinea pigs." J Virol 65(6): 3068-75.
Klinman, D. M., A. K. Yi, et al. (1996). "CpG motifs present in bacteria DNA rapidly induce lymphocytes to secrete interleukin 6, interleukin 12, and interferon gamma." Proc Natl Acad Sci U S A 93(7): 2879-83.
Kwissa, M., K. van Kampen, et al. (2000). "Efficient vaccination by intradermal or intramuscular inoculation of plasmid DNA expressing hepatitis B surface antigen under desmin promoter/enhancer control." Vaccine 18(22): 2337-44.
Laval, F., R. Paillot, et al. (2002). "Quantitative analysis of the antigen-specific IFNgamma+ T cell-mediated immune response in conventional outbred pigs: kinetics and duration of the DNA-induced IFNgamma+ CD8+ T cell response." Vet Immunol Immunopathol 90(3-4): 191-201.
Luckow, V. A. and M. D. Summers (1988). "Signals important for high-level expression of foreign genes in Autographa californica nuclear polyhedrosis virus expression vectors." Virology 167(1): 56-71.
Marshall, E., L. B. Woolford, et al. (1997). "Continuous infusion of macrophage inflammatory protein MIP-1alpha enhances leucocyte recovery and haemopoietic progenitor cell mobilization after cyclophosphamide." Br J Cancer 75(12): 1715-20.
McClements, W. L., M. E. Armstrong, et al. (1996). "Immunization with DNA vaccines encoding glycoprotein D or glycoprotein B, alone or in combination, induces protective immunity in animal models of herpes simplex virus-2 disease." Proc Natl Acad Sci U S A 93(21): 11414-20.
Miyazaki, J., S. Takaki, et al. (1989). "Expression vector system based on the chicken beta-actin promoter directs efficient production of interleukin-5." Gene 79(2): 269-77.
Moss, B. (1996). "Genetically engineered poxviruses for recombinant gene expression, vaccination, and safety." Proc Natl Acad Sci U S A 93(21): 11341-8.
Paoletti, E. (1996). "Applications of pox virus vectors to vaccination: an update." Proc Natl Acad Sci U S A 93(21): 11349-53.
Pearson, W. R. and D. J. Lipman (1988). "Improved tools for biological sequence comparison." Proc Natl Acad Sci U S A 85(8): 2444-8.
Pennock, G. D., C. Shoemaker, et al. (1984). "Strong and regulated expression of Escherichia coli beta-galactosidase in insect cells with a baculovirus vector." Mol Cell Biol 4(3): 399-406.
Perkus, M. E., K. Limbach, et al. (1989). "Cloning and expression of foreign genes in vaccinia virus, using a host range selection system." J Virol 63(9): 3829-36.
Powell, M. F. and M. J. Newman (1995). Vaccine Design, The Subunit and Adjuvant Approach. A Compendium of Vaccine Adjuvants and Excipients. F. Vogel and M. Powell. New York, Plenum Press. 6: 147, 183.
Prevec, L., M. Schneider, et al. (1989). "Use of human adenovirus-based vectors for antigen expression in animals." J Gen Virol 70 (Pt 2): 429-34.
Regelson, W., S. Kuhar, et al. (1960). "Synthetic polyelectrolytes as tumour inhibitors." Nature 186: 778-80.
Riviere, M., J. Tartaglia, et al. (1992). "Protection of mice and swine from pseudorabies virus conferred by vaccinia virus-based recombinants." J Virol 66(6): 3424-34.
Robertson, E. S., T. Ooka, et al. (1996). "Epstein-Barr virus vectors for gene delivery to B lymphocytes." Proc Natl Acad Sci U S A 93(21): 11334-40.
Robinson, H. L. and C. A. Torres (1997). "DNA vaccines." Semin Immunol 9(5): 271-83.
Roizman, B. (1996). "The function of herpes simplex virus genes: a primer for genetic engineering of novel vectors." Proc Natl Acad Sci U S A 93(21): 11307-12.
Sambrook, J. and D. W. Russell (2001). Molecular Cloning: a laboratory manual / Joseph Sambrook, David W. Russell. Cold Spring Harbor, N.Y., Cold Spring Harbor Laboratory Press.
Schneider, K., F. Puehler, et al. (2000). "cDNA cloning of biologically active chicken interleukin-18." J Interferon Cytokine Res 20(10): 879-83.
Shida, H. (1986). "Nucleotide sequence of the vaccinia virus hemagglutinin gene." Virology 150(2): 451-62.
Smith, G. E., M. D. Summers, et al. (1983). "Production of human beta interferon in insect cells infected with a baculovirus expression vector." Mol Cell Biol 3(12): 2156-65.
Snedecor, G. W. & COCHRAN, W. G. (1971) Transformation de proportions en Arcsinus. In Methodes Statistiques. 6th edn. Eds H. Boelle, E. Camhaji. Association de Coordination Technique Agricole. pp 366-367
Stickl, H. and V. Hochstein-Mintzel (1971). "[Intracutaneous smallpox vaccination with a weak pathogenic vaccinia virus ("MVA virus")]." Munch Med Wochenschr 113(35): 1149-53.
Stittelaar, K. J., L. S. Wyatt, et al. (2000). "Protective immunity in macaques vaccinated with a modified vaccinia virus Ankara-based measles virus vaccine in the presence of passively acquired antibodies." J Virol 74(9): 4236-43.
Sutter, G. and B. Moss (1992). "Nonreplicating vaccinia vector efficiently expresses recombinant genes." Proc Natl Acad Sci U S A 89(22): 10847-51.
Sutter, G., L. S. Wyatt, et al. (1994). "A recombinant vector derived from the host range-restricted and highly attenuated MVA strain of vaccinia virus stimulates protective immunity in mice to FMDV virus." Vaccine 12(11): 1032-40.
Tang, D. C., M. DeVit, et al. (1992). "Genetic immunization is a simple method for eliciting an immune response." Nature 356(6365): 152-4.
Taylor, J., R. Weinberg, et al. (1988). "Protective immunity against avian FMDV induced by a fowlpox virus recombinant." Vaccine 6(6): 504-8.
Thompson, J. D., D. G. Higgins, et al. (1994). "CLUSTAL W: improving the sensitivity of progressive multiple sequence alignment through sequence weighting, position-specific gap penalties and weight matrix choice." Nucleic Acids Res 22(22): 4673-80.
Ulmer, J. B., J. J. Donnelly, et al. (1993). "Heterologous protection against FMDV by injection of DNA encoding a viral protein." Science 259(5102): 1745-9.
Van der Zee, R., W. Van Eden, et al. (1989). "Efficient mapping and characterization of a T cell epitope by the simultaneous synthesis of multiple peptides." Eur J Immunol 19(1): 43-7.
van Ooyen, A., J. van den Berg, et al. (1979). "Comparison of total sequence of a cloned rabbit beta-globin gene and its flanking regions with a homologous mouse sequence." Science 206(4416): 337-44.
Vialard, J., M. Lalumiere, et al. (1990). "Synthesis of the membrane fusion and hemagglutinin proteins of measles virus, using a novel baculovirus vector containing the beta-galactosidase gene." J Virol 64(1): 37-50.
Xin, K. Q., K. Hamajima, et al. (1999). "IL-15 expression plasmid enhances cell-mediated immunity induced by an HIV-1 DNA vaccine." Vaccine 17(7-8): 858-66.

## Claims

1. An immunological or vaccine composition comprising a duckweed-expressed foot-and-mouth disease virus (FMDV) antigen and a pharmaceutical or veterinarily acceptable carrier, excipient or vehicle, wherein said antigen comprises a virus-like particle (VLP), further wherein said antigen is encoded by a polynucleotide having at least 80% identity to the sequence as set forth in SEQ ID NO: 2.

2. The composition of claim 1, wherein the FMDV antigen is encoded by a polynucleotide having the sequence as set forth in SEQ ID NO: 2.

3. The composition of claim 1 or claim 2, wherein the pharmaceutical or veterinarily acceptable carrier, excipient or vehicle is a water-in-oil emulsion or an oil-in-water emulsion.

4. The composition of any one of claims 1 to 3, wherein the FMDV antigen is at least 60% purified.

5. The composition of any one of claims 1 to 3, wherein the FMDV antigen is at least 90% purified.

6. The composition of any one of claims 1-5, further comprising an adjuvant.

7. The composition of any one of claims 1-6 for use in a method of vaccinating a host susceptible to ovine, bovine, caprine, or porcine FMDV.

8. The composition for the use according to claim 7 wherein said vaccination comprises a prime-boost administration protocol.

9. The composition for the use according to claim 8 wherein:
(a) the prime-boost administration comprises a prime-administration of the composition of any one of claims 1-6, and a boost-administration of a vaccine or composition comprising a recombinant viral vector that contains and expresses an FMDV antigen *in vivo,* or an inactivated viral vaccine comprising an FMDV antigen, or a DNA plasmid vaccine or composition that contains or expresses an FMDV antigen; or
(b) the prime-boost administration comprises a prime-administration of a vaccine or composition comprising a recombinant viral vector that contains and expresses an FMDV antigen *in vivo,* or an inactivated viral vaccine comprising an FMDV antigen, or a DNA plasmid vaccine or composition that contains or expresses an FMDV antigen, and a boost-administration of the composition of any one of claims 1-6; or
(c) the prime-boost administration comprises a prime-administration of the composition of any one of claims 1-6, and a boost-administration of the composition of any one of claims 1-6.

10. A duckweed plant transformed with a plasmid comprising a DNA fragment having at least 80% identity to the sequence as set forth in SEQ ID NO: 2, wherein the plasmid is for plant transformation, and wherein the transformed duckweed plant expresses an antigen encoded by said DNA fragment having at least 80% identity to the sequence as set forth in SEQ ID NO: 2.

11. A stably transformed duckweed plant transformed with:
(a) A plasmid comprising a DNA fragment having at least 80% identity to the sequence as set forth in SEQ ID NO: 2, wherein the plasmid is for plant transformation, wherein the transformed duckweed plant expresses an antigen encoded by said DNA fragment; or
(b) a gene expressing a FMDV antigen, wherein the antigen has at least 80% identity to the sequence as set forth in SEQ ID NO: 3.

## Patentansprüche

1. Immunologische Zusammensetzung oder Impfstoffzusammensetzung, umfassend ein Wasserlinsen-exprimiertes Maul-und-Klauenseuche-Virus (foot-and-mouth disease virus; FMDV)-Antigen und einen pharmazeutisch oder veterinärmedizinisch verträglichen Träger, Exzipienten oder ein pharmazeutisch oder veterinärmedizinisch verträgliches Vehikel, wobei das Antigen ein Virus-ähnliches Partikel (virus-like particle; VLP) umfasst, wobei des Weiteren das Antigen von einem Polynucleotid codiert wird, das mindestens 80% Identität mit der wie in SEQ ID NO:2 dargestellten Sequenz aufweist.

2. Zusammensetzung nach Anspruch 1, wobei das FMDV-Antigen von einem Polynucleotid codiert wird, das die wie in SEQ ID NO:2 dargestellte Sequenz aufweist.

3. Zusammensetzung nach Anspruch 1 oder 2, wobei der/das pharmazeutisch oder veterinärmedizinisch verträgliche Träger, Exzipient oder Vehikel eine Wasser-in-ÖI-Emulsion oder eine Öl-in-Wasser-Emulsion ist.

4. Zusammensetzung nach einem der Ansprüche 1 bis 3, wobei das FMDV-Antigen zu mindestens 60% gereinigt ist.

5. Zusammensetzung nach einem der Ansprüche 1 bis 3, wobei das FMDV-Antigen zu mindestens 90% gereinigt ist.

6. Zusammensetzung nach einem der Ansprüche 1 bis 5, die des Weiteren ein Adjuvans umfasst.

7. Zusammensetzung nach einem der Ansprüche 1 bis 6 zur Verwendung in einem Verfahren zum Impfen eines Wirts, der für Maul-und-Klauenseuche (FMDV) von Schaf, Rind, Ziege oder Schwein empfänglich ist.

8. Zusammensetzung zur Verwendung nach Anspruch 7, wobei die Impfung ein Prime-Boost-Verabreichungsprotokoll umfasst.

9. Zusammensetzung zur Verwendung nach Anspruch 8, wobei:
(a) die Prime-Boost-Verabreichung eine Prime-Verabreichung der Zusammensetzung nach einem der Ansprüche 1 bis 6, und eine Boost-Verabreichung eines Impfstoffs oder einer Zusammensetzung umfasst, der/die einen rekombinanten viralen Vektor umfasst, der ein FMDV-Antigen enthält und *in vivo* exprimiert, oder einen inaktivierten viralen Impfstoff, der ein FMDV-Antigen oder eine(n) DNA-Plasmid-Impfstoff oder -Zusammensetzung umfasst, der/die ein FMDV-Antigen enthält oder exprimiert; oder
(b) die Prime-Boost-Verabreichung eine Prime-Verabreichung eines Impfstoffs oder einer Zusammensetzung, der/die einen rekombinanten viralen Vektor umfasst, der ein FMDV-Antigen enthält und *in vivo* exprimiert, oder einen inaktivierten viralen Impfstoff, der ein FMDV-Antigen umfasst, oder eine(n) DNA-Plasmid-Impfstoff oder -Zusammensetzung, der/die ein FMDV-Antigen enthält oder exprimiert, und eine Boost-Verabreichung der Zusammensetzung nach einem der Ansprüche 1 bis 6 umfasst; oder
(c) die Prime-Boost-Verabreichung, die eine Prime-Verabreichung der Zusammensetzung nach einem der Ansprüche 1 bis 6 und eine Boost-Verabreichung der Zusammensetzung nach einem der Ansprüche 1 bis 6 umfasst.

10. Wasserlinsenpflanze, die mit einem Plasmid transformiert ist, das ein DNA-Fragment umfasst, das mindestens 80% Identität mit der wie in SEQ ID NO:2 dargestellten Sequenz aufweist, wobei das Plasmid für die Pflanzentransformation bestimmt ist, und wobei die transformierte Wasserlinsenpflanze ein Antigen exprimiert, das von dem DNA-Fragment codiert wird, das mindestens 80% Identität mit der wie in SEQ ID NO:2 dargestellten Sequenz aufweist.

11. Stabil transformierte Wasserlinsenpflanze, die transformiert ist mit:
(a) einem Plasmid, das ein DNA-Fragment umfasst, das mindestens 80% Identität mit der wie in SEQ ID NO:2 dargestellten Sequenz aufweist, wobei das Plasmid für die Pflanzentransformation bestimmt ist, wobei die transformierte Wasserlinsenpflanze ein Antigen exprimiert, das von dem DNA-Fragment codiert wird; oder
(b) einem Gen, das ein FMDV-Antigen exprimiert, wobei das Antigen mindestens 80% Identität mit der wie in SEQ ID NO:3 dargestellten Sequenz aufweist.

## Revendications

1. Composition immunologique ou de vaccin comprenant un antigène du virus de la fièvre aphteuse (FMDV) exprimé dans la lentille d'eau et un vecteur, excipient ou véhicule acceptable du point de vue pharmaceutique ou vétérinaire, dans laquelle ledit antigène comprend une particule analogue à un virus (VLP), dans laquelle en outre ledit antigène est codé par un polynucléotide ayant au moins 80 % d'identité avec la séquence telle que présentée dans SEQ ID NO:2.

2. Composition selon la revendication 1, dans laquelle l'antigène de FMDV est codé par un polynucléotide ayant la séquence telle que présentée dans SEQ ID NO:2.

3. Composition selon la revendication 1 ou la revendication 2, dans laquelle le vecteur, excipient ou véhicule acceptable du point de vue pharmaceutique ou vétérinaire est une émulsion eau dans huile ou une émulsion huile dans eau.

4. Composition selon l'une quelconque des revendications 1 à 3, dans laquelle l'antigène de FMDV est purifié à au moins 60 %.

5. Composition selon l'une quelconque des revendications 1 à 3, dans laquelle l'antigène de FMDV est purifié à au moins 90 %.

6. Composition selon l'une quelconque des revendications 1-5, comprenant en outre un adjuvant.

7. Composition selon l'une quelconque des revendications 1-6 destinée à être utilisée dans un procédé de vaccination d'un hôte sensible à FMDV ovin, bovin, caprin ou porcin.

8. Composition destinée à être utilisée selon la revendication 7 dans laquelle ladite vaccination comprend un protocole d'administration primo-rappel.

9. Composition destinée à être utilisée selon la revendication 8 dans laquelle :
(a) l'administration primo-rappel comprend une primo-administration de la composition selon l'une quelconque des revendications 1-6, et une administration de rappel d'un vaccin ou composition comprenant un vecteur viral recombinant qui contient et exprime un antigène de FMDV *in vivo,* ou d'un vaccin viral inactivé comprenant un antigène de FMDV, ou d'un vaccin ou composition de plasmide à ADN qui contient ou exprime un antigène de FMDV ; ou
(b) l'administration primo-rappel comprend une primo-administration d'un vaccin ou composition comprenant un vecteur viral recombinant qui contient et exprime un antigène de FMDV *in vivo,* ou d'un vaccin viral inactivé comprenant un antigène de FMDV, ou d'un vaccin ou composition de plasmide à ADN qui contient ou exprime un antigène de FMDV, et une administration de rappel de la composition selon l'une quelconque des revendications 1-6 ; ou
(c) l'administration primo-rappel comprend une primo-administration de la composition selon l'une quelconque des revendications 1-6, et une administration de rappel de la composition selon l'une quelconque des revendications 1-6.

10. Plante de lentille d'eau transformée avec un plasmide comprenant un fragment d'ADN ayant au moins 80 % d'identité avec la séquence telle que présentée dans SEQ ID NO: 2, dans laquelle le plasmide est destiné à la transformation de plante, et dans laquelle la plante de lentille d'eau transformée exprime un antigène codé par ledit fragment d'ADN ayant au moins 80 % d'identité avec la séquence telle que présentée dans SEQ ID NO : 2.

11. Plante de lentille d'eau transformée de manière stable transformée avec :
(a) un plasmide comprenant un fragment d'ADN ayant au moins 80 % d'identité avec la séquence telle que présentée dans SEQ ID NO: 2, dans laquelle le plasmide est destiné à la transformation de plante, dans laquelle la plante de lentille d'eau transformée exprime un antigène codé par ledit fragment d'ADN ; ou
(b) un gène exprimant un antigène de FMDV, dans laquelle l'antigène a au moins 80 % d'identité avec la séquence telle que présentée dans SEQ ID NO:3.


### SEQUENCE LISTING

<110> Merial Limited Biolex Therapeutics Audonnet, Jean-Christophe Guo, Xuan Michel, Bublot Feilmeier, Bradley Troupe, Karolyn Cox, Kevin
<120> FMD VIRUS RECOMBINANT VACCINES AND USES THEREOF
<130> MER 11-168
<150> 61/313,164
   <151> 2010-03-12
<150> 61/366,363
   <151> 2010-07-21
<160> 39
<170> PatentIn version 3.5
<210> 1
   <211> 3378
   <212> DNA
   <213> Artificial Sequence
<220>
   <223> pHM1119.1 FMDV P1-3C optimized for mammalian expression
<400> 1
   atgggagctg ggcaatccag cccagcaacc ggctcgcaga accagtctgg caacactggc        60
   agcataatca acaactacta catgcaacag taccagaact ccatggacac acagttggga       120
   gacaatgcca tcagtggagg ctccaacgag ggctccacgg acacaacttc aacacacaca       180
   accaacactc aaaacaatga ctggttctcg aagctcgcca gttcagcttt taccggtctg       240
   ttcggtgcac tgctcgccga caagaagaca gaggaaacga cacttcttga ggaccgcatc       300
   ctcaccaccc gcaacgggca caccacctcg acgacccaat cgagtgtggg tgtcacacac       360
   gggtactcca cagaggagga ccacgttgct gggcccaaca catcgggcct ggagacgcga       420
   gtggtgcagg cagagagatt ctacaaaaag tacttgtttg actggacaac ggacaaggca       480
   tttggacacc tggaaaagct ggagctcccg tccgaccacc acggtgtctt tggacacttg       540
   gtggactcgt acgcctatat gagaaatggc tgggatgttg aggtgtccgc tgttggcaac       600
   cagttcaacg gcgggtgcct cctggtggcc atggtacctg aatggaagga atttgacaca       660
   cgggagaaat accaactcac ccttttcccg caccagttta ttagccccag aactaacatg       720
   actgcccaca tcacggtccc ctaccttggt gtgaacaggt atgatcagta caagaagcat       780
   aagccctgga cattggttgt catggtcgtg tcgccactta cggtcaacaa cactagtgcg       840
   gcacaaatca aggtctacgc caacatagct ccgacctatg ttcacgtggc cggtgaactc       900
   ccctcgaaag aggggatttt cccggttgca tgtgcggacg gttacggagg attggtgacg       960
   acagacccga agacagctga ccctgcttat ggcaaggtgt acaacccgcc taggactaac 1020
   taccctgggc gcttcaccaa cctgttggac gtggccgaag cgtgtcccac tttcctctgc 1080
   tttgacgacg ggaaaccgta cgtcaccacg cggacggatg acacccgact tttggccaag 1140
   tttgaccttt cccttgccgc aaaacatatg tccaacacat acctgtcagg gattgctcag 1200
   tactacacac agtactctgg caccatcaat ttgcatttca tgtttacagg ttccactgat 1260
   tcaaaggccc gatacatggt ggcctacatc ccacctgggg tggagacacc accggacaca 1320
   cctgaaaggg ctgcccactg cattcacgct gaatgggaca ctggactaaa ctccaaattc 1380
   actttctcaa tcccgtacgt atccgccgcg gattacgcgt acacagcgtc tgacacggca 1440
   gaaacaatca acgtacaggg atgggtctgc atctaccaaa ttacacacgg gaaggctgaa 1500
   aatgacacct tggtcgtgtc ggttagcgcc ggcaaagact ttgagttgcg cctcccgatt 1560
   gacccccgcc agcagaccac cgctaccggg gaatcagcag acccggtcac caccaccgtg 1620
   gagaactacg gcggtgagac acaaatccag agacgtcacc acacggacat tggtttcatc 1680
   atggacagat ttgtgaagat ccaaagcttg agcccaacac atgtcattga cctcatgcag 1740
   gctcaccaac acggtctggt gggtgccttg ctgcgtgcag ccacgtacta cttttctgac 1800
   ctggaaattg ttgtacggca cgaaggcaat ctgacctggg tgcccaacgg cgcccctgaa 1860
   tcagccctgt tgaacaccag caaccccact gcctacaaca aggcaccatt cacgagactc 1920
   gctctcccct acactgcgcc gcaccgtgtg ctggcaacag tgtacaacgg gacgagtaag 1980
   tatgctgtgg gtggttcagg cagaagaggc gacatggggt ctctcgcggc gcgagtcgtg 2040
   aaacagcttc ctgcttcatt taactacggt gcaatcaagg ccgacgccat ccacgaactt 2100
   ctcgtgcgca tgaaacgggc cgagctctac tgccccagac cgctgttggc aatagaggtg 2160
   tcttcgcaag acaggcacaa gcaaaagatc attgcaccag caaagcagct tctgaatttt 2220
   gacctgctca agttggccgg agacgttgag tccaaccccg ggccattctt ctttgctgac 2280
   gttaggtcaa acttttcaaa gttggtagac acaatcaacc agatgcagga ggacatgtcc 2340
   acaaaacacg ggcccgactt caaccggttg gtgtccgcat ttgaggaatt ggccactgga 2400
   gttaaagcta tcaggaccgg tctcgacgag gccaaaccct ggtacaagct tatcaaactc 2460
   ctaagccgcc tgtcgtgcat ggccgctgtg gcagcacggt ccaaggaccc agtccttgtg 2520
   gccatcatgc tggccgacac cggtctcgag cgtcagagac ctctgaaagt gagagctaag 2580
   ctcccacagc aggaaggacc ttacgctggc ccgttggaga gacagaaacc gctgaaagtg 2640
   aaagcaaaag ccccggtcgt caaggaagga ccttacgagg gaccggtgaa gaagcctgtc 2700
   gctttgaaag tgaaagctaa gaacttgata gtcactgaga gtggtgcccc accgaccgac 2760
   ttgcaaaaga tggtcatggg caacacaaag cctgttgagc tcatccttga cgggaagaca 2820
   gtagccatct gttgtgctac tggagtgttt ggcactgctt acctcgtgcc tcgtcatctt 2880
   ttcgcagaga agtatgacaa gatcatgctg gatggcagag ccatgacaga cagtgactac      2940
   agagtgtttg agtttgagat taaagtaaaa ggacaggaca tgctctcaga cgctgcgctc      3000
   atggtgctcc accgtgggaa ccgcgtgaga gatatcacga aacactttcg tgatacagca      3060
   agaatgaaga aaggcacccc cgtcgtcggt gtggtcaaca acgccgacgt tgggagactg      3120
   attttctctg gtgaggccct cacctacaag gatattgtag tgtgcatgga cggagacacc      3180
   atgcctggcc tctttgccta caaagccgcc accaaggcag gctactgtgg aggagccgtt      3240
   ctcgccaagg acggggccga cactttcatc gtcggcactc actccgcagg aggcaatgga      3300
   gttggatact gctcatgcgt ttccaggtcc atgcttctca gaatgaaggc acacgttgac      3360
   cctgaaccac aacacgag                                                    3378
<210> 2
   <211> 3381
   <212> DNA
   <213> Artificial Sequence
<220>
   <223> FMDV - P1-3C (MerE01 - MerE04) optimized for Lemna expression
<400> 2
   atgggcgccg ggcagtcctc gcctgcgacg gggagccaga accagtcggg caacaccggc      60
   tcgatcatca acaactacta catgcagcaa taccagaaca gcatggacac ccagctcggc     120
   gataacgcca tctccggcgg gtccaacgag ggctcgaccg acacgacctc cacccacacc     180
   acgaacaccc agaacaatga ctggttcagc aagctggcct ccagcgcctt cacgggcctg     240
   ttcggggcgc tgctcgcgga caagaagacc gaggagacca cgctgctcga ggaccggatc     300
   ctcaccacgc gcaacgggca cacgaccagc acgacccagt ccagcgtcgg ggtgacccac     360
   ggctactcca cggaggagga ccacgtcgcc gggccgaaca cgagcggcct cgagacccgc     420
   gtggtccagg cggagcgctt ctacaagaag tacctgttcg actggaccac ggacaaggcg     480
   ttcgggcacc tcgagaagct ggagctgccg agcgaccacc acggcgtctt cggccacctc     540
   gtggactcct acgcgtacat gagaaatggg tgggacgtcg aggtcagcgc cgtcggcaac     600
   cagttcaacg gcgggtgcct gctcgtggcg atggtcccgg agtggaagga gttcgacacc     660
   cgcgagaagt atcagctgac cctcttcccc caccagttca tcagcccgcg caccaacatg     720
   accgcccaca tcacggtgcc gtacctcggg gtgaaccgct acgaccagta caagaagcac     780
   aagccctgga ccctcgtggt catggtggtc tccccgctga ctgtgaacaa cacgtccgcc     840
   gcgcagatca aagtctacgc caacatcgcc cccacctacg tgcacgtcgc cggcgagctg     900
   ccctccaagg agggcatctt ccccgtcgcc tgcgccgacg ggtacggggg cctggtgacc     960
   accgacccca agacggccga cccggcctac ggcaaagtgt acaacccgcc caggacgaac    1020
   taccccggtc gcttcaccaa cctcctggac gtggcggagg cctgcccgac cttcctgtgc    1080
   ttcgacgacg ggaagcccta cgtcaccacg cgcaccgacg acacgcgcct gctcgccaag    1140
   ttcgacctca gcctggccgc taagcacatg agcaacacct acctcagcgg catcgcccaa 1200
   tactataccc aatactcggg caccatcaac ctgcacttca tgttcacggg cagcaccgac 1260
   agcaaggccc ggtacatggt ggcctacatc ccgccgggcg tggagacccc tcccgacacg 1320
   cccgagcggg ctgcgcactg catccacgcc gagtgggaca ccggcctcaa cagcaagttc 1380
   acgttcagca tcccctacgt gtccgccgcg gattacgctt acaccgcctc ggacacggcc 1440
   gagacgatca acgtccaggg ctgggtctgc atctaccaga tcactcacgg caaggccgag 1500
   aacgacaccc tcgtcgtgag cgtctccgcc gggaaggact tcgagctgag gctgcccatc 1560
   gaccccaggc agcagaccac ggcgaccggg gagtccgccg accccgtgac cacgaccgtg 1620
   gagaactacg gcggggagac tcagatccag cggcgccacc acaccgacat cggcttcatc 1680
   atggaccgct tcgtgaagat ccagtccctg tcgcccaccc acgttatcga cctcatgcag 1740
   gcccaccagc acgggctcgt gggtgccctc ctgcgcgcgg ccacctacta cttcagcgac 1800
   ctcgagatcg ttgtccgcca cgaggggaac ctcacctggg tcccgaacgg tgcccccgag 1860
   agcgccctgc tcaacacctc caaccccacg gcgtacaaca aggccccctt cacgcgcctc 1920
   gccctgcctt acaccgcgcc ccaccgcgtg ctggccacgg tgtacaacgg gacctccaag 1980
   tacgccgtgg gcgggagcgg ccgcagaggc gacatgggga gcctcgccgc tagggtcgtg 2040
   aagcagctcc cggcctcctt caactacggc gccatcaagg ccgacgccat ccacgaactc 2100
   ctggtccgca tgaagcgcgc cgaactctac tgcccccggc ccctcctggc catcgaggtc 2160
   tcctcgcagg accgccacaa gcagaagatc atcgccccgg ccaagcagct gctcaacttc 2220
   gacctgctca agctcgcggg ggacgtcgag tcgaaccccg gtcccttctt cttcgccgac 2280
   gtgcggtcca acttctcgaa gctcgtggac accatcaacc agatgcagga ggacatgagc 2340
   accaagcacg ggccggactt caaccgcctc gtctcggcct tcgaggaact cgccaccggg 2400
   gtcaaggcca tcaggacggg cctggacgag gcgaagccgt ggtacaagct catcaagctc 2460
   ctgtcgcgcc tctcctgcat ggcggctgtc gcggcccgct ccaaggaccc cgtcctcgtc 2520
   gccatcatgc tcgcggacac cgggctcgag cgccagcggc ccctgaaagt gcgcgccaag 2580
   ctgccgcagc aggagggccc ctacgccggc ccgctcgaga gacagaagcc gctgaaagtc 2640
   aaggccaagg cgcccgtggt caaggagggg ccctacgagg gccccgtcaa gaagccggtg 2700
   gcgctgaaag tgaaggccaa gaacctcatc gtgaccgagt ccggtgcgcc tcccaccgac 2760
   ctgcagaaga tggtgatggg caacaccaag cccgtcgagt tgatcctcga cgggaagacc 2820
   gtggcgatct gctgcgccac cggcgtgttc ggcaccgcct acctcgtccc gagacacctg 2880
   ttcgccgaga agtatgacaa gatcatgctg gacgggcggg ccatgaccga ctcggactac 2940
   cgggtcttcg agttcgagat caaagtgaag ggccaggata tgctctccga cgccgcgctg 3000
   atggtgctcc acagaggcaa ccgcgtgcgg gacatcacca agcacttcag ggacaccgcg 3060
   cgcatgaaga aggggacccc tgtggtcggg gtcgtgaaca acgccgacgt cgggcgcctc 3120
   atcttctccg gcgaggcgct gacctacaag gacatcgtcg tgtgcatgga cggggacacg 3180
   atgccggggc tcttcgccta caaggccgcg accaaggccg ggtactgcgg gggtgcggtg 3240
   ctcgccaagg acggcgccga cacgttcatc gtgggcaccc actccgccgg cggtaacggc 3300
   gtcggctact gctcctgcgt gtcccggtcc atgctgctgc ggatgaaggc ccacgttgac 3360
   cccgagccgc agcacgagta a 3381
<210> 3
   <211> 1126
   <212> PRT
   <213> Artificial Sequence
<220>
   <223> FMDV - P1-3C (MerE01 - MerE04) translation of SEQ ID NOs:1 and 2
<400> 3
   Met Gly Ala Gly Gln Ser Ser Pro Ala Thr Gly Ser Gln Asn Gln Ser
   1               5                   10                  15
   Gly Asn Thr Gly Ser Ile Ile Asn Asn Tyr Tyr Met Gln Gln Tyr Gln
               20                  25                  30
   Asn Ser Met Asp Thr Gln Leu Gly Asp Asn Ala Ile Ser Gly Gly Ser
           35                  40                  45
   Asn Glu Gly Ser Thr Asp Thr Thr Ser Thr His Thr Thr Asn Thr Gln
       50                  55                  60
   Asn Asn Asp Trp Phe Ser Lys Leu Ala Ser Ser Ala Phe Thr Gly Leu
   65                  70                  75                  80
   Phe Gly Ala Leu Leu Ala Asp Lys Lys Thr Glu Glu Thr Thr Leu Leu
                   85                  90                  95
   Glu Asp Arg Ile Leu Thr Thr Arg Asn Gly His Thr Thr Ser Thr Thr
               100                 105                 110
   Gln Ser Ser Val Gly Val Thr His Gly Tyr Ser Thr Glu Glu Asp His
           115                 120                 125
   Val Ala Gly Pro Asn Thr Ser Gly Leu Glu Thr Arg Val Val Gln Ala
       130                 135                 140
   Glu Arg Phe Tyr Lys Lys Tyr Leu Phe Asp Trp Thr Thr Asp Lys Ala
   145                 150                 155                 160
   Phe Gly His Leu Glu Lys Leu Glu Leu Pro Ser Asp His His Gly Val
                   165                 170                 175
   Phe Gly His Leu Val Asp Ser Tyr Ala Tyr Met Arg Asn Gly Trp Asp
               180                 185                 190
   Val Glu Val Ser Ala Val Gly Asn Gln Phe Asn Gly Gly Cys Leu Leu
           195                 200                 205
   Val Ala Met Val Pro Glu Trp Lys Glu Phe Asp Thr Arg Glu Lys Tyr
       210                 215                 220
   Gln Leu Thr Leu Phe Pro His Gln Phe Ile Ser Pro Arg Thr Asn Met
   225                 230                 235                 240
   Thr Ala His Ile Thr Val Pro Tyr Leu Gly Val Asn Arg Tyr Asp Gln
                   245                 250                 255
   Tyr Lys Lys His Lys Pro Trp Thr Leu Val Val Met Val Val Ser Pro
               260                 265                 270
   Leu Thr Val Asn Asn Thr Ser Ala Ala Gln Ile Lys Val Tyr Ala Asn
           275                 280                 285
   Ile Ala Pro Thr Tyr Val His Val Ala Gly Glu Leu Pro Ser Lys Glu
       290                 295                 300
   Gly Ile Phe Pro Val Ala Cys Ala Asp Gly Tyr Gly Gly Leu Val Thr
   305                 310                 315                 320
   Thr Asp Pro Lys Thr Ala Asp Pro Ala Tyr Gly Lys Val Tyr Asn Pro
                   325                 330                 335
   Pro Arg Thr Asn Tyr Pro Gly Arg Phe Thr Asn Leu Leu Asp Val Ala
               340                 345                 350
   Glu Ala Cys Pro Thr Phe Leu Cys Phe Asp Asp Gly Lys Pro Tyr Val
           355                 360                 365
   Thr Thr Arg Thr Asp Asp Thr Arg Leu Leu Ala Lys Phe Asp Leu Ser
       370                 375                 380
   Leu Ala Ala Lys His Met Ser Asn Thr Tyr Leu Ser Gly Ile Ala Gln
   385                 390                 395                 400
   Tyr Tyr Thr Gln Tyr Ser Gly Thr Ile Asn Leu His Phe Met Phe Thr
                   405                 410                 415
   Gly Ser Thr Asp Ser Lys Ala Arg Tyr Met Val Ala Tyr Ile Pro Pro
               420                 425                 430
   Gly Val Glu Thr Pro Pro Asp Thr Pro Glu Arg Ala Ala His Cys Ile
           435                 440                 445
   His Ala Glu Trp Asp Thr Gly Leu Asn Ser Lys Phe Thr Phe Ser Ile
       450                 455                 460
   Pro Tyr Val Ser Ala Ala Asp Tyr Ala Tyr Thr Ala Ser Asp Thr Ala
   465                 470                 475                 480
   Glu Thr Ile Asn Val Gln Gly Trp Val Cys Ile Tyr Gln Ile Thr His
                   485                 490                 495
   Gly Lys Ala Glu Asn Asp Thr Leu Val Val Ser Val Ser Ala Gly Lys
               500                 505                 510
   Asp Phe Glu Leu Arg Leu Pro Ile Asp Pro Arg Gln Gln Thr Thr Ala
           515                 520                 525
   Thr Gly Glu Ser Ala Asp Pro Val Thr Thr Thr Val Glu Asn Tyr Gly
       530                 535                 540
   Gly Glu Thr Gln Ile Gln Arg Arg His His Thr Asp Ile Gly Phe Ile
   545                 550                 555                 560
   Met Asp Arg Phe Val Lys Ile Gln Ser Leu Ser Pro Thr His Val Ile
                   565                 570                 575
   Asp Leu Met Gln Ala His Gln His Gly Leu Val Gly Ala Leu Leu Arg
               580                 585                 590
   Ala Ala Thr Tyr Tyr Phe Ser Asp Leu Glu Ile Val Val Arg His Glu
           595                 600                 605
   Gly Asn Leu Thr Trp Val Pro Asn Gly Ala Pro Glu Ser Ala Leu Leu
       610                 615                 620
   Asn Thr Ser Asn Pro Thr Ala Tyr Asn Lys Ala Pro Phe Thr Arg Leu
   625                 630                 635                 640
   Ala Leu Pro Tyr Thr Ala Pro His Arg Val Leu Ala Thr Val Tyr Asn
                   645                 650                 655
   Gly Thr Ser Lys Tyr Ala Val Gly Gly Ser Gly Arg Arg Gly Asp Met
               660                 665                 670
   Gly Ser Leu Ala Ala Arg Val Val Lys Gln Leu Pro Ala Ser Phe Asn
           675                 680                 685
   Tyr Gly Ala Ile Lys Ala Asp Ala Ile His Glu Leu Leu Val Arg Met
       690                 695                 700
   Lys Arg Ala Glu Leu Tyr Cys Pro Arg Pro Leu Leu Ala Ile Glu Val
   705                 710                 715                 720
   Ser Ser Gln Asp Arg His Lys Gln Lys Ile Ile Ala Pro Ala Lys Gln
                   725                 730                 735
   Leu Leu Asn Phe Asp Leu Leu Lys Leu Ala Gly Asp Val Glu Ser Asn
               740                 745                 750
   Pro Gly Pro Phe Phe Phe Ala Asp Val Arg Ser Asn Phe Ser Lys Leu
           755                 760                 765
   Val Asp Thr Ile Asn Gln Met Gln Glu Asp Met Ser Thr Lys His Gly
       770                 775                 780
   Pro Asp Phe Asn Arg Leu Val Ser Ala Phe Glu Glu Leu Ala Thr Gly
   785                 790                 795                 800
   Val Lys Ala Ile Arg Thr Gly Leu Asp Glu Ala Lys Pro Trp Tyr Lys
                   805                 810                 815
   Leu Ile Lys Leu Leu Ser Arg Leu Ser Cys Met Ala Ala Val Ala Ala
               820                 825                 830
   Arg Ser Lys Asp Pro Val Leu Val Ala Ile Met Leu Ala Asp Thr Gly
           835                 840                 845
   Leu Glu Arg Gln Arg Pro Leu Lys Val Arg Ala Lys Leu Pro Gln Gln
       850                 855                 860
   Glu Gly Pro Tyr Ala Gly Pro Leu Glu Arg Gln Lys Pro Leu Lys Val
   865                 870                 875                 880
   Lys Ala Lys Ala Pro Val Val Lys Glu Gly Pro Tyr Glu Gly Pro Val
                   885                 890                 895
   Lys Lys Pro Val Ala Leu Lys Val Lys Ala Lys Asn Leu Ile Val Thr
               900                 905                 910
   Glu Ser Gly Ala Pro Pro Thr Asp Leu Gln Lys Met Val Met Gly Asn
           915                 920                 925
   Thr Lys Pro Val Glu Leu Ile Leu Asp Gly Lys Thr Val Ala Ile Cys
       930                 935                 940
   Cys Ala Thr Gly Val Phe Gly Thr Ala Tyr Leu Val Pro Arg His Leu
   945                 950                 955                 960
   Phe Ala Glu Lys Tyr Asp Lys Ile Met Leu Asp Gly Arg Ala Met Thr
                   965                 970                 975
   Asp Ser Asp Tyr Arg Val Phe Glu Phe Glu Ile Lys Val Lys Gly Gln
               980                 985                 990
   Asp Met Leu Ser Asp Ala Ala Leu  Met Val Leu His Arg  Gly Asn Arg
           995                 1000                 1005
   Val Arg  Asp Ile Thr Lys His  Phe Arg Asp Thr Ala  Arg Met Lys
       1010                 1015                 1020
   Lys Gly  Thr Pro Val Val Gly  Val Val Asn Asn Ala  Asp Val Gly
       1025                 1030                 1035
   Arg Leu  Ile Phe Ser Gly Glu  Ala Leu Thr Tyr Lys  Asp Ile Val
       1040                 1045                 1050
   Val Cys  Met Asp Gly Asp Thr  Met Pro Gly Leu Phe  Ala Tyr Lys
       1055                 1060                 1065
   Ala Ala  Thr Lys Ala Gly Tyr  Cys Gly Gly Ala Val  Leu Ala Lys
       1070                 1075                 1080
   Asp Gly  Ala Asp Thr Phe Ile  Val Gly Thr His Ser  Ala Gly Gly
       1085                 1090                 1095
   Asn Gly  Val Gly Tyr Cys Ser  Cys Val Ser Arg Ser  Met Leu Leu
       1100                 1105                 1110
   Arg Met  Lys Ala His Val Asp  Pro Glu Pro Gln His  Glu
       1115                 1120                 1125
<210> 4
   <211> 554
   <212> DNA
   <213> Artificial Sequence
<220>
   <223> Zea mays alcohol dehydrogenase - Nucleotides 1222-1775
<400> 4
   gatcaagtgc aaaggtccgc cttgtttctc ctctgtctct tgatctgact aatcttggtt 60
   tatgattcgt tgagtaattt tggggaaagc ttcgtccaca gttttttttt cgatgaacag 120
   tgccgcagtg gcgctgatct tgtatgctat cctgcaatcg tggtgaactt atgtctttta 180
   tatccttcac taccatgaaa agactagtaa tctttctcga tgtaacatcg tccagcactg 240
   ctattaccgt gtggtccatc cgacagtctg gctgaacaca tcatacgata ttgagcaaag 300
   atctatcttc cctgttcttt aatgaaagac gtcattttca tcagtatgat ctaagaatgt 360
   tgcaacttgc aaggaggcgt ttctttcttt gaatttaact aactcgttga gtggccctgt 420
   ttctcggacg taaggccttt gctgctccac acatgtccat tcgaatttta ccgtgtttag 480
   caagggcgaa aagtttgcat cttgatgatt tagcttgact atgcgattgc tttcctggac 540
   ccgtgcagct gcgg 554
<210> 5
   <211> 21
   <212> PRT
   <213> Artificial Sequence
<220>
   <223> Arabidopsis thaliana basic endochitinase signal peptide
<400> 5
   Met Lys Thr Asn Leu Phe Leu Phe Leu Ile Phe Ser Leu Leu Leu Ser
   1 5 10 15
   Leu Ser Ser Ala Glu
   20
<210> 6
   <211> 31
   <212> PRT
   <213> Artificial Sequence
<220>
   <223> Rice alpha-amylase signal sequence peptide (translation of SEQ ID NO:8)
<400> 6
   Met Gln Val Leu Asn Thr Met Val Asn Lys His Phe Leu Ser Leu Ser
   1 5 10 15
   Val Leu Ile Val Leu Leu Gly Leu Ser Ser Asn Leu Thr Ala Gly
   20 25 30
<210> 7
   <211> 31
   <212> PRT
   <213> Artificial Sequence
<220>
   <223> Modified rice alpha-amylase signal sequence peptide
<400> 7
   Met Gln Val Leu Asn Thr Met Val Asn Lys His Phe Leu Ser Leu Ser
   1               5                   10                  15
   Val Leu Ile Val Leu Thr Val Leu Ser Ser Asn Leu Thr Ala Gly
               20                  25                  30
<210> 8
   <211> 96
   <212> DNA
   <213> Artificial Sequence
<220>
   <223> Rice alpha-amylase signal sequence nucleic acid
<400> 8
   accatgcagg tcctgaacac gatggtcaac aagcacttcc tctccctgtc cgtcctcatc      60
   gtcctcctcg ggctgagcag caacctcacc gccggc                                96
<210> 9
   <211> 2208
   <212> DNA
   <213> Artificial Sequence
<220>
   <223> FMDV - P1 DNA optimized for Lemna expression
<400> 9
   atgggcgccg ggcagtcctc gcctgcgacg gggagccaga accagtcggg caacaccggc      60
   tcgatcatca acaactacta catgcagcaa taccagaaca gcatggacac ccagctcggc     120
   gataacgcca tctccggcgg gtccaacgag ggctcgaccg acacgacctc cacccacacc     180
   acgaacaccc agaacaatga ctggttcagc aagctggcct ccagcgcctt cacgggcctg     240
   ttcggggcgc tgctcgcgga caagaagacc gaggagacca cgctgctcga ggaccggatc     300
   ctcaccacgc gcaacgggca cacgaccagc acgacccagt ccagcgtcgg ggtgacccac     360
   ggctactcca cggaggagga ccacgtcgcc gggccgaaca cgagcggcct cgagacccgc     420
   gtggtccagg cggagcgctt ctacaagaag tacctgttcg actggaccac ggacaaggcg     480
   ttcgggcacc tcgagaagct ggagctgccg agcgaccacc acggcgtctt cggccacctc     540
   gtggactcct acgcgtacat gagaaatggg tgggacgtcg aggtcagcgc cgtcggcaac     600
   cagttcaacg gcgggtgcct gctcgtggcg atggtcccgg agtggaagga gttcgacacc     660
   cgcgagaagt atcagctgac cctcttcccc caccagttca tcagcccgcg caccaacatg     720
   accgcccaca tcacggtgcc gtacctcggg gtgaaccgct acgaccagta caagaagcac     780
   aagccctgga ccctcgtggt catggtggtc tccccgctga ctgtgaacaa cacgtccgcc     840
   gcgcagatca aagtctacgc caacatcgcc cccacctacg tgcacgtcgc cggcgagctg     900
   ccctccaagg agggcatctt ccccgtcgcc tgcgccgacg ggtacggggg cctggtgacc     960
   accgacccca agacggccga cccggcctac ggcaaagtgt acaacccgcc caggacgaac    1020
   taccccggtc gcttcaccaa cctcctggac gtggcggagg cctgcccgac cttcctgtgc    1080
   ttcgacgacg ggaagcccta cgtcaccacg cgcaccgacg acacgcgcct gctcgccaag    1140
   ttcgacctca gcctggccgc taagcacatg agcaacacct acctcagcgg catcgcccaa    1200
   tactataccc aatactcggg caccatcaac ctgcacttca tgttcacggg cagcaccgac 1260
   agcaaggccc ggtacatggt ggcctacatc ccgccgggcg tggagacccc tcccgacacg 1320
   cccgagcggg ctgcgcactg catccacgcc gagtgggaca ccggcctcaa cagcaagttc 1380
   acgttcagca tcccctacgt gtccgccgcg gattacgctt acaccgcctc ggacacggcc 1440
   gagacgatca acgtccaggg ctgggtctgc atctaccaga tcactcacgg caaggccgag 1500
   aacgacaccc tcgtcgtgag cgtctccgcc gggaaggact tcgagctgag gctgcccatc 1560
   gaccccaggc agcagaccac ggcgaccggg gagtccgccg accccgtgac cacgaccgtg 1620
   gagaactacg gcggggagac tcagatccag cggcgccacc acaccgacat cggcttcatc 1680
   atggaccgct tcgtgaagat ccagtccctg tcgcccaccc acgttatcga cctcatgcag 1740
   gcccaccagc acgggctcgt gggtgccctc ctgcgcgcgg ccacctacta cttcagcgac 1800
   ctcgagatcg ttgtccgcca cgaggggaac ctcacctggg tcccgaacgg tgcccccgag 1860
   agcgccctgc tcaacacctc caaccccacg gcgtacaaca aggccccctt cacgcgcctc 1920
   gccctgcctt acaccgcgcc ccaccgcgtg ctggccacgg tgtacaacgg gacctccaag 1980
   tacgccgtgg gcgggagcgg ccgcagaggc gacatgggga gcctcgccgc tagggtcgtg 2040
   aagcagctcc cggcctcctt caactacggc gccatcaagg ccgacgccat ccacgaactc 2100
   ctggtccgca tgaagcgcgc cgaactctac tgcccccggc ccctcctggc catcgaggtc 2160
   tcctcgcagg accgccacaa gcagaagatc atcgccccgg ccaagcag 2208
<210> 10
   <211> 736
   <212> PRT
   <213> Artificial Sequence
<220>
   <223> FMDV - P1 protein (translation of SEQ ID NOs:9 and 13)
<400> 10
   Met Gly Ala Gly Gln Ser Ser Pro Ala Thr Gly Ser Gln Asn Gln Ser
   1               5                   10                  15
   Gly Asn Thr Gly Ser Ile Ile Asn Asn Tyr Tyr Met Gln Gln Tyr Gln
               20                  25                  30
   Asn Ser Met Asp Thr Gln Leu Gly Asp Asn Ala Ile Ser Gly Gly Ser
           35                  40                  45
   Asn Glu Gly Ser Thr Asp Thr Thr Ser Thr His Thr Thr Asn Thr Gln
       50                  55                  60
   Asn Asn Asp Trp Phe Ser Lys Leu Ala Ser Ser Ala Phe Thr Gly Leu
   65                  70                  75                  80
   Phe Gly Ala Leu Leu Ala Asp Lys Lys Thr Glu Glu Thr Thr Leu Leu
                   85                  90                  95
   Glu Asp Arg Ile Leu Thr Thr Arg Asn Gly His Thr Thr Ser Thr Thr
               100                 105                 110
   Gln Ser Ser Val Gly Val Thr His Gly Tyr Ser Thr Glu Glu Asp His
           115                 120                 125
   Val Ala Gly Pro Asn Thr Ser Gly Leu Glu Thr Arg Val Val Gln Ala
       130                 135                 140
   Glu Arg Phe Tyr Lys Lys Tyr Leu Phe Asp Trp Thr Thr Asp Lys Ala
   145                 150                 155                 160
   Phe Gly His Leu Glu Lys Leu Glu Leu Pro Ser Asp His His Gly Val
                   165                 170                 175
   Phe Gly His Leu Val Asp Ser Tyr Ala Tyr Met Arg Asn Gly Trp Asp
               180                 185                 190
   Val Glu Val Ser Ala Val Gly Asn Gln Phe Asn Gly Gly Cys Leu Leu
           195                 200                 205
   Val Ala Met Val Pro Glu Trp Lys Glu Phe Asp Thr Arg Glu Lys Tyr
       210                 215                 220
   Gln Leu Thr Leu Phe Pro His Gln Phe Ile Ser Pro Arg Thr Asn Met
   225                 230                 235                 240
   Thr Ala His Ile Thr Val Pro Tyr Leu Gly Val Asn Arg Tyr Asp Gln
                   245                 250                 255
   Tyr Lys Lys His Lys Pro Trp Thr Leu Val Val Met Val Val Ser Pro
               260                 265                 270
   Leu Thr Val Asn Asn Thr Ser Ala Ala Gln Ile Lys Val Tyr Ala Asn
           275                 280                 285
   Ile Ala Pro Thr Tyr Val His Val Ala Gly Glu Leu Pro Ser Lys Glu
       290                 295                 300
   Gly Ile Phe Pro Val Ala Cys Ala Asp Gly Tyr Gly Gly Leu Val Thr
   305                 310                 315                 320
   Thr Asp Pro Lys Thr Ala Asp Pro Ala Tyr Gly Lys Val Tyr Asn Pro
                   325                 330                 335
   Pro Arg Thr Asn Tyr Pro Gly Arg Phe Thr Asn Leu Leu Asp Val Ala
               340                 345                 350
   Glu Ala Cys Pro Thr Phe Leu Cys Phe Asp Asp Gly Lys Pro Tyr Val
           355                 360                 365
   Thr Thr Arg Thr Asp Asp Thr Arg Leu Leu Ala Lys Phe Asp Leu Ser
       370                 375                 380
   Leu Ala Ala Lys His Met Ser Asn Thr Tyr Leu Ser Gly Ile Ala Gln
   385                 390                 395                 400
   Tyr Tyr Thr Gln Tyr Ser Gly Thr Ile Asn Leu His Phe Met Phe Thr
                   405                 410                 415
   Gly Ser Thr Asp Ser Lys Ala Arg Tyr Met Val Ala Tyr Ile Pro Pro
               420                 425                 430
   Gly Val Glu Thr Pro Pro Asp Thr Pro Glu Arg Ala Ala His Cys Ile
           435                 440                 445
   His Ala Glu Trp Asp Thr Gly Leu Asn Ser Lys Phe Thr Phe Ser Ile
       450                 455                 460
   Pro Tyr Val Ser Ala Ala Asp Tyr Ala Tyr Thr Ala Ser Asp Thr Ala
   465                 470                 475                 480
   Glu Thr Ile Asn Val Gln Gly Trp Val Cys Ile Tyr Gln Ile Thr His
                   485                 490                 495
   Gly Lys Ala Glu Asn Asp Thr Leu Val Val Ser Val Ser Ala Gly Lys
               500                 505                 510
   Asp Phe Glu Leu Arg Leu Pro Ile Asp Pro Arg Gln Gln Thr Thr Ala
           515                 520                 525
   Thr Gly Glu Ser Ala Asp Pro Val Thr Thr Thr Val Glu Asn Tyr Gly
       530                 535                 540
   Gly Glu Thr Gln Ile Gln Arg Arg His His Thr Asp Ile Gly Phe Ile
   545                 550                 555                 560
   Met Asp Arg Phe Val Lys Ile Gln Ser Leu Ser Pro Thr His Val Ile
                   565                 570                 575
   Asp Leu Met Gln Ala His Gln His Gly Leu Val Gly Ala Leu Leu Arg
               580                 585                 590
   Ala Ala Thr Tyr Tyr Phe Ser Asp Leu Glu Ile Val Val Arg His Glu
           595                 600                 605
   Gly Asn Leu Thr Trp Val Pro Asn Gly Ala Pro Glu Ser Ala Leu Leu
       610                 615                 620
   Asn Thr Ser Asn Pro Thr Ala Tyr Asn Lys Ala Pro Phe Thr Arg Leu
   625                 630                 635                 640
   Ala Leu Pro Tyr Thr Ala Pro His Arg Val Leu Ala Thr Val Tyr Asn
                   645                 650                 655
   Gly Thr Ser Lys Tyr Ala Val Gly Gly Ser Gly Arg Arg Gly Asp Met
               660                 665                 670
   Gly Ser Leu Ala Ala Arg Val Val Lys Gln Leu Pro Ala Ser Phe Asn
           675                 680                 685
   Tyr Gly Ala Ile Lys Ala Asp Ala Ile His Glu Leu Leu Val Arg Met
       690                 695                 700
   Lys Arg Ala Glu Leu Tyr Cys Pro Arg Pro Leu Leu Ala Ile Glu Val
   705                 710                 715                 720
   Ser Ser Gln Asp Arg His Lys Gln Lys Ile Ile Ala Pro Ala Lys Gln
                   725                 730                 735
<210> 11
   <211> 639
   <212> DNA
   <213> Artificial Sequence
<220>
   <223> FMDV - 3C DNA optimized for Lemna expression
<400> 11
   tccggtgcgc ctcccaccga cctgcagaag atggtgatgg gcaacaccaa gcccgtcgag 60
   ttgatcctcg acgggaagac cgtggcgatc tgctgcgcca ccggcgtgtt cggcaccgcc 120
   tacctcgtcc cgagacacct gttcgccgag aagtatgaca agatcatgct ggacgggcgg 180
   gccatgaccg actcggacta ccgggtcttc gagttcgaga tcaaagtgaa gggccaggat 240
   atgctctccg acgccgcgct gatggtgctc cacagaggca accgcgtgcg ggacatcacc 300
   aagcacttca gggacaccgc gcgcatgaag aaggggaccc ctgtggtcgg ggtcgtgaac 360
   aacgccgacg tcgggcgcct catcttctcc ggcgaggcgc tgacctacaa ggacatcgtc 420
   gtgtgcatgg acggggacac gatgccgggg ctcttcgcct acaaggccgc gaccaaggcc 480
   gggtactgcg ggggtgcggt gctcgccaag gacggcgccg acacgttcat cgtgggcacc 540
   cactccgccg gcggtaacgg cgtcggctac tgctcctgcg tgtcccggtc catgctgctg 600
   cggatgaagg cccacgttga ccccgagccg cagcacgag 639
<210> 12
   <211> 213
   <212> PRT
   <213> Artificial Sequence
<220>
   <223> FMDV - 3C protein (translation of SEQ ID NOs:11 and 14)
<400> 12
   Ser Gly Ala Pro Pro Thr Asp Leu Gln Lys Met Val Met Gly Asn Thr
   1               5                   10                  15
   Lys Pro Val Glu Leu Ile Leu Asp Gly Lys Thr Val Ala Ile Cys Cys
               20                  25                  30
   Ala Thr Gly Val Phe Gly Thr Ala Tyr Leu Val Pro Arg His Leu Phe
           35                  40                  45
   Ala Glu Lys Tyr Asp Lys Ile Met Leu Asp Gly Arg Ala Met Thr Asp
       50                  55                  60
   Ser Asp Tyr Arg Val Phe Glu Phe Glu Ile Lys Val Lys Gly Gln Asp
   65                  70                  75                  80
   Met Leu Ser Asp Ala Ala Leu Met Val Leu His Arg Gly Asn Arg Val
                   85                  90                  95
   Arg Asp Ile Thr Lys His Phe Arg Asp Thr Ala Arg Met Lys Lys Gly
               100                 105                 110
   Thr Pro Val Val Gly Val Val Asn Asn Ala Asp Val Gly Arg Leu Ile
           115                 120                 125
   Phe Ser Gly Glu Ala Leu Thr Tyr Lys Asp Ile Val Val Cys Met Asp
       130                 135                 140
   Gly Asp Thr Met Pro Gly Leu Phe Ala Tyr Lys Ala Ala Thr Lys Ala
   145                 150                 155                 160
   Gly Tyr Cys Gly Gly Ala Val Leu Ala Lys Asp Gly Ala Asp Thr Phe
                   165                 170                 175
   Ile Val Gly Thr His Ser Ala Gly Gly Asn Gly Val Gly Tyr Cys Ser
               180                 185                 190
   Cys Val Ser Arg Ser Met Leu Leu Arg Met Lys Ala His Val Asp Pro
           195                 200                 205
   Glu Pro Gln His Glu
       210
<210> 13
   <211> 2208
   <212> DNA
   <213> Artificial Sequence
<220>
   <223> FMDV-P1 DNA optimized for mammalian expression
<400> 13
   atgggagctg ggcaatccag cccagcaacc ggctcgcaga accagtctgg caacactggc      60
   agcataatca acaactacta catgcaacag taccagaact ccatggacac acagttggga     120
   gacaatgcca tcagtggagg ctccaacgag ggctccacgg acacaacttc aacacacaca     180
   accaacactc aaaacaatga ctggttctcg aagctcgcca gttcagcttt taccggtctg     240
   ttcggtgcac tgctcgccga caagaagaca gaggaaacga cacttcttga ggaccgcatc     300
   ctcaccaccc gcaacgggca caccacctcg acgacccaat cgagtgtggg tgtcacacac     360
   gggtactcca cagaggagga ccacgttgct gggcccaaca catcgggcct ggagacgcga     420
   gtggtgcagg cagagagatt ctacaaaaag tacttgtttg actggacaac ggacaaggca     480
   tttggacacc tggaaaagct ggagctcccg tccgaccacc acggtgtctt tggacacttg     540
   gtggactcgt acgcctatat gagaaatggc tgggatgttg aggtgtccgc tgttggcaac     600
   cagttcaacg gcgggtgcct cctggtggcc atggtacctg aatggaagga atttgacaca     660
   cgggagaaat accaactcac ccttttcccg caccagttta ttagccccag aactaacatg     720
   actgcccaca tcacggtccc ctaccttggt gtgaacaggt atgatcagta caagaagcat     780
   aagccctgga cattggttgt catggtcgtg tcgccactta cggtcaacaa cactagtgcg     840
   gcacaaatca aggtctacgc caacatagct ccgacctatg ttcacgtggc cggtgaactc     900
   ccctcgaaag aggggatttt cccggttgca tgtgcggacg gttacggagg attggtgacg     960
   acagacccga agacagctga ccctgcttat ggcaaggtgt acaacccgcc taggactaac    1020
   taccctgggc gcttcaccaa cctgttggac gtggccgaag cgtgtcccac tttcctctgc    1080
   tttgacgacg ggaaaccgta cgtcaccacg cggacggatg acacccgact tttggccaag    1140
   tttgaccttt cccttgccgc aaaacatatg tccaacacat acctgtcagg gattgctcag    1200
   tactacacac agtactctgg caccatcaat ttgcatttca tgtttacagg ttccactgat    1260
   tcaaaggccc gatacatggt ggcctacatc ccacctgggg tggagacacc accggacaca    1320
   cctgaaaggg ctgcccactg cattcacgct gaatgggaca ctggactaaa ctccaaattc    1380
   actttctcaa tcccgtacgt atccgccgcg gattacgcgt acacagcgtc tgacacggca    1440
   gaaacaatca acgtacaggg atgggtctgc atctaccaaa ttacacacgg gaaggctgaa    1500
   aatgacacct tggtcgtgtc ggttagcgcc ggcaaagact ttgagttgcg cctcccgatt    1560
   gacccccgcc agcagaccac cgctaccggg gaatcagcag acccggtcac caccaccgtg    1620
   gagaactacg gcggtgagac acaaatccag agacgtcacc acacggacat tggtttcatc 1680
   atggacagat ttgtgaagat ccaaagcttg agcccaacac atgtcattga cctcatgcag 1740
   gctcaccaac acggtctggt gggtgccttg ctgcgtgcag ccacgtacta cttttctgac 1800
   ctggaaattg ttgtacggca cgaaggcaat ctgacctggg tgcccaacgg cgcccctgaa 1860
   tcagccctgt tgaacaccag caaccccact gcctacaaca aggcaccatt cacgagactc 1920
   gctctcccct acactgcgcc gcaccgtgtg ctggcaacag tgtacaacgg gacgagtaag 1980
   tatgctgtgg gtggttcagg cagaagaggc gacatggggt ctctcgcggc gcgagtcgtg 2040
   aaacagcttc ctgcttcatt taactacggt gcaatcaagg ccgacgccat ccacgaactt 2100
   ctcgtgcgca tgaaacgggc cgagctctac tgccccagac cgctgttggc aatagaggtg 2160
   tcttcgcaag acaggcacaa gcaaaagatc attgcaccag caaagcag 2208
<210> 14
   <211> 639
   <212> DNA
   <213> Artificial Sequence
<220>
   <223> FMDV-3C DNA optimized for mammalian expression
<400> 14
   agtggtgccc caccgaccga cttgcaaaag atggtcatgg gcaacacaaa gcctgttgag 60
   ctcatccttg acgggaagac agtagccatc tgttgtgcta ctggagtgtt tggcactgct 120
   tacctcgtgc ctcgtcatct tttcgcagag aagtatgaca agatcatgct ggatggcaga 180
   gccatgacag acagtgacta cagagtgttt gagtttgaga ttaaagtaaa aggacaggac 240
   atgctctcag acgctgcgct catggtgctc caccgtggga accgcgtgag agatatcacg 300
   aaacactttc gtgatacagc aagaatgaag aaaggcaccc ccgtcgtcgg tgtggtcaac 360
   aacgccgacg ttgggagact gattttctct ggtgaggccc tcacctacaa ggatattgta 420
   gtgtgcatgg acggagacac catgcctggc ctctttgcct acaaagccgc caccaaggca 480
   ggctactgtg gaggagccgt tctcgccaag gacggggccg acactttcat cgtcggcact 540
   cactccgcag gaggcaatgg agttggatac tgctcatgcg tttccaggtc catgcttctc 600
   agaatgaagg cacacgttga ccctgaacca caacacgag 639
<210> 15
   <211> 912
   <212> DNA
   <213> Artificial Sequence
<220>
   <223> FMDV - VP0 DNA optimized for Lemna expression
<400> 15
   atgggcgccg ggcagtcctc gcctgcgacg gggagccaga accagtcggg caacaccggc        60
   tcgatcatca acaactacta catgcagcaa taccagaaca gcatggacac ccagctcggc       120
   gataacgcca tctccggcgg gtccaacgag ggctcgaccg acacgacctc cacccacacc       180
   acgaacaccc agaacaatga ctggttcagc aagctggcct ccagcgcctt cacgggcctg       240
   ttcggggcgc tgctcgcgga caagaagacc gaggagacca cgctgctcga ggaccggatc       300
   ctcaccacgc gcaacgggca cacgaccagc acgacccagt ccagcgtcgg ggtgacccac       360
   ggctactcca cggaggagga ccacgtcgcc gggccgaaca cgagcggcct cgagacccgc       420
   gtggtccagg cggagcgctt ctacaagaag tacctgttcg actggaccac ggacaaggcg       480
   ttcgggcacc tcgagaagct ggagctgccg agcgaccacc acggcgtctt cggccacctc       540
   gtggactcct acgcgtacat gagaaatggg tgggacgtcg aggtcagcgc cgtcggcaac       600
   cagttcaacg gcgggtgcct gctcgtggcg atggtcccgg agtggaagga gttcgacacc       660
   cgcgagaagt atcagctgac cctcttcccc caccagttca tcagcccgcg caccaacatg       720
   accgcccaca tcacggtgcc gtacctcggg gtgaaccgct acgaccagta caagaagcac       780
   aagccctgga ccctcgtggt catggtggtc tccccgctga ctgtgaacaa cacgtccgcc       840
   gcgcagatca aagtctacgc caacatcgcc cccacctacg tgcacgtcgc cggcgagctg       900
   ccctccaagg ag                                                           912
<210> 16
   <211> 912
   <212> DNA
   <213> Artificial Sequence
<220>
   <223> FMDV-VP0 DNA optimized for mammalian expression
<400> 16
   atgggagctg ggcaatccag cccagcaacc ggctcgcaga accagtctgg caacactggc      60
   agcataatca acaactacta catgcaacag taccagaact ccatggacac acagttggga     120
   gacaatgcca tcagtggagg ctccaacgag ggctccacgg acacaacttc aacacacaca     180
   accaacactc aaaacaatga ctggttctcg aagctcgcca gttcagcttt taccggtctg     240
   ttcggtgcac tgctcgccga caagaagaca gaggaaacga cacttcttga ggaccgcatc     300
   ctcaccaccc gcaacgggca caccacctcg acgacccaat cgagtgtggg tgtcacacac     360
   gggtactcca cagaggagga ccacgttgct gggcccaaca catcgggcct ggagacgcga     420
   gtggtgcagg cagagagatt ctacaaaaag tacttgtttg actggacaac ggacaaggca     480
   tttggacacc tggaaaagct ggagctcccg tccgaccacc acggtgtctt tggacacttg     540
   gtggactcgt acgcctatat gagaaatggc tgggatgttg aggtgtccgc tgttggcaac     600
   cagttcaacg gcgggtgcct cctggtggcc atggtacctg aatggaagga atttgacaca     660
   cgggagaaat accaactcac ccttttcccg caccagttta ttagccccag aactaacatg     720
   actgcccaca tcacggtccc ctaccttggt gtgaacaggt atgatcagta caagaagcat     780
   aagccctgga cattggttgt catggtcgtg tcgccactta cggtcaacaa cactagtgcg     840
   gcacaaatca aggtctacgc caacatagct ccgacctatg ttcacgtggc cggtgaactc     900
   ccctcgaaag ag                                                         912
<210> 17
   <211> 304
   <212> PRT
   <213> Artificial Sequence
<220>
   <223> FMDV - VP0 protein (translation of SEQ ID NOs:15 and 16)
<400> 17
   Met Gly Ala Gly Gln Ser Ser Pro Ala Thr Gly Ser Gln Asn Gln Ser
   1               5                   10                  15
   Gly Asn Thr Gly Ser Ile Ile Asn Asn Tyr Tyr Met Gln Gln Tyr Gln
               20                  25                  30
   Asn Ser Met Asp Thr Gln Leu Gly Asp Asn Ala Ile Ser Gly Gly Ser
           35                  40                  45
   Asn Glu Gly Ser Thr Asp Thr Thr Ser Thr His Thr Thr Asn Thr Gln
       50                  55                  60
   Asn Asn Asp Trp Phe Ser Lys Leu Ala Ser Ser Ala Phe Thr Gly Leu
   65                  70                  75                  80
   Phe Gly Ala Leu Leu Ala Asp Lys Lys Thr Glu Glu Thr Thr Leu Leu
                   85                  90                  95
   Glu Asp Arg Ile Leu Thr Thr Arg Asn Gly His Thr Thr Ser Thr Thr
               100                 105                 110
   Gln Ser Ser Val Gly Val Thr His Gly Tyr Ser Thr Glu Glu Asp His
           115                 120                 125
   Val Ala Gly Pro Asn Thr Ser Gly Leu Glu Thr Arg Val Val Gln Ala
       130                 135                 140
   Glu Arg Phe Tyr Lys Lys Tyr Leu Phe Asp Trp Thr Thr Asp Lys Ala
   145                 150                 155                 160
   Phe Gly His Leu Glu Lys Leu Glu Leu Pro Ser Asp His His Gly Val
                   165                 170                 175
   Phe Gly His Leu Val Asp Ser Tyr Ala Tyr Met Arg Asn Gly Trp Asp
               180                 185                 190
   Val Glu Val Ser Ala Val Gly Asn Gln Phe Asn Gly Gly Cys Leu Leu
   195 200 205
   Val Ala Met Val Pro Glu Trp Lys Glu Phe Asp Thr Arg Glu Lys Tyr
   210 215 220
   Gln Leu Thr Leu Phe Pro His Gln Phe Ile Ser Pro Arg Thr Asn Met
   225 230 235 240
   Thr Ala His Ile Thr Val Pro Tyr Leu Gly Val Asn Arg Tyr Asp Gln
   245 250 255
   Tyr Lys Lys His Lys Pro Trp Thr Leu Val Val Met Val Val Ser Pro
   260 265 270
   Leu Thr Val Asn Asn Thr Ser Ala Ala Gln Ile Lys Val Tyr Ala Asn
   275 280 285
   Ile Ala Pro Thr Tyr Val His Val Ala Gly Glu Leu Pro Ser Lys Glu
   290 295 300
<210> 18
   <211> 663
   <212> DNA
   <213> Artificial Sequence
<220>
   <223> FMDV - VP3 DNA optimized for Lemna expression
<400> 18
   ggcatcttcc ccgtcgcctg cgccgacggg tacgggggcc tggtgaccac cgaccccaag 60
   acggccgacc cggcctacgg caaagtgtac aacccgccca ggacgaacta ccccggtcgc 120
   ttcaccaacc tcctggacgt ggcggaggcc tgcccgacct tcctgtgctt cgacgacggg 180
   aagccctacg tcaccacgcg caccgacgac acgcgcctgc tcgccaagtt cgacctcagc 240
   ctggccgcta agcacatgag caacacctac ctcagcggca tcgcccaata ctatacccaa 300
   tactcgggca ccatcaacct gcacttcatg ttcacgggca gcaccgacag caaggcccgg 360
   tacatggtgg cctacatccc gccgggcgtg gagacccctc ccgacacgcc cgagcgggct 420
   gcgcactgca tccacgccga gtgggacacc ggcctcaaca gcaagttcac gttcagcatc 480
   ccctacgtgt ccgccgcgga ttacgcttac accgcctcgg acacggccga gacgatcaac 540
   gtccagggct gggtctgcat ctaccagatc actcacggca aggccgagaa cgacaccctc 600
   gtcgtgagcg tctccgccgg gaaggacttc gagctgaggc tgcccatcga ccccaggcag 660
   cag 663
<210> 19
   <211> 663
   <212> DNA
   <213> Artificial Sequence
<220>
   <223> FMDV-VP3 DNA optimized for mammalian expression
<400> 19
   gggattttcc cggttgcatg tgcggacggt tacggaggat tggtgacgac agacccgaag      60
   acagctgacc ctgcttatgg caaggtgtac aacccgccta ggactaacta ccctgggcgc     120
   ttcaccaacc tgttggacgt ggccgaagcg tgtcccactt tcctctgctt tgacgacggg     180
   aaaccgtacg tcaccacgcg gacggatgac acccgacttt tggccaagtt tgacctttcc     240
   cttgccgcaa aacatatgtc caacacatac ctgtcaggga ttgctcagta ctacacacag     300
   tactctggca ccatcaattt gcatttcatg tttacaggtt ccactgattc aaaggcccga     360
   tacatggtgg cctacatccc acctggggtg gagacaccac cggacacacc tgaaagggct     420
   gcccactgca ttcacgctga atgggacact ggactaaact ccaaattcac tttctcaatc     480
   ccgtacgtat ccgccgcgga ttacgcgtac acagcgtctg acacggcaga aacaatcaac     540
   gtacagggat gggtctgcat ctaccaaatt acacacggga aggctgaaaa tgacaccttg     600
   gtcgtgtcgg ttagcgccgg caaagacttt gagttgcgcc tcccgattga cccccgccag     660
   cag                                                                   663
<210> 20
   <211> 221
   <212> PRT
   <213> Artificial Sequence
<220>
   <223> FMDV - VP3 protein (translation of SEQ ID NOs:18 and 19)
<400> 20
   Gly Ile Phe Pro Val Ala Cys Ala Asp Gly Tyr Gly Gly Leu Val Thr
   1               5                   10                  15
   Thr Asp Pro Lys Thr Ala Asp Pro Ala Tyr Gly Lys Val Tyr Asn Pro
               20                  25                  30
   Pro Arg Thr Asn Tyr Pro Gly Arg Phe Thr Asn Leu Leu Asp Val Ala
           35                  40                  45
   Glu Ala Cys Pro Thr Phe Leu Cys Phe Asp Asp Gly Lys Pro Tyr Val
       50                  55                  60
   Thr Thr Arg Thr Asp Asp Thr Arg Leu Leu Ala Lys Phe Asp Leu Ser
   65                  70                  75                  80
   Leu Ala Ala Lys His Met Ser Asn Thr Tyr Leu Ser Gly Ile Ala Gln
                   85                  90                  95
   Tyr Tyr Thr Gln Tyr Ser Gly Thr Ile Asn Leu His Phe Met Phe Thr
               100                 105                 110
   Gly Ser Thr Asp Ser Lys Ala Arg Tyr Met Val Ala Tyr Ile Pro Pro
           115                 120                 125
   Gly Val Glu Thr Pro Pro Asp Thr Pro Glu Arg Ala Ala His Cys Ile
       130                 135                 140
   His Ala Glu Trp Asp Thr Gly Leu Asn Ser Lys Phe Thr Phe Ser Ile
   145                 150                 155                 160
   Pro Tyr Val Ser Ala Ala Asp Tyr Ala Tyr Thr Ala Ser Asp Thr Ala
                   165                 170                 175
   Glu Thr Ile Asn Val Gln Gly Trp Val Cys Ile Tyr Gln Ile Thr His
               180                 185                 190
   Gly Lys Ala Glu Asn Asp Thr Leu Val Val Ser Val Ser Ala Gly Lys
           195                 200                 205
   Asp Phe Glu Leu Arg Leu Pro Ile Asp Pro Arg Gln Gln
       210                 215                 220
<210> 21
   <211> 633
   <212> DNA
   <213> Artificial Sequence
<220>
   <223> FMDV - VP1 DNA optimized for Lemna expression
<400> 21
   accacggcga ccggggagtc cgccgacccc gtgaccacga ccgtggagaa ctacggcggg      60
   gagactcaga tccagcggcg ccaccacacc gacatcggct tcatcatgga ccgcttcgtg     120
   aagatccagt ccctgtcgcc cacccacgtt atcgacctca tgcaggccca ccagcacggg     180
   ctcgtgggtg ccctcctgcg cgcggccacc tactacttca gcgacctcga gatcgttgtc     240
   cgccacgagg ggaacctcac ctgggtcccg aacggtgccc ccgagagcgc cctgctcaac     300
   acctccaacc ccacggcgta caacaaggcc cccttcacgc gcctcgccct gccttacacc     360
   gcgccccacc gcgtgctggc cacggtgtac aacgggacct ccaagtacgc cgtgggcggg     420
   agcggccgca gaggcgacat ggggagcctc gccgctaggg tcgtgaagca gctcccggcc     480
   tccttcaact acggcgccat caaggccgac gccatccacg aactcctggt ccgcatgaag     540
   cgcgccgaac tctactgccc ccggcccctc ctggccatcg aggtctcctc gcaggaccgc     600
   cacaagcaga agatcatcgc cccggccaag cag                                   633
<210> 22
   <211> 633
   <212> DNA
   <213> Artificial Sequence
<220>
   <223> FMDV-VP1 DNA optimized for mammalian expression
<400> 22
   accaccgcta ccggggaatc agcagacccg gtcaccacca ccgtggagaa ctacggcggt      60
   gagacacaaa tccagagacg tcaccacacg gacattggtt tcatcatgga cagatttgtg     120
   aagatccaaa gcttgagccc aacacatgtc attgacctca tgcaggctca ccaacacggt     180
   ctggtgggtg ccttgctgcg tgcagccacg tactactttt ctgacctgga aattgttgta     240
   cggcacgaag gcaatctgac ctgggtgccc aacggcgccc ctgaatcagc cctgttgaac     300
   accagcaacc ccactgccta caacaaggca ccattcacga gactcgctct cccctacact     360
   gcgccgcacc gtgtgctggc aacagtgtac aacgggacga gtaagtatgc tgtgggtggt     420
   tcaggcagaa gaggcgacat ggggtctctc gcggcgcgag tcgtgaaaca gcttcctgct     480
   tcatttaact acggtgcaat caaggccgac gccatccacg aacttctcgt gcgcatgaaa     540
   cgggccgagc tctactgccc cagaccgctg ttggcaatag aggtgtcttc gcaagacagg     600
   cacaagcaaa agatcattgc accagcaaag cag                                   633
<210> 23
   <211> 211
   <212> PRT
   <213> Artificial Sequence
<220>
   <223> FMDV - VP1 protein (translation of SEQ ID NOs:21 and 22)
<400> 23
   Thr Thr Ala Thr Gly Glu Ser Ala Asp Pro Val Thr Thr Thr Val Glu
   1               5                   10                  15
   Asn Tyr Gly Gly Glu Thr Gln Ile Gln Arg Arg His His Thr Asp Ile
               20                  25                  30
   Gly Phe Ile Met Asp Arg Phe Val Lys Ile Gln Ser Leu Ser Pro Thr
           35                  40                  45
   His Val Ile Asp Leu Met Gln Ala His Gln His Gly Leu Val Gly Ala
       50                  55                  60
   Leu Leu Arg Ala Ala Thr Tyr Tyr Phe Ser Asp Leu Glu Ile Val Val
   65                  70                  75                  80
   Arg His Glu Gly Asn Leu Thr Trp Val Pro Asn Gly Ala Pro Glu Ser
                   85                  90                  95
   Ala Leu Leu Asn Thr Ser Asn Pro Thr Ala Tyr Asn Lys Ala Pro Phe
               100                 105                 110
   Thr Arg Leu Ala Leu Pro Tyr Thr Ala Pro His Arg Val Leu Ala Thr
           115                 120                 125
   Val Tyr Asn Gly Thr Ser Lys Tyr Ala Val Gly Gly Ser Gly Arg Arg
       130                 135                 140
   Gly Asp Met Gly Ser Leu Ala Ala Arg Val Val Lys Gln Leu Pro Ala
   145                 150                 155                 160
   Ser Phe Asn Tyr Gly Ala Ile Lys Ala Asp Ala Ile His Glu Leu Leu
                   165                 170                 175
   Val Arg Met Lys Arg Ala Glu Leu Tyr Cys Pro Arg Pro Leu Leu Ala
               180                 185                 190
   Ile Glu Val Ser Ser Gln Asp Arg His Lys Gln Lys Ile Ile Ala Pro
           195                 200                 205
   Ala Lys Gln
       210
<210> 24
   <211> 654
   <212> DNA
   <213> Artificial Sequence
<220>
   <223> FMDV - VP2 DNA optimized for Lemna expression
<400> 24
   gacaagaaga ccgaggagac cacgctgctc gaggaccgga tcctcaccac gcgcaacggg      60
   cacacgacca gcacgaccca gtccagcgtc ggggtgaccc acggctactc cacggaggag     120
   gaccacgtcg ccgggccgaa cacgagcggc ctcgagaccc gcgtggtcca ggcggagcgc     180
   ttctacaaga agtacctgtt cgactggacc acggacaagg cgttcgggca cctcgagaag     240
   ctggagctgc cgagcgacca ccacggcgtc ttcggccacc tcgtggactc ctacgcgtac     300
   atgagaaatg ggtgggacgt cgaggtcagc gccgtcggca accagttcaa cggcgggtgc     360
   ctgctcgtgg cgatggtccc ggagtggaag gagttcgaca cccgcgagaa gtatcagctg     420
   accctcttcc cccaccagtt catcagcccg cgcaccaaca tgaccgccca catcacggtg     480
   ccgtacctcg gggtgaaccg ctacgaccag tacaagaagc acaagccctg gaccctcgtg      540
   gtcatggtgg tctccccgct gactgtgaac aacacgtccg ccgcgcagat caaagtctac     600
   gccaacatcg cccccaccta cgtgcacgtc gccggcgagc tgccctccaa ggag           654
<210> 25
   <211> 654
   <212> DNA
   <213> Artificial Sequence
<220>
   <223> FMDV-VP2 DNA optimized for mammalian expression
<400> 25
   gacaagaaga cagaggaaac gacacttctt gaggaccgca tcctcaccac ccgcaacggg      60
   cacaccacct cgacgaccca atcgagtgtg ggtgtcacac acgggtactc cacagaggag     120
   gaccacgttg ctgggcccaa cacatcgggc ctggagacgc gagtggtgca ggcagagaga     180
   ttctacaaaa agtacttgtt tgactggaca acggacaagg catttggaca cctggaaaag     240
   ctggagctcc cgtccgacca ccacggtgtc tttggacact tggtggactc gtacgcctat     300
   atgagaaatg gctgggatgt tgaggtgtcc gctgttggca accagttcaa cggcgggtgc     360
   ctcctggtgg ccatggtacc tgaatggaag gaatttgaca cacgggagaa ataccaactc     420
   acccttttcc cgcaccagtt tattagcccc agaactaaca tgactgccca catcacggtc      480
   ccctaccttg gtgtgaacag gtatgatcag tacaagaagc ataagccctg gacattggtt     540
   gtcatggtcg tgtcgccact tacggtcaac aacactagtg cggcacaaat caaggtctac     600
   gccaacatag ctccgaccta tgttcacgtg gccggtgaac tcccctcgaa agag           654
<210> 26
   <211> 218
   <212> PRT
   <213> Artificial Sequence
<220>
   <223> FMDV - VP2 protein (translation of SEQ ID NOs:24 and 25)
<400> 26
   Asp Lys Lys Thr Glu Glu Thr Thr Leu Leu Glu Asp Arg Ile Leu Thr
   1               5                   10                  15
   Thr Arg Asn Gly His Thr Thr Ser Thr Thr Gln Ser Ser Val Gly Val
               20                  25                  30
   Thr His Gly Tyr Ser Thr Glu Glu Asp His Val Ala Gly Pro Asn Thr
           35                  40                  45
   Ser Gly Leu Glu Thr Arg Val Val Gln Ala Glu Arg Phe Tyr Lys Lys
       50                  55                  60
   Tyr Leu Phe Asp Trp Thr Thr Asp Lys Ala Phe Gly His Leu Glu Lys
   65                  70                  75                  80
   Leu Glu Leu Pro Ser Asp His His Gly Val Phe Gly His Leu Val Asp
                   85                  90                  95
   Ser Tyr Ala Tyr Met Arg Asn Gly Trp Asp Val Glu Val Ser Ala Val
               100                 105                 110
   Gly Asn Gln Phe Asn Gly Gly Cys Leu Leu Val Ala Met Val Pro Glu
           115                 120                 125
   Trp Lys Glu Phe Asp Thr Arg Glu Lys Tyr Gln Leu Thr Leu Phe Pro
       130                 135                 140
   His Gln Phe Ile Ser Pro Arg Thr Asn Met Thr Ala His Ile Thr Val
   145                 150                 155                 160
   Pro Tyr Leu Gly Val Asn Arg Tyr Asp Gln Tyr Lys Lys His Lys Pro
                   165                 170                 175
   Trp Thr Leu Val Val Met Val Val Ser Pro Leu Thr Val Asn Asn Thr
               180                 185                 190
   Ser Ala Ala Gln Ile Lys Val Tyr Ala Asn Ile Ala Pro Thr Tyr Val
           195                 200                 205
   His Val Ala Gly Glu Leu Pro Ser Lys Glu
       210                 215
<210> 27
   <211> 258
   <212> DNA
   <213> Artificial Sequence
<220>
   <223> FMDV - VP4 DNA optimized for Lemna expression
<400> 27
   atgggcgccg ggcagtcctc gcctgcgacg gggagccaga accagtcggg caacaccggc        60
   tcgatcatca acaactacta catgcagcaa taccagaaca gcatggacac ccagctcggc       120
   gataacgcca tctccggcgg gtccaacgag ggctcgaccg acacgacctc cacccacacc       180
   acgaacaccc agaacaatga ctggttcagc aagctggcct ccagcgcctt cacgggcctg       240
   ttcggggcgc tgctcgcg                                                     258
<210> 28
   <211> 258
   <212> DNA
   <213> Artificial Sequence
<220>
   <223> FMDV-VP4 DNA optimized for mammalian expression
<400> 28
   atgggagctg ggcaatccag cccagcaacc ggctcgcaga accagtctgg caacactggc        60
   agcataatca acaactacta catgcaacag taccagaact ccatggacac acagttggga       120
   gacaatgcca tcagtggagg ctccaacgag ggctccacgg acacaacttc aacacacaca       180
   accaacactc aaaacaatga ctggttctcg aagctcgcca gttcagcttt taccggtctg       240
   ttcggtgcac tgctcgcc                                                     258
<210> 29
   <211> 86
   <212> PRT
   <213> Artificial Sequence
<220>
   <223> FMDV-VP4 protein (translation of SEQ ID NOs:27 and 28)
<400> 29
   Met Gly Ala Gly Gln Ser Ser Pro Ala Thr Gly Ser Gln Asn Gln Ser
   1               5                   10                  15
   Gly Asn Thr Gly Ser Ile Ile Asn Asn Tyr Tyr Met Gln Gln Tyr Gln
               20                  25                  30
   Asn Ser Met Asp Thr Gln Leu Gly Asp Asn Ala Ile Ser Gly Gly Ser
           35                  40                  45
   Asn Glu Gly Ser Thr Asp Thr Thr Ser Thr His Thr Thr Asn Thr Gln
       50                  55                  60
   Asn Asn Asp Trp Phe Ser Lys Leu Ala Ser Ser Ala Phe Thr Gly Leu
   65                  70                  75                  80
   Phe Gly Ala Leu Leu Ala
                   85
<210> 30
   <211> 16478
   <212> DNA
   <213> Artificial Sequence
<220>
   <223> MERF01 plasmid
<400> 30
   gtaccgggcc cccccctcga cggatcaagt gcaaaggtcc gccttgtttc tcctctgtct      60
   cttgatctga ctaatcttgg tttatgattc gttgagtaat tttggggaaa gctagcttcg     120
   tccacagttt ttttttcgat gaacagtgcc gcagtggcgc tgatcttgta tgctatcctg     180
   caatcgtggt gaacttattt cttttatatc cttcactccc atgaaaaggc tagtaatctt     240
   tctcgatgta acatcgtcca gcactgctat taccgtgtgg tccatccgac agtctggctg     300
   aacacatcat acgatattga gcaaagatcg atctatcttc cctgttcttt aatgaaagac     360
   gtcattttca tcagtatgat ctaagaatgt tgcaacttgc aaggaggcgt ttctttcttt     420
   gaatttaact aactcgttga gtggccctgt ttctcggacg taaggccttt gctgctccac     480
   acatgtccat tcgaatttta ccgtgtttag caagggcgaa aagtttgcat cttgatgatt      540
   tagcttgact atgcgattgc tttcctggac ccgtgcagct gcggacggat cccccgctcg      600
   aggtcgacgg tatcgataag cttgatcaga tctgatcgaa ttcaccatgg gggccgggca      660
   gtcgtccccg gccacggggt cccagaacca gtccggcaac accggcagca tcatcaacaa      720
   ctactacatg cagcagtacc agaactccat ggacacccaa ctgggggaca acgccacgag      780
   cggcggcagc aatgagggct ccaccgacac cacgagcacc cacacgacca acacccagaa      840
   caacgactgg ttcagcaagc tcgcgtccag cgccttctcc gggctcttcg gggcgctcct       900
   ggccgacaag aagaccgagg agaccacgct gctcgaggac cgcatcctca ccacgcggaa      960
   cggccacacg acctccacca cgcagtcgag cgtcggggtc acttacggtt acgccacggc     1020
   cgaggacttc gtgagcggcc ccaacacctc cggcctcgag actcgggtcg cccaggccga     1080
   gcgcttcttc aagacgcacc tgttcgactg ggtgacctcc gaccccttcg gccgctgcca     1140
   cctcttggaa ctgcccaccg accacaaggg cgtgtacggg tcgttgacgg acagctacgc     1200
   gtacatgagg aacgggtggg acgtggaggt caccgccgtc ggcaaccagt tcaacggggg     1260
   ctgcctcctg gtggccatgg tccccgaact ctgctcgatc cagaagcggg agctgtacca     1320
   gctgaccctc ttcccgcacc agttcatcaa cccgcgcacc aacatgaccg cgcacatcac     1380
   cgtccccttc gtgggcgtga accgctacga ccagtacaaa gtccacaagc cctggacctt     1440
   ggtggtcatg gtcgtcgccc ccctcaccgt caactcggag ggggcgcccc agatcaaagt      1500
   ctacgccaac atcgccccca cgaacgtgca cgttgccggc gagttcccga gcaaggaggg     1560
   catcttcccg gttgcgtgct ccgacggcta cgggggcctc gtgacgaccg acccgaagac     1620
   cgccgaccct gcctacggca aggtgttcaa cccgcccaga aacatgctcc ctgggagatt     1680
   cacgaacttc ctcgacgtgg ccgaggcctg ccccacgttc ctgcacttcg agggggacgt    1740
   cccttacgtc accacgaaga ctgactcgga tcgcgtgctg gcccagttcg acctgtccct     1800
   cgccgctaag cacatgtcca acaccttcct cgccggcctc gcgcaatact acacccagta     1860
   cagcggcacc atcaacctcc acttcatgtt caccggcccc accgacgcca aggcccgcta     1920
   catgatcgct tacgccccgc ccggcatgga gcccccgaag acccccgagg cggccgcgca     1980
   ctgcatccac gccgagtggg acacggggct caactccaag ttcacgttct ccatccccta     2040
   cctcagcgcc gcggactacg cgtacaccgc cagcgacacc gcggagacca cgaacgtgca     2100
   gggctgggtg tgcctgttcc agatcaccca cgggaaggcc gacggcgacg ccctggtcgt     2160
   gctggcctcg gcggggaagg acttcgaact caggctgccg gtggacgccc gcacccagac     2220
   gactagcgcc ggcgagtccg cggacccggt gaccgcgacc gtcgagaact acgggggcga     2280
   gacccaagtg cagcgcagac agcacacgga cgtgtccttc atcctggatc gcttcgtgaa     2340
   agtgaccccg aaggaccaga tcaacgtgct ggacctgatg cagacgccgg cgcacaccct     2400
   cgtgggggcc ctcctgcgca ccgccacgta ctacttcgcg gacctcgagg tggcggtgaa 2460
   gcacgagggc aacctgacgt gggtgcccaa cggtgcgccg gaggccgcgc tcgacaacac 2520
   cacgaacccc accgcgtacc acaaggcccc cctgacccgg ctcgccctcc cctacaccgc 2580
   cccccacagg gtcctcgcca cggtctacaa cggcaacagc aagtacgggg acgggaccgt 2640
   cgccaacgtg cgcggggacc tccaagtgct cgcccagaag gccgcgcgcg ccctccccac 2700
   tagcttcaac tacggtgcca tcaaggccac gagggtgacg gagctgctct accgcatgaa 2760
   gcgcgccgag acctactgcc cccgccccct gctcgcgatc caccccgacc aggccagaca 2820
   caagcagaag atcgtcgccc ctgtcaagca gctcctgaac ttcgacctcc tgaagctggc 2880
   cggcgacgtg gagagcaatc cgggcccctt cttcttcgcg gacgtgcgga gcaacttctc 2940
   caagctggtg gacaccatca accagatgca ggaggacatg agcacgaagc acgggccgga 3000
   cttcaaccgg ctggtcagcg ccttcgagga gctggccacc ggggtcaagg ccatccgcac 3060
   cggtctcgac gaggccaagc cctggtacaa gctcatcaag ctgctctcgc ggctgtcctg 3120
   catggcggcc gtcgccgctc gctcgaagga ccctgtgctc gtcgccatca tgctggccga 3180
   caccggcctg gagatcctgg actccacctt cgtggtcaag aagatctcgg actcgctgtc 3240
   cagcctcttc cacgttccgg ctccggtgtt ctccttcggt gccccgatcc tcctcgccgg 3300
   cctggtcaag gtggcgtcca gcttcttccg ctccaccccg gaggacctcg agcgcgcgga 3360
   gaagcagtcc ggtgcgcctc ccaccgacct gcagaagatg gtgatgggca acaccaagcc 3420
   cgtcgagttg atcctcgacg ggaagaccgt ggcgatctgc tgcgccaccg gcgtgttcgg 3480
   caccgcctac ctcgtcccga gacacctgtt cgccgagaag tatgacaaga tcatgctgga 3540
   cgggcgggcc atgaccgact cggactaccg ggtcttcgag ttcgagatca aagtgaaggg 3600
   ccaggatatg ctctccgacg ccgcgctgat ggtgctccac agaggcaacc gcgtgcggga 3660
   catcaccaag cacttcaggg acaccgcgcg catgaagaag gggacccctg tggtcggggt 3720
   cgtgaacaac gccgacgtcg ggcgcctcat cttctccggc gaggcgctga cctacaagga 3780
   catcgtcgtg tgcatggacg gggacacgat gccggggctc ttcgcctaca aggccgcgac 3840
   caaggccggg tactgcgggg gtgcggtgct cgccaaggac ggcgccgaca cgttcatcgt 3900
   gggcacccac tccgccggcg gtaacggcgt cggctactgc tcctgcgtgt cccggtccat 3960
   gctgctgcgg atgaaggccc acgttgaccc cgagccgcag cacgagtaag agctcgaatt 4020
   tccccgatcg ttcaaacatt tggcaataaa gtttcttaag attgaatcct gttgccggtc 4080
   ttgcgatgat tatcatataa tttctgttga attacgttaa gcatgtaata attaacatgt 4140
   aatgcatgac gttatttatg agatgggttt ttatgattag agtcccgcaa ttatacattt 4200
   aatacgcgat agaaaacaaa atatagcgcg caaactagga taaattatcg cgcgcggtgt 4260
   catctatgtt actagatcgg gaattaattc agatcggctg agtggctcct tcaacgttgc 4320
   ggttctgtca gttccaaacg taaaacggct tgtcccgcgt catcggcggg ggtcataacg 4380
   tgactccctt aattctccgc tcatgatcag attgtcgttt cccgccttca gtttaaacta 4440
   tcagtgtttg acaggatata ttggcgggta aacctaagag aaaagagcgt ttattagaat 4500
   aatcggatat ttaaaagggc gtgaaaaggt ttatccgttc gtccatttgt atgtgcatgc 4560
   caaccacagg gttccccaga tctggcgccg gccagcgaga cgagcaagat tggccgccgc 4620
   ccgaaacgat ccgacagcgc gcccagcaca ggtgcgcagg caaattgcac caacgcatac 4680
   agcgccagca gaatgccata gtgggcggtg acgtcgttcg agtgaaccag atcgcgcagg 4740
   aggcccggca gcaccggcat aatcaggccg atgccgacag cgtcgagcgc gacagtgctc 4800
   agaattacga tcaggggtat gttgggtttc acgtctggcc tccggaccag cctccgctgg 4860
   tccgattgaa cgcgcggatt ctttatcact gataagttgg tggacatatt atgtttatca 4920
   gtgataaagt gtcaagcatg acaaagttgc agccgaatac agtgatccgt gccgccctgg 4980
   acctgttgaa cgaggtcggc gtagacggtc tgacgacacg caaactggcg gaacggttgg 5040
   gggttcagca gccggcgctt tactggcact tcaggaacaa gcgggcgctg ctcgacgcac 5100
   tggccgaagc catgctggcg gagaatcata cgcattcggt gccgagagcc gacgacgact 5160
   ggcgctcatt tctgatcggg aatgcccgca gcttcaggca ggcgctgctc gcctaccgcg 5220
   atggcgcgcg catccatgcc ggcacgcgac cgggcgcacc gcagatggaa acggccgacg 5280
   cgcagcttcg cttcctctgc gaggcgggtt tttcggccgg ggacgccgtc aatgcgctga 5340
   tgacaatcag ctacttcact gttggggccg tgcttgagga gcaggccggc gacagcgatg 5400
   ccggcgagcg cggcggcacc gttgaacagg ctccgctctc gccgctgttg cgggccgcga 5460
   tagacgcctt cgacgaagcc ggtccggacg cagcgttcga gcagggactc gcggtgattg 5520
   tcgatggatt ggcgaaaagg aggctcgttg tcaggaacgt tgaaggaccg agaaagggtg 5580
   acgattgatc aggaccgctg ccggagcgca acccactcac tacagcagag ccatgtagac 5640
   aacatcccct ccccctttcc accgcgtcag acgcccgtag cagcccgcta cgggcttttt 5700
   catgccctgc cctagcgtcc aagcctcacg gccgcgctcg gcctctctgg cggccttctg 5760
   gcgctcttcc gcttcctcgc tcactgactc gctgcgctcg gtcgttcggc tgcggcgagc 5820
   ggtatcagct cactcaaagg cggtaatacg gttatccaca gaatcagggg ataacgcagg 5880
   aaagaacatg tgagcaaaag gccagcaaaa ggccaggaac cgtaaaaagg ccgcgttgct 5940
   ggcgtttttc cataggctcc gcccccctga cgagcatcac aaaaatcgac gctcaagtca 6000
   gaggtggcga aacccgacag gactataaag ataccaggcg tttccccctg gaagctccct 6060
   cgtgcgctct cctgttccga ccctgccgct taccggatac ctgtccgcct ttctcccttc 6120
   gggaagcgtg gcgcttttcc gctgcataac cctgcttcgg ggtcattata gcgatttttt 6180
   cggtatatcc atcctttttc gcacgatata caggattttg ccaaagggtt cgtgtagact 6240
   ttccttggtg tatccaacgg cgtcagccgg gcaggatagg tgaagtaggc ccacccgcga 6300
   gcgggtgttc cttcttcact gtcccttatt cgcacctggc ggtgctcaac gggaatcctg 6360
   ctctgcgagg ctggccggct accgccggcg taacagatga gggcaagcgg atggctgatg 6420
   aaaccaagcc aaccaggaag ggcagcccac ctatcaaggt gtactgcctt ccagacgaac 6480
   gaagagcgat tgaggaaaag gcggcggcgg ccggcatgag cctgtcggcc tacctgctgg 6540
   ccgtcggcca gggctacaaa atcacgggcg tcgtggacta tgagcacgtc cgcgagctgg 6600
   cccgcatcaa tggcgacctg ggccgcctgg gcggcctgct gaaactctgg ctcaccgacg 6660
   acccgcgcac ggcgcggttc ggtgatgcca cgatcctcgc cctgctggcg aagatcgaag 6720
   agaagcagga cgagcttggc aaggtcatga tgggcgtggt ccgcccgagg gcagagccat 6780
   gactttttta gccgctaaaa cggccggggg gtgcgcgtga ttgccaagca cgtccccatg 6840
   cgctccatca agaagagcga cttcgcggag ctggtgaagt acatcaccga cgagcaaggc 6900
   aagaccgagc gcctttgcga cgctcaccgg gctggttgcc ctcgccgctg ggctggcggc 6960
   cgtctatggc cctgcaaacg cgccagaaac gccgtcgaag ccgtgtgcga gacaccgcgg 7020
   ccgccggcgt tgtggatacc tcgcggaaaa cttggccctc actgacagat gaggggcgga 7080
   cgttgacact tgaggggccg actcacccgg cgcggcgttg acagatgagg ggcaggctcg 7140
   atttcggccg gcgacgtgga gctggccagc ctcgcaaatc ggcgaaaacg cctgatttta 7200
   cgcgagtttc ccacagatga tgtggacaag cctggggata agtgccctgc ggtattgaca 7260
   cttgaggggc gcgactactg acagatgagg ggcgcgatcc ttgacacttg aggggcagag 7320
   tgctgacaga tgaggggcgc acctattgac atttgagggg ctgtccacag gcagaaaatc 7380
   cagcatttgc aagggtttcc gcccgttttt cggccaccgc taacctgtct tttaacctgc 7440
   ttttaaacca atatttataa accttgtttt taaccagggc tgcgccctgt gcgcgtgacc 7500
   gcgcacgccg aaggggggtg cccccccttc tcgaaccctc ccggcccgct aacgcgggcc 7560
   tcccatcccc ccaggggctg cgcccctcgg ccgcgaacgg cctcacccca aaaatggcag 7620
   cgctggcagt ccttgccatt gccgggatcg gggcagtaac gggatgggcg atcagcccga 7680
   gcgcgacgcc cggaagcatt gacgtgccgc aggtgctggc atcgacattc agcgaccagg 7740
   tgccgggcag tgagggcggc ggcctgggtg gcggcctgcc cttcacttcg gccgtcgggg 7800
   cattcacgga cttcatggcg gggccggcaa tttttacctt gggcattctt ggcatagtgg 7860
   tcgcgggtgc cgtgctcgtg ttcgggggtg cgataaaccc agcgaaccat ttgaggtgat 7920
   aggtaagatt ataccgaggt atgaaaacga gaattggacc tttacagaat tactctatga 7980
   agcgccatat ttaaaaagct accaagacga agaggatgaa gaggatgagg aggcagattg 8040
   ccttgaatat attgacaata ctgataagat aatatatctt ttatatagaa gatatcgccg 8100
   tatgtaagga tttcaggggg caaggcatag gcagcgcgct tatcaatata tctatagaat 8160
   gggcaaagca taaaaacttg catggactaa tgcttgaaac ccaggacaat aaccttatag 8220
   cttgtaaatt ctatcataat tgggtaatga ctccaactta ttgatagtgt tttatgttca 8280
   gataatgccc gatgactttg tcatgcagct ccaccgattt tgagaacgac agcgacttcc 8340
   gtcccagccg tgccaggtgc tgcctcagat tcaggttatg ccgctcaatt cgctgcgtat 8400
   atcgcttgct gattacgtgc agctttccct tcaggcggga ttcatacagc ggccagccat 8460
   ccgtcatcca tatcaccacg tcaaagggtg acagcaggct cataagacgc cccagcgtcg 8520
   ccatagtgcg ttcaccgaat acgtgcgcaa caaccgtctt ccggagactg tcatacgcgt 8580
   aaaacagcca gcgctggcgc gatttagccc cgacatagcc ccactgttcg tccatttccg 8640
   cgcagacgat gacgtcactg cccggctgta tgcgcgaggt taccgactgc ggcctgagtt 8700
   ttttaagtga cgtaaaatcg tgttgaggcc aacgcccata atgcgggctg ttgcccggca 8760
   tccaacgcca ttcatggcca tatcaatgat tttctggtgc gtaccgggtt gagaagcggt 8820
   gtaagtgaac tgcagttgcc atgttttacg gcagtgagag cagagatagc gctgatgtcc 8880
   ggcggtgctt ttgccgttac gcaccacccc gtcagtagct gaacaggagg gacagctgat 8940
   agacacagaa gccactggag cacctcaaaa acaccatcat acactaaatc agtaagttgg 9000
   cagcatcacc cataattgtg gtttcaaaat cggctccgtc gatactatgt tatacgccaa 9060
   ctttgaaaac aactttgaaa aagctgtttt ctggtattta aggttttaga atgcaaggaa 9120
   cagtgaattg gagttcgtct tgttataatt agcttcttgg ggtatcttta aatactgtag 9180
   aaaagaggaa ggaaataata aatggctaaa atgagaatat caccggaatt gaaaaaactg 9240
   atcgaaaaat accgctgcgt aaaagatacg gaaggaatgt ctcctgctaa ggtatataag 9300
   ctggtgggag aaaatgaaaa cctatattta aaaatgacgg acagccggta taaagggacc 9360
   acctatgatg tggaacggga aaaggacatg atgctatggc tggaaggaaa gctgcctgtt 9420
   ccaaaggtcc tgcactttga acggcatgat ggctggagca atctgctcat gagtgaggcc 9480
   gatggcgtcc tttgctcgga agagtatgaa gatgaacaaa gccctgaaaa gattatcgag 9540
   ctgtatgcgg agtgcatcag gctctttcac tccatcgaca tatcggattg tccctatacg 9600
   aatagcttag acagccgctt agccgaattg gattacttac tgaataacga tctggccgat 9660
   gtggattgcg aaaactggga agaagacact ccatttaaag atccgcgcga gctgtatgat 9720
   tttttaaaga cggaaaagcc cgaagaggaa cttgtctttt cccacggcga cctgggagac 9780
   agcaacatct ttgtgaaaga tggcaaagta agtggcttta ttgatcttgg gagaagcggc 9840
   agggcggaca agtggtatga cattgccttc tgcgtccggt cgatcaggga ggatatcggg 9900
   gaagaacagt atgtcgagct attttttgac ttactgggga tcaagcctga ttgggagaaa 9960
   ataaaatatt atattttact ggatgaattg ttttagtacc tagatgtggc gcaacgatgc 10020
   cggcgacaag caggagcgca ccgacttctt ccgcatcaag tgttttggct ctcaggccga 10080
   ggcccacggc aagtatttgg gcaaggggtc gctggtattc gtgcagggca agattcggaa 10140
   taccaagtac gagaaggacg gccagacggt ctacgggacc gacttcattg ccgataaggt 10200
   ggattatctg gacaccaagg caccaggcgg gtcaaatcag gaataagggc acattgcccc 10260
   ggcgtgagtc ggggcaatcc cgcaaggagg gtgaatgaat cggacgtttg accggaaggc 10320
   atacaggcaa gaactgatcg acgcggggtt ttccgccgag gatgccgaaa ccatcgcaag 10380
   ccgcaccgtc atgcgtgcgc cccgcgaaac cttccagtcc gtcggctcga tggtccagca 10440
   agctacggcc aagatcgagc gcgacagcgt gcaactggct ccccctgccc tgcccgcgcc 10500
   atcggccgcc gtggagcgtt cgcgtcgtct cgaacaggag gcggcaggtt tggcgaagtc 10560
   gatgaccatc gacacgcgag gaactatgac gaccaagaag cgaaaaaccg ccggcgagga 10620
   cctggcaaaa caggtcagcg aggccaagca ggccgcgttg ctgaaacaca cgaagcagca 10680
   gatcaaggaa atgcagcttt ccttgttcga tattgcgccg tggccggaca cgatgcgagc 10740
   gatgccaaac gacacggccc gctctgccct gttcaccacg cgcaacaaga aaatcccgcg 10800
   cgaggcgctg caaaacaagg tcattttcca cgtcaacaag gacgtgaaga tcacctacac 10860
   cggcgtcgag ctgcgggccg acgatgacga actggtgtgg cagcaggtgt tggagtacgc 10920
   gaagcgcacc cctatcggcg agccgatcac cttcacgttc tacgagcttt gccaggacct 10980
   gggctggtcg atcaatggcc ggtattacac gaaggccgag gaatgcctgt cgcgcctaca 11040
   ggcgacggcg atgggcttca cgtccgaccg cgttgggcac ctggaatcgg tgtcgctgct 11100
   gcaccgcttc cgcgtcctgg accgtggcaa gaaaacgtcc cgttgccagg tcctgatcga 11160
   cgaggaaatc gtcgtgctgt ttgctggcga ccactacacg aaattcatat gggagaagta 11220
   ccgcaagctg tcgccgacgg cccgacggat gttcgactat ttcagctcgc accgggagcc 11280
   gtacccgctc aagctggaaa ccttccgcct catgtgcgga tcggattcca cccgcgtgaa 11340
   gaagtggcgc gagcaggtcg gcgaagcctg cgaagagttg cgaggcagcg gcctggtgga 11400
   acacgcctgg gtcaatgatg acctggtgca ttgcaaacgc tagggccttg tggggtcagt 11460
   tccggctggg ggttcagcag ccagcgcttt actggcattt caggaacaag cgggcactgc 11520
   tcgacgcact tgcttcgctc agtatcgctc gggacgcacg gcgcgctcta cgaactgccg 11580
   ataaacagag gattaaaatt gacaattgtg attaaggctc agattcgacg gcttggagcg 11640
   gccgacgtgc aggatttccg cgagatccga ttgtcggccc tgaagaaagc tccagagatg 11700
   ttcgggtccg tttacgagca cgaggagaaa aagcccatgg aggcgttcgc tgaacggttg 11760
   cgagatgccg tggcattcgg cgcctacatc gacggcgaga tcattgggct gtcggtcttc 11820
   aaacaggagg acggccccaa ggacgctcac aaggcgcatc tgtccggcgt tttcgtggag 11880
   cccgaacagc gaggccgagg ggtcgccggt atgctgctgc gggcgttgcc ggcgggttta 11940
   ttgctcgtga tgatcgtccg acagattcca acgggaatct ggtggatgcg catcttcatc 12000
   ctcggcgcac ttaatatttc gctattctgg agcttgttgt ttatttcggt ctaccgcctg 12060
   ccgggcgggg tcgcggcgac ggtaggcgct gtgcagccgc tgatggtcgt gttcatctct 12120
   gccgctctgc taggtagccc gatacgattg atggcggtcc tgggggctat ttgcggaact 12180
   gcgggcgtgg cgctgttggt gttgacacca aacgcagcgc tagatcctgt cggcgtcgca 12240
   gcgggcctgg cgggggcggt ttccatggcg ttcggaaccg tgctgacccg caagtggcaa 12300
   cctcccgtgc ctctgctcac ctttaccgcc tggcaactgg cggccggagg acttctgctc 12360
   gttccagtag ctttagtgtt tgatccgcca atcccgatgc ctacaggaac caatgttctc 12420
   ggcctggcgt ggctcggcct gatcggagcg ggtttaacct acttcctttg gttccggggg 12480
   atctcgcgac tcgaacctac agttgtttcc ttactgggct ttctcagccc cagatctggg 12540
   gtcgatcagc cggggatgca tcaggccgac agtcggaact tcgggtcccc gacctgtacc 12600
   attcggtgag caatggatag gggagttgat atcgtcaacg ttcacttcta aagaaatagc 12660
   gccactcagc ttcctcagcg gctttatcca gcgatttcct attatgtcgg catagttctc 12720
   aagatcgaca gcctgtcacg gttaagcgag aaatgaataa gaaggctgat aattcggatc 12780
   tctgcgaggg agatgatatt tgatcacagg cagcaacgct ctgtcatcgt tacaatcaac 12840
   atgctaccct ccgcgagatc atccgtgttt caaacccggc agcttagttg ccgttcttcc 12900
   gaatagcatc ggtaacatga gcaaagtctg ccgccttaca acggctctcc cgctgacgcc 12960
   gtcccggact gatgggctgc ctgtatcgag tggtgatttt gtgccgagct gccggtcggg 13020
   gagctgttgg ctggctggtg gcaggatata ttgtggtgta aacaaattga cgcttagaca 13080
   acttaataac acattgcgga cgtttttaat gtactggggt ggtttttctt ttcaccagtg 13140
   agacgggcaa cagctgattg cccttcaccg cctggccctg agagagttgc agcaagcggt 13200
   ccacgctggt ttgccccagc aggcgaaaat cctgtttgat ggtggttccg aaatcggcaa 13260
   aatcccttat aaatcaaaag aatagcccga gatagggttg agtgttgttc cagtttggaa 13320
   caagagtcca ctattaaaga acgtggactc caacgtcaaa gggcgaaaaa ccgtctatca 13380
   gggcgatggc ccactacgtg aaccatcacc caaatcaagt tttttggggt cgaggtgccg 13440
   taaagcacta aatcggaacc ctaaagggag cccccgattt agagcttgac ggggaaagcc 13500
   ggcgaacgtg gcgagaaagg aagggaagaa agcgaaagga gcgggcgcca ttcaggctgc 13560
   gcaactgttg ggaagggcga tcggtgcggg cctcttcgct attacgccag ctggcgaaag 13620
   ggggatgtgc tgcaaggcga ttaagttggg taacgccagg gttttcccag tcacgacgtt 13680
   gtaaaacgac ggccagtgaa ttgccatctt gaaagaaata tagtttaaat atttattgat 13740
   aaaataagtc aggtattata gtccaagcaa aaacataatt tattgatgca aagtttaaat 13800
   tcagaaatat ttcaataact gattatatca gctggtacat tgccgtagat gaaagactga 13860
   gtgcgatatt atgtgtaata cataaattga tgatatagct agcttagctc atcgggggat 13920
   ccttaatcga ctctagctag aacgaattgt taggtggcgg tacttgggtc gatatcaaag 13980
   tgcatcactt cttcccgtat gcccaacttt gtatagagag ccactgcggg atcgtcaccg 14040
   taatctgctt gcacgtagat cacataagca ccaagcgcgt tggcctcatg cttgaggaga 14100
   ttgatgagcg cggtggcaat gccctgcctc cggtgctcgc cggagactgc gagatcatag 14160
   atatagatct cactacgcgg ctgctcaaac ctgggcagaa cgtaagccgc gagagcgcca 14220
   acaaccgctt cttggtcgaa ggcagcaagc gcgatgaatg tcttactacg gagcaagttc 14280
   ccgaggtaat cggagtccgg ctgatgttgg gagtaggtgg ctacgtctcc gaactcacga 14340
   ccgaaaagat caagagcagc ccgcatggat ttgacttggt cagggccgag cctacatgtg 14400
   cgaatgatgc ccatacttga gccacctaac tttgttttag ggcgactgcc ctgctgcgta 14460
   acatcgttgc tgctgcgtac catggagatc tggattgaga gtgaatatga gactctaatt 14520
   ggataccgag gggaatttat ggaagtcagt ggagcatttt tgacaagaaa tatttgctag 14580
   ctgatagtga ccttaggcga cttttgaacg cgcaataatg gtttctgacg tatgtgctta 14640
   gctcattaaa ctccagaaac ccgcggctga gtggctcctt caacgttgcg gttctgtcag 14700
   ttccaaacgt aaaacggctt gtcccgcgtc atcggcgggg gtcataacgt gactccctta 14760
   attctccgct catgatcttg atcccctgcg ccatcagatc cttggcggca agaaagccat 14820
   ccagtttact ttgcagggct tcccaacctt accagagggc gccccagctg gcaattccgg 14880
   ttcgcttgct gtccataaaa ccgcccagtc tagctatcgc catgtaagcc cactgcaagc 14940
   tacctgcttt ctctttgcgc ttgcgttttc ccttgtccag atagcccagt agctgacatt 15000
   catccggggt cagcaccgtt tctgcggact ggctttctac gtgttccgct tcctttagca 15060
   gcccttgcgc cctgagtgct tgcggcagcg tgaagctctg gacatcatgt tggatatgaa 15120
   acaactatta tttatctaca tgttttagat gttatctgat tatttttata ccgtagtctt 15180
   ctattgatga ggagtctaag gctatagaat tatatatcta aatgattaat atatatatta 15240
   ttaataatta acaataatta atatattata atttatatat atatatttta tattattata 15300
   ataatattct tacaaatata attattatat tcgacggtat cgataagctc gggatccctg 15360
   aaagcgacgt tggatgttaa catctacaaa ttgccttttc ttatcgacca tgtacgtaag 15420
   cgcttacgtt tttggtggac ccttgaggaa actggtagct gttgtgggcc tgtggtctca 15480
   agatggatca ttaatttcca ccttcaccta cgatgggggg catcgcaccg gtgagtaata 15540
   ttgtacggct aagagcgaat ttggcctgta ggatccctga aagcgacgtt ggatgttaac 15600
   atctacaaat tgccttttct tatcgaccat gtacgtaagc gcttacgttt ttggtggacc 15660
   cttgaggaaa ctggtagctg ttgtgggcct gtggtctcaa gatggatcat taatttccac 15720
   cttcacctac gatggggggc atcgcaccgg tgagtaatat tgtacggcta agagcgaatt 15780
   tggcctgtag gatccctgaa agcgacgttg gatgttaaca tctacaaatt gccttttctt 15840
   atcgaccatg tacgtaagcg cttacgtttt tggtggaccc ttgaggaaac tggtagctgt 15900
   tgtgggcctg tggtctcaag atggatcatt aatttccacc ttcacctacg atggggggca 15960
   tcgcaccggt gagtaatatt gtacggctaa gagcgaattt ggcctgtagg atccgcgagc 16020
   tggtcaatcc cattgctttt gaagcagctc aacattgatc tctttctcga tcgagggaga 16080
   tttttcaaat cagtgcgcaa gacgtgacgt aagtatccga gtcagttttt atttttctac    16140
   taatttggtc gtttatttcg gcgtgtagga catggcaacc gggcctgaat ttcgcgggta    16200
   ttctgtttct attccaactt tttcttgatc cgcagccatt aacgactttt gaatagatac    16260
   gctgacacgc caagcctcgc tagtcaaaag tgtaccaaac aacgctttac agcaagaacg    16320
   gaatgcgcgt gacgctcgcg gtgacgccat ttcgcctttt cagaaatgga taaatagcct    16380
   tgcttcctat tatatcttcc cccaaattaa ttaagaaact cccgaggtga gcaaggatcc    16440
   ggagtcgagc gcgaagaaga gaaagaggga aagcgcgg                            16478
<210> 31
   <211> 16821
   <212> DNA
   <213> Artificial Sequence
<220>
   <223> MERF02 plasmid
<400> 31
   gtaccgggcc cccccctcga cggatcaagt gcaaaggtcc gccttgtttc tcctctgtct 60
   cttgatctga ctaatcttgg tttatgattc gttgagtaat tttggggaaa gctagcttcg 120
   tccacagttt ttttttcgat gaacagtgcc gcagtggcgc tgatcttgta tgctatcctg 180
   caatcgtggt gaacttattt cttttatatc cttcactccc atgaaaaggc tagtaatctt 240
   tctcgatgta acatcgtcca gcactgctat taccgtgtgg tccatccgac agtctggctg 300
   aacacatcat acgatattga gcaaagatcg atctatcttc cctgttcttt aatgaaagac 360
   gtcattttca tcagtatgat ctaagaatgt tgcaacttgc aaggaggcgt ttctttcttt 420
   gaatttaact aactcgttga gtggccctgt ttctcggacg taaggccttt gctgctccac 480
   acatgtccat tcgaatttta ccgtgtttag caagggcgaa aagtttgcat cttgatgatt 540
   tagcttgact atgcgattgc tttcctggac ccgtgcagct gcggacggat cccccgctcg 600
   aggtcgacgg tatcgataag cttgatcaga tctgatcgaa ttcaccatgg gggccgggca 660
   gtcgtccccg gccacggggt cccagaacca gtccggcaac accggcagca tcatcaacaa 720
   ctactacatg cagcagtacc agaactccat ggacacccaa ctgggggaca acgccacgag 780
   cggcggcagc aatgagggct ccaccgacac cacgagcacc cacacgacca acacccagaa 840
   caacgactgg ttcagcaagc tcgcgtccag cgccttctcc gggctcttcg gggcgctcct 900
   ggccgacaag aagaccgagg agaccacgct gctcgaggac cgcatcctca ccacgcggaa 960
   cggccacacg acctccacca cgcagtcgag cgtcggggtc acttacggtt acgccacggc 1020
   cgaggacttc gtgagcggcc ccaacacctc cggcctcgag actcgggtcg cccaggccga 1080
   gcgcttcttc aagacgcacc tgttcgactg ggtgacctcc gaccccttcg gccgctgcca 1140
   cctcttggaa ctgcccaccg accacaaggg cgtgtacggg tcgttgacgg acagctacgc 1200
   gtacatgagg aacgggtggg acgtggaggt caccgccgtc ggcaaccagt tcaacggggg 1260
   ctgcctcctg gtggccatgg tccccgaact ctgctcgatc cagaagcggg agctgtacca 1320
   gctgaccctc ttcccgcacc agttcatcaa cccgcgcacc aacatgaccg cgcacatcac 1380
   cgtccccttc gtgggcgtga accgctacga ccagtacaaa gtccacaagc cctggacctt 1440
   ggtggtcatg gtcgtcgccc ccctcaccgt caactcggag gggcgccccc agatcaaagt 1500
   ctacgccaac atcgccccca cgaacgtgca cgttgccggc gagttcccga gcaaggaggg 1560
   catcttcccg gttgcgtgct ccgacggcta cgggggcctc gtgacgaccg acccgaagac 1620
   cgccgaccct gcctacggca aggtgttcaa cccgcccaga aacatgctcc ctgggagatt 1680
   cacgaacttc ctcgacgtgg ccgaggcctg ccccacgttc ctgcacttcg agggggacgt 1740
   cccttacgtc accacgaaga ctgactcgga tcgcgtgctg gcccagttcg acctgtccct 1800
   cgccgctaag cacatgtcca acaccttcct cgccggcctc gcgcaatact acacccagta 1860
   cagcggcacc atcaacctcc acttcatgtt caccggcccc accgacgcca aggcccgcta 1920
   catgatcgct tacgccccgc ccggcatgga gcccccgaag acccccgagg cggccgcgca 1980
   ctgcatccac gccgagtggg acacggggct caactccaag ttcacgttct ccatccccta 2040
   cctcagcgcc gcggactacg cgtacaccgc cagcgacacc gcggagacca cgaacgtgca 2100
   gggctgggtg tgcctgttcc agatcaccca cgggaaggcc gacggcgacg ccctggtcgt 2160
   gctggcctcg gcggggaagg acttcgaact caggctgccg gtggacgccc gcacccagac 2220
   gactagcgcc ggcgagtccg cggacccggt gaccgcgacc gtcgagaact acgggggcga 2280
   gacccaagtg cagcgcagac agcacacgga cgtgtccttc atcctggatc gcttcgtgaa 2340
   agtgaccccg aaggaccaga tcaacgtgct ggacctgatg cagacgccgg cgcacaccct 2400
   cgtgggggcc ctcctgcgca ccgccacgta ctacttcgcg gacctcgagg tggcggtgaa 2460
   gcacgagggc aacctgacgt gggtgcccaa cggtgcgccg gaggccgcgc tcgacaacac 2520
   cacgaacccc accgcgtacc acaaggcccc cctgacccgg ctcgccctcc cctacaccgc 2580
   cccccacagg gtcctcgcca cggtctacaa cggcaacagc aagtacgggg acgggaccgt 2640
   cgccaacgtg cgcggggacc tccaagtgct cgcccagaag gccgcgcgcg ccctccccac 2700
   tagcttcaac tacggtgcca tcaaggccac gagggtgacg gagctgctct accgcatgaa 2760
   gcgcgccgag acctactgcc cccgccccct gctcgcgatc caccccgacc aggccagaca 2820
   caagcagaag atcgtcgccc ctgtcaagca gctcctgaac ttcgacctcc tgaagctggc 2880
   cggcgacgtg gagagcaatc cgggcccctt cttcttcgcg gacgtgcgga gcaacttctc 2940
   caagctggtg gacaccatca accagatgca ggaggacatg agcacgaagc acgggccgga 3000
   cttcaaccgg ctggtcagcg ccttcgagga gctggccacc ggggtcaagg ccatccgcac 3060
   cggtctcgac gaggccaagc cctggtacaa gctcatcaag ctgctctcgc ggctgtcctg 3120
   catggcggcc gtcgccgctc gctcgaagga ccctgtgctc gtcgccatca tgctggccga 3180
   caccggcctg gagatcctgg actccacctt cgtggtcaag aagatctcgg actcgctgtc 3240
   cagcctcttc cacgttccgg ctccggtgtt ctccttcggt gccccgatcc tcctcgccgg 3300
   cctggtcaag gtggcgtcca gcttcttccg ctccaccccg gaggacctcg agcgcgcgga 3360
   gaagcagtcc ggtgcgcctc ccaccgacct gcagaagatg gtgatgggca acaccaagcc 3420
   cgtcgagttg atcctcgacg ggaagaccgt ggcgatctgc tgcgccaccg gcgtgttcgg 3480
   caccgcctac ctcgtcccga gacacctgtt cgccgagaag tatgacaaga tcatgctgga 3540
   cgggcgggcc atgaccgact cggactaccg ggtcttcgag ttcgagatca aagtgaaggg 3600
   ccaggatatg ctctccgacg ccgcgctgat ggtgctccac agaggcaacc gcgtgcggga 3660
   catcaccaag cacttcaggg acaccgcgcg catgaagaag gggacccctg tggtcggggt 3720
   cgtgaacaac gccgacgtcg ggcgcctcat cttctccggc gaggcgctga cctacaagga 3780
   catcgtcgtg tgcatggacg gggacacgat gccggggctc ttcgcctaca aggccgcgac 3840
   caaggccggg tactgcgggg gtgcggtgct cgccaaggac ggcgccgaca cgttcatcgt 3900
   gggcacccac tccgccggcg gtaacggcgt cggctactgc tcctgcgtgt cccggtccat 3960
   gctgctgcgg atgaaggccc acgttgaccc cgagccgcag cacgagtaag agctcgaatt 4020
   tccccgatcg ttcaaacatt tggcaataaa gtttcttaag attgaatcct gttgccggtc 4080
   ttgcgatgat tatcatataa tttctgttga attacgttaa gcatgtaata attaacatgt 4140
   aatgcatgac gttatttatg agatgggttt ttatgattag agtcccgcaa ttatacattt 4200
   aatacgcgat agaaaacaaa atatagcgcg caaactagga taaattatcg cgcgcggtgt 4260
   catctatgtt actagatcgg gaattaattc agatcggctg agtggctcct tcaacgttgc 4320
   ggttctgtca gttccaaacg taaaacggct tgtcccgcgt catcggcggg ggtcataacg 4380
   tgactccctt aattctccgc tcatgatcag attgtcgttt cccgccttca gtttaaacta 4440
   tcagtgtttg acaggatata ttggcgggta aacctaagag aaaagagcgt ttattagaat 4500
   aatcggatat ttaaaagggc gtgaaaaggt ttatccgttc gtccatttgt atgtgcatgc 4560
   caaccacagg gttccccaga tctggcgccg gccagcgaga cgagcaagat tggccgccgc 4620
   ccgaaacgat ccgacagcgc gcccagcaca ggtgcgcagg caaattgcac caacgcatac 4680
   agcgccagca gaatgccata gtgggcggtg acgtcgttcg agtgaaccag atcgcgcagg 4740
   aggcccggca gcaccggcat aatcaggccg atgccgacag cgtcgagcgc gacagtgctc 4800
   agaattacga tcaggggtat gttgggtttc acgtctggcc tccggaccag cctccgctgg 4860
   tccgattgaa cgcgcggatt ctttatcact gataagttgg tggacatatt atgtttatca 4920
   gtgataaagt gtcaagcatg acaaagttgc agccgaatac agtgatccgt gccgccctgg 4980
   acctgttgaa cgaggtcggc gtagacggtc tgacgacacg caaactggcg gaacggttgg 5040
   gggttcagca gccggcgctt tactggcact tcaggaacaa gcgggcgctg ctcgacgcac 5100
   tggccgaagc catgctggcg gagaatcata cgcattcggt gccgagagcc gacgacgact 5160
   ggcgctcatt tctgatcggg aatgcccgca gcttcaggca ggcgctgctc gcctaccgcg 5220
   atggcgcgcg catccatgcc ggcacgcgac cgggcgcacc gcagatggaa acggccgacg 5280
   cgcagcttcg cttcctctgc gaggcgggtt tttcggccgg ggacgccgtc aatgcgctga 5340
   tgacaatcag ctacttcact gttggggccg tgcttgagga gcaggccggc gacagcgatg 5400
   ccggcgagcg cggcggcacc gttgaacagg ctccgctctc gccgctgttg cgggccgcga 5460
   tagacgcctt cgacgaagcc ggtccggacg cagcgttcga gcagggactc gcggtgattg 5520
   tcgatggatt ggcgaaaagg aggctcgttg tcaggaacgt tgaaggaccg agaaagggtg 5580
   acgattgatc aggaccgctg ccggagcgca acccactcac tacagcagag ccatgtagac 5640
   aacatcccct ccccctttcc accgcgtcag acgcccgtag cagcccgcta cgggcttttt 5700
   catgccctgc cctagcgtcc aagcctcacg gccgcgctcg gcctctctgg cggccttctg 5760
   gcgctcttcc gcttcctcgc tcactgactc gctgcgctcg gtcgttcggc tgcggcgagc 5820
   ggtatcagct cactcaaagg cggtaatacg gttatccaca gaatcagggg ataacgcagg 5880
   aaagaacatg tgagcaaaag gccagcaaaa ggccaggaac cgtaaaaagg ccgcgttgct 5940
   ggcgtttttc cataggctcc gcccccctga cgagcatcac aaaaatcgac gctcaagtca 6000
   gaggtggcga aacccgacag gactataaag ataccaggcg tttccccctg gaagctccct 6060
   cgtgcgctct cctgttccga ccctgccgct taccggatac ctgtccgcct ttctcccttc 6120
   gggaagcgtg gcgcttttcc gctgcataac cctgcttcgg ggtcattata gcgatttttt 6180
   cggtatatcc atcctttttc gcacgatata caggattttg ccaaagggtt cgtgtagact 6240
   ttccttggtg tatccaacgg cgtcagccgg gcaggatagg tgaagtaggc ccacccgcga 6300
   gcgggtgttc cttcttcact gtcccttatt cgcacctggc ggtgctcaac gggaatcctg 6360
   ctctgcgagg ctggccggct accgccggcg taacagatga gggcaagcgg atggctgatg 6420
   aaaccaagcc aaccaggaag ggcagcccac ctatcaaggt gtactgcctt ccagacgaac 6480
   gaagagcgat tgaggaaaag gcggcggcgg ccggcatgag cctgtcggcc tacctgctgg 6540
   ccgtcggcca gggctacaaa atcacgggcg tcgtggacta tgagcacgtc cgcgagctgg 6600
   cccgcatcaa tggcgacctg ggccgcctgg gcggcctgct gaaactctgg ctcaccgacg 6660
   acccgcgcac ggcgcggttc ggtgatgcca cgatcctcgc cctgctggcg aagatcgaag 6720
   agaagcagga cgagcttggc aaggtcatga tgggcgtggt ccgcccgagg gcagagccat 6780
   gactttttta gccgctaaaa cggccggggg gtgcgcgtga ttgccaagca cgtccccatg 6840
   cgctccatca agaagagcga cttcgcggag ctggtgaagt acatcaccga cgagcaaggc 6900
   aagaccgagc gcctttgcga cgctcaccgg gctggttgcc ctcgccgctg ggctggcggc 6960
   cgtctatggc cctgcaaacg cgccagaaac gccgtcgaag ccgtgtgcga gacaccgcgg 7020
   ccgccggcgt tgtggatacc tcgcggaaaa cttggccctc actgacagat gaggggcgga 7080
   cgttgacact tgaggggccg actcacccgg cgcggcgttg acagatgagg ggcaggctcg 7140
   atttcggccg gcgacgtgga gctggccagc ctcgcaaatc ggcgaaaacg cctgatttta 7200
   cgcgagtttc ccacagatga tgtggacaag cctggggata agtgccctgc ggtattgaca 7260
   cttgaggggc gcgactactg acagatgagg ggcgcgatcc ttgacacttg aggggcagag 7320
   tgctgacaga tgaggggcgc acctattgac atttgagggg ctgtccacag gcagaaaatc 7380
   cagcatttgc aagggtttcc gcccgttttt cggccaccgc taacctgtct tttaacctgc 7440
   ttttaaacca atatttataa accttgtttt taaccagggc tgcgccctgt gcgcgtgacc 7500
   gcgcacgccg aaggggggtg cccccccttc tcgaaccctc ccggcccgct aacgcgggcc 7560
   tcccatcccc ccaggggctg cgcccctcgg ccgcgaacgg cctcacccca aaaatggcag 7620
   cgctggcagt ccttgccatt gccgggatcg gggcagtaac gggatgggcg atcagcccga 7680
   gcgcgacgcc cggaagcatt gacgtgccgc aggtgctggc atcgacattc agcgaccagg 7740
   tgccgggcag tgagggcggc ggcctgggtg gcggcctgcc cttcacttcg gccgtcgggg 7800
   cattcacgga cttcatggcg gggccggcaa tttttacctt gggcattctt ggcatagtgg 7860
   tcgcgggtgc cgtgctcgtg ttcgggggtg cgataaaccc agcgaaccat ttgaggtgat 7920
   aggtaagatt ataccgaggt atgaaaacga gaattggacc tttacagaat tactctatga 7980
   agcgccatat ttaaaaagct accaagacga agaggatgaa gaggatgagg aggcagattg 8040
   ccttgaatat attgacaata ctgataagat aatatatctt ttatatagaa gatatcgccg 8100
   tatgtaagga tttcaggggg caaggcatag gcagcgcgct tatcaatata tctatagaat 8160
   gggcaaagca taaaaacttg catggactaa tgcttgaaac ccaggacaat aaccttatag 8220
   cttgtaaatt ctatcataat tgggtaatga ctccaactta ttgatagtgt tttatgttca 8280
   gataatgccc gatgactttg tcatgcagct ccaccgattt tgagaacgac agcgacttcc 8340
   gtcccagccg tgccaggtgc tgcctcagat tcaggttatg ccgctcaatt cgctgcgtat 8400
   atcgcttgct gattacgtgc agctttccct tcaggcggga ttcatacagc ggccagccat 8460
   ccgtcatcca tatcaccacg tcaaagggtg acagcaggct cataagacgc cccagcgtcg 8520
   ccatagtgcg ttcaccgaat acgtgcgcaa caaccgtctt ccggagactg tcatacgcgt 8580
   aaaacagcca gcgctggcgc gatttagccc cgacatagcc ccactgttcg tccatttccg 8640
   cgcagacgat gacgtcactg cccggctgta tgcgcgagct gctgccagtg gcgataagtc 8700
   gtgtcttacc gggttggact caagacgata gttaccggat aaggcgcagc ggtcgggctg 8760
   aacggggggt tcgtgcacac agcccagctt ggagcgaacg acctacaccg aactgagata 8820
   cctacagcgt gagctatgag aaagcgccac gcttcccgaa gggagaaagg cggacaggta 8880
   tccggtaagc ggcagggtcg gaacaggaga gcgcacgagg gagcttccag ggggaaacgc 8940
   ctggtatctt tatagtcctg tcgggtttcg ccacctctga cttgagcgtc gatttttgtg 9000
   atgctcgtca ggggggcgga gcctatggaa aaacgccagc aacgcggcct ttttacggtt 9060
   cctggccttt tgctggcctt ttgctcacat gttctttcct gcgttatccc ctgattctgt 9120
   ggataaccgt attaccgcct ttgagtgagc tgataccgct cgccgcagcc gaacgaccga 9180
   gcgcagcgag tcagtgagcg aggaagcgga agagcgcctg atgcggtatt ttctccttac 9240
   gcatctgtgc ggtatttcac accgcacgac tgcggcctga gttttttaag tgacgtaaaa 9300
   tcgtgttgag gccaacgccc ataatgcggg ctgttgcccg gcatccaacg ccattcatgg 9360
   ccatatcaat gattttctgg tgcgtaccgg gttgagaagc ggtgtaagtg aactgcagtt 9420
   gccatgtttt acggcagtga gagcagagat agcgctgatg tccggcggtg cttttgccgt 9480
   tacgcaccac cccgtcagta gctgaacagg agggacagct gatagacaca gaagccactg 9540
   gagcacctca aaaacaccat catacactaa atcagtaagt tggcagcatc acccataatt 9600
   gtggtttcaa aatcggctcc gtcgatacta tgttatacgc caactttgaa aacaactttg 9660
   aaaaagctgt tttctggtat ttaaggtttt agaatgcaag gaacagtgaa ttggagttcg 9720
   tcttgttata attagcttct tggggtatct ttaaatactg tagaaaagag gaaggaaata 9780
   ataaatggct aaaatgagaa tatcaccgga attgaaaaaa ctgatcgaaa aataccgctg 9840
   cgtaaaagat acggaaggaa tgtctcctgc taaggtatat aagctggtgg gagaaaatga 9900
   aaacctatat ttaaaaatga cggacagccg gtataaaggg accacctatg atgtggaacg 9960
   ggaaaaggac atgatgctat ggctggaagg aaagctgcct gttccaaagg tcctgcactt 10020
   tgaacggcat gatggctgga gcaatctgct catgagtgag gccgatggcg tcctttgctc 10080
   ggaagagtat gaagatgaac aaagccctga aaagattatc gagctgtatg cggagtgcat 10140
   caggctcttt cactccatcg acatatcgga ttgtccctat acgaatagct tagacagccg 10200
   cttagccgaa ttggattact tactgaataa cgatctggcc gatgtggatt gcgaaaactg 10260
   ggaagaagac actccattta aagatccgcg cgagctgtat gattttttaa agacggaaaa 10320
   gcccgaagag gaacttgtct ttcccacggc cgacctggga gacagcaaca tctttgtgaa 10380
   agatggcaaa gtaagtggct ttattgatct tgggagaagc ggcagggcgg acaagtggta 10440
   tgacattgcc ttctgcgtcc ggtcgatcag ggaggatatc ggggaagaac agtatgtcga 10500
   gctatttttt gacttactgg ggatcaagcc tgattgggag aaaataaaat attatatttt 10560
   actggatgaa ttgttttagt acctagatgt ggcgcaacga tgccggcgac aagcaggagc 10620
   gcaccgactt cttccgcatc aagtgttttg gctctcaggc cgaggcccac ggcaagtatt 10680
   tgggcaaggg gtcgctggta ttcgtgcagg gcaagattcg gaataccaag tacgagaagg 10740
   acggccagac ggtctacggg accgacttca ttgccgataa ggtggattat ctggacacca 10800
   aggcaccagg cgggtcaaat caggaataag ggcacattgc cccggcgtga gtcggggcaa 10860
   tcccgcaagg agggtgaatg aatcggacgt ttgaccggaa ggcatacagg caagaactga 10920
   tcgacgcggg gttttccgcc gaggatgccg aaaccatcgc aagccgcacc gtcatgcgtg 10980
   cgccccgcga aaccttccag tccgtcggct cgatggtcca gcaagctacg gccaagatcg 11040
   agcgcgacag cgtgcaactg gctccccctg ccctgcccgc gccatcggcc gccgtggagc 11100
   gttcgcgtcg tctcgaacag gaggcggcag gtttggcgaa gtcgatgacc atcgacacgc 11160
   gaggaactat gacgaccaag aagcgaaaaa ccgccggcga ggacctggca aaacaggtca 11220
   gcgaggccaa gcaggccgcg ttgctgaaac acacgaagca gcagatcaag gaaatgcagc 11280
   tttccttgtt cgatattgcg ccgtggccgg acacgatgcg agcgatgcca aacgacacgg 11340
   cccgctctgc cctgttcacc acgcgcaaca agaaaatccc gcgcgaggcg ctgcaaaaca 11400
   aggtcatttt ccacgtcaac aaggacgtga agatcaccta caccggcgtc gagctgcggg 11460
   ccgacgatga cgaactggtg tggcagcagg tgttggagta cgcgaagcgc acccctatcg 11520
   gcgagccgat caccttcacg ttctacgagc tttgccagga cctgggctgg tcgatcaatg 11580
   gccggtatta cacgaaggcc gaggaatgcc tgtcgcgcct acaggcgacg gcgatgggct 11640
   tcacgtccga ccgcgttggg cacctggaat cggtgtcgct gctgcaccgc ttccgcgtcc 11700
   tggaccgtgg caagaaaacg tcccgttgcc aggtcctgat cgacgaggaa atcgtcgtgc 11760
   tgtttgctgg cgaccactac acgaaattca tatgggagaa gtaccgcaag ctgtcgccga 11820
   cggcccgacg gatgttcgac tatttcagct cgcaccggga gccgtacccg ctcaagctgg 11880
   aaaccttccg cctcatgtgc ggatcggatt ccacccgcgt gaagaagtgg cgcgagcagg 11940
   tcggcgaagc ctgcgaagag ttgcgaggca gcggcctggt ggaacacgcc tgggtcaatg 12000
   atgacctggt gcattgcaaa cgctagggcc ttgtggggtc agttccggct gggggttcag 12060
   cagccagcgc tttactggca tttcaggaac aagcgggcac tgctcgacgc acttgcttcg 12120
   ctcagtatcg ctcgggacgc acggcgcgct ctacgaactg ccgataaaca gaggattaaa 12180
   attgacaatt gtgattaagg ctcagattcg acggcttgga gcggccgacg tgcaggattt 12240
   ccgcgagatc cgattgtcgg ccctgaagaa agctccagag atgttcgggt ccgtttacga 12300
   gcacgaggag aaaaagccca tggaggcgtt cgctgaacgg ttgcgagatg ccgtggcatt 12360
   cggcgcctac atcgacggcg agatcattgg gctgtcggtc ttcaaacagg aggacggccc 12420
   caaggacgct cacaaggcgc atctgtccgg cgttttcgtg gagcccgaac agcgaggccg 12480
   aggggtcgcc ggtatgctgc tgcgggcgtt gccggcgggt ttattgctcg tgatgatcgt 12540
   ccgacagatt ccaacgggaa tctggtggat gcgcatcttc atcctcggcg cacttaatat 12600
   ttcgctattc tggagcttgt tgtttatttc ggtctaccgc ctgccgggcg gggtcgcggc 12660
   gacggtaggc gctgtgcagc cgctgatggt cgtgttcatc tctgccgctc tgctaggtag 12720
   cccgatacga ttgatggcgg tcctgggggc tatttgcgga actgcgggcg tggcgctgtt 12780
   ggtgttgaca ccaaacgcag cgctagatcc tgtcggcgtc gcagcgggcc tggcgggggc 12840
   ggtttccatg gcgttcggaa ccgtgctgac ccgcaagtgg caacctcccg tgcctctgct 12900
   cacctttacc gcctggcaac tggcggccgg aggacttctg ctcgttccag tagctttagt 12960
   gtttgatccg ccaatcccga tgcctacagg aaccaatgtt ctcggcctgg cgtggctcgg 13020
   cctgatcgga gcgggtttaa cctacttcct ttggttccgg gggatctcgc gactcgaacc 13080
   tacagttgtt tccttactgg gctttctcag ccccagatct ggggtcgatc agccggggat 13140
   gcatcaggcc gacagtcgga acttcgggtc cccgacctgt accattcggt gagcaatgga 13200
   taggggagtt gatatcgtca acgttcactt ctaaagaaat agcgccactc agcttcctca 13260
   gcggctttat ccagcgattt cctattatgt cggcatagtt ctcaagatcg acagcctgtc 13320
   acggttaagc gagaaatgaa taagaaggct gataattcgg atctctgcga gggagatgat 13380
   atttgatcac aggcagcaac gctctgtcat cgttacaatc aacatgctac cctccgcgag 13440
   atcatccgtg tttcaaaccc ggcagcttag ttgccgttct tccgaatagc atcggtaaca 13500
   tgagcaaagt ctgccgcctt acaacggctc tcccgctgac gccgtcccgg actgatgggc 13560
   tgcctgtatc gagtggtgat tttgtgccga gctgccggtc ggggagctgt tggctggctg 13620
   gtggcaggat atattgtggt gtaaacaaat tgacgcttag acaacttaat aacacattgc 13680
   ggacgttttt aatgtactgg ggtggttttt cttttcacca gtgagacggg caacagctga 13740
   ttgcccttca ccgcctggcc ctgagagagt tgcagcaagc ggtccacgct ggtttgcccc 13800
   agcaggcgaa aatcctgttt gatggtggtt ccgaaatcgg caaaatccct tataaatcaa 13860
   aagaatagcc cgagataggg ttgagtgttg ttccagtttg gaacaagagt ccactattaa 13920
   agaacgtgga ctccaacgtc aaagggcgaa aaaccgtcta tcagggcgat ggcccactac 13980
   gtgaaccatc acccaaatca agttttttgg ggtcgaggtg ccgtaaagca ctaaatcgga 14040
   accctaaagg gagcccccga tttagagctt gacggggaaa gccggcgaac gtggcgagaa 14100
   aggaagggaa gaaagcgaaa ggagcgggcg ccattcaggc tgcgcaactg ttgggaaggg 14160
   cgatcggtgc gggcctcttc gctattacgc cagctggcga aagggggatg tgctgcaagg 14220
   cgattaagtt gggtaacgcc agggttttcc cagtcacgac gttgtaaaac gacggccagt 14280
   gaattaattc ccatcttgaa agaaatatag tttaaatatt tattgataaa ataacaagtc 14340
   aggtattata gtccaagcaa aaacataaat ttattgatgc aagtttaaat tcagaaatat 14400
   ttcaataact gattatatca gctggtacat tgccgtagat gaaagactga gtgcgatatt 14460
   atgtgtaata cataaattga tgatatagct agcttagctc atcgggggat ccgtcgaagc 14520
   tagcttgggt cccgctcaga agaactcgtc aagaaggcga tagaaggcga tgcgctgcga 14580
   atcgggagcg gcgataccgt aaagcacgag gaagcggtca gcccattcgc cgccaagctc 14640
   ttcagcaata tcacgggtag ccaacgctat gtcctgatag cggtccgcca cacccagccg 14700
   gccacagtcg atgaatccag aaaagcggcc attttccacc atgatattcg gcaagcaggc 14760
   atcgccatgg gtcacgacga gatcctcgcc gtcgggcatg cgcgccttga gcctggcgaa 14820
   cagttcggct ggcgcgagcc cctgatgctc ttcgtccaga tcatcctgat cgacaagacc 14880
   ggcttccatc cgagtacgtg ctcgctcgat gcgatgtttc gcttggtggt cgaatgggca 14940
   ggtagccgga tcaagcgtat gcagccgccg cattgcatca gccatgatgg atactttctc 15000
   ggcaggagca aggtgagatg acaggagatc ctgccccggc acttcgccca atagcagcca 15060
   gtcccttccc gcttcagtga caacgtcgag cacagctgcg caaggaacgc ccgtcgtggc 15120
   cagccacgat agccgcgctg cctcgtcctg cagttcattc agggcaccgg acaggtcggt 15180
   cttgacaaaa agaaccgggc gcccctgcgc tgacagccgg aacacggcgg catcagagca 15240
   gccgattgtc tgttgtgccc agtcatagcc gaatagcctc tccacccaag cggccggaga 15300
   acctgcgtgc aatccatctt gttcaatcca agctcccatg ggccctcgac tagagtcgag 15360
   atctggattg agagtgaata tgagactcta attggatacc gaggggaatt tatggaacgt 15420
   cagtggagca tttttgacaa gaaatatttg ctagctgata gtgaccttag gcgacttttg 15480
   aacgcgcaat aatggtttct gacgtatgtg cttagctcat taaactccag aaacccgcgg 15540
   ctgagtggct ccttcaacgt tgcggttctg tcagttccaa acgtaaaacg gcttgtcccg 15600
   cgtcatcggc gggggtcata acgtgactcc cttaattctc cgctcatgat cttgatcccc 15660
   tgcgccatca gatccttggc ggcaagaaag ccatccagtt tactttgcag ggcttcccaa 15720
   ccttaccaga gggcgcccca gctggcaatt ccggttcgct tgctgtccat aaaaccgccc 15780
   agtctagcta tcgccatgta agcccactgc aagctacctg ctttctcttt gcgcttgcgt 15840
   tttcccttgt ccagatagcc cagtagctga cattcatccg gggtcagcac cgtttctgcg 15900
   gactggcttt ctacgtgttc cgcttccttt agcagccctt gcgccctgag tgcttgcggc 15960
   agcgtgaagc ttctgccatg catcattcat catatttagg tcatgaaatg atgcccttta 16020
   gtagctatga taggaaaaca aattctgtcg cgcaccattc atcatatttg gggccatctt 16080
   cattcttgag agggagggtc ctctttgagg aaggataatt tatcttggtt tcagagaaaa 16140
   tacttgaagt gggtgccaac ttggttgagc agtgaaacta gacataaaga ttacatgatg 16200
   ttcttggtgg tcgtaatatg tcaaatagat ggtatgctaa aaaacagtta aaactagatg 16260
   agaagattgt gaaagactga tttgccagag aaaatctagt agatatttat tggagttggc 16320
   ctagcatgtg ggcagtgcca tgcttgaatg ccccctgatc atgggtagat gtcagccaca 16380
   aggagcccaa taaaccttgg gctttgatac catgttaaat aaaaaatata tatgcctcat 16440
   atatatgatc tataaactcc taaattgcat ctaaacactc gatggaagaa gggtgaaatc 16500
   agtttatcga tttaactaaa ttaatttatt ttgggggttt ttttggcttt taaaatgggt 16560
   ttgaagataa ctctctctct ctctcccccc gcccatgcaa agaggtcggc aattttagat 16620
   aaagacgtcc attttttcga cgcgtgtcgt tacgtggcga aacgtcgtgg aaggacgagt 16680
   ctttgagggc acgcgtcaga ctatcgtggt catcctctgc ctacttatat ccctctggtc 16740
   ttcttcttct taattaagaa actcccgagg tgagcaagga tccggagtcg agcgcgaaga 16800
   agagaaagag ggaaagcgcg g 16821
<210> 32
   <211> 17675
   <212> DNA
   <213> Artificial Sequence
<220>
   <223> MERF03 plasmid
<400> 32
   cgatcgttca aacatttggc aataaagttt cttaagattg aatcctgttg ccggtcttgc 60
   gatgattatc atataatttc tgttgaatta cgttaagcat gtaataatta acatgtaatg 120
   catgacgtta tttatgagat gggtttttat gattagagtc ccgcaattat acatttaata 180
   cgcgatagaa aacaaaatat agcgcgcaaa ctaggataaa ttatcgcgcg cggtgtcatc 240
   tatgttacta gatcgggtgt acatctagaa gctctggaca tcatgttgga tatgaaacaa 300
   ctattattta tctacatgtt ttagatgtta tctgattatt tttataccgt agtcttctat 360
   tgatgaggag tctaaggcta tagaattata tatctaaatg attaatatat atattattaa 420
   taattaacaa taattaatat attataattt atatatatat attttatatt attataataa 480
   tattcttaca aatataatta ttatattcga cggtatcgat aagctcgggc ccaaatcgta 540
   cgggccggcc gtaagcttat cgatgaagga agtggaggag agaggacgcc atggtagcag 600
   aggaaggtct ggcttgatct cccgacgatt cctctctcat cagtgaaaca agagaataag 660
   aggcatcgcg attcttggaa ggtacagagg gaagttgatc aaagagaggc tccgggaaga 720
   agcagatggc ggggaagacg aaacatggcg cctgacaaca taggctatca taggataatc 780
   cactctccct ctgtctttct ctctgtttct ttctctctct ctttatctct ctctctctct 840
   cgaacatttc acacattttt gggcctgttc tttggcgtag ctagcccttt cttggtccat 900
   attttgaggc caaggccaat catgcgcagc cacgttgcat ggcgggagga ccccatctat 960
   ccattccgtc agttcctggc ttttgggaca atctgaacag tacataaacc acgggctcgg 1020
   gcttgggccc gccgaaaagc ccgtccgtac aattttctga cgtacaatat taatttccca 1080
   gaaaagaaaa ttcattaaaa aataactatg tcacccacga actcgtgatc tagatataag 1140
   gcaggcaact ttcccgtgac agtcggactt gtggggttcc cttgacggcg ccgccgtcta 1200
   ctgacggcgc ttgaatgacg tcattattac tatatttaat atatccgaga ataaatggct 1260
   ccctggatcc cccgtgaatg gtcacgtcat ccacgcgggc tttgacttcg cctaggtcgc 1320
   cacgttgggg cctcataacg tgcaagctga cgtggctatc attgcctcga tggcgatctt 1380
   gaacgaacca cataaccaat ctgagacggc gacgaggatt ctgtgttttc ctcggggatc 1440
   tctggccgtc cgatgaacta acacgcacta tttcaaaaag ggggttaaac acgattgtta 1500
   ggtttctttg agtcttccac atctccgctc cacgatcttg tacctcttct tgcgacgatc 1560
   tactcgccct cgaggtcgac accatgtccg gtgcgcctcc caccgacctg cagaagatgg 1620
   tgatgggcaa caccaagccc gtcgagttga tcctcgacgg gaagaccgtg gcgatctgct 1680
   gcgccaccgg cgtgttcggc accgcctacc tcgtcccgag acacctgttc gccgagaagt 1740
   atgacaagat catgctggac gggcgggcca tgaccgactc ggactaccgg gtcttcgagt 1800
   tcgagatcaa agtgaagggc caggatatgc tctccgacgc cgcgctgatg gtgctccaca 1860
   gaggcaaccg cgtgcgggac atcaccaagc acttcaggga caccgcgcgc atgaagaagg 1920
   ggacccctgt ggtcggggtc gtgaacaacg ccgacgtcgg gcgcctcatc ttctccggcg 1980
   aggcgctgac ctacaaggac atcgtcgtgt gcatggacgg ggacacgatg ccggggctct 2040
   tcgcctacaa ggccgcgacc aaggccgggt actgcggggg tgcggtgctc gccaaggacg 2100
   gcgccgacac gttcatcgtg ggcacccact ccgccggcgg taacggcgtc ggctactgct 2160
   cctgcgtgtc ccggtccatg ctgctgcgga tgaaggccca cgttgacccc gagccgcagc 2220
   acgagtaaaa gcttatcgat gtgcacgatc gttcaaacat ttggcaataa agtttcttaa 2280
   gattgaatcc tgttgccggt cttgcgatga ttatcatata atttctgttg aattacgtta 2340
   agcatgtaat aattaacatg taatgcatga cgttatttat gagatgggtt tttatgatta 2400
   gagtcccgca attatacatt taatacgcga tagaaaacaa aatatagcgc gcaaactagg 2460
   ataaattatc gcgcgcggtg tcatctatgt tactagatcg ggtggacaac tcgtcacgta 2520
   ctagtggcgc gcccacgtga tttaaatcgt acgggccggc cgtatactct agtggatccc 2580
   ccaattcaga tcggctgagt ggctccttca acgttgcggt tctgtcagtt ccaaacgtaa 2640
   aacggcttgt cccgcgtcat cggcgggggt cataacgtga ctcccttaat tctccgctca 2700
   tgatcagatt gtcgtttccc gccttcagtt taaactatca gtgtttgaca ggatatattg 2760
   gcgggtaaac ctaagagaaa agagcgttta ttagaataat cggatattta aaagggcgtg 2820
   aaaaggttta tccgttcgtc catttgtatg tgcatgccaa ccacagggtt ccccagatct 2880
   ggcgccggcc agcgagacga gcaagattgg ccgccgcccg aaacgatccg acagcgcgcc 2940
   cagcacaggt gcgcaggcaa attgcaccaa cgcatacagc gccagcagaa tgccatagtg 3000
   ggcggtgacg tcgttcgagt gaaccagatc gcgcaggagg cccggcagca ccggcataat 3060
   caggccgatg ccgacagcgt cgagcgcgac agtgctcaga attacgatca ggggtatgtt 3120
   gggtttcacg tctggcctcc ggaccagcct ccgctggtcc gattgaacgc gcggattctt 3180
   tatcactgat aagttggtgg acatattatg tttatcagtg ataaagtgtc aagcatgaca 3240
   aagttgcagc cgaatacagt gatccgtgcc gccctggacc tgttgaacga ggtcggcgta 3300
   gacggtctga cgacacgcaa actggcggaa cggttggggg ttcagcagcc ggcgctttac 3360
   tggcacttca ggaacaagcg ggcgctgctc gacgcactgg ccgaagccat gctggcggag 3420
   aatcatacgc attcggtgcc gagagccgac gacgactggc gctcatttct gatcgggaat 3480
   gcccgcagct tcaggcaggc gctgctcgcc taccgcgatg gcgcgcgcat ccatgccggc 3540
   acgcgaccgg gcgcaccgca gatggaaacg gccgacgcgc agcttcgctt cctctgcgag 3600
   gcgggttttt cggccgggga cgccgtcaat gcgctgatga caatcagcta cttcactgtt 3660
   ggggccgtgc ttgaggagca ggccggcgac agcgatgccg gcgagcgcgg cggcaccgtt 3720
   gaacaggctc cgctctcgcc gctgttgcgg gccgcgatag acgccttcga cgaagccggt 3780
   ccggacgcag cgttcgagca gggactcgcg gtgattgtcg atggattggc gaaaaggagg 3840
   ctcgttgtca ggaacgttga aggaccgaga aagggtgacg attgatcagg accgctgccg 3900
   gagcgcaacc cactcactac agcagagcca tgtagacaac atcccctccc cctttccacc 3960
   gcgtcagacg cccgtagcag cccgctacgg gctttttcat gccctgccct agcgtccaag 4020
   cctcacggcc gcgctcggcc tctctggcgg ccttctggcg ctcttccgct tcctcgctca 4080
   ctgactcgct gcgctcggtc gttcggctgc ggcgagcggt atcagctcac tcaaaggcgg 4140
   taatacggtt atccacagaa tcaggggata acgcaggaaa gaacatgtga gcaaaaggcc 4200
   agcaaaaggc caggaaccgt aaaaaggccg cgttgctggc gtttttccat aggctccgcc 4260
   cccctgacga gcatcacaaa aatcgacgct caagtcagag gtggcgaaac ccgacaggac 4320
   tataaagata ccaggcgttt ccccctggaa gctccctcgt gcgctctcct gttccgaccc 4380
   tgccgcttac cggatacctg tccgcctttc tcccttcggg aagcgtggcg cttttccgct 4440
   gcataaccct gcttcggggt cattatagcg attttttcgg tatatccatc ctttttcgca 4500
   cgatatacag gattttgcca aagggttcgt gtagactttc cttggtgtat ccaacggcgt 4560
   cagccgggca ggataggtga agtaggccca cccgcgagcg ggtgttcctt cttcactgtc 4620
   ccttattcgc acctggcggt gctcaacggg aatcctgctc tgcgaggctg gccggctacc 4680
   gccggcgtaa cagatgaggg caagcggatg gctgatgaaa ccaagccaac caggaagggc 4740
   agcccaccta tcaaggtgta ctgccttcca gacgaacgaa gagcgattga ggaaaaggcg 4800
   gcggcggccg gcatgagcct gtcggcctac ctgctggccg tcggccaggg ctacaaaatc 4860
   acgggcgtcg tggactatga gcacgtccgc gagctggccc gcatcaatgg cgacctgggc 4920
   cgcctgggcg gcctgctgaa actctggctc accgacgacc cgcgcacggc gcggttcggt 4980
   gatgccacga tcctcgccct gctggcgaag atcgaagaga agcaggacga gcttggcaag 5040
   gtcatgatgg gcgtggtccg cccgagggca gagccatgac ttttttagcc gctaaaacgg 5100
   ccggggggtg cgcgtgattg ccaagcacgt ccccatgcgc tccatcaaga agagcgactt 5160
   cgcggagctg gtgaagtaca tcaccgacga gcaaggcaag accgagcgcc tttgcgacgc 5220
   tcaccgggct ggttgccctc gccgctgggc tggcggccgt ctatggccct gcaaacgcgc 5280
   cagaaacgcc gtcgaagccg tgtgcgagac accgcggccg ccggcgttgt ggatacctcg 5340
   cggaaaactt ggccctcact gacagatgag gggcggacgt tgacacttga ggggccgact 5400
   cacccggcgc ggcgttgaca gatgaggggc aggctcgatt tcggccggcg acgtggagct 5460
   ggccagcctc gcaaatcggc gaaaacgcct gattttacgc gagtttccca cagatgatgt 5520
   ggacaagcct ggggataagt gccctgcggt attgacactt gaggggcgcg actactgaca 5580
   gatgaggggc gcgatccttg acacttgagg ggcagagtgc tgacagatga ggggcgcacc 5640
   tattgacatt tgaggggctg tccacaggca gaaaatccag catttgcaag ggtttccgcc 5700
   cgtttttcgg ccaccgctaa cctgtctttt aacctgcttt taaaccaata tttataaacc 5760
   ttgtttttaa ccagggctgc gccctgtgcg cgtgaccgcg cacgccgaag gggggtgccc 5820
   ccccttctcg aaccctcccg gcccgctaac gcgggcctcc catcccccca ggggctgcgc 5880
   ccctcggccg cgaacggcct caccccaaaa atggcagcgc tggcagtcct tgccattgcc 5940
   gggatcgggg cagtaacggg atgggcgatc agcccgagcg cgacgcccgg aagcattgac 6000
   gtgccgcagg tgctggcatc gacattcagc gaccaggtgc cgggcagtga gggcggcggc 6060
   ctgggtggcg gcctgccctt cacttcggcc gtcggggcat tcacggactt catggcgggg 6120
   ccggcaattt ttaccttggg cattcttggc atagtggtcg cgggtgccgt gctcgtgttc 6180
   gggggtgcga taaacccagc gaaccatttg aggtgatagg taagattata ccgaggtatg 6240
   aaaacgagaa ttggaccttt acagaattac tctatgaagc gccatattta aaaagctacc 6300
   aagacgaaga ggatgaagag gatgaggagg cagattgcct tgaatatatt gacaatactg 6360
   ataagataat atatctttta tatagaagat atcgccgtat gtaaggattt cagggggcaa 6420
   ggcataggca gcgcgcttat caatatatct atagaatggg caaagcataa aaacttgcat 6480
   ggactaatgc ttgaaaccca ggacaataac cttatagctt gtaaattcta tcataattgg 6540
   gtaatgactc caacttattg atagtgtttt atgttcagat aatgcccgat gactttgtca 6600
   tgcagctcca ccgattttga gaacgacagc gacttccgtc ccagccgtgc caggtgctgc 6660
   ctcagattca ggttatgccg ctcaattcgc tgcgtatatc gcttgctgat tacgtgcagc 6720
   tttcccttca ggcgggattc atacagcggc cagccatccg tcatccatat caccacgtca 6780
   aagggtgaca gcaggctcat aagacgcccc agcgtcgcca tagtgcgttc accgaatacg 6840
   tgcgcaacaa ccgtcttccg gagactgtca tacgcgtaaa acagccagcg ctggcgcgat 6900
   ttagccccga catagcccca ctgttcgtcc atttccgcgc agacgatgac gtcactgccc 6960
   ggctgtatgc gcgaggttac cgactgcggc ctgagttttt taagtgacgt aaaatcgtgt 7020
   tgaggccaac gcccataatg cgggctgttg cccggcatcc aacgccattc atggccatat 7080
   caatgatttt ctggtgcgta ccgggttgag aagcggtgta agtgaactgc agttgccatg 7140
   ttttacggca gtgagagcag agatagcgct gatgtccggc ggtgcttttg ccgttacgca 7200
   ccaccccgtc agtagctgaa caggagggac agctgataga cacagaagcc actggagcac 7260
   ctcaaaaaca ccatcataca ctaaatcagt aagttggcag catcacccat aattgtggtt 7320
   tcaaaatcgg ctccgtcgat actatgttat acgccaactt tgaaaacaac tttgaaaaag 7380
   ctgttttctg gtatttaagg ttttagaatg caaggaacag tgaattggag ttcgtcttgt 7440
   tataattagc ttcttggggt atctttaaat actgtagaaa agaggaagga aataataaat 7500
   ggctaaaatg agaatatcac cggaattgaa aaaactgatc gaaaaatacc gctgcgtaaa 7560
   agatacggaa ggaatgtctc ctgctaaggt atataagctg gtgggagaaa atgaaaacct 7620
   atatttaaaa atgacggaca gccggtataa agggaccacc tatgatgtgg aacgggaaaa 7680
   ggacatgatg ctatggctgg aaggaaagct gcctgttcca aaggtcctgc actttgaacg 7740
   gcatgatggc tggagcaatc tgctcatgag tgaggccgat ggcgtccttt gctcggaaga 7800
   gtatgaagat gaacaaagcc ctgaaaagat tatcgagctg tatgcggagt gcatcaggct 7860
   ctttcactcc atcgacatat cggattgtcc ctatacgaat agcttagaca gccgcttagc 7920
   cgaattggat tacttactga ataacgatct ggccgatgtg gattgcgaaa actgggaaga 7980
   agacactcca tttaaagatc cgcgcgagct gtatgatttt ttaaagacgg aaaagcccga 8040
   agaggaactt gtcttttccc acggcgacct gggagacagc aacatctttg tgaaagatgg 8100
   caaagtaagt ggctttattg atcttgggag aagcggcagg gcggacaagt ggtatgacat 8160
   tgccttctgc gtccggtcga tcagggagga tatcggggaa gaacagtatg tcgagctatt 8220
   ttttgactta ctggggatca agcctgattg ggagaaaata aaatattata ttttactgga 8280
   tgaattgttt tagtacctag atgtggcgca acgatgccgg cgacaagcag gagcgcaccg 8340
   acttcttccg catcaagtgt tttggctctc aggccgaggc ccacggcaag tatttgggca 8400
   aggggtcgct ggtattcgtg cagggcaaga ttcggaatac caagtacgag aaggacggcc 8460
   agacggtcta cgggaccgac ttcattgccg ataaggtgga ttatctggac accaaggcac 8520
   caggcgggtc aaatcaggaa taagggcaca ttgccccggc gtgagtcggg gcaatcccgc 8580
   aaggagggtg aatgaatcgg acgtttgacc ggaaggcata caggcaagaa ctgatcgacg 8640
   cggggttttc cgccgaggat gccgaaacca tcgcaagccg caccgtcatg cgtgcgcccc 8700
   gcgaaacctt ccagtccgtc ggctcgatgg tccagcaagc tacggccaag atcgagcgcg 8760
   acagcgtgca actggctccc cctgccctgc ccgcgccatc ggccgccgtg gagcgttcgc 8820
   gtcgtctcga acaggaggcg gcaggtttgg cgaagtcgat gaccatcgac acgcgaggaa 8880
   ctatgacgac caagaagcga aaaaccgccg gcgaggacct ggcaaaacag gtcagcgagg 8940
   ccaagcaggc cgcgttgctg aaacacacga agcagcagat caaggaaatg cagctttcct 9000
   tgttcgatat tgcgccgtgg ccggacacga tgcgagcgat gccaaacgac acggcccgct 9060
   ctgccctgtt caccacgcgc aacaagaaaa tcccgcgcga ggcgctgcaa aacaaggtca 9120
   ttttccacgt caacaaggac gtgaagatca cctacaccgg cgtcgagctg cgggccgacg 9180
   atgacgaact ggtgtggcag caggtgttgg agtacgcgaa gcgcacccct atcggcgagc 9240
   cgatcacctt cacgttctac gagctttgcc aggacctggg ctggtcgatc aatggccggt 9300
   attacacgaa ggccgaggaa tgcctgtcgc gcctacaggc gacggcgatg ggcttcacgt 9360
   ccgaccgcgt tgggcacctg gaatcggtgt cgctgctgca ccgcttccgc gtcctggacc 9420
   gtggcaagaa aacgtcccgt tgccaggtcc tgatcgacga ggaaatcgtc gtgctgtttg 9480
   ctggcgacca ctacacgaaa ttcatatggg agaagtaccg caagctgtcg ccgacggccc 9540
   gacggatgtt cgactatttc agctcgcacc gggagccgta cccgctcaag ctggaaacct 9600
   tccgcctcat gtgcggatcg gattccaccc gcgtgaagaa gtggcgcgag caggtcggcg 9660
   aagcctgcga agagttgcga ggcagcggcc tggtggaaca cgcctgggtc aatgatgacc 9720
   tggtgcattg caaacgctag ggccttgtgg ggtcagttcc ggctgggggt tcagcagcca 9780
   gcgctttact ggcatttcag gaacaagcgg gcactgctcg acgcacttgc ttcgctcagt 9840
   atcgctcggg acgcacggcg cgctctacga actgccgata aacagaggat taaaattgac 9900
   aattgtgatt aaggctcaga ttcgacggct tggagcggcc gacgtgcagg atttccgcga 9960
   gatccgattg tcggccctga agaaagctcc agagatgttc gggtccgttt acgagcacga 10020
   ggagaaaaag cccatggagg cgttcgctga acggttgcga gatgccgtgg cattcggcgc 10080
   ctacatcgac ggcgagatca ttgggctgtc ggtcttcaaa caggaggacg gccccaagga 10140
   cgctcacaag gcgcatctgt ccggcgtttt cgtggagccc gaacagcgag gccgaggggt 10200
   cgccggtatg ctgctgcggg cgttgccggc gggtttattg ctcgtgatga tcgtccgaca 10260
   gattccaacg ggaatctggt ggatgcgcat cttcatcctc ggcgcactta atatttcgct 10320
   attctggagc ttgttgttta tttcggtcta ccgcctgccg ggcggggtcg cggcgacggt 10380
   aggcgctgtg cagccgctga tggtcgtgtt catctctgcc gctctgctag gtagcccgat 10440
   acgattgatg gcggtcctgg gggctatttg cggaactgcg ggcgtggcgc tgttggtgtt 10500
   gacaccaaac gcagcgctag atcctgtcgg cgtcgcagcg ggcctggcgg gggcggtttc 10560
   catggcgttc ggaaccgtgc tgacccgcaa gtggcaacct cccgtgcctc tgctcacctt 10620
   taccgcctgg caactggcgg ccggaggact tctgctcgtt ccagtagctt tagtgtttga 10680
   tccgccaatc ccgatgccta caggaaccaa tgttctcggc ctggcgtggc tcggcctgat 10740
   cggagcgggt ttaacctact tcctttggtt ccgggggatc tcgcgactcg aacctacagt 10800
   tgtttcctta ctgggctttc tcagccccag atctggggtc gatcagccgg ggatgcatca 10860
   ggccgacagt cggaacttcg ggtccccgac ctgtaccatt cggtgagcaa tggatagggg 10920
   agttgatatc gtcaacgttc acttctaaag aaatagcgcc actcagcttc ctcagcggct 10980
   ttatccagcg atttcctatt atgtcggcat agttctcaag atcgacagcc tgtcacggtt 11040
   aagcgagaaa tgaataagaa ggctgataat tcggatctct gcgagggaga tgatatttga 11100
   tcacaggcag caacgctctg tcatcgttac aatcaacatg ctaccctccg cgagatcatc 11160
   cgtgtttcaa acccggcagc ttagttgccg ttcttccgaa tagcatcggt aacatgagca 11220
   aagtctgccg ccttacaacg gctctcccgc tgacgccgtc ccggactgat gggctgcctg 11280
   tatcgagtgg tgattttgtg ccgagctgcc ggtcggggag ctgttggctg gctggtggca 11340
   ggatatattg tggtgtaaac aaattgacgc ttagacaact taataacaca ttgcggacgt 11400
   ttttaatgta ctggggtggt ttttcttttc accagtgaga cgggcaacag ctgattgccc 11460
   ttcaccgcct ggccctgaga gagttgcagc aagcggtcca cgctggtttg ccccagcagg 11520
   cgaaaatcct gtttgatggt ggttccgaaa tcggcaaaat cccttataaa tcaaaagaat 11580
   agcccgagat agggttgagt gttgttccag tttggaacaa gagtccacta ttaaagaacg 11640
   tggactccaa cgtcaaaggg cgaaaaaccg tctatcaggg cgatggccca ctacgtgaac 11700
   catcacccaa atcaagtttt ttggggtcga ggtgccgtaa agcactaaat cggaacccta 11760
   aagggagccc ccgatttaga gcttgacggg gaaagccggc gaacgtggcg agaaaggaag 11820
   ggaagaaagc gaaaggagcg ggcgccattc aggctgcgca actgttggga agggcgatcg 11880
   gtgcgggcct cttcgctatt acgccagctg gcgaaagggg gatgtgctgc aaggcgatta 11940
   agttgggtaa cgccagggtt ttcccagtca cgacgttgta aaacgacggc cagtgaattg 12000
   ccatcttgaa agaaatatag tttaaatatt tattgataaa ataagtcagg tattatagtc 12060
   caagcaaaaa cataatttat tgatgcaaag tttaaattca gaaatatttc aataactgat 12120
   tatatcagct ggtacattgc cgtagatgaa agactgagtg cgatattatg tgtaatacat 12180
   aaattgatga tatagctagc ttagctcatc gggggatcct taatcgactc tagctagaac 12240
   gaattgttag gtggcggtac ttgggtcgat atcaaagtgc atcacttctt cccgtatgcc 12300
   caactttgta tagagagcca ctgcgggatc gtcaccgtaa tctgcttgca cgtagatcac 12360
   ataagcacca agcgcgttgg cctcatgctt gaggagattg atgagcgcgg tggcaatgcc 12420
   ctgcctccgg tgctcgccgg agactgcgag atcatagata tagatctcac tacgcggctg 12480
   ctcaaacctg ggcagaacgt aagccgcgag agcgccaaca accgcttctt ggtcgaaggc 12540
   agcaagcgcg atgaatgtct tactacggag caagttcccg aggtaatcgg agtccggctg 12600
   atgttgggag taggtggcta cgtctccgaa ctcacgaccg aaaagatcaa gagcagcccg 12660
   catggatttg acttggtcag ggccgagcct acatgtgcga atgatgccca tacttgagcc 12720
   acctaacttt gttttagggc gactgccctg ctgcgtaaca tcgttgctgc tgcgtaccat 12780
   ggagatctgg attgagagtg aatatgagac tctaattgga taccgagggg aatttatgga 12840
   agtcagtgga gcatttttga caagaaatat ttgctagctg atagtgacct taggcgactt 12900
   ttgaacgcgc aataatggtt tctgacgtat gtgcttagct cattaaactc cagaaacccg 12960
   cggctgagtg gctccttcaa cgttgcggtt ctgtcagttc caaacgtaaa acggcttgtc 13020
   ccgcgtcatc ggcgggggtc ataacgtgac tcccttaatt ctccgctcat gatcttgatc 13080
   ccctgcgcca tcagatcctt ggcggcaaga aagccatcca gtttactttg cagggcttcc 13140
   caaccttacc agagggcgcc ccagctggca attccggttc gcttgctgtc cataaaaccg 13200
   cccagtctag ctatcgccat gtaagcccac tgcaagctac ctgctttctc tttgcgcttg 13260
   cgttttccct tgtccagata gcccagtagc tgacattcat ccggggtcag caccgtttct 13320
   gcggactggc tttctacgtg ttccgcttcc tttagcagcc cttgcgccct gagtgcttgc 13380
   ggcagcgtga agctctggac atcatgttgg atatgaaaca actattattt atctacatgt 13440
   tttagatgtt atctgattat ttttataccg tagtcttcta ttgatgagga gtctaaggct 13500
   atagaattat atatctaaat gattaatata tatattatta ataattaaca ataattaata 13560
   tattataatt tatatatata tattttatat tattataata atattcttac aaatataatt 13620
   attatattcg acggtatcga taagctcggg atccctgaaa gcgacgttgg atgttaacat 13680
   ctacaaattg ccttttctta tcgaccatgt acgtaagcgc ttacgttttt ggtggaccct 13740
   tgaggaaact ggtagctgtt gtgggcctgt ggtctcaaga tggatcatta atttccacct 13800
   tcacctacga tggggggcat cgcaccggtg agtaatattg tacggctaag agcgaatttg 13860
   gcctgtagga tccctgaaag cgacgttgga tgttaacatc tacaaattgc cttttcttat 13920
   cgaccatgta cgtaagcgct tacgtttttg gtggaccctt gaggaaactg gtagctgttg 13980
   tgggcctgtg gtctcaagat ggatcattaa tttccacctt cacctacgat ggggggcatc 14040
   gcaccggtga gtaatattgt acggctaaga gcgaatttgg cctgtaggat ccctgaaagc 14100
   gacgttggat gttaacatct acaaattgcc ttttcttatc gaccatgtac gtaagcgctt 14160
   acgtttttgg tggacccttg aggaaactgg tagctgttgt gggcctgtgg tctcaagatg 14220
   gatcattaat ttccaccttc acctacgatg gggggcatcg caccggtgag taatattgta 14280
   cggctaagag cgaatttggc ctgtaggatc cgcgagctgg tcaatcccat tgcttttgaa 14340
   gcagctcaac attgatctct ttctcgatcg agggagattt ttcaaatcag tgcgcaagac 14400
   gtgacgtaag tatccgagtc agtttttatt tttctactaa tttggtcgtt tatttcggcg 14460
   tgtaggacat ggcaaccggg cctgaatttc gcgggtattc tgtttctatt ccaacttttt 14520
   cttgatccgc agccattaac gacttttgaa tagatacgct gacacgccaa gcctcgctag 14580
   tcaaaagtgt accaaacaac gctttacagc aagaacggaa tgcgcgtgac gctcgcggtg 14640
   acgccatttc gccttttcag aaatggataa atagccttgc ttcctattat atcttccccc 14700
   aaattaatta agaaactccc gaggtgagca aggatccgga gtcgagcgcg aagaagagaa 14760
   agagggaaag cgcgggtacc gggccccccc ctcgacggat caagtgcaaa ggtccgcctt 14820
   gtttctcctc tgtctcttga tctgactaat cttggtttat gattcgttga gtaattttgg 14880
   ggaaagctag cttcgtccac agtttttttt tcgatgaaca gtgccgcagt ggcgctgatc 14940
   ttgtatgcta tcctgcaatc gtggtgaact tatttctttt atatccttca ctcccatgaa 15000
   aaggctagta atctttctcg atgtaacatc gtccagcact gctattaccg tgtggtccat 15060
   ccgacagtct ggctgaacac atcatacgat attgagcaaa gatcgatcta tcttccctgt 15120
   tctttaatga aagacgtcat tttcatcagt atgatctaag aatgttgcaa cttgcaagga 15180
   ggcgtttctt tctttgaatt taactaactc gttgagtggc cctgtttctc ggacgtaagg 15240
   cctttgctgc tccacacatg tccattcgaa ttttaccgtg tttagcaagg gcgaaaagtt 15300
   tgcatcttga tgatttagct tgactatgcg attgctttcc tggacccgtg cagctgcgga 15360
   cggatccccc gctcgaagct agcttgatca gatctgatcg aattcaccat gggggccggg 15420
   cagtcgtccc cggccacggg gtcccagaac cagtccggca acaccggcag catcatcaac 15480
   aactactaca tgcagcagta ccagaactcc atggacaccc aactggggga caacgccacg 15540
   agcggcggca gcaatgaggg ctccaccgac accacgagca cccacacgac caacacccag 15600
   aacaacgact ggttcagcaa gctcgcgtcc agcgccttct ccgggctctt cggggcgctc 15660
   ctggccgaca agaagaccga ggagaccacg ctgctcgagg accgcatcct caccacgcgg 15720
   aacggccaca cgacctccac cacgcagtcg agcgtcgggg tcacttacgg ttacgccacg 15780
   gccgaggact tcgtgagcgg ccccaacacc tccggcctcg agactcgggt cgcccaggcc 15840
   gagcgcttct tcaagacgca cctgttcgac tgggtgacct ccgacccctt cggccgctgc 15900
   cacctcttgg aactgcccac cgaccacaag ggcgtgtacg ggtcgttgac ggacagctac 15960
   gcgtacatga ggaacgggtg ggacgtggag gtcaccgccg tcggcaacca gttcaacggg 16020
   ggctgcctcc tggtggccat ggtccccgaa ctctgctcga tccagaagcg ggagctgtac 16080
   cagctgaccc tcttcccgca ccagttcatc aacccgcgca ccaacatgac cgcgcacatc 16140
   accgtcccct tcgtgggcgt gaaccgctac gaccagtaca aagtccacaa gccctggacc 16200
   ttggtggtca tggtcgtcgc ccccctcacc gtcaactcgg agggggcgcc ccagatcaaa 16260
   gtctacgcca acatcgcccc cacgaacgtg cacgttgccg gcgagttccc gagcaaggag 16320
   ggcatcttcc cggttgcgtg ctccgacggc tacgggggcc tcgtgacgac cgacccgaag 16380
   accgccgacc ctgcctacgg caaggtgttc aacccgccca gaaacatgct ccctgggaga 16440
   ttcacgaact tcctcgacgt ggccgaggcc tgccccacgt tcctgcactt cgagggggac 16500
   gtcccttacg tcaccacgaa gactgactcg gatcgcgtgc tggcccagtt cgacctgtcc 16560
   ctcgccgcta agcacatgtc caacaccttc ctcgccggcc tcgcgcaata ctacacccag 16620
   tacagcggca ccatcaacct ccacttcatg ttcaccggcc ccaccgacgc caaggcccgc 16680
   tacatgatcg cttacgcccc gcccggcatg gagcccccga agacccccga ggcggccgcg 16740
   cactgcatcc acgccgagtg ggacacgggg ctcaactcca agttcacgtt ctccatcccc 16800
   tacctcagcg ccgcggacta cgcgtacacc gccagcgaca ccgcggagac cacgaacgtg 16860
   cagggctggg tgtgcctgtt ccagatcacc cacgggaagg ccgacggcga cgccctggtc 16920
   gtgctggcct cggcggggaa ggacttcgaa ctcaggctgc cggtggacgc ccgcacccag 16980
   acgactagcg ccggcgagtc cgcggacccg gtgaccgcga ccgtcgagaa ctacgggggc 17040
   gagacccaag tgcagcgcag acagcacacg gacgtgtcct tcatcctgga tcgcttcgtg 17100
   aaagtgaccc cgaaggacca gatcaacgtg ctggacctga tgcagacgcc ggcgcacacc 17160
   ctcgtggggg ccctcctgcg caccgccacg tactacttcg cggacctcga ggtggcggtg 17220
   aagcacgagg gcaacctgac gtgggtgccc aacggtgcgc cggaggccgc gctcgacaac    17280
   accacgaacc ccaccgcgta ccacaaggcc cccctgaccc ggctcgccct cccctacacc    17340
   gccccccaca gggtcctcgc cacggtctac aacggcaaca gcaagtacgg ggacgggacc    17400
   gtcgccaacg tgcgcgggga cctccaagtg ctcgcccaga aggccgcgcg cgccctcccc    17460
   actagcttca actacggtgc catcaaggcc acgagggtga cggagctgct ctaccgcatg    17520
   aagcgcgccg agacctactg cccccgcccc ctgctcgcga tccaccccga ccaggccaga    17580
   cacaagcaga agatcgtcgc ccctgtcaag cagctcctga acttcgacct cctgaagctg    17640
   gccggcgacg tggagagcaa tccgggctaa gagct                               17675
<210> 33
   <211> 18089
   <212> DNA
   <213> Artificial Sequence
<220>
   <223> MERF04 plasmid
<400> 33
<210> 34
   <211> 18235
   <212> DNA
   <213> Artificial Sequence
<220>
   <223> MERF05 plasmid
<400> 34
   cgatcgttca aacatttggc aataaagttt cttaagattg aatcctgttg ccggtcttgc      60
   gatgattatc atataatttc tgttgaatta cgttaagcat gtaataatta acatgtaatg     120
   catgacgtta tttatgagat gggtttttat gattagagtc ccgcaattat acatttaata     180
   cgcgatagaa aacaaaatat agcgcgcaaa ctaggataaa ttatcgcgcg cggtgtcatc     240
   tatgttacta gatcgggtgt acatctagaa gctctggaca tcatgttgga tatgaaacaa     300
   ctattattta tctacatgtt ttagatgtta tctgattatt tttataccgt agtcttctat 360
   tgatgaggag tctaaggcta tagaattata tatctaaatg attaatatat atattattaa 420
   taattaacaa taattaatat attataattt atatatatat attttatatt attataataa 480
   tattcttaca aatataatta ttatattcga cggtatcgat aagctcgggc ccaaatcgta 540
   cgggccggcc gtaagcttat cgatgaagga agtggaggag agaggacgcc atggtagcag 600
   aggaaggtct ggcttgatct cccgacgatt cctctctcat cagtgaaaca agagaataag 660
   aggcatcgcg attcttggaa ggtacagagg gaagttgatc aaagagaggc tccgggaaga 720
   agcagatggc ggggaagacg aaacatggcg cctgacaaca taggctatca taggataatc 780
   cactctccct ctgtctttct ctctgtttct ttctctctct ctttatctct ctctctctct 840
   cgaacatttc acacattttt gggcctgttc tttggcgtag ctagcccttt cttggtccat 900
   attttgaggc caaggccaat catgcgcagc cacgttgcat ggcgggagga ccccatctat 960
   ccattccgtc agttcctggc ttttgggaca atctgaacag tacataaacc acgggctcgg 1020
   gcttgggccc gccgaaaagc ccgtccgtac aattttctga cgtacaatat taatttccca 1080
   gaaaagaaaa ttcattaaaa aataactatg tcacccacga actcgtgatc tagatataag 1140
   gcaggcaact ttcccgtgac agtcggactt gtggggttcc cttgacggcg ccgccgtcta 1200
   ctgacggcgc ttgaatgacg tcattattac tatatttaat atatccgaga ataaatggct 1260
   ccctggatcc cccgtgaatg gtcacgtcat ccacgcgggc tttgacttcg cctaggtcgc 1320
   cacgttgggg cctcataacg tgcaagctga cgtggctatc attgcctcga tggcgatctt 1380
   gaacgaacca cataaccaat ctgagacggc gacgaggatt ctgtgttttc ctcggggatc 1440
   tctggccgtc cgatgaacta acacgcacta tttcaaaaag ggggttaaac acgattgtta 1500
   ggtttctttg agtcttccac atctccgctc cacgatcttg tacctcttct tgcgacgatc 1560
   tactcgccct cgaggtcgac accatgtccg gtgcgcctcc caccgacctg cagaagatgg 1620
   tgatgggcaa caccaagccc gtcgagttga tcctcgacgg gaagaccgtg gcgatctgct 1680
   gcgccaccgg cgtgttcggc accgcctacc tcgtcccgag acacctgttc gccgagaagt 1740
   atgacaagat catgctggac gggcgggcca tgaccgactc ggactaccgg gtcttcgagt 1800
   tcgagatcaa agtgaagggc caggatatgc tctccgacgc cgcgctgatg gtgctccaca 1860
   gaggcaaccg cgtgcgggac atcaccaagc acttcaggga caccgcgcgc atgaagaagg 1920
   ggacccctgt ggtcggggtc gtgaacaacg ccgacgtcgg gcgcctcatc ttctccggcg 1980
   aggcgctgac ctacaaggac atcgtcgtgt gcatggacgg ggacacgatg ccggggctct 2040
   tcgcctacaa ggccgcgacc aaggccgggt actgcggggg tgcggtgctc gccaaggacg 2100
   gcgccgacac gttcatcgtg ggcacccact ccgccggcgg taacggcgtc ggctactgct 2160
   cctgcgtgtc ccggtccatg ctgctgcgga tgaaggccca cgttgacccc gagccgcagc 2220
   acgagtaaaa gcttatcgat gtgcacgatc gttcaaacat ttggcaataa agtttcttaa 2280
   gattgaatcc tgttgccggt cttgcgatga ttatcatata atttctgttg aattacgtta 2340
   agcatgtaat aattaacatg taatgcatga cgttatttat gagatgggtt tttatgatta 2400
   gagtcccgca attatacatt taatacgcga tagaaaacaa aatatagcgc gcaaactagg 2460
   ataaattatc gcgcgcggtg tcatctatgt tactagatcg ggtggacaac tcgtcacgta 2520
   ctagtggcgc gcccacgtga tttaaatcgt acgggccggc cgtatactct agtggatccc 2580
   ccaattcaga tcggctgagt ggctccttca acgttgcggt tctgtcagtt ccaaacgtaa 2640
   aacggcttgt cccgcgtcat cggcgggggt cataacgtga ctcccttaat tctccgctca 2700
   tgatcagatt gtcgtttccc gccttcagtt taaactatca gtgtttgaca ggatatattg 2760
   gcgggtaaac ctaagagaaa agagcgttta ttagaataat cggatattta aaagggcgtg 2820
   aaaaggttta tccgttcgtc catttgtatg tgcatgccaa ccacagggtt ccccagatct 2880
   ggcgccggcc agcgagacga gcaagattgg ccgccgcccg aaacgatccg acagcgcgcc 2940
   cagcacaggt gcgcaggcaa attgcaccaa cgcatacagc gccagcagaa tgccatagtg 3000
   ggcggtgacg tcgttcgagt gaaccagatc gcgcaggagg cccggcagca ccggcataat 3060
   caggccgatg ccgacagcgt cgagcgcgac agtgctcaga attacgatca ggggtatgtt 3120
   gggtttcacg tctggcctcc ggaccagcct ccgctggtcc gattgaacgc gcggattctt 3180
   tatcactgat aagttggtgg acatattatg tttatcagtg ataaagtgtc aagcatgaca 3240
   aagttgcagc cgaatacagt gatccgtgcc gccctggacc tgttgaacga ggtcggcgta 3300
   gacggtctga cgacacgcaa actggcggaa cggttggggg ttcagcagcc ggcgctttac 3360
   tggcacttca ggaacaagcg ggcgctgctc gacgcactgg ccgaagccat gctggcggag 3420
   aatcatacgc attcggtgcc gagagccgac gacgactggc gctcatttct gatcgggaat 3480
   gcccgcagct tcaggcaggc gctgctcgcc taccgcgatg gcgcgcgcat ccatgccggc 3540
   acgcgaccgg gcgcaccgca gatggaaacg gccgacgcgc agcttcgctt cctctgcgag 3600
   gcgggttttt cggccgggga cgccgtcaat gcgctgatga caatcagcta cttcactgtt 3660
   ggggccgtgc ttgaggagca ggccggcgac agcgatgccg gcgagcgcgg cggcaccgtt 3720
   gaacaggctc cgctctcgcc gctgttgcgg gccgcgatag acgccttcga cgaagccggt 3780
   ccggacgcag cgttcgagca gggactcgcg gtgattgtcg atggattggc gaaaaggagg 3840
   ctcgttgtca ggaacgttga aggaccgaga aagggtgacg attgatcagg accgctgccg 3900
   gagcgcaacc cactcactac agcagagcca tgtagacaac atcccctccc cctttccacc 3960
   gcgtcagacg cccgtagcag cccgctacgg gctttttcat gccctgccct agcgtccaag 4020
   cctcacggcc gcgctcggcc tctctggcgg ccttctggcg ctcttccgct tcctcgctca 4080
   ctgactcgct gcgctcggtc gttcggctgc ggcgagcggt atcagctcac tcaaaggcgg 4140
   taatacggtt atccacagaa tcaggggata acgcaggaaa gaacatgtga gcaaaaggcc 4200
   agcaaaaggc caggaaccgt aaaaaggccg cgttgctggc gtttttccat aggctccgcc 4260
   cccctgacga gcatcacaaa aatcgacgct caagtcagag gtggcgaaac ccgacaggac 4320
   tataaagata ccaggcgttt ccccctggaa gctccctcgt gcgctctcct gttccgaccc 4380
   tgccgcttac cggatacctg tccgcctttc tcccttcggg aagcgtggcg cttttccgct 4440
   gcataaccct gcttcggggt cattatagcg attttttcgg tatatccatc ctttttcgca 4500
   cgatatacag gattttgcca aagggttcgt gtagactttc cttggtgtat ccaacggcgt 4560
   cagccgggca ggataggtga agtaggccca cccgcgagcg ggtgttcctt cttcactgtc 4620
   ccttattcgc acctggcggt gctcaacggg aatcctgctc tgcgaggctg gccggctacc 4680
   gccggcgtaa cagatgaggg caagcggatg gctgatgaaa ccaagccaac caggaagggc 4740
   agcccaccta tcaaggtgta ctgccttcca gacgaacgaa gagcgattga ggaaaaggcg 4800
   gcggcggccg gcatgagcct gtcggcctac ctgctggccg tcggccaggg ctacaaaatc 4860
   acgggcgtcg tggactatga gcacgtccgc gagctggccc gcatcaatgg cgacctgggc 4920
   cgcctgggcg gcctgctgaa actctggctc accgacgacc cgcgcacggc gcggttcggt 4980
   gatgccacga tcctcgccct gctggcgaag atcgaagaga agcaggacga gcttggcaag 5040
   gtcatgatgg gcgtggtccg cccgagggca gagccatgac ttttttagcc gctaaaacgg 5100
   ccggggggtg cgcgtgattg ccaagcacgt ccccatgcgc tccatcaaga agagcgactt 5160
   cgcggagctg gtgaagtaca tcaccgacga gcaaggcaag accgagcgcc tttgcgacgc 5220
   tcaccgggct ggttgccctc gccgctgggc tggcggccgt ctatggccct gcaaacgcgc 5280
   cagaaacgcc gtcgaagccg tgtgcgagac accgcggccg ccggcgttgt ggatacctcg 5340
   cggaaaactt ggccctcact gacagatgag gggcggacgt tgacacttga ggggccgact 5400
   cacccggcgc ggcgttgaca gatgaggggc aggctcgatt tcggccggcg acgtggagct 5460
   ggccagcctc gcaaatcggc gaaaacgcct gattttacgc gagtttccca cagatgatgt 5520
   ggacaagcct ggggataagt gccctgcggt attgacactt gaggggcgcg actactgaca 5580
   gatgaggggc gcgatccttg acacttgagg ggcagagtgc tgacagatga ggggcgcacc 5640
   tattgacatt tgaggggctg tccacaggca gaaaatccag catttgcaag ggtttccgcc 5700
   cgtttttcgg ccaccgctaa cctgtctttt aacctgcttt taaaccaata tttataaacc 5760
   ttgtttttaa ccagggctgc gccctgtgcg cgtgaccgcg cacgccgaag gggggtgccc 5820
   ccccttctcg aaccctcccg gcccgctaac gcgggcctcc catcccccca ggggctgcgc 5880
   ccctcggccg cgaacggcct caccccaaaa atggcagcgc tggcagtcct tgccattgcc 5940
   gggatcgggg cagtaacggg atgggcgatc agcccgagcg cgacgcccgg aagcattgac 6000
   gtgccgcagg tgctggcatc gacattcagc gaccaggtgc cgggcagtga gggcggcggc 6060
   ctgggtggcg gcctgccctt cacttcggcc gtcggggcat tcacggactt catggcgggg 6120
   ccggcaattt ttaccttggg cattcttggc atagtggtcg cgggtgccgt gctcgtgttc 6180
   gggggtgcga taaacccagc gaaccatttg aggtgatagg taagattata ccgaggtatg 6240
   aaaacgagaa ttggaccttt acagaattac tctatgaagc gccatattta aaaagctacc 6300
   aagacgaaga ggatgaagag gatgaggagg cagattgcct tgaatatatt gacaatactg 6360
   ataagataat atatctttta tatagaagat atcgccgtat gtaaggattt cagggggcaa 6420
   ggcataggca gcgcgcttat caatatatct atagaatggg caaagcataa aaacttgcat 6480
   ggactaatgc ttgaaaccca ggacaataac cttatagctt gtaaattcta tcataattgg 6540
   gtaatgactc caacttattg atagtgtttt atgttcagat aatgcccgat gactttgtca 6600
   tgcagctcca ccgattttga gaacgacagc gacttccgtc ccagccgtgc caggtgctgc 6660
   ctcagattca ggttatgccg ctcaattcgc tgcgtatatc gcttgctgat tacgtgcagc 6720
   tttcccttca ggcgggattc atacagcggc cagccatccg tcatccatat caccacgtca 6780
   aagggtgaca gcaggctcat aagacgcccc agcgtcgcca tagtgcgttc accgaatacg 6840
   tgcgcaacaa ccgtcttccg gagactgtca tacgcgtaaa acagccagcg ctggcgcgat 6900
   ttagccccga catagcccca ctgttcgtcc atttccgcgc agacgatgac gtcactgccc 6960
   ggctgtatgc gcgaggttac cgactgcggc ctgagttttt taagtgacgt aaaatcgtgt 7020
   tgaggccaac gcccataatg cgggctgttg cccggcatcc aacgccattc atggccatat 7080
   caatgatttt ctggtgcgta ccgggttgag aagcggtgta agtgaactgc agttgccatg 7140
   ttttacggca gtgagagcag agatagcgct gatgtccggc ggtgcttttg ccgttacgca 7200
   ccaccccgtc agtagctgaa caggagggac agctgataga cacagaagcc actggagcac 7260
   ctcaaaaaca ccatcataca ctaaatcagt aagttggcag catcacccat aattgtggtt 7320
   tcaaaatcgg ctccgtcgat actatgttat acgccaactt tgaaaacaac tttgaaaaag 7380
   ctgttttctg gtatttaagg ttttagaatg caaggaacag tgaattggag ttcgtcttgt 7440
   tataattagc ttcttggggt atctttaaat actgtagaaa agaggaagga aataataaat 7500
   ggctaaaatg agaatatcac cggaattgaa aaaactgatc gaaaaatacc gctgcgtaaa 7560
   agatacggaa ggaatgtctc ctgctaaggt atataagctg gtgggagaaa atgaaaacct 7620
   atatttaaaa atgacggaca gccggtataa agggaccacc tatgatgtgg aacgggaaaa 7680
   ggacatgatg ctatggctgg aaggaaagct gcctgttcca aaggtcctgc actttgaacg 7740
   gcatgatggc tggagcaatc tgctcatgag tgaggccgat ggcgtccttt gctcggaaga 7800
   gtatgaagat gaacaaagcc ctgaaaagat tatcgagctg tatgcggagt gcatcaggct 7860
   ctttcactcc atcgacatat cggattgtcc ctatacgaat agcttagaca gccgcttagc 7920
   cgaattggat tacttactga ataacgatct ggccgatgtg gattgcgaaa actgggaaga 7980
   agacactcca tttaaagatc cgcgcgagct gtatgatttt ttaaagacgg aaaagcccga 8040
   agaggaactt gtcttttccc acggcgacct gggagacagc aacatctttg tgaaagatgg 8100
   caaagtaagt ggctttattg atcttgggag aagcggcagg gcggacaagt ggtatgacat 8160
   tgccttctgc gtccggtcga tcagggagga tatcggggaa gaacagtatg tcgagctatt 8220
   ttttgactta ctggggatca agcctgattg ggagaaaata aaatattata ttttactgga 8280
   tgaattgttt tagtacctag atgtggcgca acgatgccgg cgacaagcag gagcgcaccg 8340
   acttcttccg catcaagtgt tttggctctc aggccgaggc ccacggcaag tatttgggca 8400
   aggggtcgct ggtattcgtg cagggcaaga ttcggaatac caagtacgag aaggacggcc 8460
   agacggtcta cgggaccgac ttcattgccg ataaggtgga ttatctggac accaaggcac 8520
   caggcgggtc aaatcaggaa taagggcaca ttgccccggc gtgagtcggg gcaatcccgc 8580
   aaggagggtg aatgaatcgg acgtttgacc ggaaggcata caggcaagaa ctgatcgacg 8640
   cggggttttc cgccgaggat gccgaaacca tcgcaagccg caccgtcatg cgtgcgcccc 8700
   gcgaaacctt ccagtccgtc ggctcgatgg tccagcaagc tacggccaag atcgagcgcg 8760
   acagcgtgca actggctccc cctgccctgc ccgcgccatc ggccgccgtg gagcgttcgc 8820
   gtcgtctcga acaggaggcg gcaggtttgg cgaagtcgat gaccatcgac acgcgaggaa 8880
   ctatgacgac caagaagcga aaaaccgccg gcgaggacct ggcaaaacag gtcagcgagg 8940
   ccaagcaggc cgcgttgctg aaacacacga agcagcagat caaggaaatg cagctttcct 9000
   tgttcgatat tgcgccgtgg ccggacacga tgcgagcgat gccaaacgac acggcccgct 9060
   ctgccctgtt caccacgcgc aacaagaaaa tcccgcgcga ggcgctgcaa aacaaggtca 9120
   ttttccacgt caacaaggac gtgaagatca cctacaccgg cgtcgagctg cgggccgacg 9180
   atgacgaact ggtgtggcag caggtgttgg agtacgcgaa gcgcacccct atcggcgagc 9240
   cgatcacctt cacgttctac gagctttgcc aggacctggg ctggtcgatc aatggccggt 9300
   attacacgaa ggccgaggaa tgcctgtcgc gcctacaggc gacggcgatg ggcttcacgt 9360
   ccgaccgcgt tgggcacctg gaatcggtgt cgctgctgca ccgcttccgc gtcctggacc 9420
   gtggcaagaa aacgtcccgt tgccaggtcc tgatcgacga ggaaatcgtc gtgctgtttg 9480
   ctggcgacca ctacacgaaa ttcatatggg agaagtaccg caagctgtcg ccgacggccc 9540
   gacggatgtt cgactatttc agctcgcacc gggagccgta cccgctcaag ctggaaacct 9600
   tccgcctcat gtgcggatcg gattccaccc gcgtgaagaa gtggcgcgag caggtcggcg 9660
   aagcctgcga agagttgcga ggcagcggcc tggtggaaca cgcctgggtc aatgatgacc 9720
   tggtgcattg caaacgctag ggccttgtgg ggtcagttcc ggctgggggt tcagcagcca 9780
   gcgctttact ggcatttcag gaacaagcgg gcactgctcg acgcacttgc ttcgctcagt 9840
   atcgctcggg acgcacggcg cgctctacga actgccgata aacagaggat taaaattgac 9900
   aattgtgatt aaggctcaga ttcgacggct tggagcggcc gacgtgcagg atttccgcga 9960
   gatccgattg tcggccctga agaaagctcc agagatgttc gggtccgttt acgagcacga 10020
   ggagaaaaag cccatggagg cgttcgctga acggttgcga gatgccgtgg cattcggcgc 10080
   ctacatcgac ggcgagatca ttgggctgtc ggtcttcaaa caggaggacg gccccaagga 10140
   cgctcacaag gcgcatctgt ccggcgtttt cgtggagccc gaacagcgag gccgaggggt 10200
   cgccggtatg ctgctgcggg cgttgccggc gggtttattg ctcgtgatga tcgtccgaca 10260
   gattccaacg ggaatctggt ggatgcgcat cttcatcctc ggcgcactta atatttcgct 10320
   attctggagc ttgttgttta tttcggtcta ccgcctgccg ggcggggtcg cggcgacggt 10380
   aggcgctgtg cagccgctga tggtcgtgtt catctctgcc gctctgctag gtagcccgat 10440
   acgattgatg gcggtcctgg gggctatttg cggaactgcg ggcgtggcgc tgttggtgtt 10500
   gacaccaaac gcagcgctag atcctgtcgg cgtcgcagcg ggcctggcgg gggcggtttc 10560
   catggcgttc ggaaccgtgc tgacccgcaa gtggcaacct cccgtgcctc tgctcacctt 10620
   taccgcctgg caactggcgg ccggaggact tctgctcgtt ccagtagctt tagtgtttga 10680
   tccgccaatc ccgatgccta caggaaccaa tgttctcggc ctggcgtggc tcggcctgat 10740
   cggagcgggt ttaacctact tcctttggtt ccgggggatc tcgcgactcg aacctacagt 10800
   tgtttcctta ctgggctttc tcagccccag atctggggtc gatcagccgg ggatgcatca 10860
   ggccgacagt cggaacttcg ggtccccgac ctgtaccatt cggtgagcaa tggatagggg 10920
   agttgatatc gtcaacgttc acttctaaag aaatagcgcc actcagcttc ctcagcggct 10980
   ttatccagcg atttcctatt atgtcggcat agttctcaag atcgacagcc tgtcacggtt 11040
   aagcgagaaa tgaataagaa ggctgataat tcggatctct gcgagggaga tgatatttga 11100
   tcacaggcag caacgctctg tcatcgttac aatcaacatg ctaccctccg cgagatcatc 11160
   cgtgtttcaa acccggcagc ttagttgccg ttcttccgaa tagcatcggt aacatgagca 11220
   aagtctgccg ccttacaacg gctctcccgc tgacgccgtc ccggactgat gggctgcctg 11280
   tatcgagtgg tgattttgtg ccgagctgcc ggtcggggag ctgttggctg gctggtggca 11340
   ggatatattg tggtgtaaac aaattgacgc ttagacaact taataacaca ttgcggacgt 11400
   ttttaatgta ctggggtggt ttttcttttc accagtgaga cgggcaacag ctgattgccc 11460
   ttcaccgcct ggccctgaga gagttgcagc aagcggtcca cgctggtttg ccccagcagg 11520
   cgaaaatcct gtttgatggt ggttccgaaa tcggcaaaat cccttataaa tcaaaagaat 11580
   agcccgagat agggttgagt gttgttccag tttggaacaa gagtccacta ttaaagaacg 11640
   tggactccaa cgtcaaaggg cgaaaaaccg tctatcaggg cgatggccca ctacgtgaac 11700
   catcacccaa atcaagtttt ttggggtcga ggtgccgtaa agcactaaat cggaacccta 11760
   aagggagccc ccgatttaga gcttgacggg gaaagccggc gaacgtggcg agaaaggaag 11820
   ggaagaaagc gaaaggagcg ggcgccattc aggctgcgca actgttggga agggcgatcg 11880
   gtgcgggcct cttcgctatt acgccagctg gcgaaagggg gatgtgctgc aaggcgatta 11940
   agttgggtaa cgccagggtt ttcccagtca cgacgttgta aaacgacggc cagtgaatta 12000
   attcccatct tgaaagaaat atagtttaaa tatttattga taaaataaca agtcaggtat 12060
   tatagtccaa gcaaaaacat aaatttattg atgcaagttt aaattcagaa atatttcaat 12120
   aactgattat atcagctggt acattgccgt agatgaaaga ctgagtgcga tattatgtgt 12180
   aatacataaa ttgatgatat agctagctta gctcatcggg ggatccgtcg aagctagctt 12240
   gggtcccgct cagaagaact cgtcaagaag gcgatagaag gcgatgcgct gcgaatcggg 12300
   agcggcgata ccgtaaagca cgaggaagcg gtcagcccat tcgccgccaa gctcttcagc 12360
   aatatcacgg gtagccaacg ctatgtcctg atagcggtcc gccacaccca gccggccaca 12420
   gtcgatgaat ccagaaaagc ggccattttc caccatgata ttcggcaagc aggcatcgcc 12480
   atgggtcacg acgagatcct cgccgtcggg catgcgcgcc ttgagcctgg cgaacagttc 12540
   ggctggcgcg agcccctgat gctcttcgtc cagatcatcc tgatcgacaa gaccggcttc 12600
   catccgagta cgtgctcgct cgatgcgatg tttcgcttgg tggtcgaatg ggcaggtagc 12660
   cggatcaagc gtatgcagcc gccgcattgc atcagccatg atggatactt tctcggcagg 12720
   agcaaggtga gatgacagga gatcctgccc cggcacttcg cccaatagca gccagtccct 12780
   tcccgcttca gtgacaacgt cgagcacagc tgcgcaagga acgcccgtcg tggccagcca 12840
   cgatagccgc gctgcctcgt cctgcagttc attcagggca ccggacaggt cggtcttgac 12900
   aaaaagaacc gggcgcccct gcgctgacag ccggaacacg gcggcatcag agcagccgat 12960
   tgtctgttgt gcccagtcat agccgaatag cctctccacc caagcggccg gagaacctgc 13020
   gtgcaatcca tcttgttcaa tccaagctcc catgggccct cgactagagt cgagatctgg 13080
   attgagagtg aatatgagac tctaattgga taccgagggg aatttatgga acgtcagtgg 13140
   agcatttttg acaagaaata tttgctagct gatagtgacc ttaggcgact tttgaacgcg 13200
   caataatggt ttctgacgta tgtgcttagc tcattaaact ccagaaaccc gcggctgagt 13260
   ggctccttca acgttgcggt tctgtcagtt ccaaacgtaa aacggcttgt cccgcgtcat 13320
   cggcgggggt cataacgtga ctcccttaat tctccgctca tgatcttgat cccctgcgcc 13380
   atcagatcct tggcggcaag aaagccatcc agtttacttt gcagggcttc ccaaccttac 13440
   cagagggcgc cccagctggc aattccggtt cgcttgctgt ccataaaacc gcccagtcta 13500
   gctatcgcca tgtaagccca ctgcaagcta cctgctttct ctttgcgctt gcgttttccc 13560
   ttgtccagat agcccagtag ctgacattca tccggggtca gcaccgtttc tgcggactgg 13620
   ctttctacgt gttccgcttc ctttagcagc ccttgcgccc tgagtgcttg cggcagcgtg 13680
   aagctctgga catcatgttg gatatgaaac aactattatt tatctacatg ttttagatgt 13740
   tatctgatta tttttatacc gtagtcttct attgatgagg agtctaaggc tatagaatta 13800
   tatatctaaa tgattaatat atatattatt aataattaac aataattaat atattataat 13860
   ttatatatat atattttata ttattataat aatattctta caaatataat tattatattc 13920
   gacggtatcg atgatggaca gataatgaga tgaattagaa aaaaaaaatt cgtgttgtaa 13980
   gatagaatac ttgctatcta ctgatgaatg cagttcagtt ttcctcacga tcttaaagat 14040
   cgcgcactat cctcagcttc actctggaaa ttttgattct cttcttctgc tcagcagcct 14100
   cgactctgtc tagggtttcg tacaatcgga cgccattcta catgaatcga gcacagggaa 14160
   tgaagacaat taggagatcc tcgatgtcct ccgacttact tgcatgactt gacggggaag 14220
   atctcgagca gggaagcgac gcctctccgg aggactcgcc tcgccgagag gacctcctcc 14280
   gcgacacgga ccatggcctc cacggggtag aagctggccc tgttctttat tctcttgagg 14340
   atcatcggcc gaagcctccg caaatccatc cccgaggagt agaatctcgc ctgcaggaag 14400
   catctgtcga gatcctcgcc gaggcggcgg agatacctcg ccggcgccgc catggcgccg 14460
   gggacggagc accaccacgg agaagaagaa ccctaaccca aggcattaac gaagttgcgc 14520
   agattataca aaagccctca aatatctttc attttctatt tcactgatac attttcatta 14580
   ttgtatatga gtgtttattt aaattattcc gtattagaaa agcacctcca gaacccgaca 14640
   aaatagggtg acgtcatcat ggtgtcatga ccgcccaaca gccgcagatt taaaatcggt 14700
   ggatgagtgc ggccacgcca cgaaagcgat gggccttcgt cgatgccgtg agaatccatc 14760
   tgacataaag taaacggcgc cgtcagtatt gacggcgtat gacacgtgga aagaagctat 14820
   tggttcacgc atcggtggtt ccgctagcct ccgtccaccg ctagtactat aaatacggtc 14880
   ccgaggcctc ctcaccactc gcacatatcc tctttgtttt cctctccgtg aaagaagcga 14940
   ggaagcgcgt cgtctctccc aaggtaagga gcagatctct ttgatcgttt ttgttcttct 15000
   tttgttttgt tttttttttc tgcggatctt cggttgcatc atgccttggc tgtttttatt 15060
   agtttaggat atcctcgttt ggatctgagc cgatcatata tgttaaaggt tgtgttcgat 15120
   ctctttgttc attttcgcat gaaaaggatg tatccttttg atgtgaggcg atcttctatg 15180
   gttaagactt tgttcggtct attgatcatt tctgttcttc gtttttgagt ttttttctgc 15240
   ggatatcgca tcatccctag gtttttgctt tggttaggat gcatcctttg gatttgagcc 15300
   gatctccctt ggttaaggct gtgtctgttg cagaggagaa agtctgtcga ggtccttatg 15360
   caggctttgt ccagatgcgc gtgctctctc atgctatgaa tttatgtttt gagaactcct 15420
   cccggttttt ctagatccgg atttgaagta ttcattgcgg ttccccttcg gttttatgta 15480
   tttctcgagt tgatttggtc catgatcgtg ttctgtccag atctctcttg atatggatga 15540
   gatattcgtt acctctttca aacatcggtg gatgttcttt ttagtcttgg ctcaccttta 15600
   tctagaaatt aattttcggt ttgaaacccc tgcttgttaa ggtgatgtat tccttcttta 15660
   tagatttcgg tgtgttattt cttaacggtg atctgtccga tccatgtgtt gcacctcttg 15720
   ttttctgtgt aatcctctgt gaattataat tatgttttga aaacgtactt aagtaagggg 15780
   catgttcccc gtttaaaact tttgttctat caatttgtgg ttaatagatc ctgatttgtg 15840
   gtcgccttat tctgtcttta atcgtggatt ttatttatct tgagcgcgtc cttttctttt 15900
   aaaatcatgt gtttaacctt tcagtcgtca tatgttccat caggtcgacg tacaagggcg 15960
   aattcaccat gggggccggg cagtcgtccc cggccacggg gtcccagaac cagtccggca 16020
   acaccggcag catcatcaac aactactaca tgcagcagta ccagaactcc atggacaccc 16080
   aactggggga caacgccacg agcggcggca gcaatgaggg ctccaccgac accacgagca 16140
   cccacacgac caacacccag aacaacgact ggttcagcaa gctcgcgtcc agcgccttct 16200
   ccgggctctt cggggcgctc ctggccgaca agaagaccga ggagaccacg ctgctcgagg 16260
   accgcatcct caccacgcgg aacggccaca cgacctccac cacgcagtcg agcgtcgggg 16320
   tcacttacgg ttacgccacg gccgaggact tcgtgagcgg ccccaacacc tccggcctcg 16380
   agactcgggt cgcccaggcc gagcgcttct tcaagacgca cctgttcgac tgggtgacct 16440
   ccgacccctt cggccgctgc cacctcttgg aactgcccac cgaccacaag ggcgtgtacg 16500
   ggtcgttgac ggacagctac gcgtacatga ggaacgggtg ggacgtggag gtcaccgccg 16560
   tcggcaacca gttcaacggg ggctgcctcc tggtggccat ggtccccgaa ctctgctcga 16620
   tccagaagcg ggagctgtac cagctgaccc tcttcccgca ccagttcatc aacccgcgca 16680
   ccaacatgac cgcgcacatc accgtcccct tcgtgggcgt gaaccgctac gaccagtaca 16740
   aagtccacaa gccctggacc ttggtggtca tggtcgtcgc ccccctcacc gtcaactcgg 16800
   agggggcgcc ccagatcaaa gtctacgcca acatcgcccc cacgaacgtg cacgttgccg 16860
   gcgagttccc gagcaaggag ggcatcttcc cggttgcgtg ctccgacggc tacgggggcc 16920
   tcgtgacgac cgacccgaag accgccgacc ctgcctacgg caaggtgttc aacccgccca 16980
   gaaacatgct ccctgggaga ttcacgaact tcctcgacgt ggccgaggcc tgccccacgt 17040
   tcctgcactt cgagggggac gtcccttacg tcaccacgaa gactgactcg gatcgcgtgc 17100
   tggcccagtt cgacctgtcc ctcgccgcta agcacatgtc caacaccttc ctcgccggcc 17160
   tcgcgcaata ctacacccag tacagcggca ccatcaacct ccacttcatg ttcaccggcc 17220
   ccaccgacgc caaggcccgc tacatgatcg cttacgcccc gcccggcatg gagcccccga 17280
   agacccccga ggcggccgcg cactgcatcc acgccgagtg ggacacgggg ctcaactcca 17340
   agttcacgtt ctccatcccc tacctcagcg ccgcggacta cgcgtacacc gccagcgaca 17400
   ccgcggagac cacgaacgtg cagggctggg tgtgcctgtt ccagatcacc cacgggaagg 17460
   ccgacggcga cgccctggtc gtgctggcct cggcggggaa ggacttcgaa ctcaggctgc 17520
   cggtggacgc ccgcacccag acgactagcg ccggcgagtc cgcggacccg gtgaccgcga 17580
   ccgtcgagaa ctacgggggc gagacccaag tgcagcgcag acagcacacg gacgtgtcct 17640
   tcatcctgga tcgcttcgtg aaagtgaccc cgaaggacca gatcaacgtg ctggacctga 17700
   tgcagacgcc ggcgcacacc ctcgtggggg ccctcctgcg caccgccacg tactacttcg 17760
   cggacctcga ggtggcggtg aagcacgagg gcaacctgac gtgggtgccc aacggtgcgc 17820
   cggaggccgc gctcgacaac accacgaacc ccaccgcgta ccacaaggcc cccctgaccc     17880
   ggctcgccct cccctacacc gccccccaca gggtcctcgc cacggtctac aacggcaaca     17940
   gcaagtacgg ggacgggacc gtcgccaacg tgcgcgggga cctccaagtg ctcgcccaga     18000
   aggccgcgcg cgccctcccc actagcttca actacggtgc catcaaggcc acgagggtga     18060
   cggagctgct ctaccgcatg aagcgcgccg agacctactg cccccgcccc ctgctcgcga     18120
   tccaccccga ccaggccaga cacaagcaga agatcgtcgc ccctgtcaag cagctcctga     18180
   acttcgacct cctgaagctg gccggcgacg tggagagcaa tccgggctaa gagct          18235
<210> 35
   <211> 18649
   <212> DNA
   <213> Artificial Sequence
<220>
   <223> MERF06 plasmid
<400> 35
<210> 36
   <211> 16496
   <212> DNA
   <213> Artificial Sequence
<220>
   <223> MERE01 Plasmid
<400> 36
   ggacatggca accgggcctg aatttcgcgg gtattctgtt tctattccaa ctttttcttg      60
   atccgcagcc attaacgact tttgaataga tacgctgaca cgccaagcct cgctagtcaa     120
   aagtgtacca aacaacgctt tacagcaaga acggaatgcg cgtgacgctc gcggtgacgc     180
   catttcgcct tttcagaaat ggataaatag ccttgcttcc tattatatct tcccccaaat     240
   taattaagaa actcccgagg tgagcaagga tccggagtcg agcgcgaaga agagaaagag     300
   ggaaagcgcg ggtaccgggc ccccccctcg acggatcaag tgcaaaggtc cgccttgttt     360
   ctcctctgtc tcttgatctg actaatcttg gtttatgatt cgttgagtaa ttttggggaa     420
   agctagcttc gtccacagtt tttttttcga tgaacagtgc cgcagtggcg ctgatcttgt      480
   atgctatcct gcaatcgtgg tgaacttatt tcttttatat ccttcactcc catgaaaagg     540
   ctagtaatct ttctcgatgt aacatcgtcc agcactgcta ttaccgtgtg gtccatccga     600
   cagtctggct gaacacatca tacgatattg agcaaagatc gatctatctt ccctgttctt     660
   taatgaaaga cgtcattttc atcagtatga tctaagaatg ttgcaacttg caaggaggcg     720
   tttctttctt tgaatttaac taactcgttg agtggccctg tttctcggac gtaaggcctt     780
   tgctgctcca cacatgtcca ttcgaatttt accgtgttta gcaagggcga aaagtttgca     840
   tcttgatgat ttagcttgac tatgcgattg ctttcctgga cccgtgcagc tgcggacgga     900
   tcccccgctc gaggtcgacg gtatcgataa gcttgatcag atctgatcga attcaccatg     960
   ggcgccgggc agtcctcgcc tgcgacgggg agccagaacc agtcgggcaa caccggctcg    1020
   atcatcaaca actactacat gcagcaatac cagaacagca tggacaccca gctcggcgat    1080
   aacgccatct ccggcgggtc caacgagggc tcgaccgaca cgacctccac ccacaccacg    1140
   aacacccaga acaatgactg gttcagcaag ctggcctcca gcgccttcac gggcctgttc    1200
   ggggcgctgc tcgcggacaa gaagaccgag gagaccacgc tgctcgagga ccggatcctc    1260
   accacgcgca acgggcacac gaccagcacg acccagtcca gcgtcggggt gacccacggc    1320
   tactccacgg aggaggacca cgtcgccggg ccgaacacga gcggcctcga gacccgcgtg    1380
   gtccaggcgg agcgcttcta caagaagtac ctgttcgact ggaccacgga caaggcgttc    1440
   gggcacctcg agaagctgga gctgccgagc gaccaccacg gcgtcttcgg ccacctcgtg    1500
   gactcctacg cgtacatgag aaatgggtgg gacgtcgagg tcagcgccgt cggcaaccag    1560
   ttcaacggcg ggtgcctgct cgtggcgatg gtcccggagt ggaaggagtt cgacacccgc    1620
   gagaagtatc agctgaccct cttcccccac cagttcatca gcccgcgcac caacatgacc    1680
   gcccacatca cggtgccgta cctcggggtg aaccgctacg accagtacaa gaagcacaag    1740
   ccctggaccc tcgtggtcat ggtggtctcc ccgctgactg tgaacaacac gtccgccgcg    1800
   cagatcaaag tctacgccaa catcgccccc acctacgtgc acgtcgccgg cgagctgccc 1860
   tccaaggagg gcatcttccc cgtcgcctgc gccgacgggt acgggggcct ggtgaccacc 1920
   gaccccaaga cggccgaccc ggcctacggc aaagtgtaca acccgcccag gacgaactac 1980
   cccggtcgct tcaccaacct cctggacgtg gcggaggcct gcccgacctt cctgtgcttc 2040
   gacgacggga agccctacgt caccacgcgc accgacgaca cgcgcctgct cgccaagttc 2100
   gacctcagcc tggccgctaa gcacatgagc aacacctacc tcagcggcat cgcccaatac 2160
   tatacccaat actcgggcac catcaacctg cacttcatgt tcacgggcag caccgacagc 2220
   aaggcccggt acatggtggc ctacatcccg ccgggcgtgg agacccctcc cgacacgccc 2280
   gagcgggctg cgcactgcat ccacgccgag tgggacaccg gcctcaacag caagttcacg 2340
   ttcagcatcc cctacgtgtc cgccgcggat tacgcttaca ccgcctcgga cacggccgag 2400
   acgatcaacg tccagggctg ggtctgcatc taccagatca ctcacggcaa ggccgagaac 2460
   gacaccctcg tcgtgagcgt ctccgccggg aaggacttcg agctgaggct gcccatcgac 2520
   cccaggcagc agaccacggc gaccggggag tccgccgacc ccgtgaccac gaccgtggag 2580
   aactacggcg gggagactca gatccagcgg cgccaccaca ccgacatcgg cttcatcatg 2640
   gaccgcttcg tgaagatcca gtccctgtcg cccacccacg ttatcgacct catgcaggcc 2700
   caccagcacg ggctcgtggg tgccctcctg cgcgcggcca cctactactt cagcgacctc 2760
   gagatcgttg tccgccacga ggggaacctc acctgggtcc cgaacggtgc ccccgagagc 2820
   gccctgctca acacctccaa ccccacggcg tacaacaagg cccccttcac gcgcctcgcc 2880
   ctgccttaca ccgcgcccca ccgcgtgctg gccacggtgt acaacgggac ctccaagtac 2940
   gccgtgggcg ggagcggccg cagaggcgac atggggagcc tcgccgctag ggtcgtgaag 3000
   cagctcccgg cctccttcaa ctacggcgcc atcaaggccg acgccatcca cgaactcctg 3060
   gtccgcatga agcgcgccga actctactgc ccccggcccc tcctggccat cgaggtctcc 3120
   tcgcaggacc gccacaagca gaagatcatc gccccggcca agcagctgct caacttcgac 3180
   ctgctcaagc tcgcggggga cgtcgagtcg aaccccggtc ccttcttctt cgccgacgtg 3240
   cggtccaact tctcgaagct cgtggacacc atcaaccaga tgcaggagga catgagcacc 3300
   aagcacgggc cggacttcaa ccgcctcgtc tcggccttcg aggaactcgc caccggggtc 3360
   aaggccatca ggacgggcct ggacgaggcg aagccgtggt acaagctcat caagctcctg 3420
   tcgcgcctct cctgcatggc ggctgtcgcg gcccgctcca aggaccccgt cctcgtcgcc 3480
   atcatgctcg cggacaccgg gctcgagcgc cagcggcccc tgaaagtgcg cgccaagctg 3540
   ccgcagcagg agggccccta cgccggcccg ctcgagagac agaagccgct gaaagtcaag 3600
   gccaaggcgc ccgtggtcaa ggaggggccc tacgagggcc ccgtcaagaa gccggtggcg 3660
   ctgaaagtga aggccaagaa cctcatcgtg accgagtccg gtgcgcctcc caccgacctg 3720
   cagaagatgg tgatgggcaa caccaagccc gtcgagttga tcctcgacgg gaagaccgtg 3780
   gcgatctgct gcgccaccgg cgtgttcggc accgcctacc tcgtcccgag acacctgttc 3840
   gccgagaagt atgacaagat catgctggac gggcgggcca tgaccgactc ggactaccgg 3900
   gtcttcgagt tcgagatcaa agtgaagggc caggatatgc tctccgacgc cgcgctgatg 3960
   gtgctccaca gaggcaaccg cgtgcgggac atcaccaagc acttcaggga caccgcgcgc 4020
   atgaagaagg ggacccctgt ggtcggggtc gtgaacaacg ccgacgtcgg gcgcctcatc 4080
   ttctccggcg aggcgctgac ctacaaggac atcgtcgtgt gcatggacgg ggacacgatg 4140
   ccggggctct tcgcctacaa ggccgcgacc aaggccgggt actgcggggg tgcggtgctc 4200
   gccaaggacg gcgccgacac gttcatcgtg ggcacccact ccgccggcgg taacggcgtc 4260
   ggctactgct cctgcgtgtc ccggtccatg ctgctgcgga tgaaggccca cgttgacccc 4320
   gagccgcagc acgagtaaga gctcgaattt ccccgatcgt tcaaacattt ggcaataaag 4380
   tttcttaaga ttgaatcctg ttgccggtct tgcgatgatt atcatataat ttctgttgaa 4440
   ttacgttaag catgtaataa ttaacatgta atgcatgacg ttatttatga gatgggtttt 4500
   tatgattaga gtcccgcaat tatacattta atacgcgata gaaaacaaaa tatagcgcgc 4560
   aaactaggat aaattatcgc gcgcggtgtc atctatgtta ctagatcggg aattaattca 4620
   gatcggctga gtggctcctt caacgttgcg gttctgtcag ttccaaacgt aaaacggctt 4680
   gtcccgcgtc atcggcgggg gtcataacgt gactccctta attctccgct catgatcaga 4740
   ttgtcgtttc ccgccttcag tttaaactat cagtgtttga caggatatat tggcgggtaa 4800
   acctaagaga aaagagcgtt tattagaata atcggatatt taaaagggcg tgaaaaggtt 4860
   tatccgttcg tccatttgta tgtgcatgcc aaccacaggg ttccccagat ctggcgccgg 4920
   ccagcgagac gagcaagatt ggccgccgcc cgaaacgatc cgacagcgcg cccagcacag 4980
   gtgcgcaggc aaattgcacc aacgcataca gcgccagcag aatgccatag tgggcggtga 5040
   cgtcgttcga gtgaaccaga tcgcgcagga ggcccggcag caccggcata atcaggccga 5100
   tgccgacagc gtcgagcgcg acagtgctca gaattacgat caggggtatg ttgggtttca 5160
   cgtctggcct ccggaccagc ctccgctggt ccgattgaac gcgcggattc tttatcactg 5220
   ataagttggt ggacatatta tgtttatcag tgataaagtg tcaagcatga caaagttgca 5280
   gccgaataca gtgatccgtg ccgccctgga cctgttgaac gaggtcggcg tagacggtct 5340
   gacgacacgc aaactggcgg aacggttggg ggttcagcag ccggcgcttt actggcactt 5400
   caggaacaag cgggcgctgc tcgacgcact ggccgaagcc atgctggcgg agaatcatac 5460
   gcattcggtg ccgagagccg acgacgactg gcgctcattt ctgatcggga atgcccgcag 5520
   cttcaggcag gcgctgctcg cctaccgcga tggcgcgcgc atccatgccg gcacgcgacc 5580
   gggcgcaccg cagatggaaa cggccgacgc gcagcttcgc ttcctctgcg aggcgggttt 5640
   ttcggccggg gacgccgtca atgcgctgat gacaatcagc tacttcactg ttggggccgt 5700
   gcttgaggag caggccggcg acagcgatgc cggcgagcgc ggcggcaccg ttgaacaggc 5760
   tccgctctcg ccgctgttgc gggccgcgat agacgccttc gacgaagccg gtccggacgc 5820
   agcgttcgag cagggactcg cggtgattgt cgatggattg gcgaaaagga ggctcgttgt 5880
   caggaacgtt gaaggaccga gaaagggtga cgattgatca ggaccgctgc cggagcgcaa 5940
   cccactcact acagcagagc catgtagaca acatcccctc cccctttcca ccgcgtcaga 6000
   cgcccgtagc agcccgctac gggctttttc atgccctgcc ctagcgtcca agcctcacgg 6060
   ccgcgctcgg cctctctggc ggccttctgg cgctcttccg cttcctcgct cactgactcg 6120
   ctgcgctcgg tcgttcggct gcggcgagcg gtatcagctc actcaaaggc ggtaatacgg 6180
   ttatccacag aatcagggga taacgcagga aagaacatgt gagcaaaagg ccagcaaaag 6240
   gccaggaacc gtaaaaaggc cgcgttgctg gcgtttttcc ataggctccg cccccctgac 6300
   gagcatcaca aaaatcgacg ctcaagtcag aggtggcgaa acccgacagg actataaaga 6360
   taccaggcgt ttccccctgg aagctccctc gtgcgctctc ctgttccgac cctgccgctt 6420
   accggatacc tgtccgcctt tctcccttcg ggaagcgtgg cgcttttccg ctgcataacc 6480
   ctgcttcggg gtcattatag cgattttttc ggtatatcca tcctttttcg cacgatatac 6540
   aggattttgc caaagggttc gtgtagactt tccttggtgt atccaacggc gtcagccggg 6600
   caggataggt gaagtaggcc cacccgcgag cgggtgttcc ttcttcactg tcccttattc 6660
   gcacctggcg gtgctcaacg ggaatcctgc tctgcgaggc tggccggcta ccgccggcgt 6720
   aacagatgag ggcaagcgga tggctgatga aaccaagcca accaggaagg gcagcccacc 6780
   tatcaaggtg tactgccttc cagacgaacg aagagcgatt gaggaaaagg cggcggcggc 6840
   cggcatgagc ctgtcggcct acctgctggc cgtcggccag ggctacaaaa tcacgggcgt 6900
   cgtggactat gagcacgtcc gcgagctggc ccgcatcaat ggcgacctgg gccgcctggg 6960
   cggcctgctg aaactctggc tcaccgacga cccgcgcacg gcgcggttcg gtgatgccac 7020
   gatcctcgcc ctgctggcga agatcgaaga gaagcaggac gagcttggca aggtcatgat 7080
   gggcgtggtc cgcccgaggg cagagccatg acttttttag ccgctaaaac ggccgggggg 7140
   tgcgcgtgat tgccaagcac gtccccatgc gctccatcaa gaagagcgac ttcgcggagc 7200
   tggtgaagta catcaccgac gagcaaggca agaccgagcg cctttgcgac gctcaccggg 7260
   ctggttgccc tcgccgctgg gctggcggcc gtctatggcc ctgcaaacgc gccagaaacg 7320
   ccgtcgaagc cgtgtgcgag acaccgcggc cgccggcgtt gtggatacct cgcggaaaac 7380
   ttggccctca ctgacagatg aggggcggac gttgacactt gaggggccga ctcacccggc 7440
   gcggcgttga cagatgaggg gcaggctcga tttcggccgg cgacgtggag ctggccagcc 7500
   tcgcaaatcg gcgaaaacgc ctgattttac gcgagtttcc cacagatgat gtggacaagc 7560
   ctggggataa gtgccctgcg gtattgacac ttgaggggcg cgactactga cagatgaggg 7620
   gcgcgatcct tgacacttga ggggcagagt gctgacagat gaggggcgca cctattgaca 7680
   tttgaggggc tgtccacagg cagaaaatcc agcatttgca agggtttccg cccgtttttc 7740
   ggccaccgct aacctgtctt ttaacctgct tttaaaccaa tatttataaa ccttgttttt 7800
   aaccagggct gcgccctgtg cgcgtgaccg cgcacgccga aggggggtgc ccccccttct 7860
   cgaaccctcc cggcccgcta acgcgggcct cccatccccc caggggctgc gcccctcggc 7920
   cgcgaacggc ctcaccccaa aaatggcagc gctggcagtc cttgccattg ccgggatcgg 7980
   ggcagtaacg ggatgggcga tcagcccgag cgcgacgccc ggaagcattg acgtgccgca 8040
   ggtgctggca tcgacattca gcgaccaggt gccgggcagt gagggcggcg gcctgggtgg 8100
   cggcctgccc ttcacttcgg ccgtcggggc attcacggac ttcatggcgg ggccggcaat 8160
   ttttaccttg ggcattcttg gcatagtggt cgcgggtgcc gtgctcgtgt tcgggggtgc 8220
   gataaaccca gcgaaccatt tgaggtgata ggtaagatta taccgaggta tgaaaacgag 8280
   aattggacct ttacagaatt actctatgaa gcgccatatt taaaaagcta ccaagacgaa 8340
   gaggatgaag aggatgagga ggcagattgc cttgaatata ttgacaatac tgataagata 8400
   atatatcttt tatatagaag atatcgccgt atgtaaggat ttcagggggc aaggcatagg 8460
   cagcgcgctt atcaatatat ctatagaatg ggcaaagcat aaaaacttgc atggactaat 8520
   gcttgaaacc caggacaata accttatagc ttgtaaattc tatcataatt gggtaatgac 8580
   tccaacttat tgatagtgtt ttatgttcag ataatgcccg atgactttgt catgcagctc 8640
   caccgatttt gagaacgaca gcgacttccg tcccagccgt gccaggtgct gcctcagatt 8700
   caggttatgc cgctcaattc gctgcgtata tcgcttgctg attacgtgca gctttccctt 8760
   caggcgggat tcatacagcg gccagccatc cgtcatccat atcaccacgt caaagggtga 8820
   cagcaggctc ataagacgcc ccagcgtcgc catagtgcgt tcaccgaata cgtgcgcaac 8880
   aaccgtcttc cggagactgt catacgcgta aaacagccag cgctggcgcg atttagcccc 8940
   gacatagccc cactgttcgt ccatttccgc gcagacgatg acgtcactgc ccggctgtat 9000
   gcgcgaggtt accgactgcg gcctgagttt tttaagtgac gtaaaatcgt gttgaggcca 9060
   acgcccataa tgcgggctgt tgcccggcat ccaacgccat tcatggccat atcaatgatt 9120
   ttctggtgcg taccgggttg agaagcggtg taagtgaact gcagttgcca tgttttacgg 9180
   cagtgagagc agagatagcg ctgatgtccg gcggtgcttt tgccgttacg caccaccccg 9240
   tcagtagctg aacaggaggg acagctgata gacacagaag ccactggagc acctcaaaaa 9300
   caccatcata cactaaatca gtaagttggc agcatcaccc ataattgtgg tttcaaaatc 9360
   ggctccgtcg atactatgtt atacgccaac tttgaaaaca actttgaaaa agctgttttc 9420
   tggtatttaa ggttttagaa tgcaaggaac agtgaattgg agttcgtctt gttataatta 9480
   gcttcttggg gtatctttaa atactgtaga aaagaggaag gaaataataa atggctaaaa 9540
   tgagaatatc accggaattg aaaaaactga tcgaaaaata ccgctgcgta aaagatacgg 9600
   aaggaatgtc tcctgctaag gtatataagc tggtgggaga aaatgaaaac ctatatttaa 9660
   aaatgacgga cagccggtat aaagggacca cctatgatgt ggaacgggaa aaggacatga 9720
   tgctatggct ggaaggaaag ctgcctgttc caaaggtcct gcactttgaa cggcatgatg 9780
   gctggagcaa tctgctcatg agtgaggccg atggcgtcct ttgctcggaa gagtatgaag 9840
   atgaacaaag ccctgaaaag attatcgagc tgtatgcgga gtgcatcagg ctctttcact 9900
   ccatcgacat atcggattgt ccctatacga atagcttaga cagccgctta gccgaattgg 9960
   attacttact gaataacgat ctggccgatg tggattgcga aaactgggaa gaagacactc 10020
   catttaaaga tccgcgcgag ctgtatgatt ttttaaagac ggaaaagccc gaagaggaac 10080
   ttgtcttttc ccacggcgac ctgggagaca gcaacatctt tgtgaaagat ggcaaagtaa 10140
   gtggctttat tgatcttggg agaagcggca gggcggacaa gtggtatgac attgccttct 10200
   gcgtccggtc gatcagggag gatatcgggg aagaacagta tgtcgagcta ttttttgact 10260
   tactggggat caagcctgat tgggagaaaa taaaatatta tattttactg gatgaattgt 10320
   tttagtacct agatgtggcg caacgatgcc ggcgacaagc aggagcgcac cgacttcttc 10380
   cgcatcaagt gttttggctc tcaggccgag gcccacggca agtatttggg caaggggtcg 10440
   ctggtattcg tgcagggcaa gattcggaat accaagtacg agaaggacgg ccagacggtc 10500
   tacgggaccg acttcattgc cgataaggtg gattatctgg acaccaaggc accaggcggg 10560
   tcaaatcagg aataagggca cattgccccg gcgtgagtcg gggcaatccc gcaaggaggg 10620
   tgaatgaatc ggacgtttga ccggaaggca tacaggcaag aactgatcga cgcggggttt 10680
   tccgccgagg atgccgaaac catcgcaagc cgcaccgtca tgcgtgcgcc ccgcgaaacc 10740
   ttccagtccg tcggctcgat ggtccagcaa gctacggcca agatcgagcg cgacagcgtg 10800
   caactggctc cccctgccct gcccgcgcca tcggccgccg tggagcgttc gcgtcgtctc 10860
   gaacaggagg cggcaggttt ggcgaagtcg atgaccatcg acacgcgagg aactatgacg 10920
   accaagaagc gaaaaaccgc cggcgaggac ctggcaaaac aggtcagcga ggccaagcag 10980
   gccgcgttgc tgaaacacac gaagcagcag atcaaggaaa tgcagctttc cttgttcgat 11040
   attgcgccgt ggccggacac gatgcgagcg atgccaaacg acacggcccg ctctgccctg 11100
   ttcaccacgc gcaacaagaa aatcccgcgc gaggcgctgc aaaacaaggt cattttccac 11160
   gtcaacaagg acgtgaagat cacctacacc ggcgtcgagc tgcgggccga cgatgacgaa 11220
   ctggtgtggc agcaggtgtt ggagtacgcg aagcgcaccc ctatcggcga gccgatcacc 11280
   ttcacgttct acgagctttg ccaggacctg ggctggtcga tcaatggccg gtattacacg 11340
   aaggccgagg aatgcctgtc gcgcctacag gcgacggcga tgggcttcac gtccgaccgc 11400
   gttgggcacc tggaatcggt gtcgctgctg caccgcttcc gcgtcctgga ccgtggcaag 11460
   aaaacgtccc gttgccaggt cctgatcgac gaggaaatcg tcgtgctgtt tgctggcgac 11520
   cactacacga aattcatatg ggagaagtac cgcaagctgt cgccgacggc ccgacggatg 11580
   ttcgactatt tcagctcgca ccgggagccg tacccgctca agctggaaac cttccgcctc 11640
   atgtgcggat cggattccac ccgcgtgaag aagtggcgcg agcaggtcgg cgaagcctgc 11700
   gaagagttgc gaggcagcgg cctggtggaa cacgcctggg tcaatgatga cctggtgcat 11760
   tgcaaacgct agggccttgt ggggtcagtt ccggctgggg gttcagcagc cagcgcttta 11820
   ctggcatttc aggaacaagc gggcactgct cgacgcactt gcttcgctca gtatcgctcg 11880
   ggacgcacgg cgcgctctac gaactgccga taaacagagg attaaaattg acaattgtga 11940
   ttaaggctca gattcgacgg cttggagcgg ccgacgtgca ggatttccgc gagatccgat 12000
   tgtcggccct gaagaaagct ccagagatgt tcgggtccgt ttacgagcac gaggagaaaa 12060
   agcccatgga ggcgttcgct gaacggttgc gagatgccgt ggcattcggc gcctacatcg 12120
   acggcgagat cattgggctg tcggtcttca aacaggagga cggccccaag gacgctcaca 12180
   aggcgcatct gtccggcgtt ttcgtggagc ccgaacagcg aggccgaggg gtcgccggta 12240
   tgctgctgcg ggcgttgccg gcgggtttat tgctcgtgat gatcgtccga cagattccaa 12300
   cgggaatctg gtggatgcgc atcttcatcc tcggcgcact taatatttcg ctattctgga 12360
   gcttgttgtt tatttcggtc taccgcctgc cgggcggggt cgcggcgacg gtaggcgctg 12420
   tgcagccgct gatggtcgtg ttcatctctg ccgctctgct aggtagcccg atacgattga 12480
   tggcggtcct gggggctatt tgcggaactg cgggcgtggc gctgttggtg ttgacaccaa 12540
   acgcagcgct agatcctgtc ggcgtcgcag cgggcctggc gggggcggtt tccatggcgt 12600
   tcggaaccgt gctgacccgc aagtggcaac ctcccgtgcc tctgctcacc tttaccgcct 12660
   ggcaactggc ggccggagga cttctgctcg ttccagtagc tttagtgttt gatccgccaa 12720
   tcccgatgcc tacaggaacc aatgttctcg gcctggcgtg gctcggcctg atcggagcgg 12780
   gtttaaccta cttcctttgg ttccggggga tctcgcgact cgaacctaca gttgtttcct 12840
   tactgggctt tctcagcccc agatctgggg tcgatcagcc ggggatgcat caggccgaca 12900
   gtcggaactt cgggtccccg acctgtacca ttcggtgagc aatggatagg ggagttgata 12960
   tcgtcaacgt tcacttctaa agaaatagcg ccactcagct tcctcagcgg ctttatccag 13020
   cgatttccta ttatgtcggc atagttctca agatcgacag cctgtcacgg ttaagcgaga 13080
   aatgaataag aaggctgata attcggatct ctgcgaggga gatgatattt gatcacaggc 13140
   agcaacgctc tgtcatcgtt acaatcaaca tgctaccctc cgcgagatca tccgtgtttc 13200
   aaacccggca gcttagttgc cgttcttccg aatagcatcg gtaacatgag caaagtctgc 13260
   cgccttacaa cggctctccc gctgacgccg tcccggactg atgggctgcc tgtatcgagt 13320
   ggtgattttg tgccgagctg ccggtcgggg agctgttggc tggctggtgg caggatatat 13380
   tgtggtgtaa acaaattgac gcttagacaa cttaataaca cattgcggac gtttttaatg 13440
   tactggggtg gtttttcttt tcaccagtga gacgggcaac agctgattgc ccttcaccgc 13500
   ctggccctga gagagttgca gcaagcggtc cacgctggtt tgccccagca ggcgaaaatc 13560
   ctgtttgatg gtggttccga aatcggcaaa atcccttata aatcaaaaga atagcccgag 13620
   atagggttga gtgttgttcc agtttggaac aagagtccac tattaaagaa cgtggactcc 13680
   aacgtcaaag ggcgaaaaac cgtctatcag ggcgatggcc cactacgtga accatcaccc 13740
   aaatcaagtt ttttggggtc gaggtgccgt aaagcactaa atcggaaccc taaagggagc 13800
   ccccgattta gagcttgacg gggaaagccg gcgaacgtgg cgagaaagga agggaagaaa 13860
   gcgaaaggag cgggcgccat tcaggctgcg caactgttgg gaagggcgat cggtgcgggc 13920
   ctcttcgcta ttacgccagc tggcgaaagg gggatgtgct gcaaggcgat taagttgggt 13980
   aacgccaggg ttttcccagt cacgacgttg taaaacgacg gccagtgaat tgccatcttg 14040
   aaagaaatat agtttaaata tttattgata aaataagtca ggtattatag tccaagcaaa 14100
   aacataattt attgatgcaa agtttaaatt cagaaatatt tcaataactg attatatcag 14160
   ctggtacatt gccgtagatg aaagactgag tgcgatatta tgtgtaatac ataaattgat 14220
   gatatagcta gcttagctca tcgggggatc cttaatcgac tctagctaga acgaattgtt 14280
   aggtggcggt acttgggtcg atatcaaagt gcatcacttc ttcccgtatg cccaactttg 14340
   tatagagagc cactgcggga tcgtcaccgt aatctgcttg cacgtagatc acataagcac 14400
   caagcgcgtt ggcctcatgc ttgaggagat tgatgagcgc ggtggcaatg ccctgcctcc 14460
   ggtgctcgcc ggagactgcg agatcataga tatagatctc actacgcggc tgctcaaacc 14520
   tgggcagaac gtaagccgcg agagcgccaa caaccgcttc ttggtcgaag gcagcaagcg 14580
   cgatgaatgt cttactacgg agcaagttcc cgaggtaatc ggagtccggc tgatgttggg 14640
   agtaggtggc tacgtctccg aactcacgac cgaaaagatc aagagcagcc cgcatggatt 14700
   tgacttggtc agggccgagc ctacatgtgc gaatgatgcc catacttgag ccacctaact 14760
   ttgttttagg gcgactgccc tgctgcgtaa catcgttgct gctgcgtacc atggagatct 14820
   ggattgagag tgaatatgag actctaattg gataccgagg ggaatttatg gaagtcagtg 14880
   gagcattttt gacaagaaat atttgctagc tgatagtgac cttaggcgac ttttgaacgc 14940
   gcaataatgg tttctgacgt atgtgcttag ctcattaaac tccagaaacc cgcggctgag 15000
   tggctccttc aacgttgcgg ttctgtcagt tccaaacgta aaacggcttg tcccgcgtca 15060
   tcggcggggg tcataacgtg actcccttaa ttctccgctc atgatcttga tcccctgcgc 15120
   catcagatcc ttggcggcaa gaaagccatc cagtttactt tgcagggctt cccaacctta 15180
   ccagagggcg ccccagctgg caattccggt tcgcttgctg tccataaaac cgcccagtct 15240
   agctatcgcc atgtaagccc actgcaagct acctgctttc tctttgcgct tgcgttttcc 15300
   cttgtccaga tagcccagta gctgacattc atccggggtc agcaccgttt ctgcggactg 15360
   gctttctacg tgttccgctt cctttagcag cccttgcgcc ctgagtgctt gcggcagcgt 15420
   gaagctctgg acatcatgtt ggatatgaaa caactattat ttatctacat gttttagatg 15480
   ttatctgatt atttttatac cgtagtcttc tattgatgag gagtctaagg ctatagaatt 15540
   atatatctaa atgattaata tatatattat taataattaa caataattaa tatattataa 15600
   tttatatata tatattttat attattataa taatattctt acaaatataa ttattatatt 15660
   cgacggtatc gataagctcg ggatccctga aagcgacgtt ggatgttaac atctacaaat 15720
   tgccttttct tatcgaccat gtacgtaagc gcttacgttt ttggtggacc cttgaggaaa 15780
   ctggtagctg ttgtgggcct gtggtctcaa gatggatcat taatttccac cttcacctac 15840
   gatggggggc atcgcaccgg tgagtaatat tgtacggcta agagcgaatt tggcctgtag 15900
   gatccctgaa agcgacgttg gatgttaaca tctacaaatt gccttttctt atcgaccatg 15960
   tacgtaagcg cttacgtttt tggtggaccc ttgaggaaac tggtagctgt tgtgggcctg 16020
   tggtctcaag atggatcatt aatttccacc ttcacctacg atggggggca tcgcaccggt 16080
   gagtaatatt gtacggctaa gagcgaattt ggcctgtagg atccctgaaa gcgacgttgg 16140
   atgttaacat ctacaaattg ccttttctta tcgaccatgt acgtaagcgc ttacgttttt 16200
   ggtggaccct tgaggaaact ggtagctgtt gtgggcctgt ggtctcaaga tggatcatta 16260
   atttccacct tcacctacga tggggggcat cgcaccggtg agtaatattg tacggctaag 16320
   agcgaatttg gcctgtagga tccgcgagct ggtcaatccc attgcttttg aagcagctca 16380
   acattgatct ctttctcgat cgagggagat ttttcaaatc agtgcgcaag acgtgacgta 16440
   agtatccgag tcagttttta tttttctact aatttggtcg tttatttcgg cgtgta 16496
<210> 37
   <211> 15344
   <212> DNA
   <213> Artificial Sequence
<220>
   <223> MERE02 Plasmid
<400> 37
   ggtcgtttat ttcggcgtgt aggacatggc aaccgggcct gaatttcgcg ggtattctgt 60
   ttctattcca actttttctt gatccgcagc cattaacgac ttttgaatag atacgctgac 120
   acgccaagcc tcgctagtca aaagtgtacc aaacaacgct ttacagcaag aacggaatgc 180
   gcgtgacgct cgcggtgacg ccatttcgcc ttttcagaaa tggataaata gccttgcttc 240
   ctattatatc ttcccccaaa ttaattaaga aactcccgag gtgagcaagg atccggagtc 300
   gagcgcgaag aagagaaaga gggaaagcgc gggtaccggg cccccccctc gacggatcaa 360
   gtgcaaaggt ccgccttgtt tctcctctgt ctcttgatct gactaatctt ggtttatgat 420
   tcgttgagta attttgggga aagctagctt cgtccacagt ttttttttcg atgaacagtg 480
   ccgcagtggc gctgatcttg tatgctatcc tgcaatcgtg gtgaacttat ttcttttata 540
   tccttcactc ccatgaaaag gctagtaatc tttctcgatg taacatcgtc cagcactgct 600
   attaccgtgt ggtccatccg acagtctggc tgaacacatc atacgatatt gagcaaagat 660
   cgatctatct tccctgttct ttaatgaaag acgtcatttt catcagtatg atctaagaat 720
   gttgcaactt gcaaggaggc gtttctttct ttgaatttaa ctaactcgtt gagtggccct 780
   gtttctcgga cgtaaggcct ttgctgctcc acacatgtcc attcgaattt taccgtgttt 840
   agcaagggcg aaaagtttgc atcttgatga tttagcttga ctatgcgatt gctttcctgg 900
   acccgtgcag ctgcggacgg atcccccgct cgaggtcgac ggtatcgata agcttgatca 960
   gatctgatcg aattcaccat gcaggtcctg aacacgatgg gcgccgggca gtcctcgcct 1020
   gcgacgggga gccagaacca gtcgggcaac accggctcga tcatcaacaa ctactacatg 1080
   cagcaatacc agaacagcat ggacacccag ctcggcgata acgccatctc cggcgggtcc 1140
   aacgagggct cgaccgacac gacctccacc cacaccacga acacccagaa caatgactgg 1200
   ttcagcaagc tggcctccag cgccttcacg ggcctgttcg gggcgctgct cgcggacaag 1260
   aagaccgagg agaccacgct gctcgaggac cggatcctca ccacgcgcaa cgggcacacg 1320
   accagcacga cccagtccag cgtcggggtg acccacggct actccacgga ggaggaccac 1380
   gtcgccgggc cgaacacgag cggcctcgag acccgcgtgg tccaggcgga gcgcttctac 1440
   aagaagtacc tgttcgactg gaccacggac aaggcgttcg ggcacctcga gaagctggag 1500
   ctgccgagcg accaccacgg cgtcttcggc cacctcgtgg actcctacgc gtacatgaga 1560
   aatgggtggg acgtcgaggt cagcgccgtc ggcaaccagt tcaacggcgg gtgcctgctc 1620
   gtggcgatgg tcccggagtg gaaggagttc gacacccgcg agaagtatca gctgaccctc 1680
   ttcccccacc agttcatcag cccgcgcacc aacatgaccg cccacatcac ggtgccgtac 1740
   ctcggggtga accgctacga ccagtacaag aagcacaagc cctggaccct cgtggtcatg 1800
   gtggtctccc cgctgactgt gaacaacacg tccgccgcgc agatcaaagt ctacgccaac 1860
   atcgccccca cctacgtgca cgtcgccggc gagctgccct ccaaggaggg catcttcccc 1920
   gtcgcctgcg ccgacgggta cgggggcctg gtgaccaccg accccaagac ggccgacccg 1980
   gcctacggca aagtgtacaa cccgcccagg acgaactacc ccggtcgctt caccaacctc 2040
   ctggacgtgg cggaggcctg cccgaccttc ctgtgcttcg acgacgggaa gccctacgtc 2100
   accacgcgca ccgacgacac gcgcctgctc gccaagttcg acctcagcct ggccgctaag 2160
   cacatgagca acacctacct cagcggcatc gcccaatact atacccaata ctcgggcacc 2220
   atcaacctgc acttcatgtt cacgggcagc accgacagca aggcccggta catggtggcc 2280
   tacatcccgc cgggcgtgga gacccctccc gacacgcccg agcgggctgc gcactgcatc 2340
   cacgccgagt gggacaccgg cctcaacagc aagttcacgt tcagcatccc ctacgtgtcc 2400
   gccgcggatt acgcttacac cgcctcggac acggccgaga cgatcaacgt ccagggctgg 2460
   gtctgcatct accagatcac tcacggcaag gccgagaacg acaccctcgt cgtgagcgtc 2520
   tccgccggga aggacttcga gctgaggctg cccatcgacc ccaggcagca gaccacggcg 2580
   accggggagt ccgccgaccc cgtgaccacg accgtggaga actacggcgg ggagactcag 2640
   atccagcggc gccaccacac cgacatcggc ttcatcatgg accgcttcgt gaagatccag 2700
   tccctgtcgc ccacccacgt tatcgacctc atgcaggccc accagcacgg gctcgtgggt 2760
   gccctcctgc gcgcggccac ctactacttc agcgacctcg agatcgttgt ccgccacgag 2820
   gggaacctca cctgggtccc gaacggtgcc cccgagagcg ccctgctcaa cacctccaac 2880
   cccacggcgt acaacaaggc ccccttcacg cgcctcgccc tgccttacac cgcgccccac 2940
   cgcgtgctgg ccacggtgta caacgggacc tccaagtacg ccgtgggcgg gagcggccgc 3000
   agaggcgaca tggggagcct cgccgctagg gtcgtgaagc agctcccggc ctccttcaac 3060
   tacggcgcca tcaaggccga cgccatccac gaactcctgg tccgcatgaa gcgcgccgaa 3120
   ctctactgcc cccggcccct cctggccatc gaggtctcct cgcaggaccg ccacaagcag 3180
   aagatcatcg ccccggccaa gcagtaagag ctcgaatttc cccgatcgtt caaacatttg 3240
   gcaataaagt ttcttaagat tgaatcctgt tgccggtctt gcgatgatta tcatataatt 3300
   tctgttgaat tacgttaagc atgtaataat taacatgtaa tgcatgacgt tatttatgag 3360
   atgggttttt atgattagag tcccgcaatt atacatttaa tacgcgatag aaaacaaaat 3420
   atagcgcgca aactaggata aattatcgcg cgcggtgtca tctatgttac tagatcggga 3480
   attaattcag atcggctgag tggctccttc aacgttgcgg ttctgtcagt tccaaacgta 3540
   aaacggcttg tcccgcgtca tcggcggggg tcataacgtg actcccttaa ttctccgctc 3600
   atgatcagat tgtcgtttcc cgccttcagt ttaaactatc agtgtttgac aggatatatt 3660
   ggcgggtaaa cctaagagaa aagagcgttt attagaataa tcggatattt aaaagggcgt 3720
   gaaaaggttt atccgttcgt ccatttgtat gtgcatgcca accacagggt tccccagatc 3780
   tggcgccggc cagcgagacg agcaagattg gccgccgccc gaaacgatcc gacagcgcgc 3840
   ccagcacagg tgcgcaggca aattgcacca acgcatacag cgccagcaga atgccatagt 3900
   gggcggtgac gtcgttcgag tgaaccagat cgcgcaggag gcccggcagc accggcataa 3960
   tcaggccgat gccgacagcg tcgagcgcga cagtgctcag aattacgatc aggggtatgt 4020
   tgggtttcac gtctggcctc cggaccagcc tccgctggtc cgattgaacg cgcggattct 4080
   ttatcactga taagttggtg gacatattat gtttatcagt gataaagtgt caagcatgac 4140
   aaagttgcag ccgaatacag tgatccgtgc cgccctggac ctgttgaacg aggtcggcgt 4200
   agacggtctg acgacacgca aactggcgga acggttgggg gttcagcagc cggcgcttta 4260
   ctggcacttc aggaacaagc gggcgctgct cgacgcactg gccgaagcca tgctggcgga 4320
   gaatcatacg cattcggtgc cgagagccga cgacgactgg cgctcatttc tgatcgggaa 4380
   tgcccgcagc ttcaggcagg cgctgctcgc ctaccgcgat ggcgcgcgca tccatgccgg 4440
   cacgcgaccg ggcgcaccgc agatggaaac ggccgacgcg cagcttcgct tcctctgcga 4500
   ggcgggtttt tcggccgggg acgccgtcaa tgcgctgatg acaatcagct acttcactgt 4560
   tggggccgtg cttgaggagc aggccggcga cagcgatgcc ggcgagcgcg gcggcaccgt 4620
   tgaacaggct ccgctctcgc cgctgttgcg ggccgcgata gacgccttcg acgaagccgg 4680
   tccggacgca gcgttcgagc agggactcgc ggtgattgtc gatggattgg cgaaaaggag 4740
   gctcgttgtc aggaacgttg aaggaccgag aaagggtgac gattgatcag gaccgctgcc 4800
   ggagcgcaac ccactcacta cagcagagcc atgtagacaa catcccctcc ccctttccac 4860
   cgcgtcagac gcccgtagca gcccgctacg ggctttttca tgccctgccc tagcgtccaa 4920
   gcctcacggc cgcgctcggc ctctctggcg gccttctggc gctcttccgc ttcctcgctc 4980
   actgactcgc tgcgctcggt cgttcggctg cggcgagcgg tatcagctca ctcaaaggcg 5040
   gtaatacggt tatccacaga atcaggggat aacgcaggaa agaacatgtg agcaaaaggc 5100
   cagcaaaagg ccaggaaccg taaaaaggcc gcgttgctgg cgtttttcca taggctccgc 5160
   ccccctgacg agcatcacaa aaatcgacgc tcaagtcaga ggtggcgaaa cccgacagga 5220
   ctataaagat accaggcgtt tccccctgga agctccctcg tgcgctctcc tgttccgacc 5280
   ctgccgctta ccggatacct gtccgccttt ctcccttcgg gaagcgtggc gcttttccgc 5340
   tgcataaccc tgcttcgggg tcattatagc gattttttcg gtatatccat cctttttcgc 5400
   acgatataca ggattttgcc aaagggttcg tgtagacttt ccttggtgta tccaacggcg 5460
   tcagccgggc aggataggtg aagtaggccc acccgcgagc gggtgttcct tcttcactgt 5520
   cccttattcg cacctggcgg tgctcaacgg gaatcctgct ctgcgaggct ggccggctac 5580
   cgccggcgta acagatgagg gcaagcggat ggctgatgaa accaagccaa ccaggaaggg 5640
   cagcccacct atcaaggtgt actgccttcc agacgaacga agagcgattg aggaaaaggc 5700
   ggcggcggcc ggcatgagcc tgtcggccta cctgctggcc gtcggccagg gctacaaaat 5760
   cacgggcgtc gtggactatg agcacgtccg cgagctggcc cgcatcaatg gcgacctggg 5820
   ccgcctgggc ggcctgctga aactctggct caccgacgac ccgcgcacgg cgcggttcgg 5880
   tgatgccacg atcctcgccc tgctggcgaa gatcgaagag aagcaggacg agcttggcaa 5940
   ggtcatgatg ggcgtggtcc gcccgagggc agagccatga cttttttagc cgctaaaacg 6000
   gccggggggt gcgcgtgatt gccaagcacg tccccatgcg ctccatcaag aagagcgact 6060
   tcgcggagct ggtgaagtac atcaccgacg agcaaggcaa gaccgagcgc ctttgcgacg 6120
   ctcaccgggc tggttgccct cgccgctggg ctggcggccg tctatggccc tgcaaacgcg 6180
   ccagaaacgc cgtcgaagcc gtgtgcgaga caccgcggcc gccggcgttg tggatacctc 6240
   gcggaaaact tggccctcac tgacagatga ggggcggacg ttgacacttg aggggccgac 6300
   tcacccggcg cggcgttgac agatgagggg caggctcgat ttcggccggc gacgtggagc 6360
   tggccagcct cgcaaatcgg cgaaaacgcc tgattttacg cgagtttccc acagatgatg 6420
   tggacaagcc tggggataag tgccctgcgg tattgacact tgaggggcgc gactactgac 6480
   agatgagggg cgcgatcctt gacacttgag gggcagagtg ctgacagatg aggggcgcac 6540
   ctattgacat ttgaggggct gtccacaggc agaaaatcca gcatttgcaa gggtttccgc 6600
   ccgtttttcg gccaccgcta acctgtcttt taacctgctt ttaaaccaat atttataaac 6660
   cttgttttta accagggctg cgccctgtgc gcgtgaccgc gcacgccgaa ggggggtgcc 6720
   cccccttctc gaaccctccc ggcccgctaa cgcgggcctc ccatcccccc aggggctgcg 6780
   cccctcggcc gcgaacggcc tcaccccaaa aatggcagcg ctggcagtcc ttgccattgc 6840
   cgggatcggg gcagtaacgg gatgggcgat cagcccgagc gcgacgcccg gaagcattga 6900
   cgtgccgcag gtgctggcat cgacattcag cgaccaggtg ccgggcagtg agggcggcgg 6960
   cctgggtggc ggcctgccct tcacttcggc cgtcggggca ttcacggact tcatggcggg 7020
   gccggcaatt tttaccttgg gcattcttgg catagtggtc gcgggtgccg tgctcgtgtt 7080
   cgggggtgcg ataaacccag cgaaccattt gaggtgatag gtaagattat accgaggtat 7140
   gaaaacgaga attggacctt tacagaatta ctctatgaag cgccatattt aaaaagctac 7200
   caagacgaag aggatgaaga ggatgaggag gcagattgcc ttgaatatat tgacaatact 7260
   gataagataa tatatctttt atatagaaga tatcgccgta tgtaaggatt tcagggggca 7320
   aggcataggc agcgcgctta tcaatatatc tatagaatgg gcaaagcata aaaacttgca 7380
   tggactaatg cttgaaaccc aggacaataa ccttatagct tgtaaattct atcataattg 7440
   ggtaatgact ccaacttatt gatagtgttt tatgttcaga taatgcccga tgactttgtc 7500
   atgcagctcc accgattttg agaacgacag cgacttccgt cccagccgtg ccaggtgctg 7560
   cctcagattc aggttatgcc gctcaattcg ctgcgtatat cgcttgctga ttacgtgcag 7620
   ctttcccttc aggcgggatt catacagcgg ccagccatcc gtcatccata tcaccacgtc 7680
   aaagggtgac agcaggctca taagacgccc cagcgtcgcc atagtgcgtt caccgaatac 7740
   gtgcgcaaca accgtcttcc ggagactgtc atacgcgtaa aacagccagc gctggcgcga 7800
   tttagccccg acatagcccc actgttcgtc catttccgcg cagacgatga cgtcactgcc 7860
   cggctgtatg cgcgaggtta ccgactgcgg cctgagtttt ttaagtgacg taaaatcgtg 7920
   ttgaggccaa cgcccataat gcgggctgtt gcccggcatc caacgccatt catggccata 7980
   tcaatgattt tctggtgcgt accgggttga gaagcggtgt aagtgaactg cagttgccat 8040
   gttttacggc agtgagagca gagatagcgc tgatgtccgg cggtgctttt gccgttacgc 8100
   accaccccgt cagtagctga acaggaggga cagctgatag acacagaagc cactggagca 8160
   cctcaaaaac accatcatac actaaatcag taagttggca gcatcaccca taattgtggt 8220
   ttcaaaatcg gctccgtcga tactatgtta tacgccaact ttgaaaacaa ctttgaaaaa 8280
   gctgttttct ggtatttaag gttttagaat gcaaggaaca gtgaattgga gttcgtcttg 8340
   ttataattag cttcttgggg tatctttaaa tactgtagaa aagaggaagg aaataataaa 8400
   tggctaaaat gagaatatca ccggaattga aaaaactgat cgaaaaatac cgctgcgtaa 8460
   aagatacgga aggaatgtct cctgctaagg tatataagct ggtgggagaa aatgaaaacc 8520
   tatatttaaa aatgacggac agccggtata aagggaccac ctatgatgtg gaacgggaaa 8580
   aggacatgat gctatggctg gaaggaaagc tgcctgttcc aaaggtcctg cactttgaac 8640
   ggcatgatgg ctggagcaat ctgctcatga gtgaggccga tggcgtcctt tgctcggaag 8700
   agtatgaaga tgaacaaagc cctgaaaaga ttatcgagct gtatgcggag tgcatcaggc 8760
   tctttcactc catcgacata tcggattgtc cctatacgaa tagcttagac agccgcttag 8820
   ccgaattgga ttacttactg aataacgatc tggccgatgt ggattgcgaa aactgggaag 8880
   aagacactcc atttaaagat ccgcgcgagc tgtatgattt tttaaagacg gaaaagcccg 8940
   aagaggaact tgtcttttcc cacggcgacc tgggagacag caacatcttt gtgaaagatg 9000
   gcaaagtaag tggctttatt gatcttggga gaagcggcag ggcggacaag tggtatgaca 9060
   ttgccttctg cgtccggtcg atcagggagg atatcgggga agaacagtat gtcgagctat 9120
   tttttgactt actggggatc aagcctgatt gggagaaaat aaaatattat attttactgg 9180
   atgaattgtt ttagtaccta gatgtggcgc aacgatgccg gcgacaagca ggagcgcacc 9240
   gacttcttcc gcatcaagtg ttttggctct caggccgagg cccacggcaa gtatttgggc 9300
   aaggggtcgc tggtattcgt gcagggcaag attcggaata ccaagtacga gaaggacggc 9360
   cagacggtct acgggaccga cttcattgcc gataaggtgg attatctgga caccaaggca 9420
   ccaggcgggt caaatcagga ataagggcac attgccccgg cgtgagtcgg ggcaatcccg 9480
   caaggagggt gaatgaatcg gacgtttgac cggaaggcat acaggcaaga actgatcgac 9540
   gcggggtttt ccgccgagga tgccgaaacc atcgcaagcc gcaccgtcat gcgtgcgccc 9600
   cgcgaaacct tccagtccgt cggctcgatg gtccagcaag ctacggccaa gatcgagcgc 9660
   gacagcgtgc aactggctcc ccctgccctg cccgcgccat cggccgccgt ggagcgttcg 9720
   cgtcgtctcg aacaggaggc ggcaggtttg gcgaagtcga tgaccatcga cacgcgagga 9780
   actatgacga ccaagaagcg aaaaaccgcc ggcgaggacc tggcaaaaca ggtcagcgag 9840
   gccaagcagg ccgcgttgct gaaacacacg aagcagcaga tcaaggaaat gcagctttcc 9900
   ttgttcgata ttgcgccgtg gccggacacg atgcgagcga tgccaaacga cacggcccgc 9960
   tctgccctgt tcaccacgcg caacaagaaa atcccgcgcg aggcgctgca aaacaaggtc 10020
   attttccacg tcaacaagga cgtgaagatc acctacaccg gcgtcgagct gcgggccgac 10080
   gatgacgaac tggtgtggca gcaggtgttg gagtacgcga agcgcacccc tatcggcgag 10140
   ccgatcacct tcacgttcta cgagctttgc caggacctgg gctggtcgat caatggccgg 10200
   tattacacga aggccgagga atgcctgtcg cgcctacagg cgacggcgat gggcttcacg 10260
   tccgaccgcg ttgggcacct ggaatcggtg tcgctgctgc accgcttccg cgtcctggac 10320
   cgtggcaaga aaacgtcccg ttgccaggtc ctgatcgacg aggaaatcgt cgtgctgttt 10380
   gctggcgacc actacacgaa attcatatgg gagaagtacc gcaagctgtc gccgacggcc 10440
   cgacggatgt tcgactattt cagctcgcac cgggagccgt acccgctcaa gctggaaacc 10500
   ttccgcctca tgtgcggatc ggattccacc cgcgtgaaga agtggcgcga gcaggtcggc 10560
   gaagcctgcg aagagttgcg aggcagcggc ctggtggaac acgcctgggt caatgatgac 10620
   ctggtgcatt gcaaacgcta gggccttgtg gggtcagttc cggctggggg ttcagcagcc 10680
   agcgctttac tggcatttca ggaacaagcg ggcactgctc gacgcacttg cttcgctcag 10740
   tatcgctcgg gacgcacggc gcgctctacg aactgccgat aaacagagga ttaaaattga 10800
   caattgtgat taaggctcag attcgacggc ttggagcggc cgacgtgcag gatttccgcg 10860
   agatccgatt gtcggccctg aagaaagctc cagagatgtt cgggtccgtt tacgagcacg 10920
   aggagaaaaa gcccatggag gcgttcgctg aacggttgcg agatgccgtg gcattcggcg 10980
   cctacatcga cggcgagatc attgggctgt cggtcttcaa acaggaggac ggccccaagg 11040
   acgctcacaa ggcgcatctg tccggcgttt tcgtggagcc cgaacagcga ggccgagggg 11100
   tcgccggtat gctgctgcgg gcgttgccgg cgggtttatt gctcgtgatg atcgtccgac 11160
   agattccaac gggaatctgg tggatgcgca tcttcatcct cggcgcactt aatatttcgc 11220
   tattctggag cttgttgttt atttcggtct accgcctgcc gggcggggtc gcggcgacgg 11280
   taggcgctgt gcagccgctg atggtcgtgt tcatctctgc cgctctgcta ggtagcccga 11340
   tacgattgat ggcggtcctg ggggctattt gcggaactgc gggcgtggcg ctgttggtgt 11400
   tgacaccaaa cgcagcgcta gatcctgtcg gcgtcgcagc gggcctggcg ggggcggttt 11460
   ccatggcgtt cggaaccgtg ctgacccgca agtggcaacc tcccgtgcct ctgctcacct 11520
   ttaccgcctg gcaactggcg gccggaggac ttctgctcgt tccagtagct ttagtgtttg 11580
   atccgccaat cccgatgcct acaggaacca atgttctcgg cctggcgtgg ctcggcctga 11640
   tcggagcggg tttaacctac ttcctttggt tccgggggat ctcgcgactc gaacctacag 11700
   ttgtttcctt actgggcttt ctcagcccca gatctggggt cgatcagccg gggatgcatc 11760
   aggccgacag tcggaacttc gggtccccga cctgtaccat tcggtgagca atggataggg 11820
   gagttgatat cgtcaacgtt cacttctaaa gaaatagcgc cactcagctt cctcagcggc 11880
   tttatccagc gatttcctat tatgtcggca tagttctcaa gatcgacagc ctgtcacggt 11940
   taagcgagaa atgaataaga aggctgataa ttcggatctc tgcgagggag atgatatttg 12000
   atcacaggca gcaacgctct gtcatcgtta caatcaacat gctaccctcc gcgagatcat 12060
   ccgtgtttca aacccggcag cttagttgcc gttcttccga atagcatcgg taacatgagc 12120
   aaagtctgcc gccttacaac ggctctcccg ctgacgccgt cccggactga tgggctgcct 12180
   gtatcgagtg gtgattttgt gccgagctgc cggtcgggga gctgttggct ggctggtggc 12240
   aggatatatt gtggtgtaaa caaattgacg cttagacaac ttaataacac attgcggacg 12300
   tttttaatgt actggggtgg tttttctttt caccagtgag acgggcaaca gctgattgcc 12360
   cttcaccgcc tggccctgag agagttgcag caagcggtcc acgctggttt gccccagcag 12420
   gcgaaaatcc tgtttgatgg tggttccgaa atcggcaaaa tcccttataa atcaaaagaa 12480
   tagcccgaga tagggttgag tgttgttcca gtttggaaca agagtccact attaaagaac 12540
   gtggactcca acgtcaaagg gcgaaaaacc gtctatcagg gcgatggccc actacgtgaa 12600
   ccatcaccca aatcaagttt tttggggtcg aggtgccgta aagcactaaa tcggaaccct 12660
   aaagggagcc cccgatttag agcttgacgg ggaaagccgg cgaacgtggc gagaaaggaa 12720
   gggaagaaag cgaaaggagc gggcgccatt caggctgcgc aactgttggg aagggcgatc 12780
   ggtgcgggcc tcttcgctat tacgccagct ggcgaaaggg ggatgtgctg caaggcgatt 12840
   aagttgggta acgccagggt tttcccagtc acgacgttgt aaaacgacgg ccagtgaatt 12900
   gccatcttga aagaaatata gtttaaatat ttattgataa aataagtcag gtattatagt 12960
   ccaagcaaaa acataattta ttgatgcaaa gtttaaattc agaaatattt caataactga 13020
   ttatatcagc tggtacattg ccgtagatga aagactgagt gcgatattat gtgtaataca 13080
   taaattgatg atatagctag cttagctcat cgggggatcc ttaatcgact ctagctagaa 13140
   cgaattgtta ggtggcggta cttgggtcga tatcaaagtg catcacttct tcccgtatgc 13200
   ccaactttgt atagagagcc actgcgggat cgtcaccgta atctgcttgc acgtagatca 13260
   cataagcacc aagcgcgttg gcctcatgct tgaggagatt gatgagcgcg gtggcaatgc 13320
   cctgcctccg gtgctcgccg gagactgcga gatcatagat atagatctca ctacgcggct 13380
   gctcaaacct gggcagaacg taagccgcga gagcgccaac aaccgcttct tggtcgaagg 13440
   cagcaagcgc gatgaatgtc ttactacgga gcaagttccc gaggtaatcg gagtccggct 13500
   gatgttggga gtaggtggct acgtctccga actcacgacc gaaaagatca agagcagccc 13560
   gcatggattt gacttggtca gggccgagcc tacatgtgcg aatgatgccc atacttgagc 13620
   cacctaactt tgttttaggg cgactgccct gctgcgtaac atcgttgctg ctgcgtacca 13680
   tggagatctg gattgagagt gaatatgaga ctctaattgg ataccgaggg gaatttatgg 13740
   aagtcagtgg agcatttttg acaagaaata tttgctagct gatagtgacc ttaggcgact 13800
   tttgaacgcg caataatggt ttctgacgta tgtgcttagc tcattaaact ccagaaaccc 13860
   gcggctgagt ggctccttca acgttgcggt tctgtcagtt ccaaacgtaa aacggcttgt 13920
   cccgcgtcat cggcgggggt cataacgtga ctcccttaat tctccgctca tgatcttgat 13980
   cccctgcgcc atcagatcct tggcggcaag aaagccatcc agtttacttt gcagggcttc 14040
   ccaaccttac cagagggcgc cccagctggc aattccggtt cgcttgctgt ccataaaacc 14100
   gcccagtcta gctatcgcca tgtaagccca ctgcaagcta cctgctttct ctttgcgctt 14160
   gcgttttccc ttgtccagat agcccagtag ctgacattca tccggggtca gcaccgtttc 14220
   tgcggactgg ctttctacgt gttccgcttc ctttagcagc ccttgcgccc tgagtgcttg    14280
   cggcagcgtg aagctctgga catcatgttg gatatgaaac aactattatt tatctacatg    14340
   ttttagatgt tatctgatta tttttatacc gtagtcttct attgatgagg agtctaaggc    14400
   tatagaatta tatatctaaa tgattaatat atatattatt aataattaac aataattaat    14460
   atattataat ttatatatat atattttata ttattataat aatattctta caaatataat    14520
   tattatattc gacggtatcg ataagctcgg gatccctgaa agcgacgttg gatgttaaca    14580
   tctacaaatt gccttttctt atcgaccatg tacgtaagcg cttacgtttt tggtggaccc    14640
   ttgaggaaac tggtagctgt tgtgggcctg tggtctcaag atggatcatt aatttccacc    14700
   ttcacctacg atggggggca tcgcaccggt gagtaatatt gtacggctaa gagcgaattt    14760
   ggcctgtagg atccctgaaa gcgacgttgg atgttaacat ctacaaattg ccttttctta    14820
   tcgaccatgt acgtaagcgc ttacgttttt ggtggaccct tgaggaaact ggtagctgtt    14880
   gtgggcctgt ggtctcaaga tggatcatta atttccacct tcacctacga tggggggcat    14940
   cgcaccggtg agtaatattg tacggctaag agcgaatttg gcctgtagga tccctgaaag    15000
   cgacgttgga tgttaacatc tacaaattgc cttttcttat cgaccatgta cgtaagcgct    15060
   tacgtttttg gtggaccctt gaggaaactg gtagctgttg tgggcctgtg gtctcaagat    15120
   ggatcattaa tttccacctt cacctacgat ggggggcatc gcaccggtga gtaatattgt    15180
   acggctaaga gcgaatttgg cctgtaggat ccgcgagctg gtcaatccca ttgcttttga    15240
   agcagctcaa cattgatctc tttctcgatc gagggagatt tttcaaatca gtgcgcaaga    15300
   cgtgacgtaa gtatccgagt cagtttttat ttttctacta attt                      15344
<210> 38
   <211> 17728
   <212> DNA
   <213> Artificial Sequence
<220>
   <223> MERE03 Plasmid
<400> 38
   ctaaggctat agaattatat atctaaatga ttaatatata tattattaat aattaacaat      60
   aattaatata ttataattta tatatatata ttttatatta ttataataat attcttacaa     120
   atataattat tatattcgac ggtatcgata agctcgggat ccctgaaagc gacgttggat     180
   gttaacatct acaaattgcc ttttcttatc gaccatgtac gtaagcgctt acgtttttgg     240
   tggacccttg aggaaactgg tagctgttgt gggcctgtgg tctcaagatg gatcattaat     300
   ttccaccttc acctacgatg gggggcatcg caccggtgag taatattgta cggctaagag     360
   cgaatttggc ctgtaggatc cctgaaagcg acgttggatg ttaacatcta caaattgcct     420
   tttcttatcg accatgtacg taagcgctta cgtttttggt ggacccttga ggaaactggt     480
   agctgttgtg ggcctgtggt ctcaagatgg atcattaatt tccaccttca cctacgatgg     540
   ggggcatcgc accggtgagt aatattgtac ggctaagagc gaatttggcc tgtaggatcc      600
   ctgaaagcga cgttggatgt taacatctac aaattgcctt ttcttatcga ccatgtacgt      660
   aagcgcttac gtttttggtg gacccttgag gaaactggta gctgttgtgg gcctgtggtc      720
   tcaagatgga tcattaattt ccaccttcac ctacgatggg gggcatcgca ccggtgagta      780
   atattgtacg gctaagagcg aatttggcct gtaggatccg cgagctggtc aatcccattg      840
   cttttgaagc agctcaacat tgatctcttt ctcgatcgag ggagattttt caaatcagtg      900
   cgcaagacgt gacgtaagta tccgagtcag tttttatttt tctactaatt tggtcgttta      960
   tttcggcgtg taggacatgg caaccgggcc tgaatttcgc gggtattctg tttctattcc     1020
   aactttttct tgatccgcag ccattaacga cttttgaata gatacgctga cacgccaagc     1080
   ctcgctagtc aaaagtgtac caaacaacgc tttacagcaa gaacggaatg cgcgtgacgc     1140
   tcgcggtgac gccatttcgc cttttcagaa atggataaat agccttgctt cctattatat     1200
   cttcccccaa attaattaag aaactcccga ggtgagcaag gatccggagt cgagcgcgaa     1260
   gaagagaaag agggaaagcg cgggtaccgg gcccccccct cgacggatca agtgcaaagg     1320
   tccgccttgt ttctcctctg tctcttgatc tgactaatct tggtttatga ttcgttgagt     1380
   aattttgggg aaagctagct tcgtccacag tttttttttc gatgaacagt gccgcagtgg     1440
   cgctgatctt gtatgctatc ctgcaatcgt ggtgaactta tttcttttat atccttcact     1500
   cccatgaaaa ggctagtaat ctttctcgat gtaacatcgt ccagcactgc tattaccgtg     1560
   tggtccatcc gacagtctgg ctgaacacat catacgatat tgagcaaaga tcgatctatc     1620
   ttccctgttc tttaatgaaa gacgtcattt tcatcagtat gatctaagaa tgttgcaact     1680
   tgcaaggagg cgtttctttc tttgaattta actaactcgt tgagtggccc tgtttctcgg     1740
   acgtaaggcc tttgctgctc cacacatgtc cattcgaatt ttaccgtgtt tagcaagggc     1800
   gaaaagtttg catcttgatg atttagcttg actatgcgat tgctttcctg gacccgtgca     1860
   gctgcggacg gatcccccgc tcgaagctag cttgatcaga tctgatcgaa ttcaccatgg     1920
   gcgccgggca gtcctcgcct gcgacgggga gccagaacca gtcgggcaac accggctcga     1980
   tcatcaacaa ctactacatg cagcaatacc agaacagcat ggacacccag ctcggcgata     2040
   acgccatctc cggcgggtcc aacgagggct cgaccgacac gacctccacc cacaccacga     2100
   acacccagaa caatgactgg ttcagcaagc tggcctccag cgccttcacg ggcctgttcg     2160
   gggcgctgct cgcggacaag aagaccgagg agaccacgct gctcgaggac cggatcctca     2220
   ccacgcgcaa cgggcacacg accagcacga cccagtccag cgtcggggtg acccacggct     2280
   actccacgga ggaggaccac gtcgccgggc cgaacacgag cggcctcgag acccgcgtgg     2340
   tccaggcgga gcgcttctac aagaagtacc tgttcgactg gaccacggac aaggcgttcg     2400
   ggcacctcga gaagctggag ctgccgagcg accaccacgg cgtcttcggc cacctcgtgg     2460
   actcctacgc gtacatgaga aatgggtggg acgtcgaggt cagcgccgtc ggcaaccagt 2520
   tcaacggcgg gtgcctgctc gtggcgatgg tcccggagtg gaaggagttc gacacccgcg 2580
   agaagtatca gctgaccctc ttcccccacc agttcatcag cccgcgcacc aacatgaccg 2640
   cccacatcac ggtgccgtac ctcggggtga accgctacga ccagtacaag aagcacaagc 2700
   cctggaccct cgtggtcatg gtggtctccc cgctgactgt gaacaacacg tccgccgcgc 2760
   agatcaaagt ctacgccaac atcgccccca cctacgtgca cgtcgccggc gagctgccct 2820
   ccaaggaggg catcttcccc gtcgcctgcg ccgacgggta cgggggcctg gtgaccaccg 2880
   accccaagac ggccgacccg gcctacggca aagtgtacaa cccgcccagg acgaactacc 2940
   ccggtcgctt caccaacctc ctggacgtgg cggaggcctg cccgaccttc ctgtgcttcg 3000
   acgacgggaa gccctacgtc accacgcgca ccgacgacac gcgcctgctc gccaagttcg 3060
   acctcagcct ggccgctaag cacatgagca acacctacct cagcggcatc gcccaatact 3120
   atacccaata ctcgggcacc atcaacctgc acttcatgtt cacgggcagc accgacagca 3180
   aggcccggta catggtggcc tacatcccgc cgggcgtgga gacccctccc gacacgcccg 3240
   agcgggctgc gcactgcatc cacgccgagt gggacaccgg cctcaacagc aagttcacgt 3300
   tcagcatccc ctacgtgtcc gccgcggatt acgcttacac cgcctcggac acggccgaga 3360
   cgatcaacgt ccagggctgg gtctgcatct accagatcac tcacggcaag gccgagaacg 3420
   acaccctcgt cgtgagcgtc tccgccggga aggacttcga gctgaggctg cccatcgacc 3480
   ccaggcagca gaccacggcg accggggagt ccgccgaccc cgtgaccacg accgtggaga 3540
   actacggcgg ggagactcag atccagcggc gccaccacac cgacatcggc ttcatcatgg 3600
   accgcttcgt gaagatccag tccctgtcgc ccacccacgt tatcgacctc atgcaggccc 3660
   accagcacgg gctcgtgggt gccctcctgc gcgcggccac ctactacttc agcgacctcg 3720
   agatcgttgt ccgccacgag gggaacctca cctgggtccc gaacggtgcc cccgagagcg 3780
   ccctgctcaa cacctccaac cccacggcgt acaacaaggc ccccttcacg cgcctcgccc 3840
   tgccttacac cgcgccccac cgcgtgctgg ccacggtgta caacgggacc tccaagtacg 3900
   ccgtgggcgg gagcggccgc agaggcgaca tggggagcct cgccgctagg gtcgtgaagc 3960
   agctcccggc ctccttcaac tacggcgcca tcaaggccga cgccatccac gaactcctgg 4020
   tccgcatgaa gcgcgccgaa ctctactgcc cccggcccct cctggccatc gaggtctcct 4080
   cgcaggaccg ccacaagcag aagatcatcg ccccggccaa gcagctgctc aacttcgacc 4140
   tgctcaagct cgcgggggac gtcgagtcga accccggtta agagctcaga gctcgaattt 4200
   ccccgatcgt tcaaacattt ggcaataaag tttcttaaga ttgaatcctg ttgccggtct 4260
   tgcgatgatt atcatataat ttctgttgaa ttacgttaag catgtaataa ttaacatgta 4320
   atgcatgacg ttatttatga gatgggtttt tatgattaga gtcccgcaat tatacattta 4380
   atacgcgata gaaaacaaaa tatagcgcgc aaactaggat aaattatcgc gcgcggtgtc 4440
   atctgtacat ctagatgctt gcggcagcgt gaagctctgg acatcatgtt ggatatgaaa 4500
   caactattat ttatctacat gttttagatg ttatctgatt atttttatac cgtagtcttc 4560
   tattgatgag gagtctaagg ctatagaatt atatatctaa atgattaata tatatattat 4620
   taataattaa caataattaa tatattataa tttatatata tatattttat attattataa 4680
   taatattctt acaaatataa ttattatatt cgacgcccgg gctagtacta taaatacggt 4740
   cccgaggcct cctcaccact cgcacatatc ctctttgttt tcctctccgt gaaagaagcg 4800
   aggaagcgcg tcgtctctcc caaggtaagg agcagatctc tttgatcgtt tttgttcttc 4860
   ttttgttttg tttttttttt ctgcggatct tcggttgcat catgccttgg ctgtttttat 4920
   tagtttagga tatcctcgtt tggatctgag ccgatcatat atgttaaagg ttgtgttcga 4980
   tctctttgtt cattttcgca tgaaaaggat gtatcctttt gatgtgaggc gatcttctat 5040
   ggttaagact ttgttcggtc tattgatcat ttctgttctt cgtttttgag tttttttctg 5100
   cggatatcgc atcatcccta ggtttttgct ttggttagga tgcatccttt ggatttgagc 5160
   cgatctccct tggttaaggc tgtgtctgtt gcagaggaga aagtctgtcg aggtccttat 5220
   gcaggctttg tccagatgcg cgtgctctct catgctatga atttatgttt tgagaactcc 5280
   tcccggtttt tctagatccg gatttgaagt attcattgcg gttccccttc ggttttatgt 5340
   atttctcgag ttgatttggt ccatgatcgt gttctgtcca gatctctctt gatatggatg 5400
   agatattcgt tacctctttc aaacatcggt ggatgttctt tttagtcttg gctcaccttt 5460
   atctagaaat taattttcgg tttgaaaccc ctgcttgtta aggtgatgta ttccttcttt 5520
   atagatttcg gtgtgttatt tcttaacggt gatctgtccg atccatgtgt tgcacctctt 5580
   gttttctgtg taatcctctg tgaattataa ttatgttttg aaaacgtact taagtaaggg 5640
   gcatgttccc cgtttaaaac ttttgttcta tcaatttgtg gttaatagat cctgatttgt 5700
   ggtcgcctta ttctgtcttt aatcgtggat tttatttatc ttgagcgcgt ccttttcttt 5760
   taaaatcatg tgtttaacct ttcagtcgtc atatgttcca tcagggtacc gtcgacacca 5820
   tgtccggggc gccccccacc gacctgcaga agatggtgat gggcaacacc aagcccgtcg 5880
   agttgatcct cgacgggaag accgtggcga tctgctgcgc caccggcgtg ttcggcaccg 5940
   cctacctcgt cccgagacac ctgttcgccg agaagtatga caagatcatg ctggacgggc 6000
   gggccatgac cgactcggac taccgggtct tcgagttcga gatcaaagtg aagggccagg 6060
   atatgctctc cgacgccgcg ctgatggtgc tccacagagg caaccgcgtg cgggacatca 6120
   ccaagcactt cagggacacc gcgcgcatga agaaggggac ccctgtggtc ggggtcgtga 6180
   acaacgccga cgtcgggcgc ctcatcttct ccggcgaggc gctgacctac aaggacatcg 6240
   tcgtgtgcat ggacggggac acgatgccgg ggctcttcgc ctacaaggcc gcgaccaagg 6300
   ccgggtactg cgggggtgcg gtgctcgcca aggacggcgc cgacacgttc atcgtgggca 6360
   cccactccgc cggcggtaac ggcgtcggct actgctcctg cgtgtcccgg tccatgctgc 6420
   tgcggatgaa ggcccacgtt gaccccgagc cgcagcacga gtaaaagctt atcgatgtgc 6480
   acgatcgttc aaacatttgg caataaagtt tcttaagatt gaatcctgtt gccggtcttg 6540
   cgatgattat catataattt ctgttgaatt acgttaagca tgtaataatt aacatgtaat 6600
   gcatgacgtt atttatgaga tgggttttta tgattagagt cccgcaatta tacatttaat 6660
   acgcgataga aaacaaaata tagcgcgcaa actaggataa attatcgcgc gcggtgtcat 6720
   ctatgttact agatcgggtg gacaactcgt cacgtactag tggcgcgccc acgtgattta 6780
   aatcgtacgg gccggccgta tactctagtg gatcccccaa ttcagatcgg ctgagtggct 6840
   ccttcaacgt tgcggttctg tcagttccaa acgtaaaacg gcttgtcccg cgtcatcggc 6900
   gggggtcata acgtgactcc cttaattctc cgctcatgat cagattgtcg tttcccgcct 6960
   tcagtttaaa ctatcagtgt ttgacaggat atattggcgg gtaaacctaa gagaaaagag 7020
   cgtttattag aataatcgga tatttaaaag ggcgtgaaaa ggtttatccg ttcgtccatt 7080
   tgtatgtgca tgccaaccac agggttcccc agatctggcg ccggccagcg agacgagcaa 7140
   gattggccgc cgcccgaaac gatccgacag cgcgcccagc acaggtgcgc aggcaaattg 7200
   caccaacgca tacagcgcca gcagaatgcc atagtgggcg gtgacgtcgt tcgagtgaac 7260
   cagatcgcgc aggaggcccg gcagcaccgg cataatcagg ccgatgccga cagcgtcgag 7320
   cgcgacagtg ctcagaatta cgatcagggg tatgttgggt ttcacgtctg gcctccggac 7380
   cagcctccgc tggtccgatt gaacgcgcgg attctttatc actgataagt tggtggacat 7440
   attatgttta tcagtgataa agtgtcaagc atgacaaagt tgcagccgaa tacagtgatc 7500
   cgtgccgccc tggacctgtt gaacgaggtc ggcgtagacg gtctgacgac acgcaaactg 7560
   gcggaacggt tgggggttca gcagccggcg ctttactggc acttcaggaa caagcgggcg 7620
   ctgctcgacg cactggccga agccatgctg gcggagaatc atacgcattc ggtgccgaga 7680
   gccgacgacg actggcgctc atttctgatc gggaatgccc gcagcttcag gcaggcgctg 7740
   ctcgcctacc gcgatggcgc gcgcatccat gccggcacgc gaccgggcgc accgcagatg 7800
   gaaacggccg acgcgcagct tcgcttcctc tgcgaggcgg gtttttcggc cggggacgcc 7860
   gtcaatgcgc tgatgacaat cagctacttc actgttgggg ccgtgcttga ggagcaggcc 7920
   ggcgacagcg atgccggcga gcgcggcggc accgttgaac aggctccgct ctcgccgctg 7980
   ttgcgggccg cgatagacgc cttcgacgaa gccggtccgg acgcagcgtt cgagcaggga 8040
   ctcgcggtga ttgtcgatgg attggcgaaa aggaggctcg ttgtcaggaa cgttgaagga 8100
   ccgagaaagg gtgacgattg atcaggaccg ctgccggagc gcaacccact cactacagca 8160
   gagccatgta gacaacatcc cctccccctt tccaccgcgt cagacgcccg tagcagcccg 8220
   ctacgggctt tttcatgccc tgccctagcg tccaagcctc acggccgcgc tcggcctctc 8280
   tggcggcctt ctggcgctct tccgcttcct cgctcactga ctcgctgcgc tcggtcgttc 8340
   ggctgcggcg agcggtatca gctcactcaa aggcggtaat acggttatcc acagaatcag 8400
   gggataacgc aggaaagaac atgtgagcaa aaggccagca aaaggccagg aaccgtaaaa 8460
   aggccgcgtt gctggcgttt ttccataggc tccgcccccc tgacgagcat cacaaaaatc 8520
   gacgctcaag tcagaggtgg cgaaacccga caggactata aagataccag gcgtttcccc 8580
   ctggaagctc cctcgtgcgc tctcctgttc cgaccctgcc gcttaccgga tacctgtccg 8640
   cctttctccc ttcgggaagc gtggcgcttt tccgctgcat aaccctgctt cggggtcatt 8700
   atagcgattt tttcggtata tccatccttt ttcgcacgat atacaggatt ttgccaaagg 8760
   gttcgtgtag actttccttg gtgtatccaa cggcgtcagc cgggcaggat aggtgaagta 8820
   ggcccacccg cgagcgggtg ttccttcttc actgtccctt attcgcacct ggcggtgctc 8880
   aacgggaatc ctgctctgcg aggctggccg gctaccgccg gcgtaacaga tgagggcaag 8940
   cggatggctg atgaaaccaa gccaaccagg aagggcagcc cacctatcaa ggtgtactgc 9000
   cttccagacg aacgaagagc gattgaggaa aaggcggcgg cggccggcat gagcctgtcg 9060
   gcctacctgc tggccgtcgg ccagggctac aaaatcacgg gcgtcgtgga ctatgagcac 9120
   gtccgcgagc tggcccgcat caatggcgac ctgggccgcc tgggcggcct gctgaaactc 9180
   tggctcaccg acgacccgcg cacggcgcgg ttcggtgatg ccacgatcct cgccctgctg 9240
   gcgaagatcg aagagaagca ggacgagctt ggcaaggtca tgatgggcgt ggtccgcccg 9300
   agggcagagc catgactttt ttagccgcta aaacggccgg ggggtgcgcg tgattgccaa 9360
   gcacgtcccc atgcgctcca tcaagaagag cgacttcgcg gagctggtga agtacatcac 9420
   cgacgagcaa ggcaagaccg agcgcctttg cgacgctcac cgggctggtt gccctcgccg 9480
   ctgggctggc ggccgtctat ggccctgcaa acgcgccaga aacgccgtcg aagccgtgtg 9540
   cgagacaccg cggccgccgg cgttgtggat acctcgcgga aaacttggcc ctcactgaca 9600
   gatgaggggc ggacgttgac acttgagggg ccgactcacc cggcgcggcg ttgacagatg 9660
   aggggcaggc tcgatttcgg ccggcgacgt ggagctggcc agcctcgcaa atcggcgaaa 9720
   acgcctgatt ttacgcgagt ttcccacaga tgatgtggac aagcctgggg ataagtgccc 9780
   tgcggtattg acacttgagg ggcgcgacta ctgacagatg aggggcgcga tccttgacac 9840
   ttgaggggca gagtgctgac agatgagggg cgcacctatt gacatttgag gggctgtcca 9900
   caggcagaaa atccagcatt tgcaagggtt tccgcccgtt tttcggccac cgctaacctg 9960
   tcttttaacc tgcttttaaa ccaatattta taaaccttgt ttttaaccag ggctgcgccc 10020
   tgtgcgcgtg accgcgcacg ccgaaggggg gtgccccccc ttctcgaacc ctcccggccc 10080
   gctaacgcgg gcctcccatc cccccagggg ctgcgcccct cggccgcgaa cggcctcacc 10140
   ccaaaaatgg cagcgctggc agtccttgcc attgccggga tcggggcagt aacgggatgg 10200
   gcgatcagcc cgagcgcgac gcccggaagc attgacgtgc cgcaggtgct ggcatcgaca 10260
   ttcagcgacc aggtgccggg cagtgagggc ggcggcctgg gtggcggcct gcccttcact 10320
   tcggccgtcg gggcattcac ggacttcatg gcggggccgg caatttttac cttgggcatt 10380
   cttggcatag tggtcgcggg tgccgtgctc gtgttcgggg gtgcgataaa cccagcgaac 10440
   catttgaggt gataggtaag attataccga ggtatgaaaa cgagaattgg acctttacag 10500
   aattactcta tgaagcgcca tatttaaaaa gctaccaaga cgaagaggat gaagaggatg 10560
   aggaggcaga ttgccttgaa tatattgaca atactgataa gataatatat cttttatata 10620
   gaagatatcg ccgtatgtaa ggatttcagg gggcaaggca taggcagcgc gcttatcaat 10680
   atatctatag aatgggcaaa gcataaaaac ttgcatggac taatgcttga aacccaggac 10740
   aataacctta tagcttgtaa attctatcat aattgggtaa tgactccaac ttattgatag 10800
   tgttttatgt tcagataatg cccgatgact ttgtcatgca gctccaccga ttttgagaac 10860
   gacagcgact tccgtcccag ccgtgccagg tgctgcctca gattcaggtt atgccgctca 10920
   attcgctgcg tatatcgctt gctgattacg tgcagctttc ccttcaggcg ggattcatac 10980
   agcggccagc catccgtcat ccatatcacc acgtcaaagg gtgacagcag gctcataaga 11040
   cgccccagcg tcgccatagt gcgttcaccg aatacgtgcg caacaaccgt cttccggaga 11100
   ctgtcatacg cgtaaaacag ccagcgctgg cgcgatttag ccccgacata gccccactgt 11160
   tcgtccattt ccgcgcagac gatgacgtca ctgcccggct gtatgcgcga ggttaccgac 11220
   tgcggcctga gttttttaag tgacgtaaaa tcgtgttgag gccaacgccc ataatgcggg 11280
   ctgttgcccg gcatccaacg ccattcatgg ccatatcaat gattttctgg tgcgtaccgg 11340
   gttgagaagc ggtgtaagtg aactgcagtt gccatgtttt acggcagtga gagcagagat 11400
   agcgctgatg tccggcggtg cttttgccgt tacgcaccac cccgtcagta gctgaacagg 11460
   agggacagct gatagacaca gaagccactg gagcacctca aaaacaccat catacactaa 11520
   atcagtaagt tggcagcatc acccataatt gtggtttcaa aatcggctcc gtcgatacta 11580
   tgttatacgc caactttgaa aacaactttg aaaaagctgt tttctggtat ttaaggtttt 11640
   agaatgcaag gaacagtgaa ttggagttcg tcttgttata attagcttct tggggtatct 11700
   ttaaatactg tagaaaagag gaaggaaata ataaatggct aaaatgagaa tatcaccgga 11760
   attgaaaaaa ctgatcgaaa aataccgctg cgtaaaagat acggaaggaa tgtctcctgc 11820
   taaggtatat aagctggtgg gagaaaatga aaacctatat ttaaaaatga cggacagccg 11880
   gtataaaggg accacctatg atgtggaacg ggaaaaggac atgatgctat ggctggaagg 11940
   aaagctgcct gttccaaagg tcctgcactt tgaacggcat gatggctgga gcaatctgct 12000
   catgagtgag gccgatggcg tcctttgctc ggaagagtat gaagatgaac aaagccctga 12060
   aaagattatc gagctgtatg cggagtgcat caggctcttt cactccatcg acatatcgga 12120
   ttgtccctat acgaatagct tagacagccg cttagccgaa ttggattact tactgaataa 12180
   cgatctggcc gatgtggatt gcgaaaactg ggaagaagac actccattta aagatccgcg 12240
   cgagctgtat gattttttaa agacggaaaa gcccgaagag gaacttgtct tttcccacgg 12300
   cgacctggga gacagcaaca tctttgtgaa agatggcaaa gtaagtggct ttattgatct 12360
   tgggagaagc ggcagggcgg acaagtggta tgacattgcc ttctgcgtcc ggtcgatcag 12420
   ggaggatatc ggggaagaac agtatgtcga gctatttttt gacttactgg ggatcaagcc 12480
   tgattgggag aaaataaaat attatatttt actggatgaa ttgttttagt acctagatgt 12540
   ggcgcaacga tgccggcgac aagcaggagc gcaccgactt cttccgcatc aagtgttttg 12600
   gctctcaggc cgaggcccac ggcaagtatt tgggcaaggg gtcgctggta ttcgtgcagg 12660
   gcaagattcg gaataccaag tacgagaagg acggccagac ggtctacggg accgacttca 12720
   ttgccgataa ggtggattat ctggacacca aggcaccagg cgggtcaaat caggaataag 12780
   ggcacattgc cccggcgtga gtcggggcaa tcccgcaagg agggtgaatg aatcggacgt 12840
   ttgaccggaa ggcatacagg caagaactga tcgacgcggg gttttccgcc gaggatgccg 12900
   aaaccatcgc aagccgcacc gtcatgcgtg cgccccgcga aaccttccag tccgtcggct 12960
   cgatggtcca gcaagctacg gccaagatcg agcgcgacag cgtgcaactg gctccccctg 13020
   ccctgcccgc gccatcggcc gccgtggagc gttcgcgtcg tctcgaacag gaggcggcag 13080
   gtttggcgaa gtcgatgacc atcgacacgc gaggaactat gacgaccaag aagcgaaaaa 13140
   ccgccggcga ggacctggca aaacaggtca gcgaggccaa gcaggccgcg ttgctgaaac 13200
   acacgaagca gcagatcaag gaaatgcagc tttccttgtt cgatattgcg ccgtggccgg 13260
   acacgatgcg agcgatgcca aacgacacgg cccgctctgc cctgttcacc acgcgcaaca 13320
   agaaaatccc gcgcgaggcg ctgcaaaaca aggtcatttt ccacgtcaac aaggacgtga 13380
   agatcaccta caccggcgtc gagctgcggg ccgacgatga cgaactggtg tggcagcagg 13440
   tgttggagta cgcgaagcgc acccctatcg gcgagccgat caccttcacg ttctacgagc 13500
   tttgccagga cctgggctgg tcgatcaatg gccggtatta cacgaaggcc gaggaatgcc 13560
   tgtcgcgcct acaggcgacg gcgatgggct tcacgtccga ccgcgttggg cacctggaat 13620
   cggtgtcgct gctgcaccgc ttccgcgtcc tggaccgtgg caagaaaacg tcccgttgcc 13680
   aggtcctgat cgacgaggaa atcgtcgtgc tgtttgctgg cgaccactac acgaaattca 13740
   tatgggagaa gtaccgcaag ctgtcgccga cggcccgacg gatgttcgac tatttcagct 13800
   cgcaccggga gccgtacccg ctcaagctgg aaaccttccg cctcatgtgc ggatcggatt 13860
   ccacccgcgt gaagaagtgg cgcgagcagg tcggcgaagc ctgcgaagag ttgcgaggca 13920
   gcggcctggt ggaacacgcc tgggtcaatg atgacctggt gcattgcaaa cgctagggcc 13980
   ttgtggggtc agttccggct gggggttcag cagccagcgc tttactggca tttcaggaac 14040
   aagcgggcac tgctcgacgc acttgcttcg ctcagtatcg ctcgggacgc acggcgcgct 14100
   ctacgaactg ccgataaaca gaggattaaa attgacaatt gtgattaagg ctcagattcg 14160
   acggcttgga gcggccgacg tgcaggattt ccgcgagatc cgattgtcgg ccctgaagaa 14220
   agctccagag atgttcgggt ccgtttacga gcacgaggag aaaaagccca tggaggcgtt 14280
   cgctgaacgg ttgcgagatg ccgtggcatt cggcgcctac atcgacggcg agatcattgg 14340
   gctgtcggtc ttcaaacagg aggacggccc caaggacgct cacaaggcgc atctgtccgg 14400
   cgttttcgtg gagcccgaac agcgaggccg aggggtcgcc ggtatgctgc tgcgggcgtt 14460
   gccggcgggt ttattgctcg tgatgatcgt ccgacagatt ccaacgggaa tctggtggat 14520
   gcgcatcttc atcctcggcg cacttaatat ttcgctattc tggagcttgt tgtttatttc 14580
   ggtctaccgc ctgccgggcg gggtcgcggc gacggtaggc gctgtgcagc cgctgatggt 14640
   cgtgttcatc tctgccgctc tgctaggtag cccgatacga ttgatggcgg tcctgggggc 14700
   tatttgcgga actgcgggcg tggcgctgtt ggtgttgaca ccaaacgcag cgctagatcc 14760
   tgtcggcgtc gcagcgggcc tggcgggggc ggtttccatg gcgttcggaa ccgtgctgac 14820
   ccgcaagtgg caacctcccg tgcctctgct cacctttacc gcctggcaac tggcggccgg 14880
   aggacttctg ctcgttccag tagctttagt gtttgatccg ccaatcccga tgcctacagg 14940
   aaccaatgtt ctcggcctgg cgtggctcgg cctgatcgga gcgggtttaa cctacttcct 15000
   ttggttccgg gggatctcgc gactcgaacc tacagttgtt tccttactgg gctttctcag 15060
   ccccagatct ggggtcgatc agccggggat gcatcaggcc gacagtcgga acttcgggtc 15120
   cccgacctgt accattcggt gagcaatgga taggggagtt gatatcgtca acgttcactt 15180
   ctaaagaaat agcgccactc agcttcctca gcggctttat ccagcgattt cctattatgt 15240
   cggcatagtt ctcaagatcg acagcctgtc acggttaagc gagaaatgaa taagaaggct 15300
   gataattcgg atctctgcga gggagatgat atttgatcac aggcagcaac gctctgtcat 15360
   cgttacaatc aacatgctac cctccgcgag atcatccgtg tttcaaaccc ggcagcttag 15420
   ttgccgttct tccgaatagc atcggtaaca tgagcaaagt ctgccgcctt acaacggctc 15480
   tcccgctgac gccgtcccgg actgatgggc tgcctgtatc gagtggtgat tttgtgccga 15540
   gctgccggtc ggggagctgt tggctggctg gtggcaggat atattgtggt gtaaacaaat 15600
   tgacgcttag acaacttaat aacacattgc ggacgttttt aatgtactgg ggtggttttt 15660
   cttttcacca gtgagacggg caacagctga ttgcccttca ccgcctggcc ctgagagagt 15720
   tgcagcaagc ggtccacgct ggtttgcccc agcaggcgaa aatcctgttt gatggtggtt 15780
   ccgaaatcgg caaaatccct tataaatcaa aagaatagcc cgagataggg ttgagtgttg 15840
   ttccagtttg gaacaagagt ccactattaa agaacgtgga ctccaacgtc aaagggcgaa 15900
   aaaccgtcta tcagggcgat ggcccactac gtgaaccatc acccaaatca agttttttgg 15960
   ggtcgaggtg ccgtaaagca ctaaatcgga accctaaagg gagcccccga tttagagctt 16020
   gacggggaaa gccggcgaac gtggcgagaa aggaagggaa gaaagcgaaa ggagcgggcg 16080
   ccattcaggc tgcgcaactg ttgggaaggg cgatcggtgc gggcctcttc gctattacgc 16140
   cagctggcga aagggggatg tgctgcaagg cgattaagtt gggtaacgcc agggttttcc 16200
   cagtcacgac gttgtaaaac gacggccagt gaattgccat cttgaaagaa atatagttta 16260
   aatatttatt gataaaataa gtcaggtatt atagtccaag caaaaacata atttattgat 16320
   gcaaagttta aattcagaaa tatttcaata actgattata tcagctggta cattgccgta 16380
   gatgaaagac tgagtgcgat attatgtgta atacataaat tgatgatata gctagcttag 16440
   ctcatcgggg gatccttaat cgactctagc tagaacgaat tgttaggtgg cggtacttgg 16500
   gtcgatatca aagtgcatca cttcttcccg tatgcccaac tttgtataga gagccactgc 16560
   gggatcgtca ccgtaatctg cttgcacgta gatcacataa gcaccaagcg cgttggcctc 16620
   atgcttgagg agattgatga gcgcggtggc aatgccctgc ctccggtgct cgccggagac 16680
   tgcgagatca tagatataga tctcactacg cggctgctca aacctgggca gaacgtaagc 16740
   cgcgagagcg ccaacaaccg cttcttggtc gaaggcagca agcgcgatga atgtcttact 16800
   acggagcaag ttcccgaggt aatcggagtc cggctgatgt tgggagtagg tggctacgtc 16860
   tccgaactca cgaccgaaaa gatcaagagc agcccgcatg gatttgactt ggtcagggcc 16920
   gagcctacat gtgcgaatga tgcccatact tgagccacct aactttgttt tagggcgact 16980
   gccctgctgc gtaacatcgt tgctgctgcg taccatggag atctggattg agagtgaata 17040
   tgagactcta attggatacc gaggggaatt tatggaagtc agtggagcat ttttgacaag 17100
   aaatatttgc tagctgatag tgaccttagg cgacttttga acgcgcaata atggtttctg 17160
   acgtatgtgc ttagctcatt aaactccaga aacccgcggc tgagtggctc cttcaacgtt 17220
   gcggttctgt cagttccaaa cgtaaaacgg cttgtcccgc gtcatcggcg ggggtcataa 17280
   cgtgactccc ttaattctcc gctcatgatc ttgatcccct gcgccatcag atccttggcg 17340
   gcaagaaagc catccagttt actttgcagg gcttcccaac cttaccagag ggcgccccag 17400
   ctggcaattc cggttcgctt gctgtccata aaaccgccca gtctagctat cgccatgtaa 17460
   gcccactgca agctacctgc tttctctttg cgcttgcgtt ttcccttgtc cagatagccc 17520
   agtagctgac attcatccgg ggtcagcacc gtttctgcgg actggctttc tacgtgttcc 17580
   gcttccttta gcagcccttg cgccctgagt gcttgcggca gcgtgaagct ctggacatca 17640
   tgttggatat gaaacaacta ttatttatct acatgtttta gatgttatct gattattttt 17700
   ataccgtagt cttctattga tgaggagt 17728
<210> 39
   <211> 17746
   <212> DNA
   <213> Artificial Sequence
<220>
   <223> MERE04 Plasmid
<400> 39